# EUROPEAN PATENT APPLICATION

(11) **EP 4 289 823 A1**
(43) Date of publication of application: **13.12.2023**
(21) Application number: 22749169.3
(22) Date of filing: 29.01.2022
(51) Int. Cl.: C07D 237/20, C07D 401/04, C07D 401/14, C07D 403/12, A61K 31/501, A61P 25/16

(54) **SUBSTITUTED PYRIDAZINE PHENOL DERIVATIVES**

(30) Priority: 08.02.2021 CN 202110172932; 30.07.2021 CN 202110875431; 20.08.2021 CN 202110962973; 30.09.2021 CN 202111162651; 03.12.2021 CN 202111466804
(71) Applicant: Medshine Discovery Inc., Nanjing, Jiangsu 210032 (CN)
(72) Inventor: WU, Lingyun, Shanghai 200131 (CN); ZHAO, Lele, Shanghai 200131 (CN); SUN, Jianjun, Shanghai 200131 (CN); QIAO, Chuang, Shanghai 200131 (CN); CHEN, Shuhui, Shanghai 200131 (CN)
(74) Representative: Dehns
(86) International application number: PCT/CN2022/075004
(87) International publication number: WO 2022/166890

(57) **Abstract**

Substituted pyridazine phenol derivatives and a preparation method therefor, specifically relating to a compound as shown in formula (VI) and a pharmaceutically acceptable salt thereof, which can be used as an NLRP3 inhibitor.

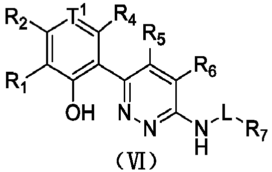

## Description

The present application claims the right of the following priorities:
CN202110172932.4, February 08, 2021;
CN202110875431.2, July 30, 2021;
CN202110962973.3, August 20, 2021;
CN202111162651.7, September 30, 2021;
CN202111466804.7, December 03, 2021.

### TECHNICAL FIELD

The present disclosure relates to a series of substituted pyridazine phenol derivatives and a preparation method therefor, in particular to a compound of formula (VI) and a pharmaceutically acceptable salt thereof.

### BACKGROUND

NLRP3 inflammasome is a multiprotein complex that plays an important role in the development of innate immunity and inflammation-related diseases. NLRP3 inflammasome is composed of NOD-like receptors (NLRs), apoptosis-associated speck-like protein containing a CARD (ASC), and Caspase-1. NLRP3 may be activated by exogenous pathogens or endogenous risk factors such as mitochondrial reactive oxygen species, oxidized mitochondrial DNA, β-amyloid, or α-synuclein. Activated NLRP3 forms activated NLRP3 inflammasome with ASC and Caspase-1, and further hydrolyzes IL-1β precursor (pro-IL-1β) and IL-18 precursor (pro-IL-18) through Caspase-1 to release active cytokines IL-1β and IL-18. The secretion of these cytokines can lead to pyroptosis. NLRP3 inflammasome plays an important role in various autoimmune diseases, cardiovascular diseases, neurodegenerative diseases, and tumorigenesis (Nature Reviews Drug Discovery, 2018, 17(8): 588-606.).

Currently, there is no drug molecule marketed as NLRP3 inhibitors, and drugs such as OLT-1177, Inzomelid, and IFM-2427 are in the clinical research stage. The development of NLRP3 inhibitors has broad application prospects.

### CONTENT OF THE PRESENT INVENTION

The present disclosure provides a compound of formula (VI) or a pharmaceutically acceptable salt thereof, 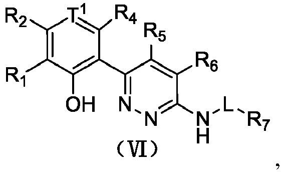 wherein
T¹ is selected from N and CR₃;
L is selected from a single bond and C(=O);
R₁ is selected from H, F, Cl, Br, I, -OH, -NH₂, -CN, C₁₋₃ alkyl, and C₁₋₃ haloalkyl;
R₂ is selected from F, Cl, Br, I, -OH, -NH₂, -CN, C₁₋₄ alkyl, C₁₋₄ alkoxy, -S(=O)₂-C₁₋₃ alkyl, C₃₋₆ cycloalkyl, C₃₋₆ cycloalkenyl, phenyl, and 5- to 6-membered heteroaryl, wherein the C₁₋₄ alkyl, C₁₋₄ alkoxy, -S(=O)₂-C₁₋₃ alkyl, C₃₋₆ cycloalkyl, C₃₋₆ cycloalkenyl, phenyl, and 5- to 6-membered heteroaryl are each independently and optionally substituted by 1, 2, 3, or 4 Rₐ;
or, R₁ and R₂ together with the carbon atom to which they are attached form ring A, wherein the ring A is selected from C₃₋₁₂ cycloalkyl, C₃₋₁₂ cycloalkenyl, 3- to 12-membered heterocycloalkyl, 3- to 12-membered heterocycloalkenyl, C₆₋₁₂ aryl, and 5- to 12-membered heteroaryl, wherein the C₃₋₁₂ cycloalkyl, C₃₋₁₂ cycloalkenyl, 3- to 12-membered heterocycloalkyl, 3- to 12-membered heterocycloalkenyl, C₆₋₁₂ aryl, and 5- to 12-membered heteroaryl are each independently and optionally substituted by 1, 2, 3, or 4 R_{b};
provided that when L is selected from the single bond, R₂ is not selected from Cl, CH₃, CF₃, and -OCF₃;
R₃ and R₄ are each independently selected from H, F, Cl, Br, I, -OH, -NH₂, -CN, and C₁₋₃ alkyl, wherein the C₁₋₃ alkyl is optionally substituted by 1, 2, or 3 R_{c};
or, R₂ and R₃ together with the carbon atom to which they are attached form ring B, wherein the ring B is selected from phenyl, wherein the phenyl is optionally substituted by 1, 2, 3, or 4 R_{b};
R₅ and R₆ are each independently selected from H, F, Cl, Br, I, -OH, -NH₂, -CN, C₁₋₃ alkyl, and C₁₋₃ haloalkyl;
R₇ is selected from C₁₋₃ alkyl, C₃₋₆ cycloalkyl, 3- to 12-membered heterocycloalkyl, and 5- to 6-membered heteroaryl, wherein the C₁₋₃ alkyl, C₃₋₆ cycloalkyl, 3- to 12-membered heterocycloalkyl, and 5- to 6-membered heteroaryl are each independently and optionally substituted by 1, 2, or 3 R_{d};
Rₐ is each independently selected from F, Cl, Br, I, =O, -OH, -NH₂, -CN, and -CH₃;
R_{b} is each independently selected from F, Cl, Br, I, =O, -OH, -NH₂, -CN, and -CH₃;
R_{c} is each independently selected from F, Cl, Br, I, =O, -OH, -NH₂, and -CN;
R_{d} is each independently selected from F, Cl, Br, I, =O, -OH, -NH₂, -CN, C₁₋₃ alkyl, C₁₋₃ alkoxy, C₁₋₃ alkylamino, 3- to 6-membered heterocycloalkyl, C₃₋₆ cycloalkyl, 3- to 6-membered heterocycloalkyl-CH₂-, C₃₋₆ cycloalkyl-CH₂-, and 3- to 6-membered heterocycloalkyl-C(=O)-, wherein the C₁₋₃ alkyl, C₁₋₃ alkoxy, C₁₋₃ alkylamino, 3- to 6-membered heterocycloalkyl, C₃₋₆ cycloalkyl, 3- to 6-membered heterocycloalkyl-CH₂-, C₃₋₆ cycloalkyl-CH₂-, and C₃₋₆ cycloalkyl-C(=O)- are each independently and optionally substituted by 1, 2, or 3 R;
R is each independently selected from F, Cl, Br, I, =O, -OH, -NH₂, -CN, -C(=O)NH₂, -CH₃, -OCH₃, and -N(CH₃)₂;
the 5- to 6-membered heteroaryl, 3- to 12-membered heterocycloalkyl, 3- to 12-membered heterocycloalkenyl, 5- to 12-membered heteroaryl, 3- to 6-membered heterocycloalkyl, and 3- to 6-membered heterocycloalkyl-CH₂- each independently comprises 1, 2, 3, or 4 atoms or atom groups independently selected from N, O, S, and NH.

The present disclosure provides a compound of formula (VI) or a pharmaceutically acceptable salt thereof, 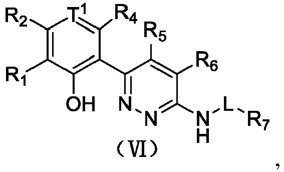 wherein
T¹ is selected from N and CR₃;
L is selected from a single bond and C(=O);
R₁ is selected from H, F, Cl, Br, I, -OH, -NH₂, -CN, C₁₋₃ alkyl, and C₁₋₃ haloalkyl;
R₂ is selected from H, F, Cl, Br, I, -OH, -NH₂, -CN, C₁₋₄ alkyl, C₁₋₄ alkoxy, -S(=O)₂-C₁₋₃ alkyl, C₃₋₆ cycloalkyl, C₃₋₆ cycloalkenyl, phenyl, and 5- to 6-membered heteroaryl, wherein the C₁₋₄ alkyl, C₁₋₄ alkoxy, -S(=O)₂-C₁₋₃ alkyl, C₃₋₆ cycloalkyl, C₃₋₆ cycloalkenyl, phenyl, and 5- to 6-membered heteroaryl are each independently and optionally substituted by 1, 2, 3, or 4 Rₐ;
or, R₁ and R₂ together with the carbon atom to which they are attached form ring A, wherein the ring A is selected from C₃₋₁₂ cycloalkyl, C₃₋₁₂ cycloalkenyl, 3- to 12-membered heterocycloalkyl, 3- to 12-membered heterocycloalkenyl, C₆₋₁₂ aryl, and 5- to 12-membered heteroaryl, wherein the C₃₋₁₂ cycloalkyl, C₃₋₁₂ cycloalkenyl, 3- to 12-membered heterocycloalkyl, 3- to 12-membered heterocycloalkenyl, C₆₋₁₂ aryl, and 5- to 12-membered heteroaryl are each independently and optionally substituted by 1, 2, 3, or 4 R_{b};
provided that when L is selected from the single bond, R₂ is not selected from Cl, CH₃, CF₃, and -OCF₃;
R₃ and R₄ are each independently selected from H, F, Cl, Br, I, -OH, -NH₂, -CN, and C₁₋₃ alkyl, wherein the C₁₋₃ alkyl is optionally substituted by 1, 2, or 3 R_{c};
or, R₂ and R₃ together with the carbon atom to which they are attached form ring B, wherein the ring B is selected from phenyl, wherein the phenyl is optionally substituted by 1, 2, 3, or 4 R_{b};
R₅ and R₆ are each independently selected H, F, Cl, Br, I, -OH, -NH₂, -CN, C₁₋₃ alkyl, and C₁₋₃ haloalkyl;
R₇ is selected from H, C₁₋₃ alkyl, C₃₋₆ cycloalkyl, 3- to 12-membered heterocycloalkyl, and 5- to 6-membered heteroaryl, wherein the C₁₋₃ alkyl, C₃₋₆ cycloalkyl, 3- to 12-membered heterocycloalkyl, and 5- to 6-membered heteroaryl are each independently and optionally substituted by 1, 2, or 3 R_{d};
Rₐ is each independently selected from F, Cl, Br, I, =O, -OH, -NH₂, -CN, and -CH₃;
R_{b} is each independently selected from F, Cl, Br, I, =O, -OH, -NH₂, -CN, and -CH₃;
R_{c} is each independently selected from F, Cl, Br, I, =O, -OH, -NH₂, and -CN;
R_{d} is each independently selected from F, Cl, Br, I, =O, -OH, -NH₂, -CN, C₁₋₃ alkyl, C₁₋₃ alkoxy, C₁₋₃ alkylamino, 3- to 6-membered heterocycloalkyl, C₃₋₆ cycloalkyl, 3- to 6-membered heterocycloalkyl-CH₂-, C₃₋₆ cycloalkyl-CH₂-, and 3- to 6-membered heterocycloalkyl-C(=O)-, wherein the C₁₋₃ alkyl, C₁₋₃ alkoxy, C₁₋₃ alkylamino, 3- to 6-membered heterocycloalkyl, C₃₋₆ cycloalkyl, 3- to 6-membered heterocycloalkyl-CH₂-, C₃₋₆ cycloalkyl-CH₂-, and C₃₋₆ cycloalkyl-C(=O)- are each independently and optionally substituted by 1, 2, or 3 R;
R is each independently selected from F, Cl, Br, I, =O, -OH, -NH₂, -CN, -C(=O)NH₂, -CH₃, -OCH₃, and -N(CH₃)₂;
the 5- to 6-membered heteroaryl, 3- to 12-membered heterocycloalkyl, 3- to 12-membered heterocycloalkenyl, 5- to 12-membered heteroaryl, 3- to 6-membered heterocycloalkyl, and 3- to 6-membered heterocycloalkyl-CH₂- each independently comprises 1, 2, 3, or 4 atoms or atom groups independently selected from N, O, S, and NH.

The present disclosure provides a compound of formula (I) or a pharmaceutically acceptable salt thereof, 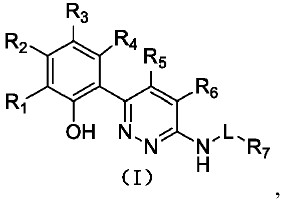 wherein
L is selected from a single bond and C(=O);
R₁ is selected from H, F, Cl, Br, I, -OH, -NH₂, -CN, C₁₋₃ alkyl, and C₁₋₃ haloalkyl;
R₂ is selected from H, F, Cl, Br, I, -OH, -NH₂, -CN, C₁₋₄ alkyl, C₁₋₄ alkoxy, -S(=O)₂-C₁₋₃ alkyl, C₃₋₆ cycloalkyl, C₃₋₆ cycloalkenyl, phenyl, and 5- to 6-membered heteroaryl, wherein the C₁₋₄ alkyl, C₁₋₄ alkoxy, -S(=O)₂-C₁₋₃ alkyl, C₃₋₆ cycloalkyl, C₃₋₆ cycloalkenyl, phenyl, and 5- to 6-membered heteroaryl are each independently and optionally substituted by 1, 2, 3, or 4 Rₐ;
or, R₁ and R₂ together with the carbon atom to which they are attached form ring A, wherein the ring A is selected from C₃₋₁₂ cycloalkyl, C₃₋₁₂ cycloalkenyl, 3- to 12-membered heterocycloalkyl, 3- to 12-membered heterocycloalkenyl, C₆₋₁₂ aryl, and 5- to 12-membered heteroaryl, wherein the C₃₋₁₂ cycloalkyl, C₃₋₁₂ cycloalkenyl, 3- to 12-membered heterocycloalkyl, 3- to 12-membered heterocycloalkenyl, C₆₋₁₂ aryl, and 5- to 12-membered heteroaryl are each independently and optionally substituted by 1, 2, 3, or 4 R_{b};
provided that when L is selected from the single bond, R₂ is not selected from Cl, CH₃, CF₃, and -OCF₃;
R₃ and R₄ are each independently selected from H, F, Cl, Br, I, -OH, -NH₂, -CN, and C₁₋₃ alkyl, wherein the C₁₋₃ alkyl is optionally substituted by 1, 2, or 3 R_{c};
or, R₂ and R₃ together with the carbon atom to which they are attached form ring B, wherein the ring B is selected from phenyl, wherein the phenyl is optionally substituted by 1, 2, 3, or 4 R_{b};
R₅ and R₆ are each independently selected H, F, Cl, Br, I, -OH, -NH₂, -CN, C₁₋₃ alkyl, and C₁₋₃ haloalkyl;
R₇ is selected from H, C₁₋₃ alkyl, C₃₋₆ cycloalkyl, and 3- to 12-membered heterocycloalkyl, wherein the C₁₋₃ alkyl, C₃₋₆ cycloalkyl, and 3- to 12-membered heterocycloalkyl are each independently and optionally substituted by 1, 2, or 3 R_{d};
Rₐ is each independently selected from F, Cl, Br, I, =O, -OH, -NH₂, -CN, and -CH₃;
R_{b} is each independently selected from F, Cl, Br, I, =O, -OH, -NH₂, -CN, and -CH₃;
R_{c} is each independently selected from F, Cl, Br, I, =O, -OH, -NH₂, and -CN;
R_{d} is each independently selected from F, Cl, Br, I, =O, -OH, -NH₂, -CN, C₁₋₃ alkyl, C₁₋₃ alkoxy, 3- to 6-membered heterocycloalkyl, C₃₋₆ cycloalkyl, 3- to 6-membered heterocycloalkyl-CH₂-, C₃₋₆ cycloalkyl-CH₂-, and 3- to 6-membered heterocycloalkyl-C(=O)-, wherein the C₁₋₃ alkyl, C₁₋₃ alkoxy, 3- to 6-membered heterocycloalkyl, C₃₋₆ cycloalkyl, 3- to 6-membered heterocycloalkyl-CH₂-, C₃₋₆ cycloalkyl-CH₂-, and C₃₋₆ cycloalkyl-C(=O)- are each independently and optionally substituted by 1, 2, or 3 R;
R is each independently selected from F, Cl, Br, I, =O, -OH, -NH₂, -CN, -C(=O)NH₂, -CH₃, -OCH₃, and -N(CH₃)₂;
the 5- to 6-membered heteroaryl, 3- to 12-membered heterocycloalkyl, 3- to 12-membered heterocycloalkenyl, 5- to 12-membered heteroaryl, 3- to 6-membered heterocycloalkyl, and 3- to 6-membered heterocycloalkyl-CH₂- each independently comprises 1, 2, 3, or 4 atoms or atom groups independently selected from N, O, S, and NH.

The present disclosure provides a compound of formula (I) or a pharmaceutically acceptable salt thereof, 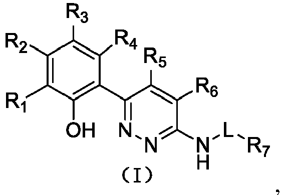 wherein
L is selected from a single bond and C(=O);
R₁ is selected from H, F, Cl, Br, I, -OH, -NH₂, -CN, C₁₋₃ alkyl, and C₁₋₃ haloalkyl;
R₂ is selected from H, F, Cl, Br, I, -OH, -NH₂, -CN, C₁₋₄ alkyl, C₁₋₄ alkoxy, -S(=O)₂-C₁₋₃ alkyl, C₃₋₆ cycloalkyl, C₃₋₆ cycloalkenyl, phenyl, and 5- to 6-membered heteroaryl, wherein the C₁₋₄ alkyl, C₁₋₄ alkoxy, -S(=O)₂-C₁₋₃ alkyl, C₃₋₆ cycloalkyl, C₃₋₆ cycloalkenyl, phenyl, and 5- to 6-membered heteroaryl are each independently and optionally substituted by 1, 2, 3, or 4 Rₐ;
or, R₁ and R₂ together with the carbon atom to which they are attached form ring A, wherein the ring A is selected from C₃₋₁₂ cycloalkyl, C₃₋₁₂ cycloalkenyl, 3- to 12-membered heterocycloalkyl, 3- to 12-membered heterocycloalkenyl, C₆₋₁₂ aryl, and 5- to 12-membered heteroaryl, wherein the C₃₋₁₂ cycloalkyl, C₃₋₁₂ cycloalkenyl, 3- to 12-membered heterocycloalkyl, 3- to 12-membered heterocycloalkenyl, C₆₋₁₂ aryl, and 5- to 12-membered heteroaryl are each independently and optionally substituted by 1, 2, 3, or 4 R_{b};
provided that when L is selected from the single bond, R₂ is not selected from Cl, CH₃, CF₃, and -OCF₃;
R₃ and R₄ are each independently selected from H, F, Cl, Br, I, -OH, -NH₂, -CN, and C₁₋₃ alkyl, wherein the C₁₋₃ alkyl is optionally substituted by 1, 2, or 3 R_{c};
or, R₂ and R₃ together with the carbon atoms to which they are attached form ring B, wherein the ring B is selected from phenyl, wherein the phenyl is optionally substituted by 1, 2, 3, or 4 R_{b};
R₅ and R₆ are each independently selected from H, F, Cl, Br, I, -OH, -NH₂, -CN, C₁₋₃ alkyl, and C₁₋₃ haloalkyl;
R₇ is selected from H, C₁₋₃ alkyl, C₃₋₆ cycloalkyl, and 3- to 12-membered heterocycloalkyl, wherein the C₁₋₃ alkyl, C₃₋₆ cycloalkyl, and 3- to 12-membered heterocycloalkyl are each independently and optionally substituted by 1, 2, or 3 R_{d};
Rₐ is each independently selected from F, Cl, Br, I, =O, -OH, -NH₂, -CN, and -CH₃;
R_{b} is each independently selected from F, Cl, Br, I, =O, -OH, -NH₂, -CN, and -CH₃;
R_{c} is each independently selected from F, Cl, Br, I, =O, -OH, -NH₂, and -CN;
R_{d} is each independently selected from F, Cl, Br, I, =O, -OH, -NH₂, -CN, C₁₋₃ alkyl, C₁₋₃ alkoxy, 3- to 6-membered heterocycloalkyl, C₃₋₆ cycloalkyl, 3- to 6-membered heterocycloalkyl-CH₂-, C₃₋₆ cycloalkyl-CH₂-, and 3- to 6-membered heterocycloalkyl-C(=O)-, wherein the C₁₋₃ alkyl, C₁₋₃ alkoxy, 3- to 6-membered heterocycloalkyl, C₃₋₆ cycloalkyl, 3- to 6-membered heterocycloalkyl-CH₂-, C₃₋₆ cycloalkyl-CH₂-, and C₃₋₆ cycloalkyl-C(=O)- are each independently and optionally substituted by 1, 2, or 3 R;
R is each independently selected from F, Cl, Br, I, =O, -OH, -NH₂, -CN, -C(=O)NH₂, and -CH₃;
the 5- to 6-membered heteroaryl, 3- to 12-membered heterocycloalkyl, 3- to 12-membered heterocycloalkenyl, 5- to 12-membered heteroaryl, 3- to 6-membered heterocycloalkyl, and 3- to 6-membered heterocycloalkyl-CH₂- each independently comprises 1, 2, 3, or 4 atoms or atom groups independently selected from N, O, S, and NH.

The present disclosure also provides a compound of formula (I) or a pharmaceutically acceptable salt thereof, 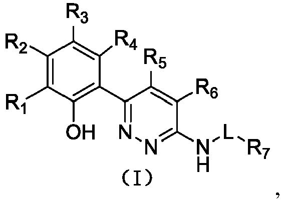 wherein
L is selected from a single bond and C(=O);
R₁ is selected from H, F, Cl, Br, I, -OH, -NH₂, -CN, C₁₋₃ alkyl, and C₁₋₃ haloalkyl;
R₂ is selected from H, F, Cl, Br, I, -OH, -NH₂, -CN, C₁₋₄ alkyl, C₁₋₄ alkoxy, -S(=O)₂-C₁₋₃ alkyl, C₃₋₆ cycloalkyl, C₃₋₆ cycloalkenyl, phenyl, and 5- to 6-membered heteroaryl, wherein the C₁₋₄ alkyl, C₁₋₄ alkoxy, -S(=O)₂-C₁₋₃ alkyl, C₃₋₆ cycloalkyl, C₃₋₆ cycloalkenyl, phenyl, and 5- to 6-membered heteroaryl are each independently and optionally substituted by 1, 2, 3, or 4 Rₐ;
or, R₁ and R₂ together with the carbon atom to which they are attached form ring A, wherein the ring A is selected from C₃₋₁₂ cycloalkyl, C₃₋₁₂ cycloalkenyl, 3- to 12-membered heterocycloalkyl, 3- to 12-membered heterocycloalkenyl, C₆₋₁₂ aryl, and 5- to 12-membered heteroaryl, wherein the C₃₋₁₂ cycloalkyl, C₃₋₁₂ cycloalkenyl, 3- to 12-membered heterocycloalkyl, 3- to 12-membered heterocycloalkenyl, C₆₋₁₂ aryl, and 5- to 12-membered heteroaryl are each independently and optionally substituted by 1, 2, 3, or 4 R_{b};
provided that when L is selected from the single bond, R₂ is not selected from Cl, CH₃, CF₃, and -OCF₃;
R₃ and R₄ are each independently selected from H, F, Cl, Br, I, -OH, -NH₂, -CN, and C₁₋₃ alkyl, wherein the C₁₋₃ alkyl is optionally substituted by 1, 2, or 3 R_{c};
or, R₂ and R₃ together with the carbon atoms to which they are attached form ring B, wherein the ring B is selected from phenyl, wherein the phenyl is optionally substituted by 1, 2, 3, or 4 R_{b};
R₅ and R₆ are each independently selected from H, F, Cl, Br, I, -OH, -NH₂, -CN, C₁₋₃ alkyl, and C₁₋₃ haloalkyl;
R₇ is selected from H, C₁₋₃ alkyl, C₃₋₆ cycloalkyl, and 3- to 12-membered heterocycloalkyl, wherein the C₁₋₃ alkyl, C₃₋₆ cycloalkyl, and 3- to 12-membered heterocycloalkyl are each independently and optionally substituted by 1, 2, or 3 R_{d};
Rₐ is each independently selected from F, Cl, Br, I, =O, -OH, -NH₂, -CN, and -CH₃;
R_{b} is each independently selected from F, Cl, Br, I, =O, -OH, -NH₂, and -CN;
R_{c} is each independently selected from F, Cl, Br, I, =O, -OH, -NH₂, and -CN;
R_{d} is each independently selected from F, Cl, Br, I, =O, -OH, -NH₂, -CN, C₁₋₃ alkyl, C₁₋₃ alkoxy, 3- to 6-membered heterocycloalkyl, C₃₋₆ cycloalkyl, and 3- to 6-membered heterocycloalkyl-CH₂-, wherein the C₁₋₃ alkyl, C₁₋₃ alkoxy, 3- to 6-membered heterocycloalkyl, C₃₋₆ cycloalkyl, and 3- to 6-membered heterocycloalkyl-CH₂- are each independently and optionally substituted by 1, 2, or 3 R;
R is each independently selected from F, Cl, Br, I, =O, -OH, -NH₂, -CN, and - C(=O)NH₂;
the 5- to 6-membered heteroaryl, 3- to 12-membered heterocycloalkyl, 3- to 12-membered heterocycloalkenyl, 5- to 12-membered heteroaryl, 3- to 6-membered heterocycloalkyl, and 3- to 6-membered heterocycloalkyl-CH₂- each independently comprises 1, 2, 3, or 4 atoms or atom groups independently selected from N, O, S, and NH.

The present disclosure also provides a compound of formula (I) or a pharmaceutically acceptable salt thereof, 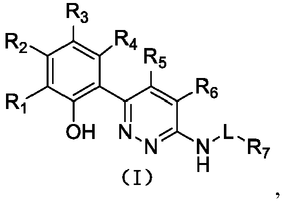 wherein
L is selected from a single bond and C(=O);
R₁ is selected from H, F, Cl, Br, I, -OH, -NH₂, -CN, C₁₋₃ alkyl, and C₁₋₃ haloalkyl;
R₂ is selected from H, F, Cl, Br, I, -OH, -NH₂, -CN, C₁₋₄ alkyl, C₁₋₄ alkoxy, -S(=O)₂-C₁₋₃ alkyl, cyclopropyl, phenyl, and 5- to 6-membered heteroaryl, wherein the C₁₋₄ alkyl, C₁₋₄ alkoxy, -S(=O)₂-C₁₋₃ alkyl, cyclopropyl, phenyl, and 5- to 6-membered heteroaryl are each independently and optionally substituted by 1, 2, or 3 Rₐ;
or, R₁ and R₂ together with the carbon atom to which they are attached form ring A, wherein the ring A is selected from C₃₋₁₂ cycloalkyl, C₃₋₁₂ cycloalkenyl, 3- to 12-membered heterocycloalkyl, 3- to 12-membered heterocycloalkenyl, C₆₋₁₂ aryl, and 5- to 12-membered heteroaryl, wherein the C₃₋₁₂ cycloalkyl, C₃₋₁₂ cycloalkenyl, 3- to 12-membered heterocycloalkyl, 3- to 12-membered heterocycloalkenyl, C₆₋₁₂ aryl, and 5- to 12-membered heteroaryl are each independently and optionally substituted by 1, 2, 3, or 4 R_{b};
provided that when L is selected from the single bond, R₂ is not selected from Cl, CH₃, CF₃, and -OCF₃;
R₃ and R₄ are each independently selected from H, F, Cl, Br, I, -OH, -NH₂, -CN, and C₁₋₃ alkyl, wherein the C₁₋₃ alkyl is optionally substituted by 1, 2, or 3 R_{c};
or, R₂ and R₃ together with the carbon atom to which they are attached form ring B, wherein the ring B is selected from phenyl, wherein the phenyl is optionally substituted by 1, 2, 3, or 4 R_{b};
R₅ and R₆ are each independently selected from H, F, Cl, Br, I, -OH, -NH₂, -CN, C₁₋₃ alkyl, and C₁₋₃ haloalkyl;
R₇ is selected from H, C₁₋₃ alkyl, C₃₋₆ cycloalkyl, and 3- to 12-membered heterocycloalkyl, wherein the C₁₋₃ alkyl, C₃₋₆ cycloalkyl, and 3- to 12-membered heterocycloalkyl are each independently and optionally substituted by 1, 2, or 3 R_{d};
Rₐ is each independently selected from F, Cl, Br, I, =O, -OH, -NH₂, and -CN;
R_{b} is each independently selected from F, Cl, Br, I, =O, -OH, -NH₂, and -CN;
R_{c} is each independently selected from F, Cl, Br, I, =O, -OH, -NH₂, and -CN;
R_{d} is each independently selected from F, Cl, Br, I, =O, -OH, -NH₂, -CN, C₁₋₃ alkyl, C₁₋₃ alkoxy, and 3- to 6-membered heterocycloalkyl, wherein the C₁₋₃ alkyl, C₁₋₃ alkoxy, and 3-to 6-membered heterocycloalkyl are each independently and optionally substituted by 1, 2, or 3 R;
R is each independently selected from F, Cl, Br, I, =O, -OH, -NH₂, -CN, and - C(=O)NH₂;
the 5- to 6-membered heteroaryl, 3- to 12-membered heterocycloalkyl, 3- to 12-membered heterocycloalkenyl, 5- to 12-membered heteroaryl, and 3- to 6-membered heterocycloalkyl each independently comprises 1, 2, 3, or 4 atoms or atom groups independently selected from N, O, S, and NH.

The present disclosure also provides a compound of formula (I) or a pharmaceutically acceptable salt thereof, 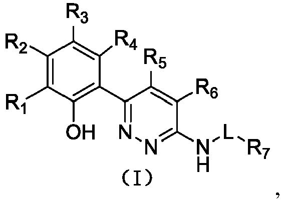 wherein
L is selected from a single bond and C(=O);
R₁ is selected from H, F, Cl, Br, I, -OH, -NH₂, -CN, C₁₋₃ alkyl, and C₁₋₃ haloalkyl;
R₂ is selected from H, F, Cl, Br, I, -OH, -NH₂, -CN, C₁₋₃ alkyl, C₁₋₃ alkoxy, phenyl, and 5- to 6-membered heteroaryl, wherein the C₁₋₃ alkyl, C₁₋₃ alkoxy, phenyl, and 5- to 6-membered heteroaryl are each independently and optionally substituted by 1, 2, or 3 Rₐ;
or, R₁ and R₂ together with the carbon atom to which they are attached form ring A, wherein the ring A is selected from C₃₋₁₂ cycloalkyl, C₃₋₁₂ cycloalkenyl, 3- to 12-membered heterocycloalkyl, 3- to 12-membered heterocycloalkenyl, C₆₋₁₂ aryl, and 5- to 12-membered heteroaryl, wherein the C₃₋₁₂ cycloalkyl, C₃₋₁₂ cycloalkenyl, 3- to 12-membered heterocycloalkyl, 3- to 12-membered heterocycloalkenyl, C₆₋₁₂ aryl, and 5- to 12-membered heteroaryl are each independently and optionally substituted by 1, 2, 3, or 4 R_{b};
provided that when L is selected from the single bond, R₂ is not selected from Cl, CH₃, CF₃, and -OCF₃;
R₃ and R₄ are each independently selected from H, F, Cl, Br, I, -OH, -NH₂, -CN, and C₁₋₃ alkyl, wherein the C₁₋₃ alkyl is optionally substituted by 1, 2, or 3 R_{c};
R₅ and R₆ are each independently selected from H, F, Cl, Br, I, -OH, -NH₂, -CN, C₁₋₃ alkyl, and C₁₋₃ haloalkyl;
R₇ is selected from H, C₁₋₃ alkyl, C₃₋₆ cycloalkyl, and 3- to 6-membered heterocycloalkyl, wherein the C₁₋₃ alkyl, C₃₋₆ cycloalkyl, and 3- to 6-membered heterocycloalkyl are each independently and optionally substituted by 1, 2, or 3 R_{d};
Rₐ is each independently selected from F, Cl, Br, I, =O, -OH, -NH₂, and -CN;
R_{b} is each independently selected from F, Cl, Br, I, =O, -OH, -NH₂, and -CN;
R_{c} is each independently selected from F, Cl, Br, I, =O, -OH, -NH₂, and -CN;
R_{d} is each independently selected from F, Cl, Br, I, =O, -OH, -NH₂, -CN, C₁₋₃ alkyl, and C₁₋₃ alkoxy, wherein the C₁₋₃ alkyl and C₁₋₃ alkoxy are each independently and optionally substituted by 1, 2, or 3 R;
R is each independently selected from F, Cl, Br, I, =O, -OH, -NH₂, and -CN;
the 5- to 6-membered heteroaryl, 3- to 12-membered heterocycloalkyl, 3- to 12-membered heterocycloalkenyl, 5- to 12-membered heteroaryl, and 3- to 6-membered heterocycloalkyl each independently comprises 1, 2, 3, or 4 atoms or atom groups independently selected from N, O, S, and NH.

In some embodiments of the present disclosure, the L is selected from the single bond, and other variables are as defined in the present disclosure.

In some embodiments of the present disclosure, the R₁ is selected from H, and other variables are as defined in the present disclosure.

In some embodiments of the present disclosure, the Rₐ is each independently selected from F, Cl, Br, -CN, and -CH₃, and other variables are as defined in the present disclosure.

In some embodiments of the present disclosure, the Rₐ is selected from H and F, and other variables are as defined in the present disclosure.

In some embodiments of the present disclosure, the R_{b} is selected from -CH₃, and other variables are as defined in the present disclosure.

In some embodiments of the present disclosure, the R_{b} is selected from F and -CH₃, and other variables are as defined in the present disclosure.

In some embodiments of the present disclosure, the R₂ is selected from H, -CN, -CH₃, 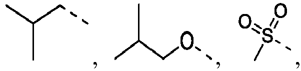 cyclopropyl, cyclobutyl, cyclopentyl, cyclohexyl, cyclopentenyl, cyclohexenyl, phenyl, and pyridyl, wherein the -CH₃, 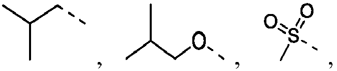 cyclopropyl, cyclobutyl, cyclopentyl, cyclohexyl, cyclobutenyl, cyclopentenyl, cyclohexenyl, phenyl, and pyridyl are each independently and optionally substituted by 1, 2, 3, or 4 Rₐ, and Rₐ and other variables are as defined in the present disclosure.

In some embodiments of the present disclosure, the R₂ is selected from -CN, -CH₃, 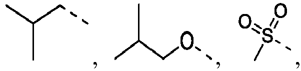 cyclopropyl, cyclobutyl, cyclopentyl, cyclohexyl, cyclopentenyl, cyclohexenyl, phenyl, and pyridyl, wherein the -CH₃, 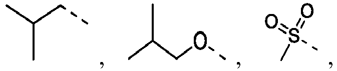 cyclopropyl, cyclobutyl, cyclopentyl, cyclohexyl, cyclobutenyl, cyclopentenyl, cyclohexenyl, phenyl, and pyridyl are each independently and optionally substituted by 1, 2, 3, or 4 Rₐ, and Rₐ and other variables are as defined in the present disclosure.

In some embodiments of the present disclosure, the R₂ is selected from H, -CH₃, 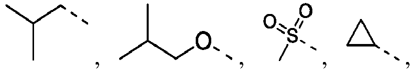 phenyl, and pyridyl, wherein the -CH₃, 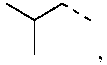 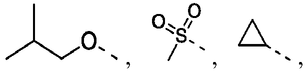 phenyl, and pyridyl are each independently and optionally substituted by 1, 2, or 3 Rₐ, and Rₐ and other variables are as defined in the present disclosure.

In some embodiments of the present disclosure, the R₂ is selected from H, -CH₃, phenyl, and pyridyl, wherein the -CH₃, phenyl, and pyridyl are each independently and optionally substituted by 1, 2, or 3 Rₐ, and Rₐ and other variables are as defined in the present disclosure.

In some embodiments of the present disclosure, the R₂ is selected from H, -CN, -CH₃, 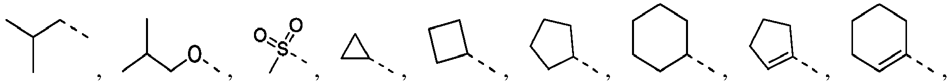 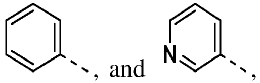 wherein the -CH₃, 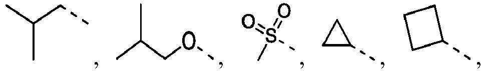 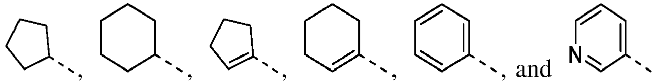 are each independently and optionally substituted by 1, 2, 3, or 4 Rₐ, and Rₐ and other variables are as defined in the present disclosure.

In some embodiments of the present disclosure, the R₂ is selected from -CN, -CH₃, 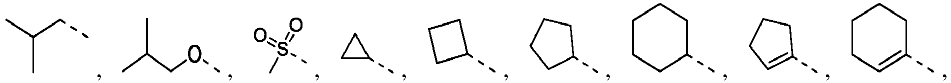 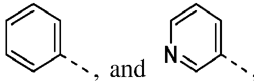 wherein the -CH₃, 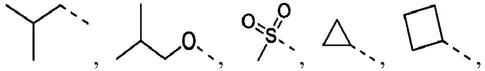 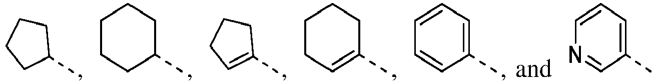 are each independently and optionally substituted by 1, 2, 3, or 4 Rₐ, and Rₐ and other variables are as defined in the present disclosure.

In some embodiments of the present disclosure, the R₂ is selected from H, -CN, -CF₃, 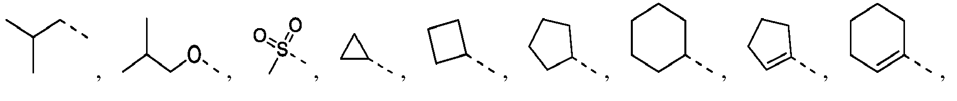 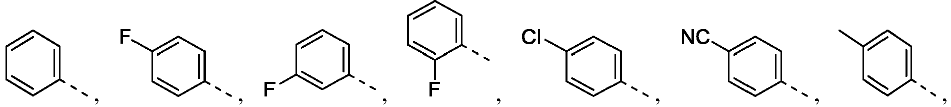 and 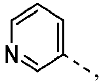 and other variables are as defined in the present disclosure.

In some embodiments of the present disclosure, the R₂ is selected from H, -CF₃, 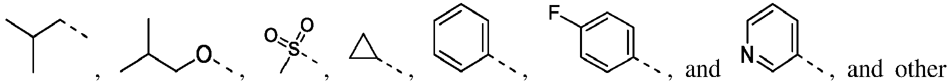 variables are as defined in the present disclosure.

In some embodiments of the present disclosure, the R₂ is selected from H, -CF₃, 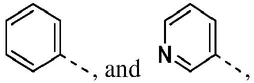 and other variables are as defined in the present disclosure.

In some embodiments of the present disclosure, the ring A is selected from C₅₋₆ cycloalkenyl, 5- to 6-membered heterocycloalkenyl, phenyl, and 5- to 6-membered heteroaryl, wherein the C₅₋₆ cycloalkenyl, 5- to 6-membered heterocycloalkenyl, phenyl, and 5- to 6-membered heteroaryl are each independently and optionally substituted by 1, 2, 3, or 4 R_{b}, and R_{b} and other variables are as defined in the present disclosure.

In some embodiments of the present disclosure, the ring A is selected from 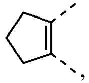 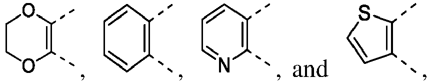 wherein the 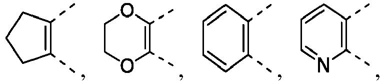 and 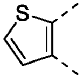 are each independently and optionally substituted by 1, 2, 3, or 4 R_{b}, and R_{b} and other variables are as defined in the present disclosure.

In some embodiments of the present disclosure, the ring A is selected from 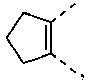 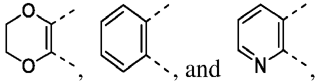 wherein the 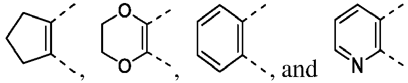 are each independently and optionally substituted by 1, 2, 3, or 4 R_{b}, and R_{b} and other variables are as defined in the present disclosure.

In some embodiments of the present disclosure, the ring A is selected from 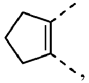 wherein the 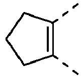 is optionally substituted by 1, 2, 3, or 4 R_{b}, and R_{b} and other variables are as defined in the present disclosure.

In some embodiments of the present disclosure, the ring A is selected from 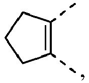 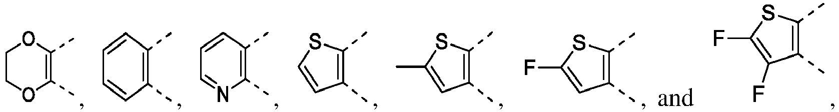 and other variables are as defined in the present disclosure.

In some embodiments of the present disclosure, the ring A is selected from 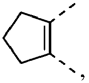 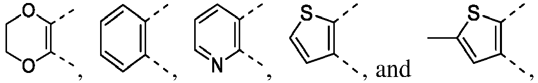 and other variables are as defined in the present disclosure.

In some embodiments of the present disclosure, the ring A is selected from 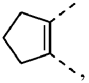 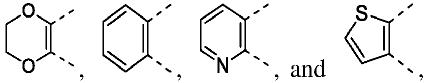 and other variables are as defined in the present disclosure.

In some embodiments of the present disclosure, the ring A is selected from 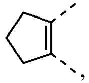 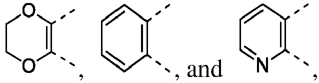 and other variables are as defined in the present disclosure.

In some embodiments of the present disclosure, the ring B is selected from 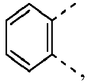 and other variables are as defined in the present disclosure.

In some embodiments of the present disclosure, the structural moiety 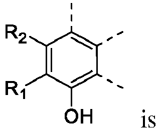 selected from 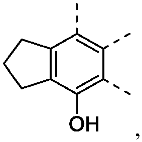 and other variables are as defined in the present disclosure.

In some embodiments of the present disclosure, the R₃ is selected from H, and other variables are as defined in the present disclosure.

In some embodiments of the present disclosure, the R₄ is selected from H and -CH₃, and other variables are as defined in the present disclosure.

In some embodiments of the present disclosure, the R₄ is selected from H, and other variables are as defined in the present disclosure.

In some embodiments of the present disclosure, the R₅ is selected from H and -CH₃, and other variables are as defined in the present disclosure.

In some embodiments of the present disclosure, the R₅ is selected from -CH₃, and other variables are as defined in the present disclosure.

In some embodiments of the present disclosure, the R₆ is selected from H and -CH₃, and other variables are as defined in the present disclosure.

In some embodiments of the present disclosure, the R₆ is selected from H, and other variables are as defined in the present disclosure.

In some embodiments of the present disclosure, the R is selected from F, Cl, Br, -OH, -CN, -C(=O)NH₂, and -CH₃, and other variables are as defined in the present disclosure.

In some embodiments of the present disclosure, the R_{d} is selected from F, Cl, Br, -OH, -CH₃, -CH₂-CH₃, -CH(CH₃)₂, -CH₂-CH₂-CH₃, -N(CH₃)₂, 4- to 6-membered heterocycloalkyl, cyclobutyl, 6-membered heterocycloalkyl-CH₂-, cyclopropyl-CH₂-, and cyclopropyl-C(=O)-, wherein the -CH₃, -CH₂-CH₃, -CH(CH₃)₂, -CH₂-CH₂-CH₃, -N(CH₃)₂, 4- to 6-membered heterocycloalkyl, C₄₋₆ cycloalkyl, 6-membered heterocycloalkyl-CH₂-, cyclopropyl-CH₂-, and cyclopropyl-C(=O)- are each independently and optionally substituted by 1, 2 or 3 R.

In some embodiments of the present disclosure, the R_{d} is selected from -OH, -CH₃, - CH₂-CH₃, -CH(CH₃)₂, -CH₂-CH₂-CH₃, 4- to 6-membered heterocycloalkyl, cyclobutyl, 6-membered heterocycloalkyl-CH₂-, cyclopropyl-CH₂-, and cyclopropyl-C(=O)-, wherein the - CH₃, -CH₂-CH₃, -CH(CH₃)₂, -CH₂-CH₂-CH₃, 4- to 6-membered heterocycloalkyl, C₄₋₆ cycloalkyl, 6-membered heterocycloalkyl-CH₂-, cyclopropyl-CH₂-, and cyclopropyl-C(=O)-are each independently and optionally substituted by 1, 2 or 3 R.

In some embodiments of the present disclosure, the R_{d} is selected from F, Cl, Br, -OH, -CH₃, -CH₂-CH₃, -CH(CH₃)₂, -CH₂-CH₂-CH₃, -N(CH₃)₂, 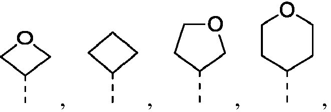 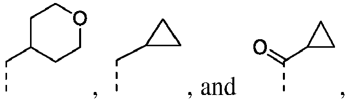 wherein the -CH₃, -CH₂-CH₃, -CH(CH₃)₂, -CH₂-CH₂-CH₃, -N(CH₃)₂, 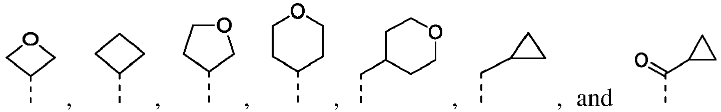 are each independently and optionally substituted by 1, 2, or 3 R, and R and other variables are as defined in the present disclosure.

In some embodiments of the present disclosure, the R_{d} is selected from -OH, -CH₃, - CH₂-CH₃, -CH(CH₃)₂, -CH₂-CH₂-CH₃, 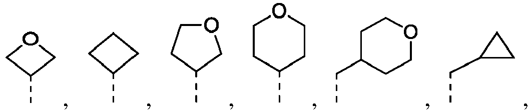 and 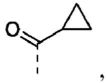 wherein the -CH₃, -CH₂-CH₃, -CH(CH₃)₂, -CH₂-CH₂-CH₃, 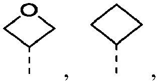 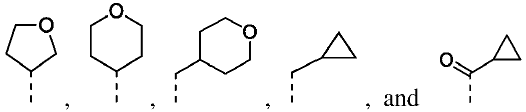 are each independently and optionally substituted by 1, 2, or 3 R, and R and other variables are as defined in the present disclosure.

In some embodiments of the present disclosure, the R_{d} is selected from 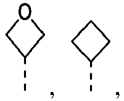 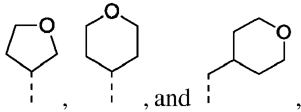 wherein the -CH₃, 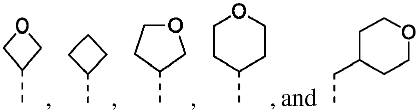 are each independently and optionally substituted by 1, 2, or 3 R, and R and other variables are as defined in the present disclosure.

In some embodiments of the present disclosure, the R_{d} is selected from -CH₃ and 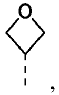 wherein the -CH₃ and 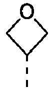 are each independently and optionally substituted by 1, 2, or 3 R, and R and other variables are as defined in the present disclosure.

In some embodiments of the present disclosure, the R_{d} is selected from -CH₃, wherein the -CH₃ is optionally substituted by 1, 2, or 3 R, and R and other variables are as defined in the present disclosure.

In some embodiments of the present disclosure, the R_{d} is selected from F, Cl, Br, -OH, -CH₃, -CH₂C(=O)NH₂, -CH₂CN, -CH₂-CH₃, -CH₂CF₃, -CH(CH₃)₂, -CH₂-CH₂-CH₃, -N(CH₃)₂, 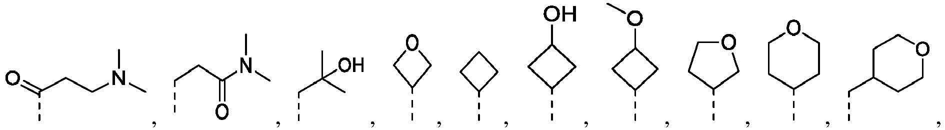 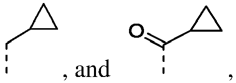 and other variables are as defined in the present disclosure.

In some embodiments of the present disclosure, the R_{d} is selected from -OH, -CH₃, - CH₂C(=O)NH₂, -CH₂CN, -CH₂-CH₃, -CH₂CF₃, -CH(CH₃)₂, -CH₂-CH₂-CH₃, 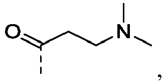 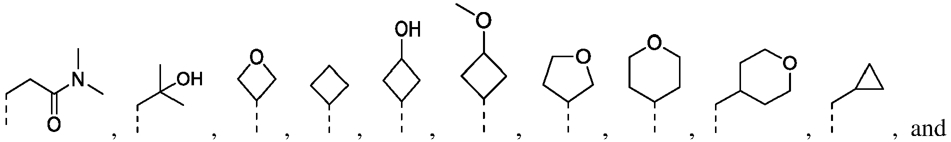 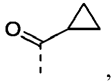 and other variables are as defined in the present disclosure.

In some embodiments of the present disclosure, the R_{d} is selected from -OH, -CH₃, - CH₂C(=O)NH₂, -CH₂CN, -CH₂-CH₃, -CH₂CF₃, -CH(CH₃)₂, -CH₂-CH₂-CH₃, 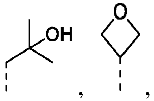 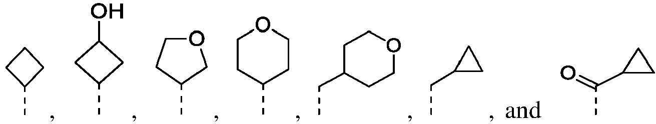 , and other variables are as defined in the present disclosure.

In some embodiments of the present disclosure, the R_{d} is selected from -CH₃, - CH₂C(=O)NH₂, -CH₂CN, 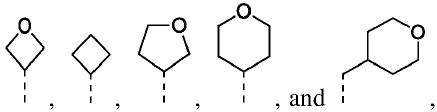 and other variables are as defined in the present disclosure.

In some embodiments of the present disclosure, the R_{d} is selected from -CH₃, - CH₂C(=O)NH₂, -CH₂CN, and 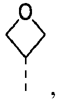 and other variables are as defined in the present disclosure.

In some embodiments of the present disclosure, the R_{d} is selected from -CH₃, and other variables are as defined in the present disclosure.

In some embodiments of the present disclosure, the R₇ is selected from C₁₋₃ alkyl, C₅₋₆ cycloalkyl, and 5- to 10-membered heterocycloalkyl, wherein the C₁₋₃ alkyl, C₅₋₆ cycloalkyl, and 5- to 10-membered heterocycloalkyl are each independently and optionally substituted by 1, 2, or 3 R_{d}, and R_{d} and other variables are as defined in the present disclosure.

In some embodiments of the present disclosure, the R₇ is selected from H, -CH₂-CH₃, -CH(CH₃)₂, -CH₂-CH₂-CH₃, pyrrolidinyl, tetrahydrofuryl, piperidinyl, quinuclidinyl, 2-oxa-8-azaspiro[4.5]decyl, cyclohexyl, octahydroindolizinyl, and pyridyl, wherein the -CH₂-CH₃, - CH(CH₃)₂, -CH₂-CH₂-CH₃, pyrrolidinyl, tetrahydrofuryl, piperidinyl, quinuclidinyl, 2-oxa-8-azaspiro[4.5]decyl, 1-oxa-8-azaspiro[4.5]decyl, cyclohexyl, octahydroindolizinyl, and pyridyl are each independently and optionally substituted by 1, 2, or 3 R_{d}, and R_{d} and other variables are as defined in the present disclosure.

In some embodiments of the present disclosure, the R₇ is selected from -CH₂-CH₃, - CH(CH₃)₂, -CH₂-CH₂-CH₃, pyrrolidinyl, tetrahydrofuryl, piperidinyl, quinuclidinyl, 2-oxa-8-azaspiro[4.5]decyl, cyclohexyl, octahydroindolizinyl, and pyridyl, wherein the -CH₂-CH₃, - CH(CH₃)₂, -CH₂-CH₂-CH₃, pyrrolidinyl, tetrahydrofuryl, piperidinyl, quinuclidinyl, 2-oxa-8-azaspiro[4.5]decyl, 1-oxa-8-azaspiro[4.5]decyl, cyclohexyl, octahydroindolizinyl, and pyridyl are each independently and optionally substituted by 1, 2, or 3 R_{d}, and R_{d} and other variables are as defined in the present disclosure.

In some embodiments of the present disclosure, the R₇ is selected from H, piperidinyl, quinuclidinyl, 2-oxa-8-azaspiro[4.5]decyl, cyclohexyl, and octahydroindolizinyl, wherein the piperidinyl, quinuclidinyl, 2-oxa-8-azaspiro[4.5]decyl, cyclohexyl, and octahydroindolizinyl are each independently and optionally substituted by 1, 2, or 3 R_{d}, and R_{d} and other variables are as defined in the present disclosure.

In some embodiments of the present disclosure, the R₇ is selected from H, piperidinyl, quinuclidinyl, and 2-oxa-8-azaspiro[4.5]decyl, wherein the piperidinyl, quinuclidinyl, and 2-oxa-8-azaspiro[4.5]decyl are each independently and optionally substituted by 1, 2, or 3 R_{d}, and R_{d} and other variables are as defined in the present disclosure.

In some embodiments of the present disclosure, the R₇ is selected from H and piperidinyl, wherein the piperidinyl is optionally substituted by 1, 2, or 3 R_{d}, and R_{d} and other variables are as defined in the present disclosure.

In some embodiments of the present disclosure, the R₇ is selected from H, -CH₂-CH₃, -CH(CH₃)₂, -CH₂-CH₂-CH₃, 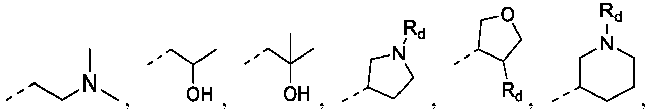 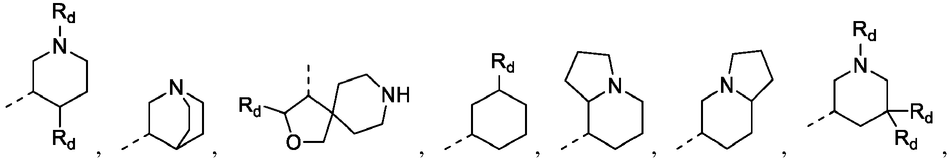 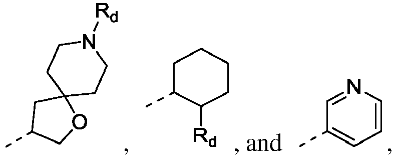 and R_{d} and other variables are as defined in the present disclosure.

In some embodiments of the present disclosure, the R₇ is selected from -CH₂-CH₃, - CH(CH₃)₂, -CH₂-CH₂-CH₃, 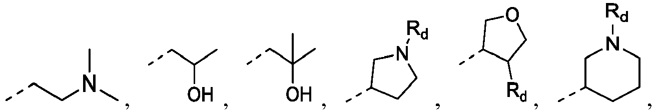 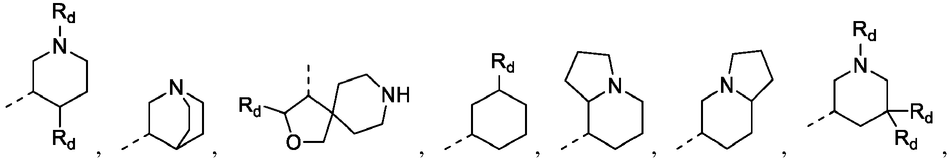 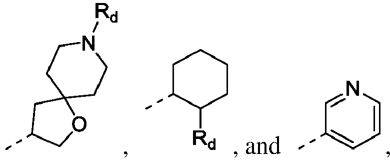 and R_{d} and other variables are as defined in the present disclosure.

In some embodiments of the present disclosure, the R₇ is selected from H, 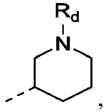 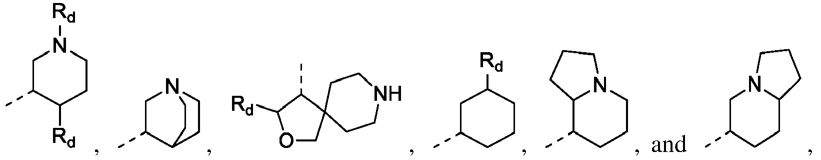 and R_{d} and other variables are as defined in the present disclosure.

In some embodiments of the present disclosure, the R₇ is selected from H, 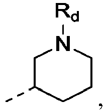 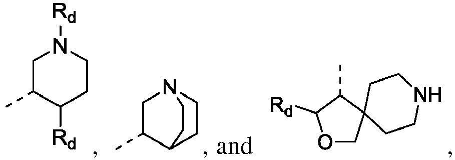 and R_{d} and other variables are as defined in the present disclosure.

In some embodiments of the present disclosure, the R₇ is selected from H and 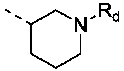 , R_{d} and other variables are as defined in the present disclosure.

In some embodiments of the present disclosure, the R₇ is selected from H, 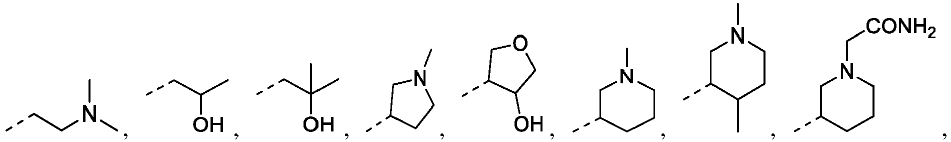 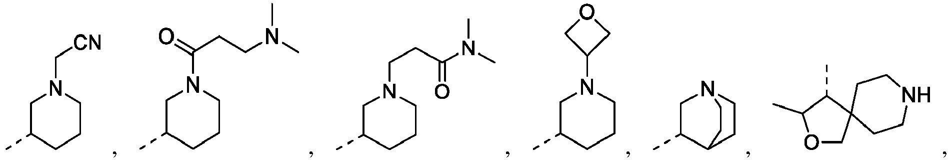 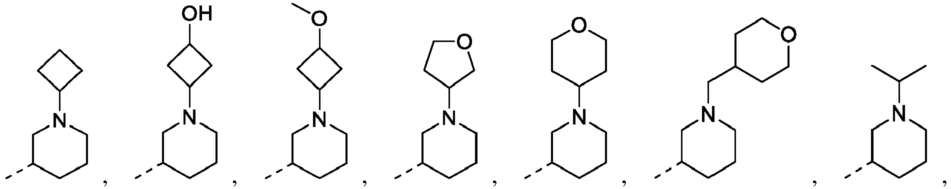 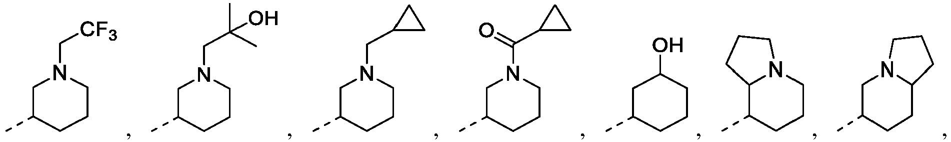 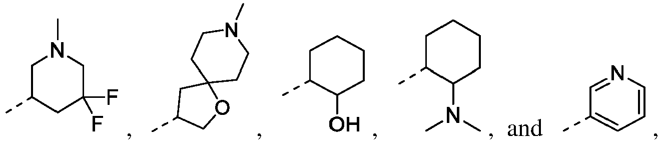 and other variables are as defined in the present disclosure.

In some embodiments of the present disclosure, the R₇ is selected from 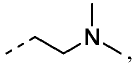 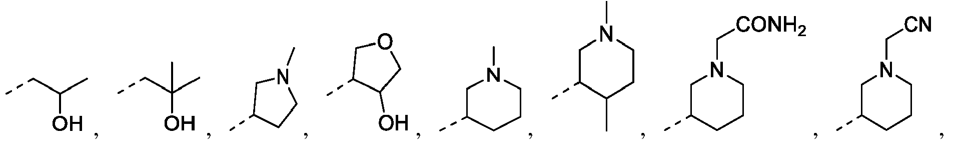 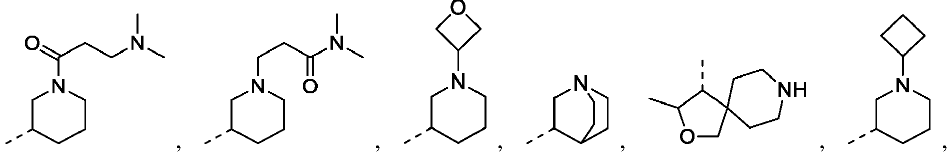 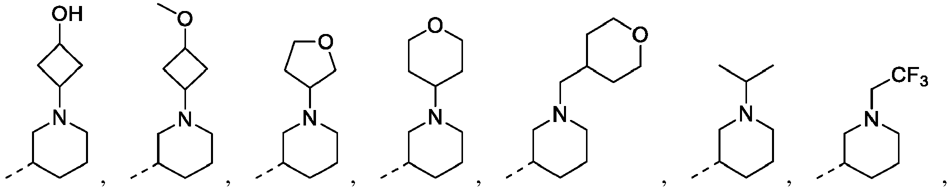 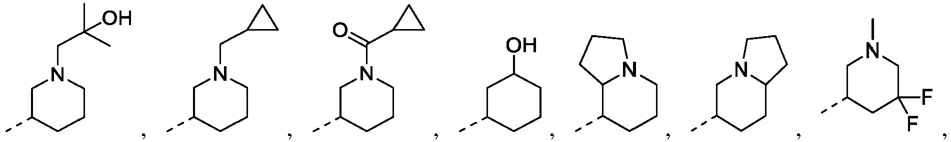 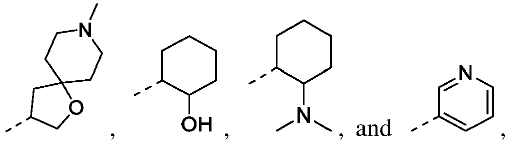 and other variables are as defined in the present disclosure.

In some embodiments of the present disclosure, the R₇ is selected from H, 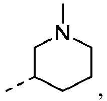 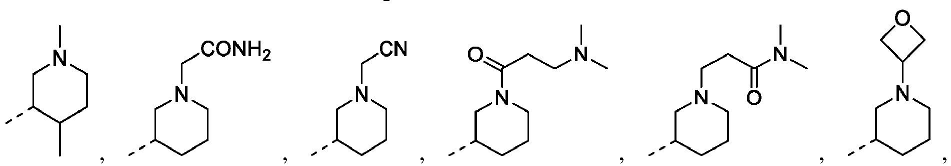 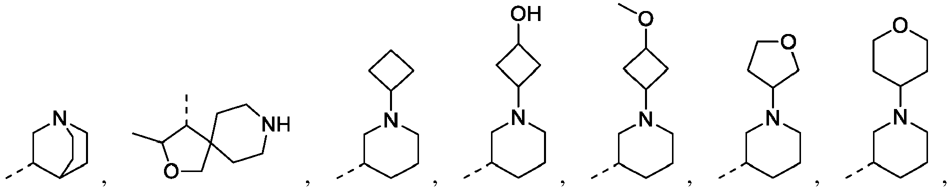 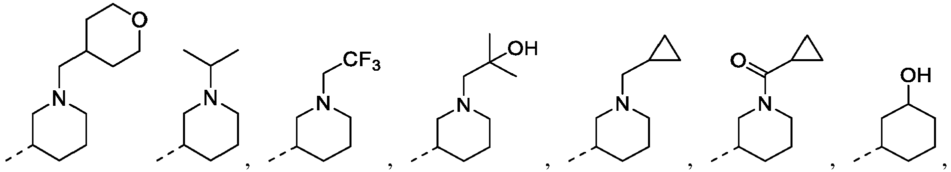 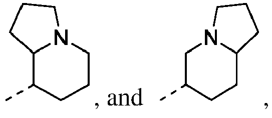 and other variables are as defined in the present disclosure.

In some embodiments of the present disclosure, the R₇ is selected from H, 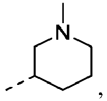 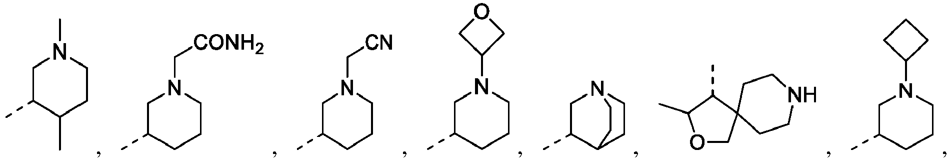 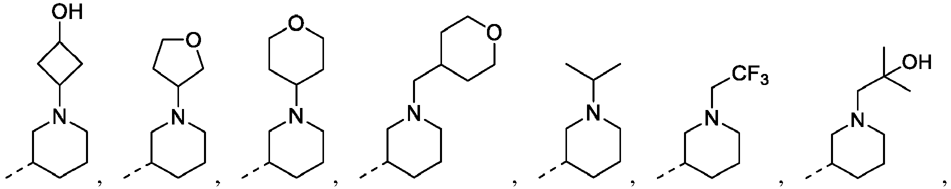 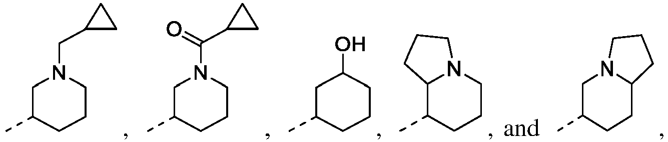 and other variables are as defined in the present disclosure.

In some embodiments of the present disclosure, the R₇ is selected from H, 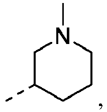 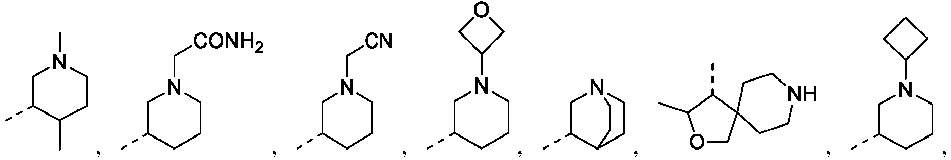 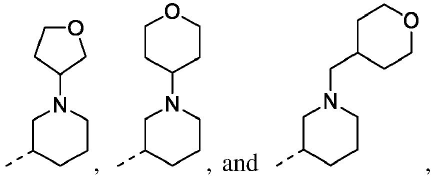 and other variables are as defined in the present disclosure.

In some embodiments of the present disclosure, the R₇ is selected from H, 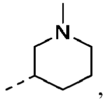 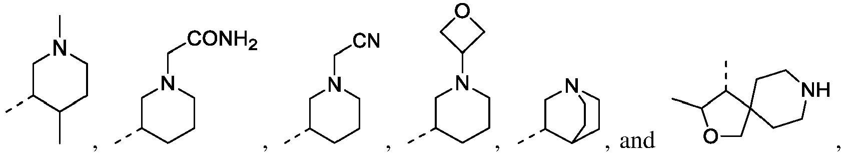 and other variables are as defined in the present disclosure.

In some embodiments of the present disclosure, the R₇ is selected from H and 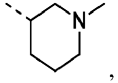 and other variables are as defined in the present disclosure.

In some embodiments of the present disclosure, the structural moiety 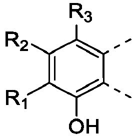 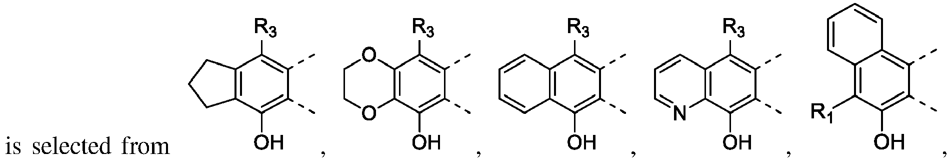 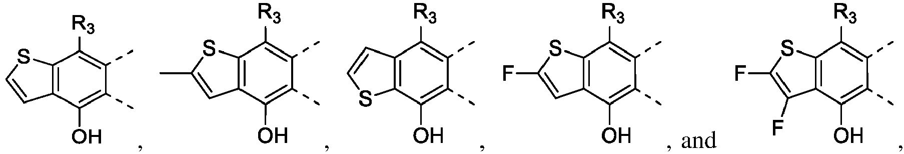 and R₁, R₃, and other variables are as defined in the present disclosure.

In some embodiments of the present disclosure, the structural moiety 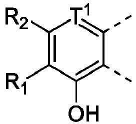 is selected from 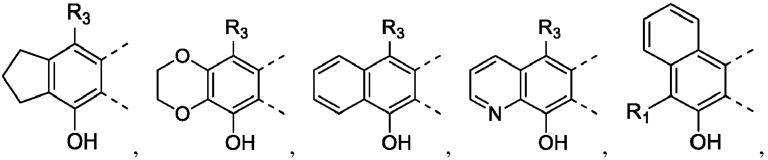 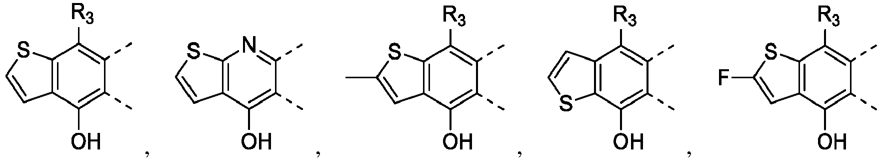 , and 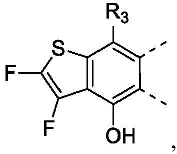 and R₁, R₃, and other variables are as defined in the present disclosure.

In some embodiments of the present disclosure, the structural moiety 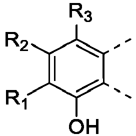 is selected from 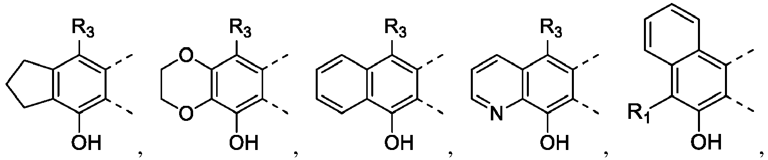 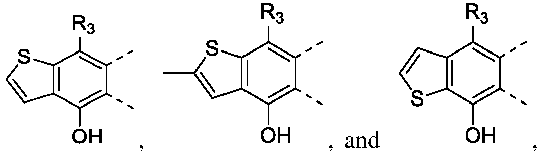 and R₁, R₃, and other variables are as defined in the present disclosure.

In some embodiments of the present disclosure, the structural moiety 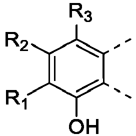 is selected from 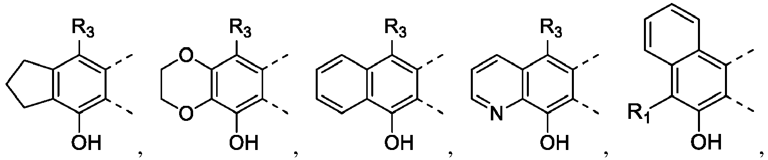 and 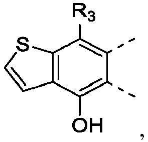 and R₁, R₃, and other variables are as defined in the present disclosure.

In some embodiments of the present disclosure, the structural moiety 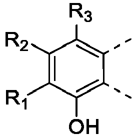 is selected from 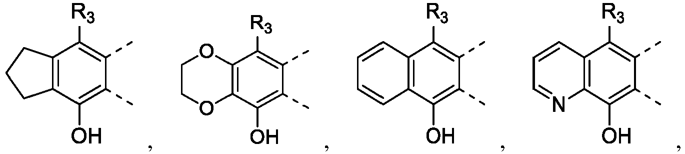 and 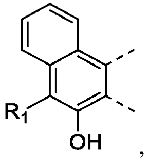 and R₁, R₃, and other variables are as defined in the present disclosure.

In some embodiments of the present disclosure, the structural moiety 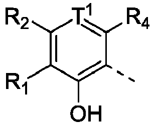 is selected from 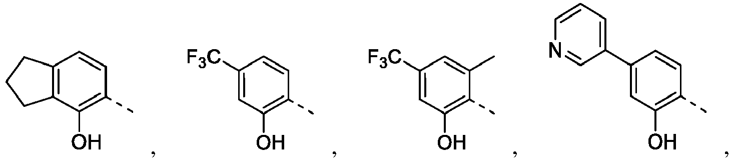 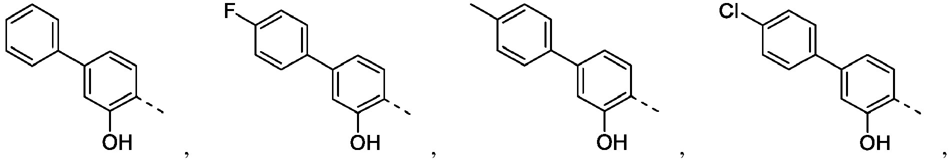 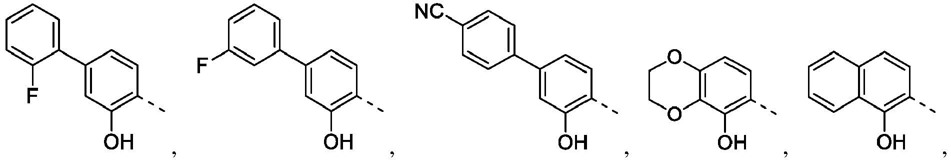 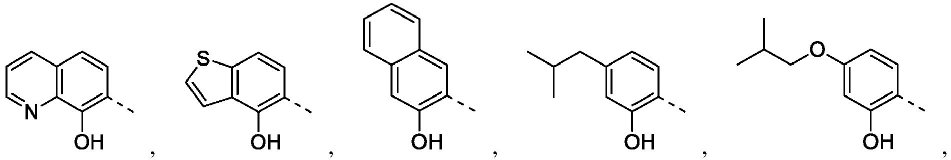 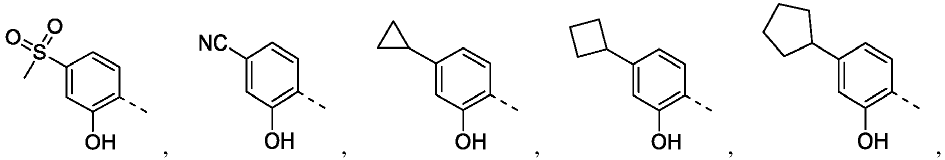 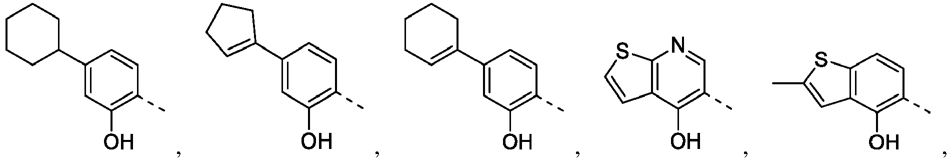 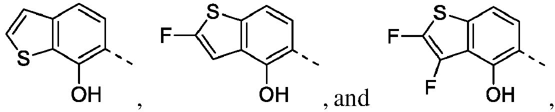 and other variables are as defined in the present disclosure.

In some embodiments of the present disclosure, the structural moiety 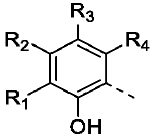 is selected from 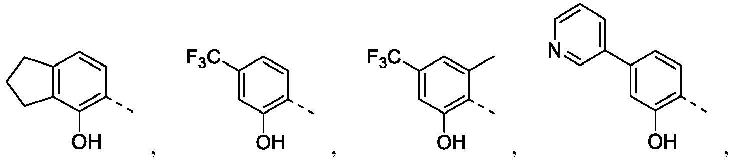 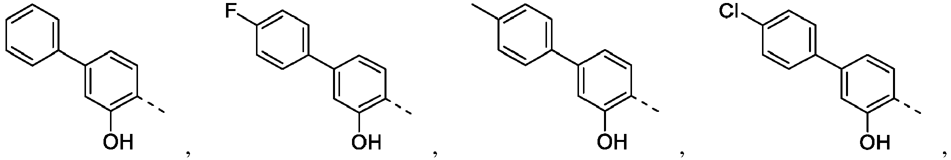 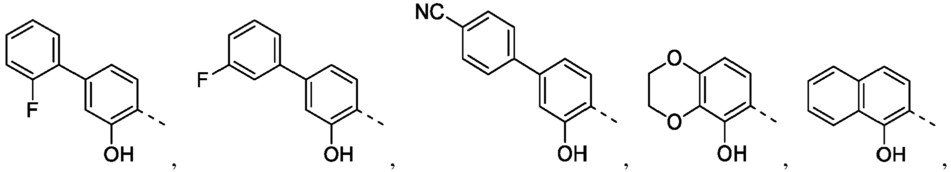 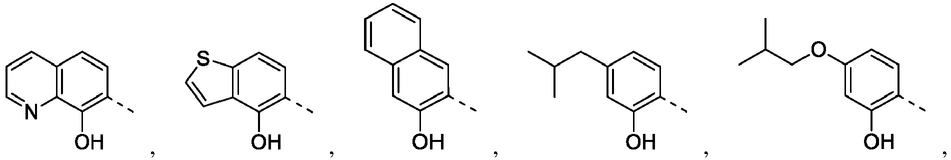 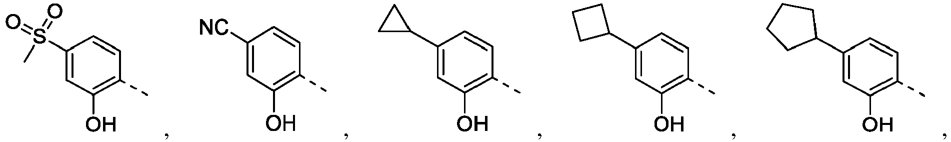 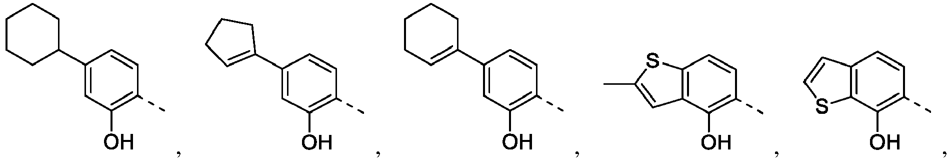 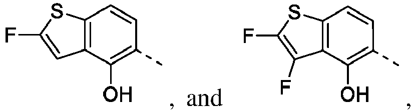 and other variables are as defined in the present disclosure.

In some embodiments of the present disclosure, the structural moiety 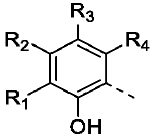 is selected from 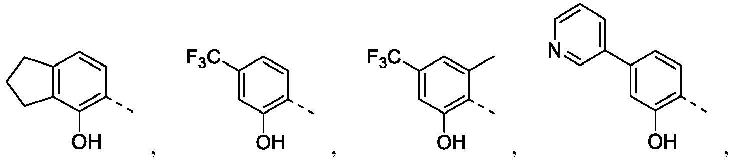 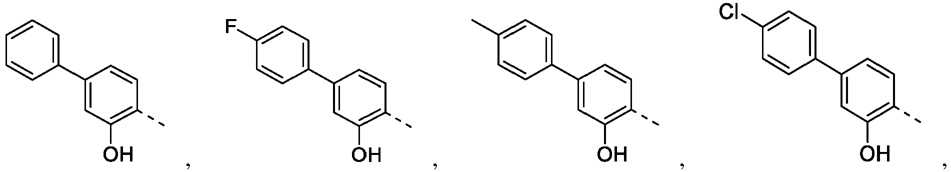 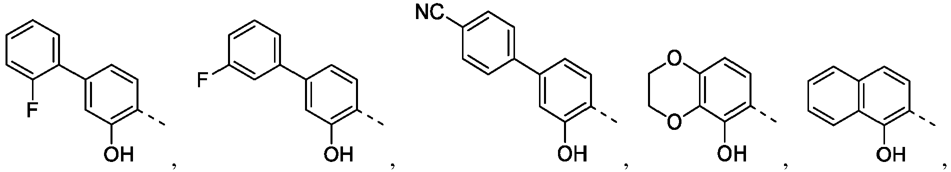 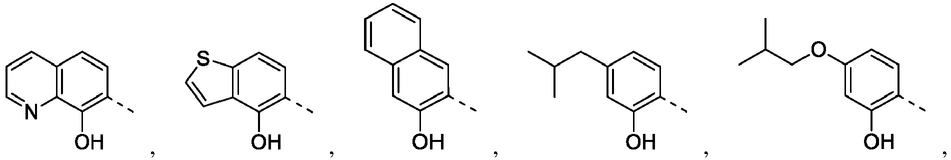 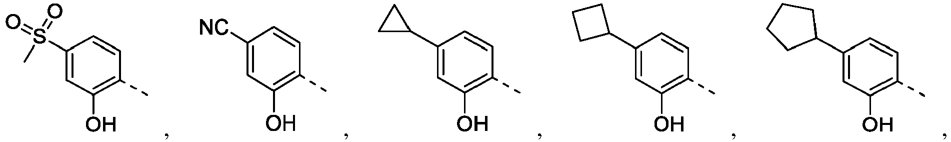 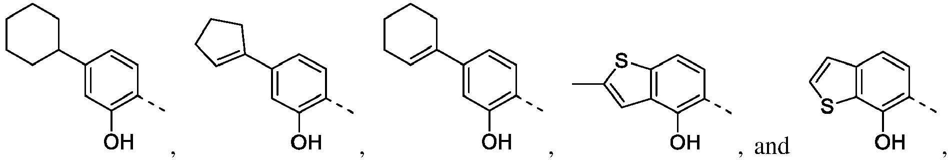 and other variables are as defined in the present disclosure.

In some embodiments of the present disclosure, the structural moiety 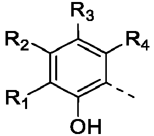 is selected from 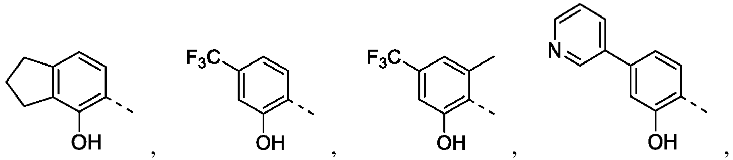 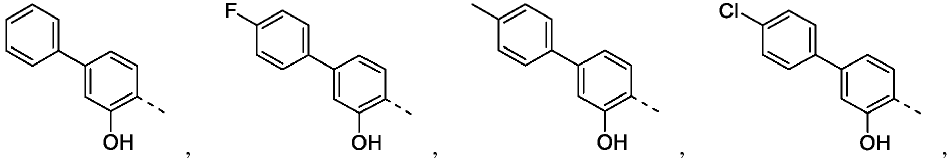 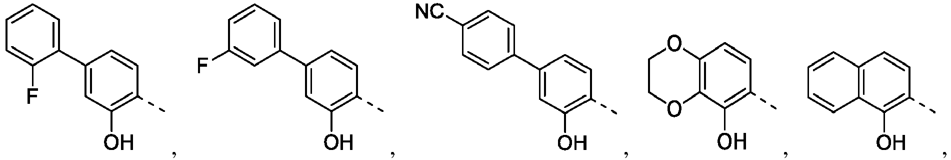 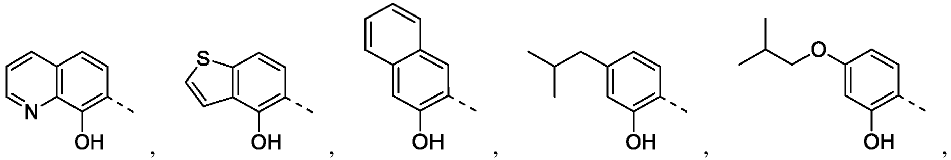 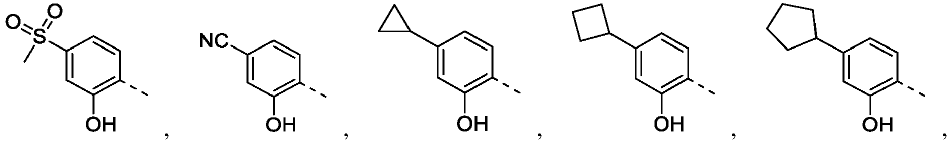 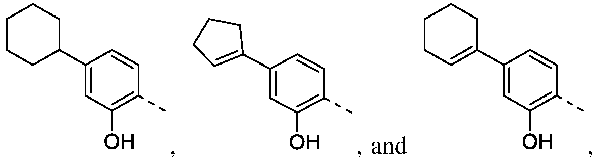 and other variables are as defined in the present disclosure.

In some embodiments of the present disclosure, the structural moiety 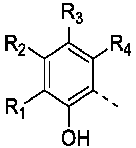 is selected from 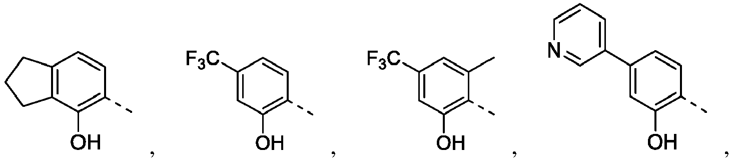 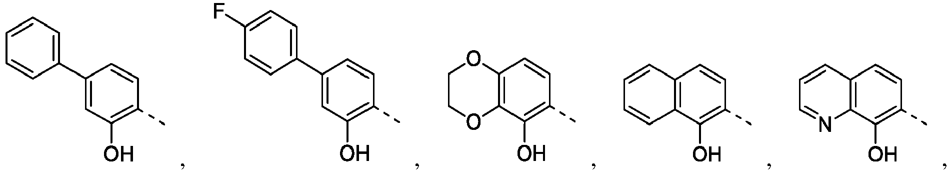 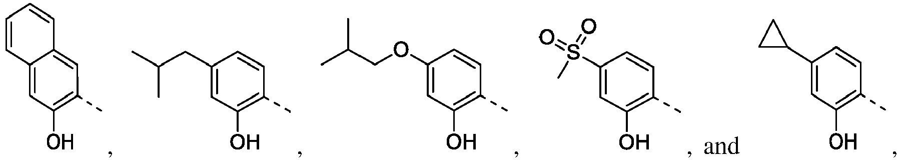 and other variables are as defined in the present disclosure.

In some embodiments of the present disclosure, the structural moiety 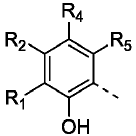 is selected from 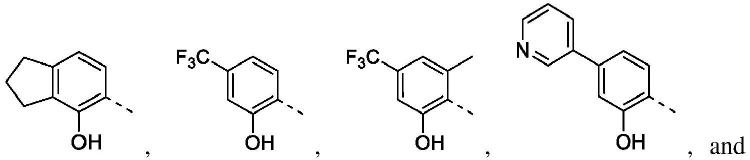 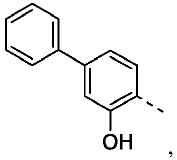 and other variables are as defined in the present disclosure.

In some embodiments of the present disclosure, the compound has a structure of formula (I) or (VI-1): 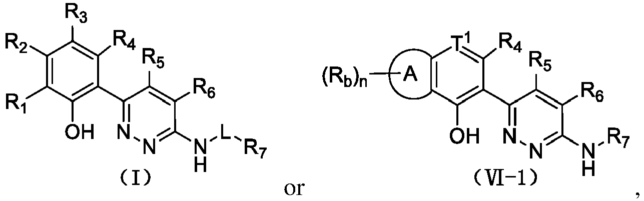 wherein T¹, L, R₁, R₂, R₃, R₄, R₅, R₆, R₇, R_{b}, n, and ring A are as defined in the present disclosure.

In some embodiments of the present disclosure, the compound has a structure of formula (I-1), (I-2), or (I-4): 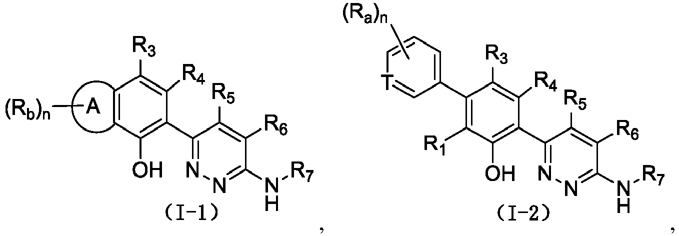 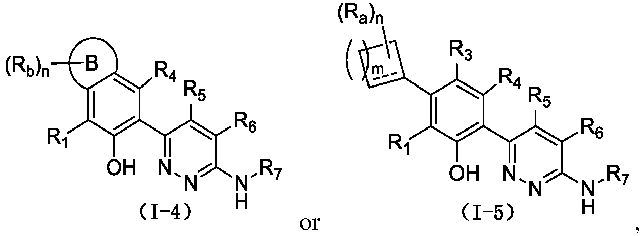
wherein T is selected from CH and N;
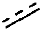 is a single bond or a double bond;
n is selected from 0, 1, 2, 3, or 4;
m is selected from 0, 1, 2, 3, or 4;
ring A, ring B, Rₐ, R_{b}, R₁, R₃, R₄, R₅, R₆, and R₇ are as defined in the present disclosure.

In some embodiments of the present disclosure, the compound has a structure of formula (I-3): 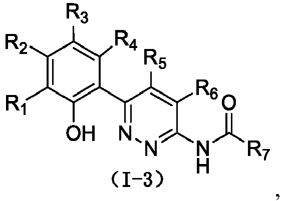 wherein R₁, R₂, R₃, R₄, R₅, R₆, and R₇ are as defined in the present disclosure.

In some embodiments of the present disclosure, the compound has a structure of formula (I-1A): 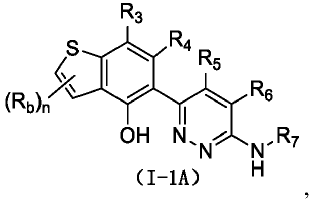 wherein
n is selected from 0, 1, or 2;
R_{b}, R₃, R₄, R₅, R₆, and R₇ are as defined in the present disclosure.

In some embodiments of the present disclosure, the compound has a structure of formula (I-1A-1): 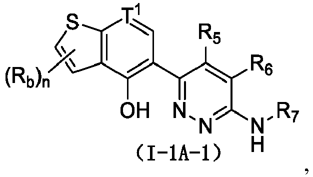 wherein
n is selected from 0, 1, or 2;
T¹ is selected from N and CH;
R₅ is selected from N and -CH₃;
R₆ is selected from N and -CH₃;
R_{b} and R₇ are as defined in the present disclosure. There are still some embodiments of the present disclosure which are obtained by any combination of the above variables.

The present disclosure also provides a compound of the following formula or a pharmaceutically acceptable salt thereof,

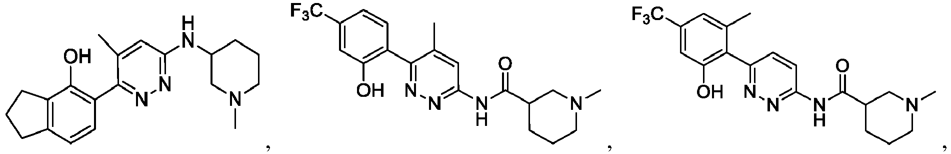

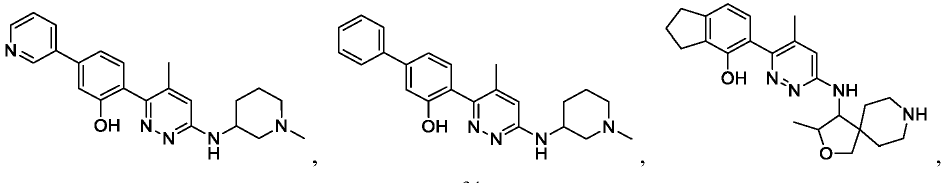

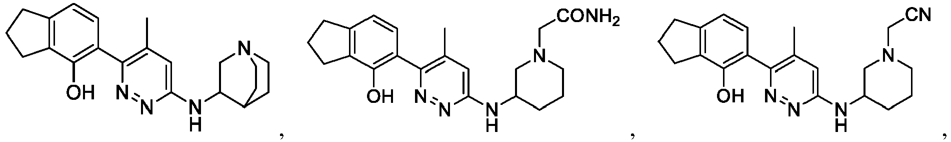

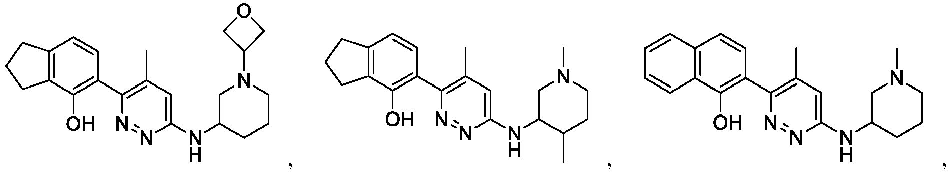

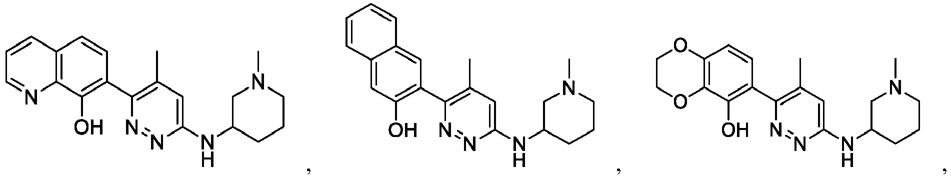

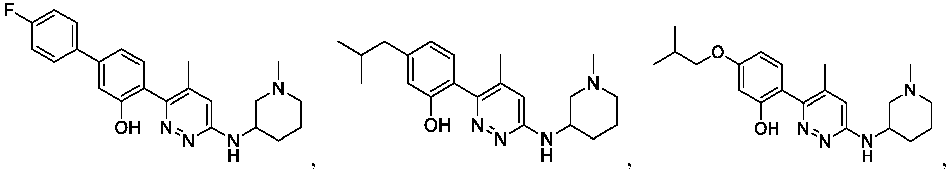

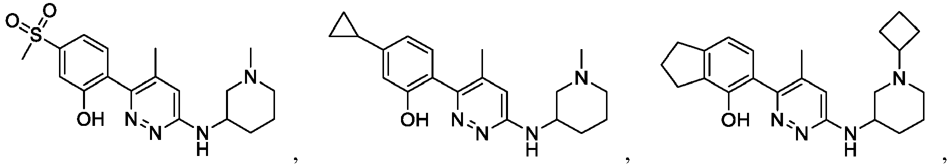

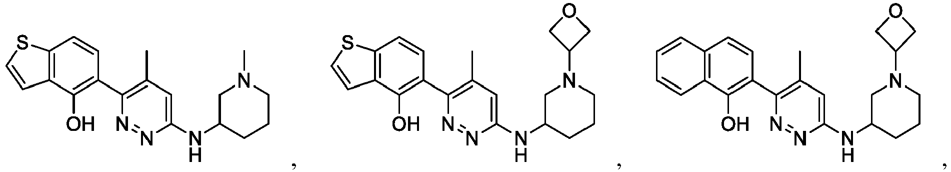

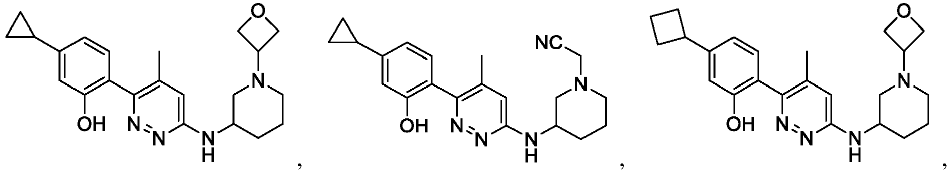

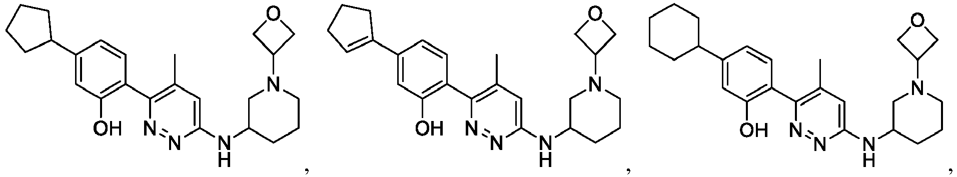

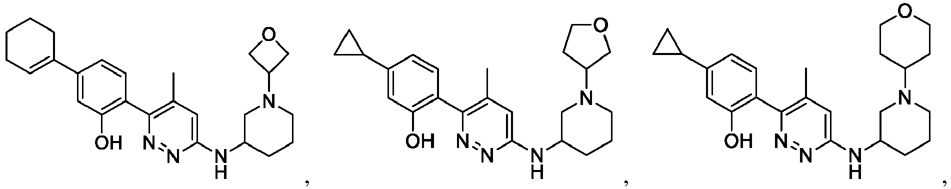

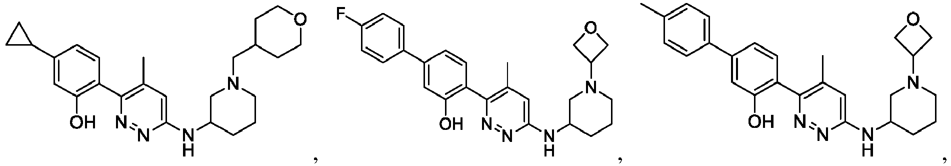

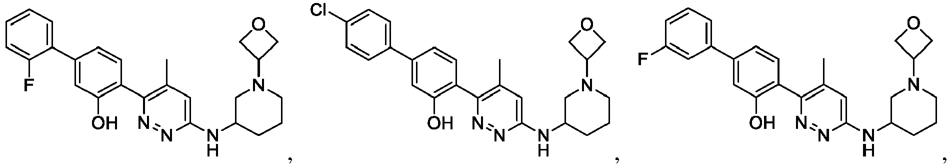

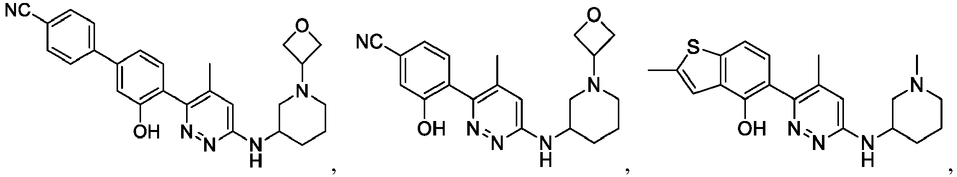

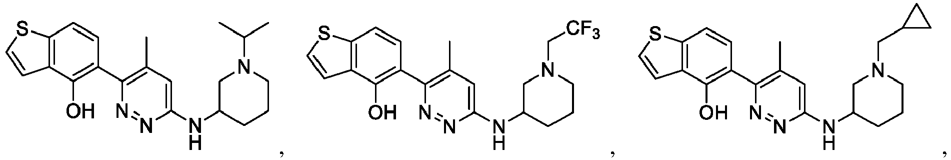

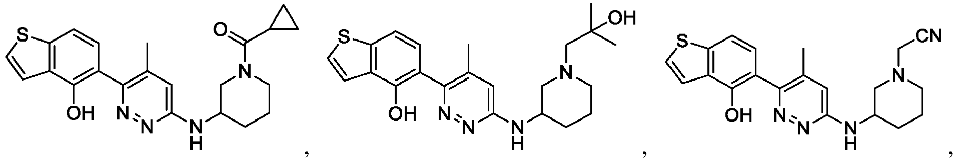

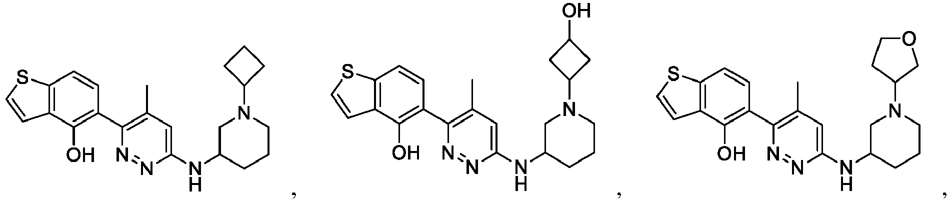

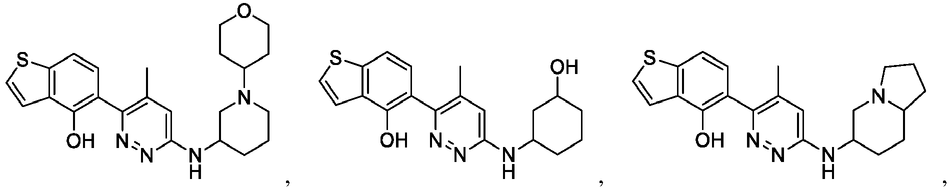

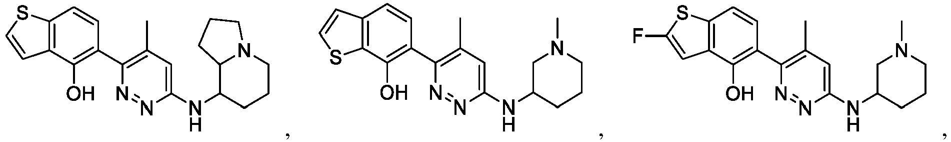

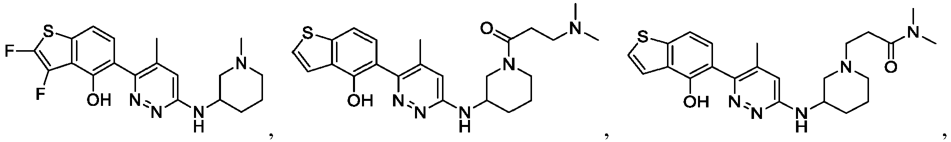

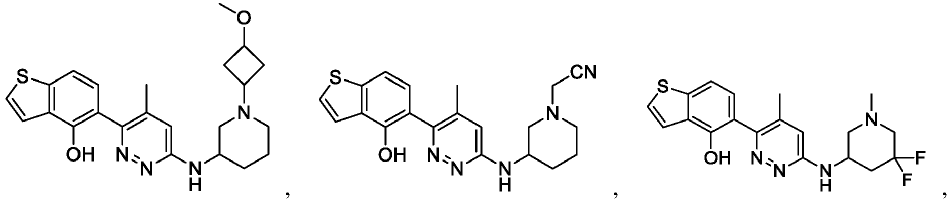

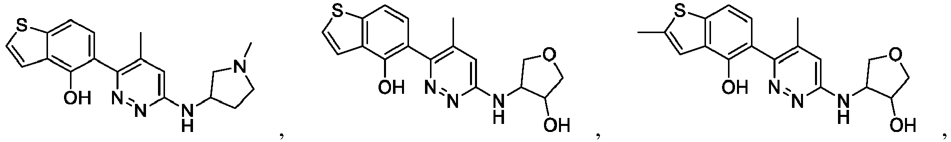

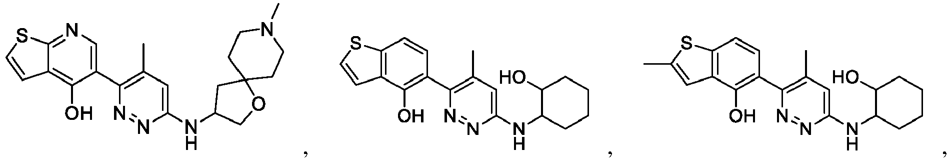

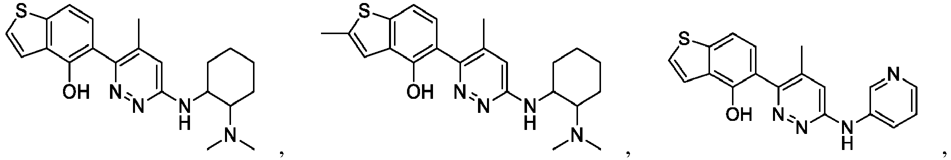

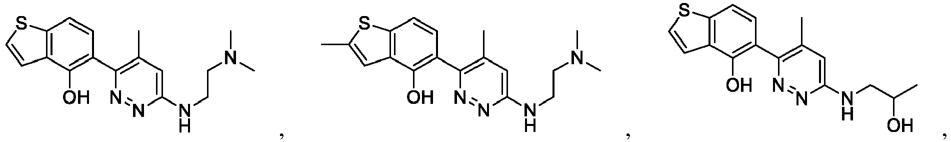

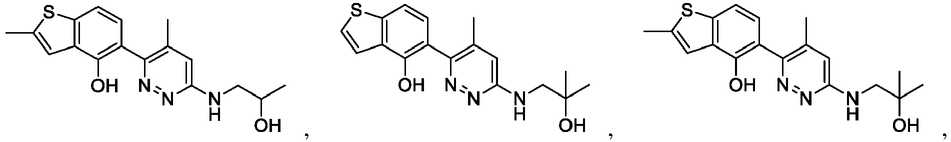

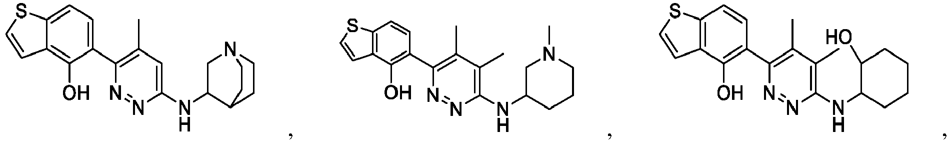

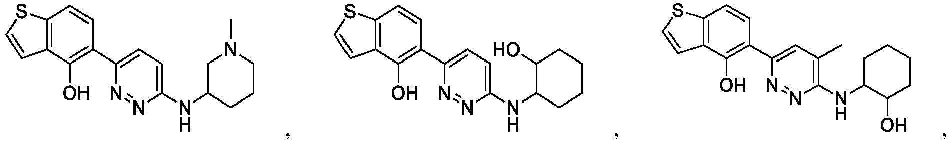

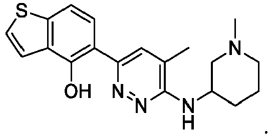

The present disclosure also provides a compound of the following formula or a pharmaceutically acceptable salt thereof,

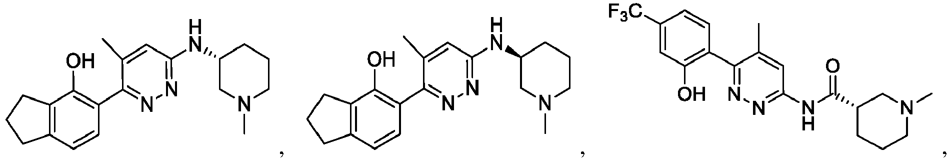

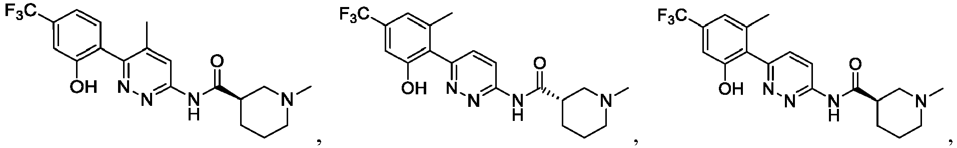

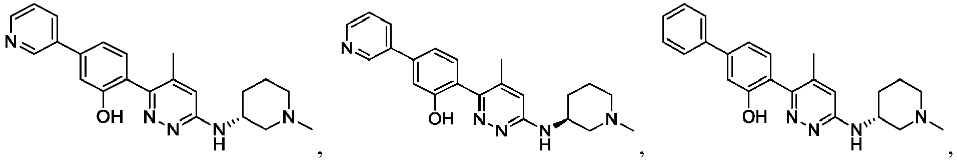

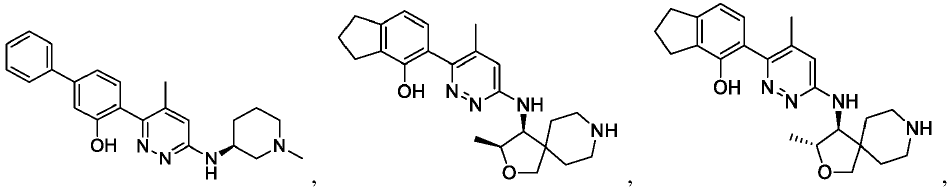

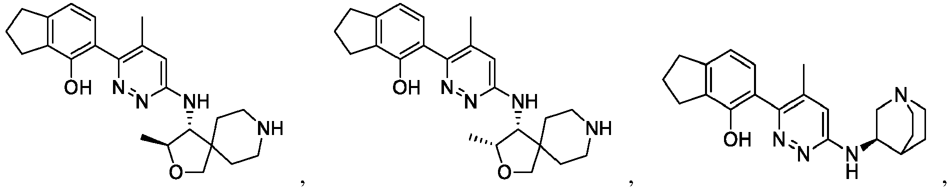

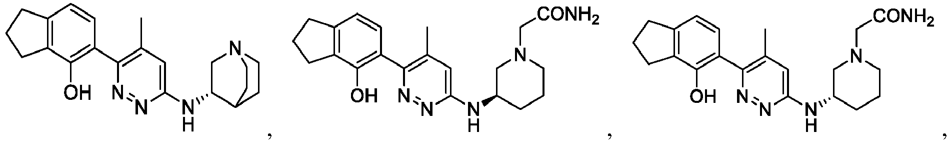

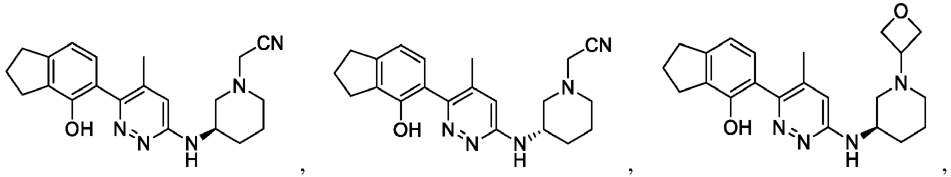

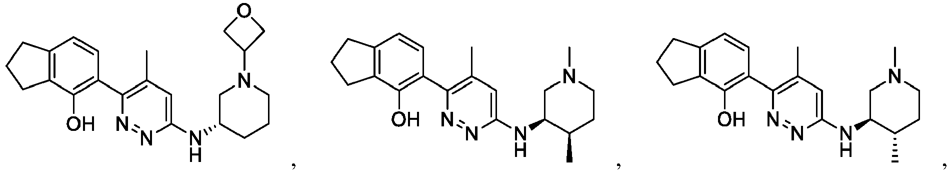

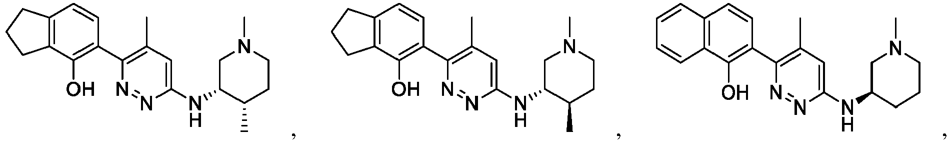

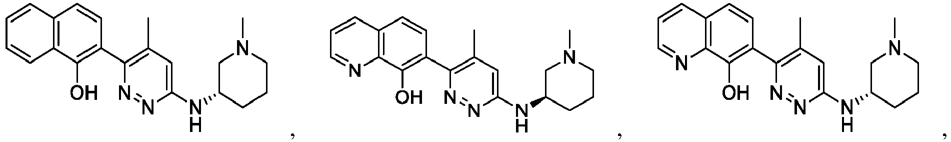

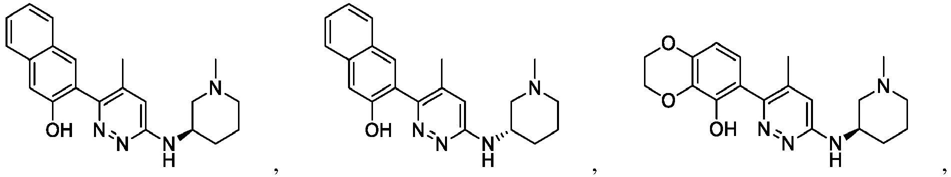

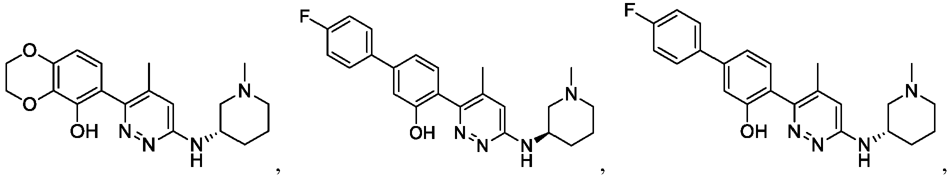

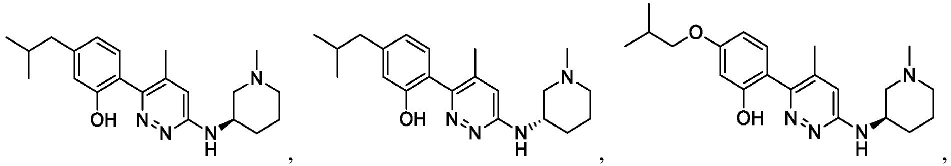

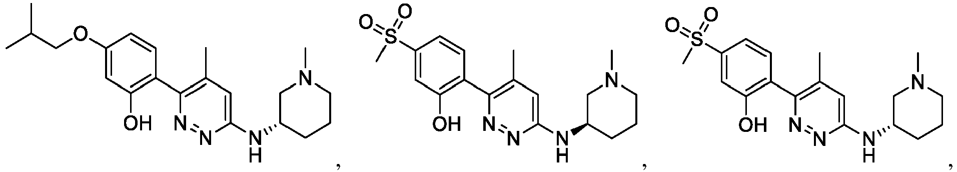

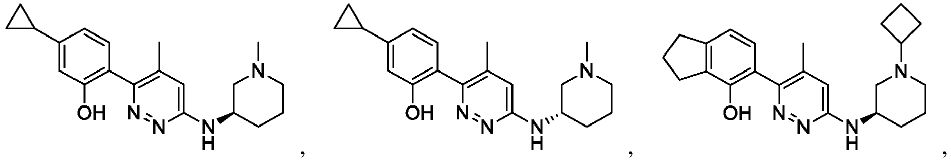

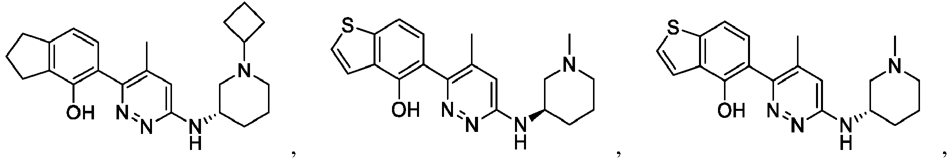

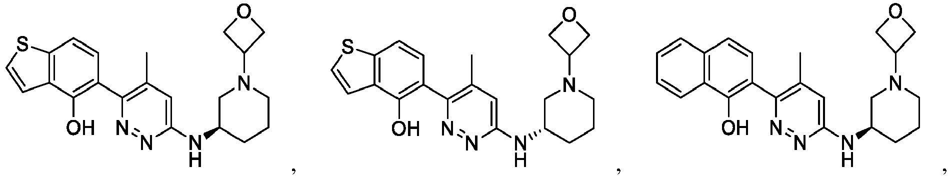

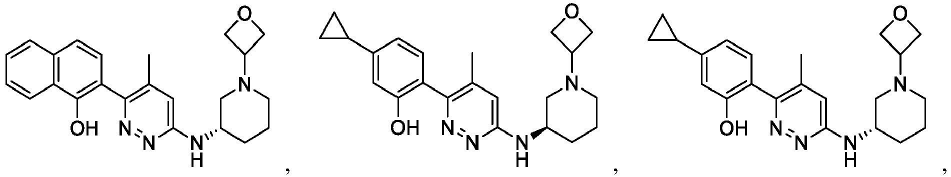

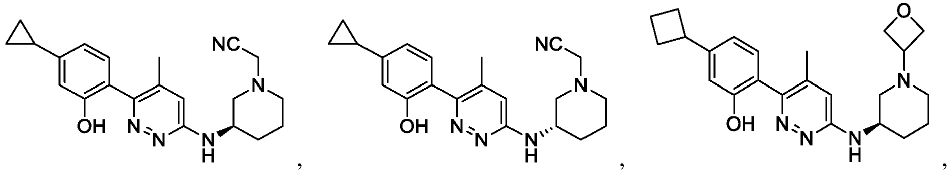

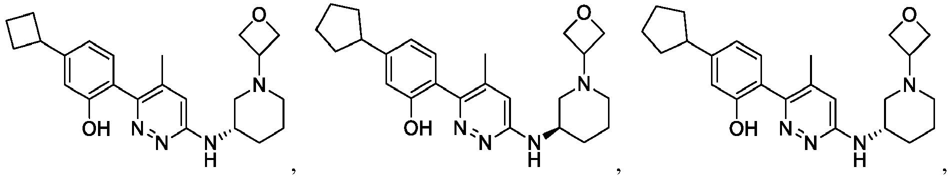

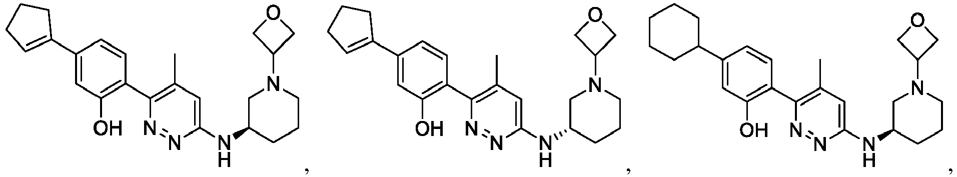

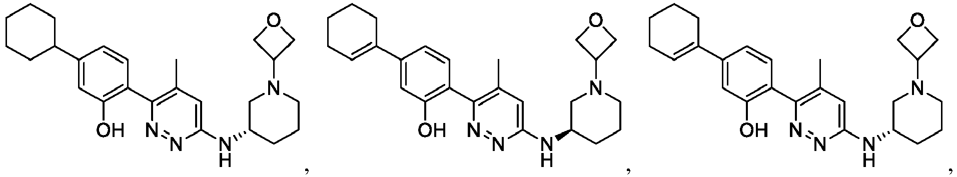

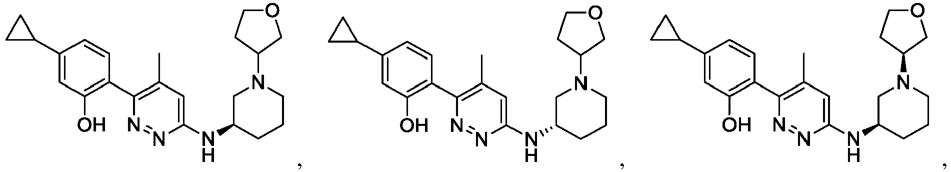

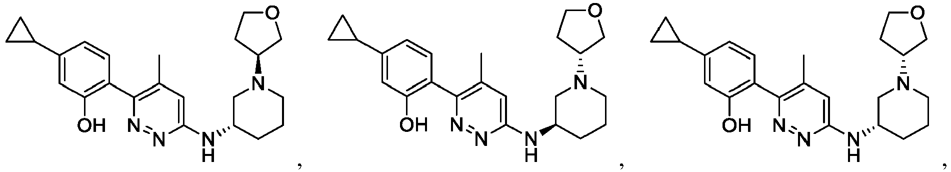

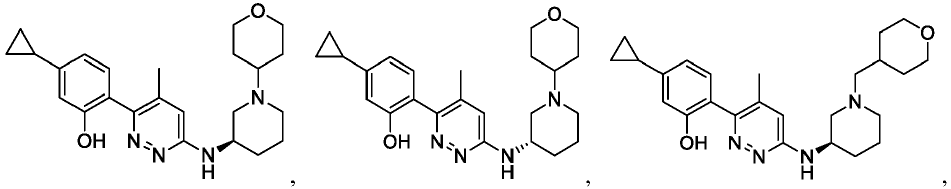

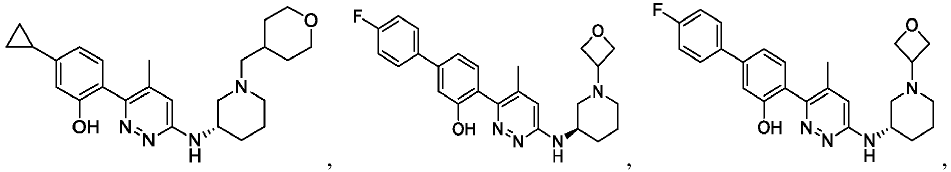

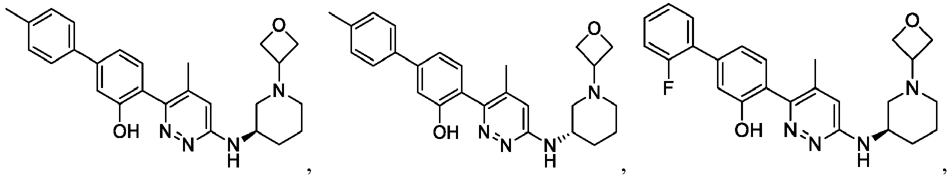

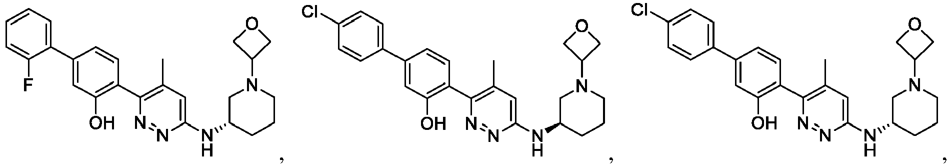

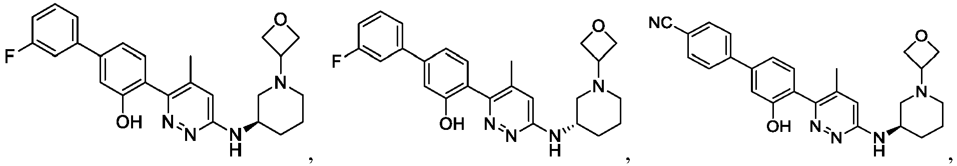

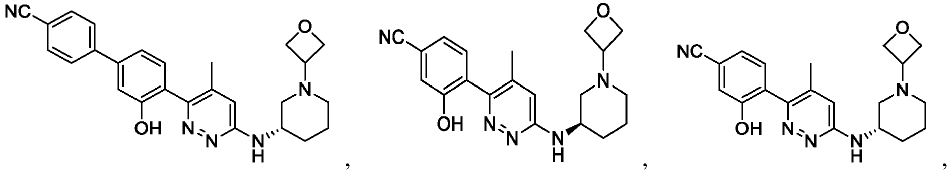

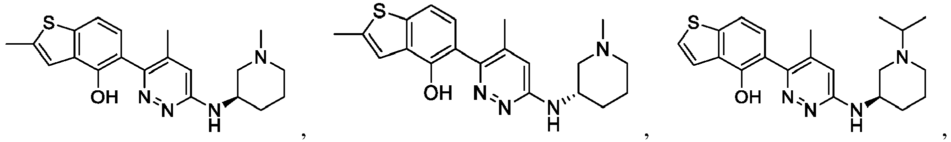

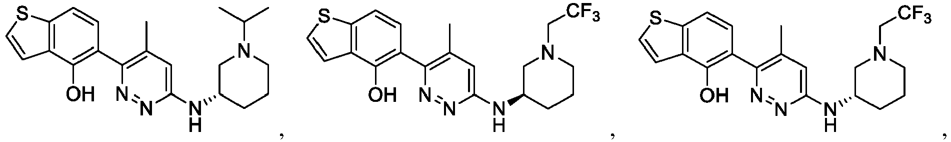

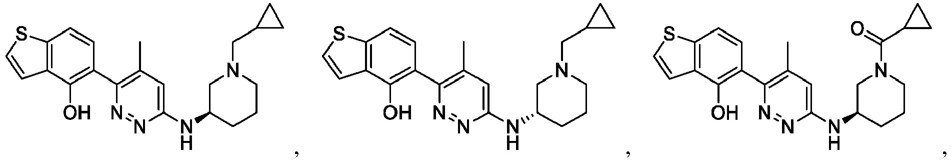

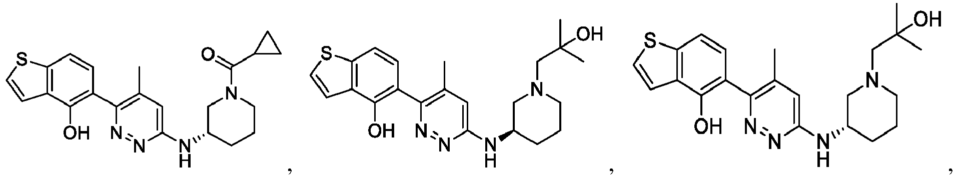

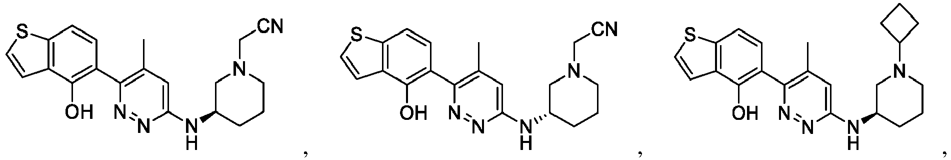

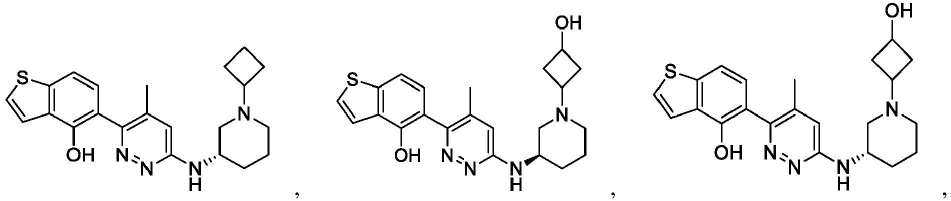

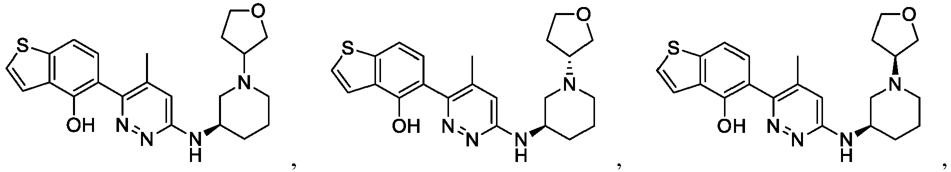

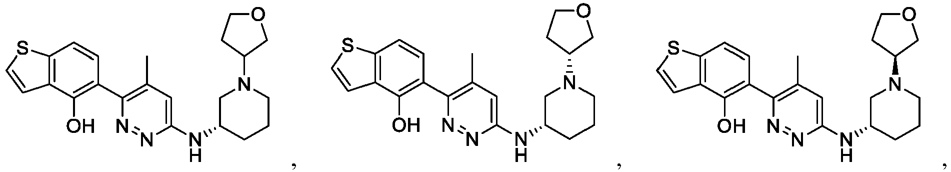

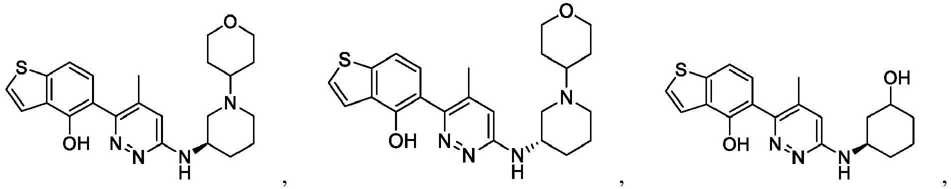

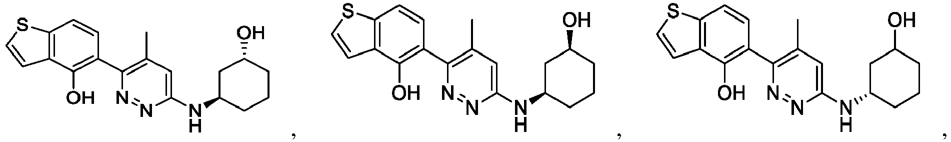

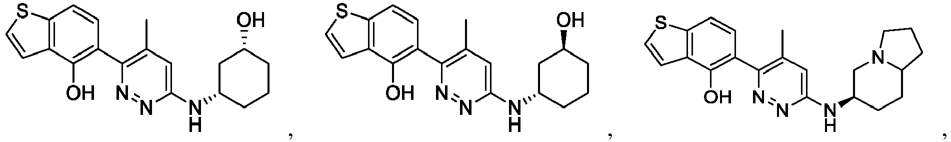

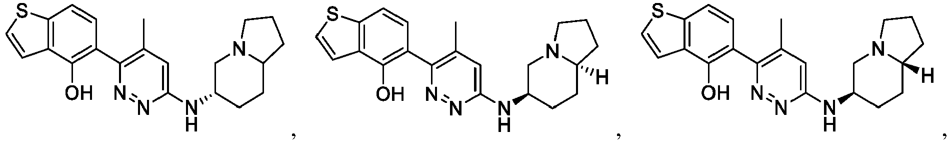

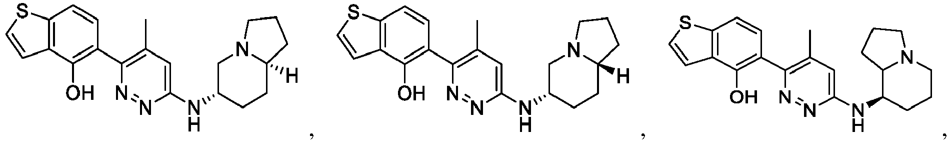

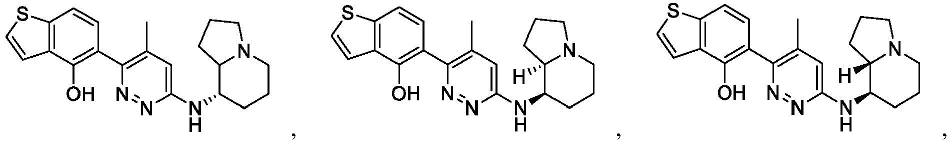

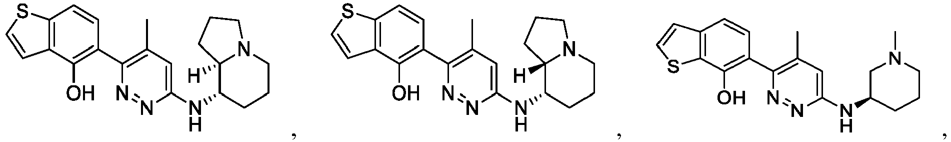

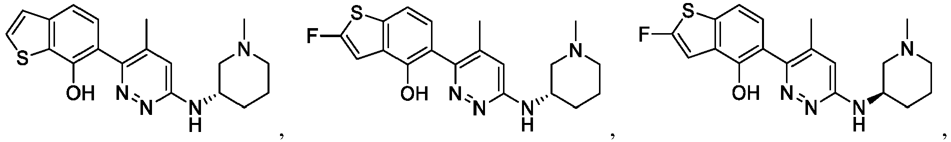

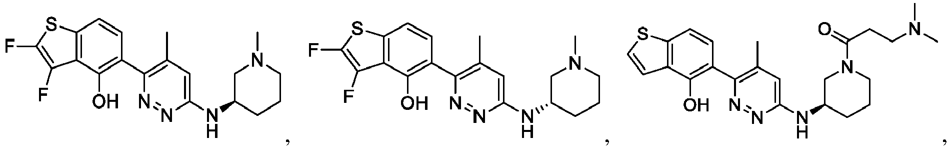

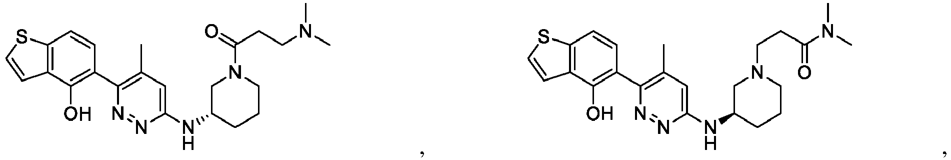

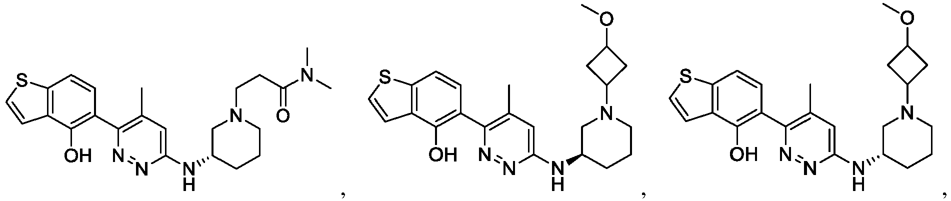

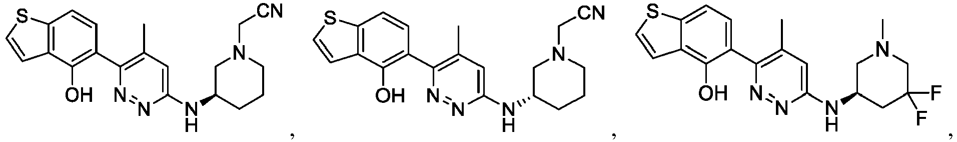

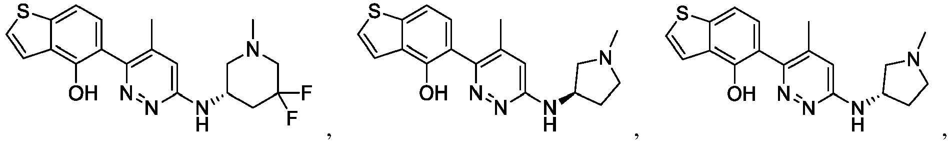

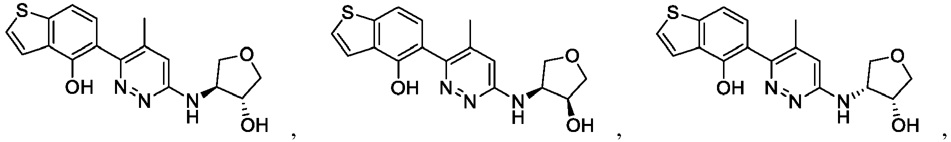

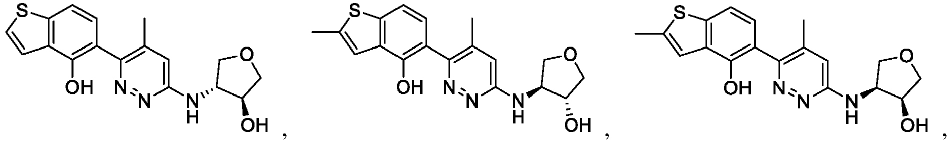

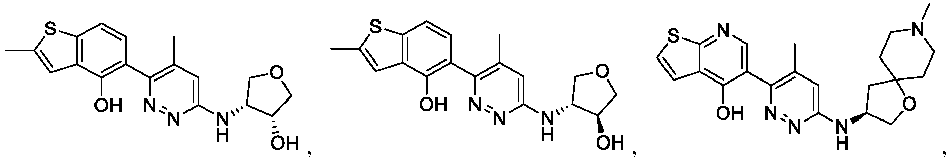

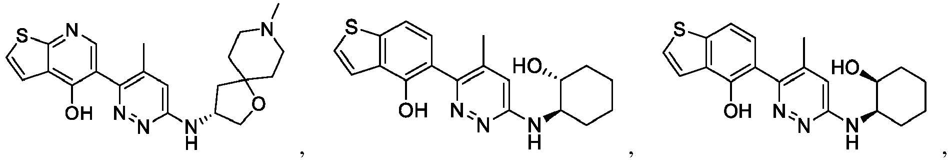

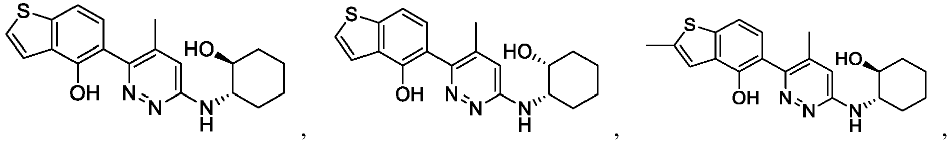

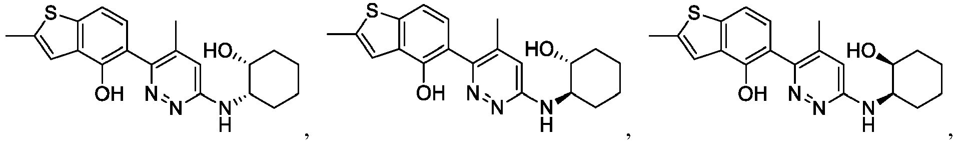

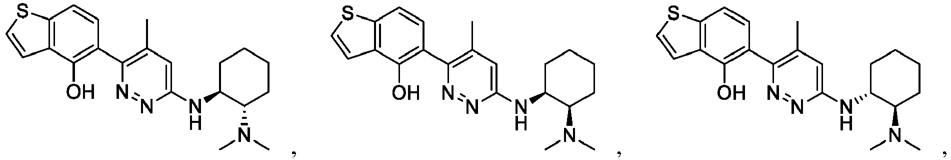

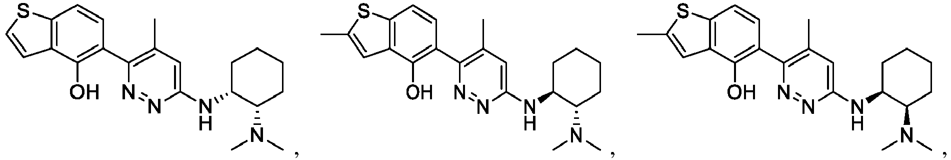

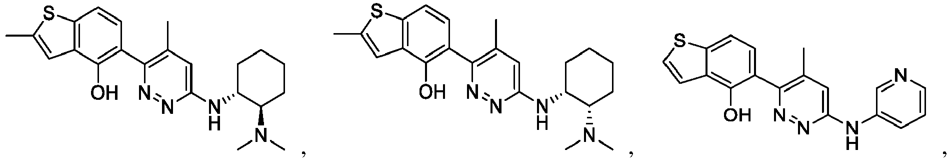

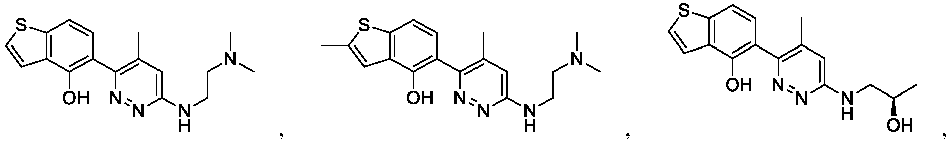

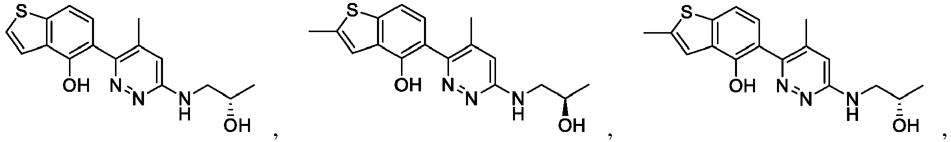

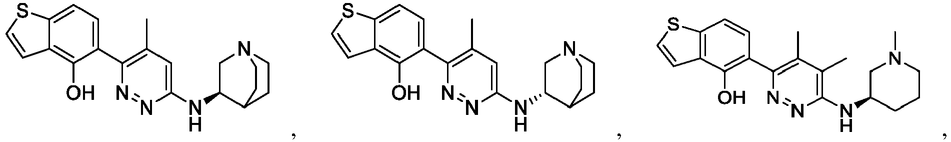

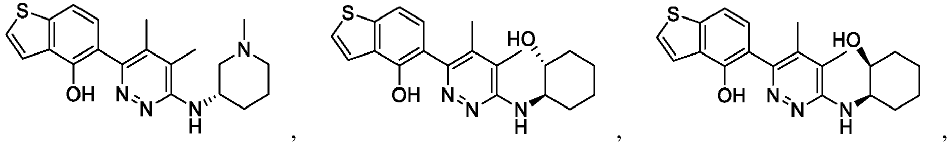

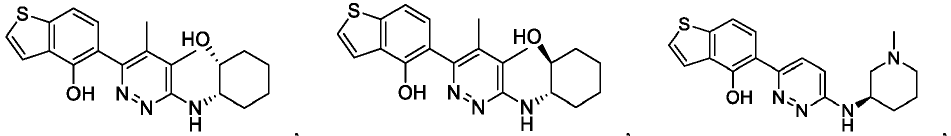

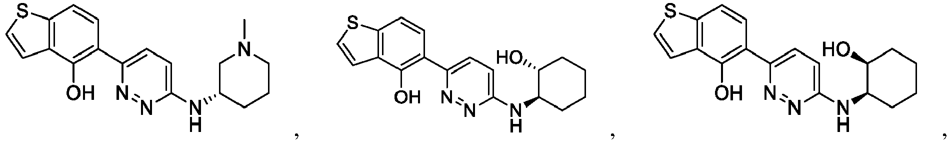

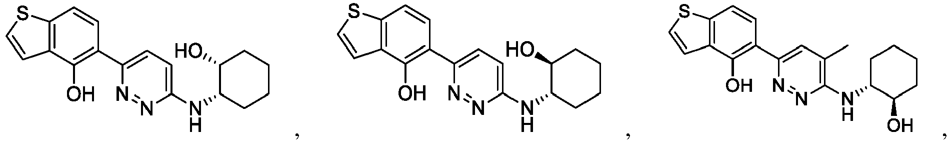

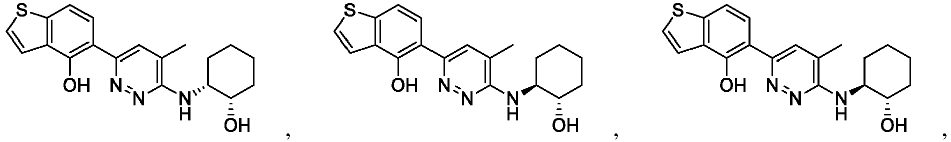

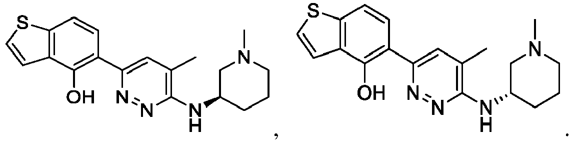

The present disclosure also provides a use of the compound or the pharmaceutically acceptable salt thereof in the manufacture of a medicament for the treatment of Parkinson's disease.

The present disclosure also provides the following experimental test method for the compound or the pharmaceutically acceptable salt thereof:

### Experimental test: Behavioral and histological examination of model of 6-hydroxydopamine-induced Parkinson's disease in rats

### I. Experimental purpose:

The model of Parkinson's disease (PD) is induced by unilateral administration of 6-hydroxydopamine (6-OHDA) in two brain regions of substantia nigra (SN) and striatum (Str) of rats, and the corresponding behavioral tests are carried out (apomorphine-asymmetric rotation test, balance beam test, rotarod test). Some animals may be selected for histological evaluation (tyrosine hydroxylase (TH), microglia (Iba-1) immunofluorescence staining and western blot sampling, and neurotransmitter assay sampling).

### II. Experimental objects and grouping:

69 SD male rats (180 to 220 g), and the specific administration regimen is shown in Table 1.

**Table 1 Detailed list of administration regimens for experimental subjects**

| Group | Animal | Administration group | Dose | Number of animals | Route of administration | Time point of administration |
|---|---|---|---|---|---|---|
| 1 | SD Rat | Sham surgery group | / | 12 | / | / |
| 2 | SD Rat | 6-OHDA modeling group | / | 12 | / | / |
| 3 | SD Rat | 6-OHDA + Hydrochloride of compound **20** | 5 mpk | 15 | p.o. | Twice a day / 21 consecutive days after modeling |
| 4 | SD Rat | 6-OHDA + Hydrochloride of compound **60** | 5 mpk | 15 | p.o. | Twice a day / 21 consecutive days after modeling |

### III. Experimental method:

### 1. Stereotaxic administration in SN and Str regions (unilateral administration, right side)

SN: AP = -5.0 mm; ML = ±1.9 mm; DV = -8.5 mm;
Str: AP = +0.5 mm; ML = ±3.0 mm; DV = -6.0 mm;
Note: the front-to-back distance of bregma at AP position (Y axis), the left-to-right distance lateral to the sagittal suture of ML (X axis), and the vertical downward distance of the skull surface at DV position (Z axis)
6-OHDA (20 µg/8 µL) is dissolved in 0.9% normal saline (NS) (containing 0.02% ascorbic acid) at 0.4 µL/min, before and after 10 min, 4 µL is given to SN and Str of each animal; 0.9% NS (containing 0.02% ascorbic acid) is given to Sham group; the details are as follows:
   1) Animal anesthesia after weighing: animal anesthesia;
   2) rat head fixation: to ensure that the head of the animal does not move, and adjust the brain surface to be flat;
   3) determine the bregma: the head of the rat is shaved, and a skin incision is made along the sagittal suture to expose the bregma;
   4) positioning of SN and Str regions: positioning the glass electrode to bregma, resetting each axis of the coordinate display to zero, and positioning according to the coordinates;
   5) injection: after slowly inserting the needle to locate in SN and Str, 10 min is waited before administering the drug at a rate of 0.4 µL/min, and then the needle is slowly withdrawn after another 10 min dwell after administration.

### 2. Apomorphine - Asymmetric rotation test

1) Drug injection: Intraperitoneal injection of apomorphine (0.5 mg/kg).
2) Adaptation to the environment: Animals are placed in the test room for 30 to 60 minutes before testing to adapt to the environment;
3) Experimental method: Apomorphine (APO) is injected intraperitoneally (0.5 mg/kg), and the behavioral changes are observed.
4) Analysis of the results: When the apomorphine-induced rotation behavior is positive, rats mostly rotate in situ to the opposite side of the injury using the forelimb on the rotating side as a support point. The rotation behavior of animals is monitored continuously for 30 min, more than 5 times/min, as a quantitative indicator of successful PD modeling.

### 3. Balance beam test

1) Two days before the start of the experiment, mice are placed on a balance beam for 10 minutes per day to acclimatize, and trained to cross the beam 2 times each time. Typically, the mice cross the beam with minimal pauses. When the mice stop, sniff or look around without taking any action to move forward, the experimenter should wear gloves to poke or push from behind to encourage the mice to continue moving forward.
2) The mice are placed on the balance beam at the beginning of the experiment, and the time taken for the mice to pass the balance beam and the number of foot slips are recorded.
3) After the experiment of each animal, the feces are removed, the balance beam is sprayed with 75% alcohol and dried with clean gauze.
4) Evaluation criteria: The average time of two successful passes on the balance beam, the number of foot slips (foot leaving the top of the balance beam).

### 4. Rotarod test (muscle strength test)

1) Adaptation to the environment: Animals are placed in the test room for 30 to 60 minutes before testing to adapt to the environment;
2) adaptive training: each experimental animal is placed on a rotarod fatigue instrument for adaptive training for 5 min;
3) formal testing: parameters of the rotarod fatigue instrument are set to: speed: 20 rpm/min, test time: 5 min, mice are placed on the rotarod in batches for testing, and feces and urine must be cleared after each round, and disinfected with 75% alcohol;
analysis of the results: Statistics of the time each animal is on the rotarod.

### 5. Immunofluorescence staining (TH, Iba-1)

1) Animal anesthesia after weighing: animal anesthesia;
2) Fixation and perfusion of mice: the mice are fixed on a dissecting board, the chest cavity is opened, the right auricle is cut open, the left ventricle is perfused with NS at 30 rpm/min, and then 4% paraformaldehyde (PFA) is perfused after the blood is washed away, and the intact brain tissue is stripped out after fixation;
3) Brain fixation and sugar precipitation: the stripped brain tissue is soaked in 4% PFA and placed in a refrigerator at 4°C overnight, and then the brain tissue is changed to 20%, 30%, and 35% gradient sucrose solutions for sugar precipitation (the time or concentration is appropriately increased according to the sugar precipitation of brain tissue);
4) The brain tissue is taken out, embedded with OCT embedding agent, and sliced with a cryostat, with a thickness of 16 µm. The brain tissue is frozen at -20°C or -80°C after cutting;
5) Slices are rewarmed for 30 min.
6) Sealing: 10% serum +0.3% TritonX-100 at room temperature for 1 hour.
7) The slices are spun, added with primary antibody, overnight at 4°C.
8) Slices are rewarmed for 30 min.
9) The primary antibody is washed for 5 min × 3 times.
10) A secondary antibody is added backlight at room temperature for 2 hours.
11) The secondary antibody is washed for 5 min × 3 times.
12) 4',6-Diamidino-2-phenylindole (DAPI) is added at room temperature for 10 min.
13) DAPI is washed for 5 min × 3 times.
14) Sealing: Glycerin (70%) is used to avoid air bubbles.

Conclusion: Compared with the 6-hydroxydopamine modeling group, the compounds of the present disclosure have the effect of improving behavioral indicators and increasing the expression of TH in striatum.

### Technical effect

The NLRP3 inhibitor provided in the present disclosure can effectively inhibit the activity of NLRP3 and the activation of downstream caspase-1, thereby inhibiting the maturation and secretion of IL-1β, and has good pharmacokinetic properties, thus can be used for the treatment of diseases associated with abnormal activation of NLRP3 inflammasome.

### Definition and description

Unless otherwise specified, the following terms and phrases when used herein have the following meanings. A specific term or phrase should not be considered indefinite or unclear in the absence of a particular definition, but should be understood according to the common meaning. When a trading name appears herein, it is intended to refer to its corresponding commercial product or active ingredient thereof.

The term "pharmaceutically acceptable" is used herein in terms of those compounds, materials, compositions, and/or dosage forms, which are suitable for use in contact with human and animal tissues within the scope of reliable medical judgment, without excessive toxicity, irritation, anaphylactic reaction, or other problems or complications, commensurate with a reasonable benefit/risk ratio.

The term "pharmaceutically acceptable salt" refers to a salt of the compound of the present disclosure that is prepared by reacting the compound having a specific substituent of the present disclosure with a relatively non-toxic acid or base. When the compound of the present disclosure contains a relatively acidic functional group, a base addition salt can be obtained by contacting the compound with a sufficient amount of a base in a pure solution or a suitable inert solvent. The pharmaceutically acceptable base addition salt includes a salt of sodium, potassium, calcium, ammonium, organic amine, magnesium, or similar salts. When the compound of the present disclosure contains a relatively basic functional group, an acid addition salt can be obtained by contacting the compound with a sufficient amount of acid in a pure solution or a suitable inert solvent. Examples of the pharmaceutically acceptable acid addition salt include an inorganic acid salt, wherein the inorganic acid includes, for example, hydrochloric acid, hydrobromic acid, nitric acid, carbonic acid, bicarbonate, phosphoric acid, monohydrogen phosphate, dihydrogen phosphate, sulfuric acid, hydrogen sulfate, hydroiodic acid, phosphorous acid; and an organic acid salt, wherein the organic acid includes, for example, acetic acid, propionic acid, isobutyric acid, maleic acid, malonic acid, benzoic acid, succinic acid, suberic acid, fumaric acid, lactic acid, mandelic acid, phthalic acid, benzenesulfonic acid, p-toluenesulfonic acid, citric acid, tartaric acid, and methanesulfonic acid; and salts of amino acid (such as arginine), and a salt of an organic acid such as glucuronic acid. Certain specific compounds of the present disclosure contain both basic and acidic functional groups, thus can be converted to any base or acid addition salt.

The pharmaceutically acceptable salt of the present disclosure can be prepared from the parent compound that contains an acidic or basic moiety by a conventional chemical method. Generally, such salt can be prepared by reacting the free acid or base form of the compound with a stoichiometric amount of an appropriate base or acid in water or an organic solvent or a mixture thereof.

The compounds of the present disclosure may exist in specific geometric or stereoisomeric forms. The present disclosure contemplates all such compounds, including cis and trans isomers, (-)- and (+)-enantiomers, (R)- and (S)-enantiomers, diastereoisomers, (D)-isomers, (L)-isomers, and racemic and other mixtures thereof, such as enantiomers or diastereomer enriched mixtures, all of which are within the scope of the present disclosure. Additional asymmetric carbon atoms may be present in substituents such as alkyl. All these isomers and their mixtures are encompassed within the scope of the present disclosure.

Unless otherwise specified, the term "enantiomer" or "optical isomer" refers to stereoisomers that are mirror images of each other.

Unless otherwise specified, the term "cis-trans isomer" or "geometric isomer" is caused by the inability to rotate freely of double bonds or single bonds of ring-forming carbon atoms.

Unless otherwise specified, the term "diastereomer" refers to a stereoisomer in which a molecule has two or more chiral centers and the relationship between the molecules is not mirror images.

Unless otherwise specified, "(+)" refers to dextrorotation, "(-)" refers to levorotation, and "(±)" refers to racemic.

Unless otherwise specified, the absolute configuration of a stereogenic center is represented by a wedged solid bond ( 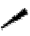 ) and a wedged dashed bond ( 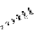 ), and the relative configuration of a stereogenic center is represented by a straight solid bond ( 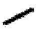 ) and a straight dashed bond ( 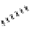 ), a wave line ( 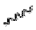 ) is used to represent a wedged solid bond ( 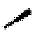 ) or a wedged dashed bond ( 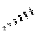 ), or the wave line ( 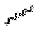 ) is used to represent a straight solid bond ( 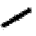 ) and a straight dashed bond ( 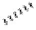 ).

The compounds of the present disclosure may exist in specific. Unless otherwise specified, the term "tautomer" or "tautomeric form" means that at room temperature, the isomers of different functional groups are in dynamic equilibrium and can be transformed into each other quickly. If tautomers possibly exist (such as in solution), the chemical equilibrium of tautomers can be reached. For example, proton tautomer (also called prototropic tautomer) includes interconversion through proton migration, such as keto-enol isomerization and imineenamine isomerization. Valence tautomer includes some recombination of bonding electrons for mutual transformation. A specific example of keto-enol tautomerization is the tautomerism between two tautomers of pentane-2,4-dione and 4-hydroxypent-3-en-2-one.

Unless otherwise specified, the terms "enriched in one isomer", "enriched in isomers", "enriched in one enantiomer", or "enriched in enantiomers" refer to the content of one of the isomers or enantiomers is less than 100%, and the content of the isomer or enantiomer is greater than or equal to 60%, or greater than or equal to 70%, or greater than or equal to 80%, or greater than or equal to 90%, or greater than or equal to 95%, or greater than or equal to 96%, or greater than or equal to 97%, or greater than or equal to 98%, or greater than or equal to 99%, or greater than or equal to 99.5%, or greater than or equal to 99.6%, or greater than or equal to 99.7%, or greater than or equal to 99.8%, or greater than or equal to 99.9%.

Unless otherwise specified, the term "isomer excess" or "enantiomeric excess" refers to the difference between the relative percentages of two isomers or two enantiomers. For example, if the content of one isomer or enantiomer is 90%, and the content of the other isomer or enantiomer is 10%, the isomer or enantiomer excess (ee value) is 80%.

Optically active (*R*)- and (*S*)-isomer, or *D* and *L* isomer can be prepared using chiral synthesis or chiral reagents or other conventional techniques. If one kind of enantiomer of certain compound of the present disclosure is to be obtained, the pure desired enantiomer can be obtained by asymmetric synthesis or derivative action of chiral auxiliary followed by separating the resulting diastereomeric mixture and cleaving the auxiliary group. Alternatively, when the molecule contains a basic functional group (such as amino) or an acidic functional group (such as carboxyl), the compound reacts with an appropriate optically active acid or base to form a salt of the diastereomeric isomer which is then subjected to diastereomeric resolution through the conventional method in the art to obtain the pure enantiomer. In addition, the enantiomer and the diastereoisomer are generally isolated through chromatography which uses a chiral stationary phase and optionally combines with a chemical derivative method (such as carbamate generated from amine).

The compound of the present disclosure may contain an unnatural proportion of atomic isotope at one or more than one atom that constitutes the compound. For example, the compound can be radiolabeled with a radioactive isotope, such as tritium (³H), iodine-125 (¹²⁵I), or C-14 (¹⁴C). For another example, deuterated drugs can be formed by replacing hydrogen with heavy hydrogen, the bond formed by deuterium and carbon is stronger than that of ordinary hydrogen and carbon, compared with non-deuterated drugs, deuterated drugs have the advantages of reduced toxic and side effects, increased drug stability, enhanced efficacy, extended biological half-life of drugs, etc. All isotopic variations of the compound of the present disclosure, whether radioactive or not, are encompassed within the scope of the present disclosure.

The term "optional" or "optionally" means that the subsequently described event or circumstance may, but does not necessarily, occur, and the description includes instances where the event or circumstance occurs and instances where it does not.

The term "substituted" means one or more than one hydrogen atom(s) on a specific atom are substituted with the substituent, including deuterium and hydrogen variables, as long as the valence of the specific atom is normal and the substituted compound is stable. When the substituent is oxygen (i.e., =O), it means two hydrogen atoms are substituted. Positions on an aromatic ring cannot be substituted with a ketone.

The term "optionally substituted" means an atom can be substituted with a substituent or not, unless otherwise specified, the type and number of the substituent may be arbitrary as long as being chemically achievable.

When any variable (such as R) occurs in the constitution or structure of the compound more than once, the definition of the variable at each occurrence is independent. Thus, for example, if a group is substituted with 0 to 2 R, the group can be optionally substituted with up to two R, wherein the definition of R at each occurrence is independent. Moreover, a combination of the substituent and/or the variant thereof is allowed only when the combination results in a stable compound.

When the number of a linking group is 0, such as -(CRR)₀-, it means that the linking group is a single bond.

When one of the variables is selected from a single bond, it means that the two groups linked by the single bond are connected directly. For example, when L in A-L-Z represents a single bond, the structure of A-L-Z is actually A-Z.

When a substituent is vacant, it means that the substituent does not exist, for example, when X is vacant in A-X, the structure of A-X is actually A. When the enumerative substituent does not indicate by which atom it is linked to the group to be substituted, such substituent can be bonded by any atom thereof. For example, when pyridyl acts as a substituent, it can be linked to the group to be substituted by any carbon atom on the pyridine ring.

When the enumerative linking group does not indicate the direction for linking, the direction for linking is arbitrary, for example, the linking group L contained in 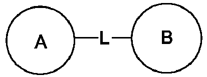 is -M-W-, then -M-W- can link ring A and ring B to form 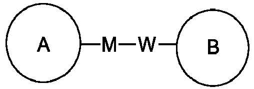 in the direction same as left-to-right reading order and form 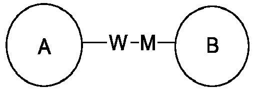 in the direction contrary to left-to-right reading order. A combination of the linking groups, substituents, and/or variables thereof is allowed only when such combination can result in a stable compound.

When the enumerative linking fused ring group does not indicate the linking direction, the linking direction is arbitrary. For example, the fused ring A in 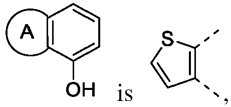 and at this time, 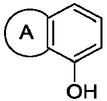 includes two structural moieties, 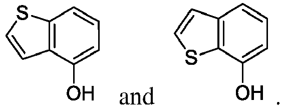 A combination of the linking groups, substituents, and/or variables thereof is allowed only when such combination can result in a stable compound.

Unless otherwise specified, when a group has one or more than one linkable site, any one or more than one site of the group can be linked to other groups through chemical bonds. When the linking site of the chemical bond is not positioned, and there is an H atom at the linkable site, then the number of H atoms at the site will decrease correspondingly with the number of chemical bonds linking thereto so as to meet the corresponding valence. The chemical bond between the site and other groups can be represented by a straight solid bond 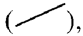 a straight dashed bond 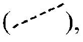 or a wavy line. 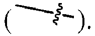 For example, the straight solid bond in -OCH₃ means that it is linked to other groups through the oxygen atom in the group; the straight dashed bonds in 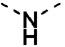 mean that it is linked to other groups through the two ends of nitrogen atom in the group; the wavy lines in 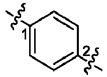 mean that the phenyl group is linked to other groups through carbon atoms at position 1 and position 2. 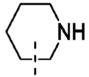 means that it can be linked to other groups through any linkable sites on the piperidinyl by one chemical bond, including at least four types of linkage, including 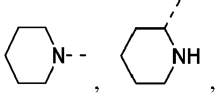 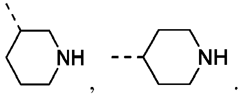 Even though the H atom is drawn on the -N-, 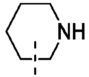 still includes the linkage of 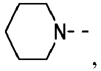 merely when one chemical bond was connected, the H of this site will be reduced by one to the corresponding monovalent piperidinyl.

When the chemical bond of a substituent intersects the chemical bond connecting two atoms in the ring, it means that the substituent may be bonded to any atom on the ring. When the atom to which a substituent is attached is not specified, the substituent may be bonded to any atom. If the atom to which the substituent is attached is in a bicyclic or tricyclic system, it means that the substituent may be bonded to any atom on any ring in the system. A combination of substituents and/or variables thereof is allowed only when such combination can result in a stable compound. For example, the structural moiety 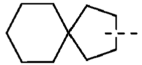 or 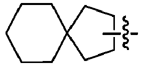 means that it can be substituted at any position on cyclohexyl or cyclopentyl.

Unless otherwise specified, the number of atoms in a ring is usually defined as the number of ring members, for example, "5- to 7-membered ring" refers to a "ring" in which 5 to 7 atoms are arranged around.

Unless otherwise specified, Cₙ₋ₙ₊ₘ or Cₙ-Cₙ₊ₘ includes any specific case of n to n+m carbons, for example, C₁₋₁₂ includes C₁, C₂, C₃, C₄, C₅, C₆, C₇, C₈, C₉, C₁₀, C₁₁, and C₁₂, and any range from n to n+m is also included, for example, C₁₋₁₂ includes C₁₋₃, C₁₋₆, C₁₋₉, C₃₋₆, C₃₋₉, C₃₋₁₂, C₆₋₉, C₆₋₁₂, and C₉₋₁₂, etc.; similarly, n-membered to n+m-membered means that the number of atoms on the ring is from n to n+m, for example, 3- to 12-membered ring includes 3-membered ring, 4-membered ring, 5-membered ring, 6-membered ring, 7-membered ring, 8-membered ring, 9-membered ring, 10-membered ring, 11-membered ring, and 12-membered ring, and any range from n to n+m is also included, for example, 3- to 12-membered ring includes 3- to 6-membered ring, 3- to 9-membered ring, 5- to 6-membered ring, 5- to 7-membered ring, 6- to 7-membered ring, 6- to 8-membered ring, 6- to 10-membered ring, etc.

Unless otherwise specified, the term "C₁₋₃ alkyl" refers to a linear or branched saturated hydrocarbon group consisting of 1 to 3 carbon atoms. The C₁₋₃ alkyl includes C₁₋₂, C₂₋₃ alkyl, etc.; it can be monovalent (such as methyl), divalent (such as methylene), or multivalent (such as methine). Examples of C₁₋₃ alkyl include, but are not limited to, methyl (Me), ethyl (Et), propyl (including *n*-propyl and isopropyl), etc.

Unless otherwise specified, the term "C₁₋₄ alkyl" refers to a linear or branched saturated hydrocarbon group consisting of 1 to 4 carbon atoms. The C₁₋₄ alkyl includes C₁₋₂, C₁₋₃, and C₂₋₃ alkyl, etc.; it can be monovalent (such as methyl), divalent (such as methylene), or multivalent (such as methine). Examples of C₁₋₄ alkyl include, but are not limited to, methyl (Me), ethyl (Et), propyl (including *n*-propyl and isopropyl), butyl (including *n*-butyl, isobutyl, *s*-butyl, and *t*-butyl), etc.

Unless otherwise specified, the term "heteroalkyl" by itself or in combination with another term refers to a stable linear or branched chain alkyl atomic group or a combination thereof consisting of a certain number of carbon atoms and at least one heteroatom or group of heteroatoms. In some embodiments, the heteroatom is selected from B, O, N, and S, wherein the nitrogen and sulfur atoms are optionally oxidized, and the nitrogen heteroatom is optionally quaternized. In other embodiments, the group of heteroatoms is selected from -C(=O)O-, - C(=O)-, -C(=S)-, -S(=O), -S(=O)₂-, -C(=O)N(H)-, -N(H)-, -C(=NH)-, -S(=O)₂N(H)-, and - S(=O)N(H)-. In some embodiments, the heteroalkyl is C₁₋₆ heteroalkyl; in other embodiments, the heteroalkyl is C₁₋₃ heteroalkyl. The heteroatom or group of heteroatoms may be located at any internal position within the heteroalkyl, including the position at which the alkyl is attached to the rest of the molecule, but the terms "alkoxy", "alkylamino", and "alkylthio" (or thioalkoxy) are customary expressions referring to those alkyl groups attached to the rest of the molecule via an oxygen, amino, or sulfur atom, respectively. Examples of heteroalkyl include, but are not limited to, -OCH₃, -OCH₂CH₃, -OCH₂CH₂CH₃, -OCH₂(CH₃)₂, -CH₂-CH₂-O-CH₃, -NHCH₃, -N(CH₃)₂, -NHCH₂CH₃, -N(CH₃)(CH₂CH₃), -CH₂-CH₂-NH-CH₃, -CH₂-CH₂-N(CH₃)-CH₃, -SCH₃, -SCH₂CH₃, -SCH₂CH₂CH₃, -SCH₂(CH₃)₂, -CH₂-S-CH₂-CH₃, -CH₂-CH₂, -S(=O)-CH₃, and -CH₂-CH₂-S(=O)₂-CH₃. Up to two heteroatoms may be consecutive, such as -CH₂-NH-OCH₃.

Unless otherwise specified, the term "C₁₋₃ alkoxy" refers to an alkyl group containing 1 to 3 carbon atoms that are connected to the rest of the molecule through an oxygen atom. The C₁₋₃ alkoxy includes C₁₋₂, C₂₋₃, C₃, and C₂ alkoxy, etc. Examples of C₁₋₃ alkoxy include, but are not limited to, methoxy, ethoxy, propoxy (including n-propoxy and isopropoxy), etc.

Unless otherwise specified, the term "C₁₋₄ alkoxy" refers to an alkyl group containing 1 to 4 carbon atoms that are connected to the rest of the molecule through an oxygen atom. The C₁₋₄ alkoxy includes C₁₋₃, C₁₋₂, C₂₋₄, C₄, and C₃ alkoxy, etc. Examples of C₁₋₄ alkoxy include, but are not limited to, methoxy, ethoxy, propoxy (including n-propoxy and isopropoxy), butoxy (including n-butoxy, isobutoxy, *s*-butoxy, and *t*-butoxy), etc.

Unless otherwise specified, the term "C₁₋₃ alkylamino" refers to an alkyl group containing 1 to 3 carbon atoms attached to the rest of the molecule through an amino group. The C₁₋₃ alkylamino includes C₁₋₂, C₃, and C₂ alkylamino, etc. Examples of C₁₋₃ alkylamino include, but are not limited to, -NHCH₃, -N(CH₃)₂, -NHCH₂CH₃, -N(CH₃)CH₂CH₃, - NHCH₂CH₂CH₃, -NHCH₂(CH₃)₂, etc.

Unless otherwise specified, the term "halo" or "halogen" by itself or as part of another substituent refers to fluorine, chlorine, bromine, or iodine atom.

Unless otherwise specified, the term "C₁₋₃ haloalkyl" refers to monohaloalkyl and polyhaloalkyl containing 1 to 3 carbon atoms. The C₁₋₃ haloalkyl includes C₁₋₂, C₂₋₃, C₃, C₂, and C₁ haloalkyl, etc. Examples of C₁₋₃ haloalkyl include, but are not limited to, trifluoromethyl, trichloromethyl, 2,2,2-trifluoroethyl, pentafluoroethyl, pentachloroethyl, 3-bromopropyl, etc.

Unless otherwise specified, "C₃₋₁₂ cycloalkyl" refers to a saturated cyclic hydrocarbon group consisting of 3 to 12 carbon atoms, including monocyclic, bicyclic, and tricyclic systems, wherein the bicyclic and tricyclic systems include spiro ring, fused ring, and bridged ring. The C₃₋₁₂ cycloalkyl includes C₃₋₁₀, C₃₋₈, C₃₋₆, C₃₋₅, C₄₋₁₀, C₄₋₈, C₄₋₆, C₄₋₅, C₅₋₈, and C₅₋₆ cycloalkyl, etc.; it may be monovalent, divalent, or multivalent. Examples of C₃₋₁₂ cycloalkyl include, but are not limited to, cyclopropyl, cyclobutyl, cyclopentyl, cyclohexyl, cycloheptyl, norbornyl, [2.2.2]dicyclooctyl, [4.4.0]bicyclodecyl, etc.

Unless otherwise specified, "C₃₋₆ cycloalkyl" refers to a saturated cyclic hydrocarbon group consisting of 3 to 6 carbon atoms, which is a monocyclic and bicyclic system, and the C₃₋₆ cycloalkyl includes C₃₋₅, C₄₋₅, and C₅₋₆ cycloalkyl, etc.; it can be monovalent, divalent, or multivalent. Examples of C₃₋₆ cycloalkyl include, but are not limited to, cyclopropyl, cyclobutyl, cyclopentyl, cyclohexyl, etc.

Unless otherwise specified, "C₄₋₆ cycloalkyl" refers to a saturated cyclic hydrocarbon group composing of 4 to 6 carbon atoms, which is a monocyclic and bicyclic system, and the C₄₋₆ cycloalkyl includes C₄₋₅ and C₅₋₆ cycloalkyl, etc.; it can be monovalent, divalent, or multivalent. Examples of C₄₋₆ cycloalkyl include, but are not limited to, cyclobutyl, cyclopentyl, cyclohexyl, etc.

Unless otherwise specified, "C₅₋₆ cycloalkyl" refers to a saturated cyclic hydrocarbon group consisting of 5 to 6 carbon atoms, which is a monocyclic and bicyclic system, and the C₅₋₆ cycloalkyl includes C₅ and C₆ cycloalkyl, etc.; it can be monovalent, divalent, or multivalent. Examples of C₅₋₆ cycloalkyl include, but are not limited to, cyclopentyl, cyclohexyl, etc.

Unless otherwise specified, the term "3- to 12-membered heterocycloalkyl" by itself or in combination with other terms refers to a saturated cyclic group consisting of 3 to 12 ring atoms, wherein 1, 2, 3, or 4 ring atoms are heteroatoms independently selected from O, S, and N, and the rest are carbon atoms, wherein nitrogen atoms are optionally quaternized, and carbon, nitrogen, and sulfur heteroatoms may be optionally oxidized (i.e., C(=O), NO, and S(O)ₚ, p is 1 or 2). The 3- to 12-membered heterocycloalkyl includes monocyclic, bicyclic, and tricyclic systems, wherein the bicyclic and tricyclic systems include a spiro ring, a fused ring, and a bridged ring. In addition, with regard to the "3- to 12-membered heterocycloalkyl", a heteroatom may occupy the connection position of the heterocycloalkyl with the rest of the molecule. The 3- to 12-membered heterocycloalkyl includes 3- to 10-membered, 3- to 8-membered, 3- to 6-membered, 3- to 5-membered, 4- to 6-membered, 5- to 6-membered, 4-membered, 5-membered, and 6-membered heterocycloalkyl, etc. Examples of 3- to 12-membered heterocycloalkyl include, but are not limited to, azetidinyl, oxetanyl, thietanyl, pyrrolidinyl, pyrazolidinyl, imidazolidinyl, tetrahydrothienyl (including tetrahydrothiophen-2-yl and tetrahydrothiophen-3-yl, etc.), tetrahydrofuranyl (including tetrahydrofuran-2-yl, etc.), tetrahydropyranyl, piperidinyl (including 1-piperidinyl, 2-piperidinyl, and 3-piperidinyl, etc.), piperazinyl (including 1-piperazinyl and 2-piperazinyl, etc.), morpholinyl (including 3-morpholinyl and 4-morpholinyl, etc.), dioxinyl, dithianyl, isoxazolidinyl, isothiazolidinyl, 1,2-oxazinyl, 1,2-thiazinyl, hexahydropyridazinyl, homopiperazinyl, homopiperidinyl, or dioxacycloheptyl, etc.

Unless otherwise specified, the term "5- to 10-membered heterocycloalkyl" by itself or in combination with other terms refers to a saturated cyclic group consisting of 5 to 10 ring atoms, wherein 1, 2, 3, or 4 ring atoms are heteroatoms independently selected from O, S, and N, and the rest are carbon atoms, wherein nitrogen atoms are optionally quaternized, and carbon, nitrogen, and sulfur heteroatoms may be optionally oxidized (i.e., C(=O), NO, and S(O)ₚ, p is 1 or 2). The 5- to 10-membered heterocycloalkyl includes monocyclic, bicyclic, and tricyclic systems, wherein the bicyclic and tricyclic systems include a spiro ring, a fused ring, and a bridged ring. In addition, with regard to the "5- to 10-membered heterocycloalkyl", a heteroatom may occupy the connection position of the heterocycloalkyl with the rest of the molecule. The 5- to 10-membered heterocycloalkyl includes 5- to 6-membered, 5- to 7-membered, 5- to 8-membered, 5- to 9-membered, 6- to 7-membered, 6- to 8-membered, 6- to 9-membered, 6- to 10-membered, 5-membered, 6-membered, 7-membered, 8-membered, 9-membered, and 10-membered heterocycloalkyl, etc. Examples of 5- to 10-membered heterocycloalkyl include, but are not limited to, pyrrolidinyl, pyrazolidinyl, imidazolidinyl, tetrahydrothienyl (including tetrahydrothiophen-2-yl and tetrahydrothiophen-3-yl, etc.), tetrahydrofuranyl (including tetrahydrofuran-2-yl, etc.), tetrahydropyranyl, piperidinyl (including 1-piperidinyl, 2-piperidinyl and 3-piperidinyl, etc.), piperazinyl (including 1-piperazinyl and 2-piperazinyl, etc.), morpholinyl (including 3-morpholinyl and 4-morpholinyl, etc.), dioxinyl, dithianyl, isoxazolidinyl, isothiazolidinyl, 1,2-oxazinyl, 1,2-thiazinyl, hexahydropyridazinyl, homopiperazinyl, homopiperidinyl, or dioxacycloheptyl, etc.

Unless otherwise specified, the term "3- to 6-membered heterocycloalkyl" by itself or in combination with other terms refers to a saturated cyclic group consisting of 3 to 6 ring atoms, wherein 1, 2, 3, or 4 ring atoms are heteroatoms independently selected from O, S, and N, and the rest are carbon atoms, wherein nitrogen atoms are optionally quaternized, and carbon, nitrogen, and sulfur heteroatoms may be optionally oxidized (i.e., C(=O), NO, and S(O)ₚ, p is 1 or 2). The 3- to 6-membered heterocycloalkyl includes monocyclic and bicyclic systems, wherein the bicyclic systems include a spiro ring, a fused ring, and a bridged ring. In addition, with regard to the "3- to 6-membered heterocycloalkyl", a heteroatom may occupy the connection position of the heterocycloalkyl with the rest of the molecule. The 3- to 6-membered heterocycloalkyl includes 4- to 6-membered, 5- to 6-membered, 4-membered, 5-membered, and 6-membered heterocycloalkyl, etc. Examples of 3- to 6-membered heterocycloalkyl include, but are not limited to, azetidinyl, oxetanyl, thietanyl, pyrrolidinyl, pyrazolidinyl, imidazolidinyl, tetrahydrothienyl (including tetrahydrothiophen-2-yl and tetrahydrothiophen-3-yl, etc.), tetrahydrofuranyl (including tetrahydrofuran-2-yl, etc.), tetrahydropyranyl, piperidinyl (including 1-piperidinyl, 2-piperidinyl, and 3-piperidinyl, etc.), piperazinyl (including 1-piperazinyl and 2-piperazinyl, etc.), morpholinyl (including 3-morpholinyl and 4-morpholinyl, etc.), dioxinyl, dithianyl, isoxazolidinyl, isothiazolidinyl, 1,2-oxazinyl, 1,2-thiazinyl, or hexahydropyridazinyl, etc.

Unless otherwise specified, the term "4- to 6-membered heterocycloalkyl" by itself or in combination with other terms refers to a saturated cyclic group consisting of 4 to 6 ring atoms, wherein 1, 2, 3, or 4 ring atoms are heteroatoms independently selected from O, S, and N, and the rest are carbon atoms, wherein nitrogen atoms are optionally quaternized, and carbon, nitrogen, and sulfur heteroatoms may be optionally oxidized (i.e., C(=O), NO, and S(=O)ₚ, p is 1 or 2). The 4- to 6-membered heterocycloalkyl includes monocyclic and bicyclic systems, wherein the bicyclic systems include a spiro ring, a fused ring, and a bridged ring. In addition, with regard to the "4- to 6-membered heterocycloalkyl", a heteroatom may occupy the connection position of the heterocycloalkyl with the rest of the molecule. The 4- to 6-membered heterocycloalkyl includes 4- to 5-membered, 5- to 6-membered, 4-membered, 5-membered, and 6-membered heterocycloalkyl, etc. Examples of 4- to 6-membered heterocycloalkyl include, but are not limited to, azetidinyl, oxetanyl, thietanyl, pyrrolidinyl, pyrazolidinyl, imidazolidinyl, tetrahydrothienyl (including tetrahydrothiophen-2-yl and tetrahydrothiophen-3-yl, etc.), tetrahydrofuranyl (including tetrahydrofuran-2-yl, etc.), tetrahydropyranyl, piperidinyl (including 1-piperidinyl, 2-piperidinyl, and 3-piperidinyl, etc.), piperazinyl (including 1-piperazinyl and 2-piperazinyl, etc.), morpholinyl (including 3-morpholinyl and 4-morpholinyl, etc.), dioxinyl, dithianyl, isoxazolidinyl, isothiazolidinyl, 1,2-oxazinyl, 1,2-thiazinyl, or hexahydropyridazinyl, etc.

Unless otherwise specified, the term "6-membered heterocycloalkyl" by itself or in combination with other terms refers to a saturated cyclic group consisting of 6 ring atoms, wherein 1, 2, 3, or 4 ring atoms are heteroatoms independently selected from O, S, and N, and the rest are carbon atoms, wherein nitrogen atoms are optionally quaternized, and carbon, nitrogen, and sulfur heteroatoms may be optionally oxidized (i.e., C(=O), NO, and S(=O)ₚ, p is 1 or 2). The 6-membered heterocycloalkyl includes monocyclic and bicyclic systems, wherein the bicyclic systems include a spiro ring, a fused ring, and a bridged ring. In addition, with regard to the "6-membered heterocycloalkyl", a heteroatom may occupy the connection position of the heterocycloalkyl with the rest of the molecule. Examples of 6-membered heterocycloalkyl include, but are not limited to, tetrahydropyranyl, piperidinyl (including 1-piperidinyl, 2-piperidinyl, and 3-piperidinyl, etc.), piperazinyl (including 1-piperazinyl and 2-piperazinyl, etc.), morpholinyl (including 3-morpholinyl and 4-morpholinyl, etc.), dioxinyl, dithianyl, isoxazolidinyl, isothiazolidinyl, 1,2-oxazinyl, 1,2-thiazinyl, or hexahydropyridazinyl, etc.

Unless otherwise specified, "C₃₋₁₂ cycloalkenyl" refers to a partially unsaturated cyclic hydrocarbon group consisting of 3 to 12 carbon atoms containing at least one carbon-carbon double bond, including monocyclic, bicyclic, and tricyclic systems, wherein the bicyclic and tricyclic systems include a spiro ring, a fused ring, and a bridged ring, and any ring in this system is non-aromatic. The C₃₋₁₂ cycloalkenyl includes C₃₋₁₀, C₃₋₈, C₃₋₆, and C₃₋₅ cycloalkenyl, etc.; it may be monovalent, divalent, or multivalent. Examples of C₃₋₁₂ cycloalkenyl include, but are not limited to, cyclopropenyl, cyclobutenyl, cyclopentenyl, cyclopentadienyl, cyclohexenyl, cyclohexadienyl, etc.

Unless otherwise specified, "C₃₋₆ cycloalkenyl" refers to a partially unsaturated cyclic hydrocarbon group consisting of 3 to 6 carbon atoms containing at least one carbon-carbon double bond, including monocyclic and bicyclic systems, wherein the bicyclic system includes a spiro ring, a fused ring, and a bridged ring, and any ring in this system is non-aromatic. The C₃₋₆ cycloalkenyl includes C₄₋₆, C₄₋₅, or C₅₋₆ cycloalkenyl, etc.; it may be monovalent, divalent, or multivalent. Examples of C₃₋₆ cycloalkenyl include, but are not limited to, cyclopropenyl, cyclobutenyl, cyclopentenyl, cyclopentadienyl, cyclohexenyl, cyclohexadienyl, etc.

Unless otherwise specified, "C₅₋₆ cycloalkenyl" refers to a partially unsaturated cyclic hydrocarbon group consisting of 5 to 6 carbon atoms containing at least one carbon-carbon double bond, including monocyclic and bicyclic systems, wherein the bicyclic system includes a spiro ring, a fused ring, and a bridged ring, and any ring in this system is non-aromatic. The C₅₋₆ cycloalkenyl includes C₅ or C₆ cycloalkenyl, etc.; it may be monovalent, divalent, or multivalent. Examples of C₅₋₆ cycloalkenyl include, but are not limited to, cyclopentenyl, cyclopentadienyl, cyclohexenyl, cyclohexadienyl, etc.

Unless otherwise specified, the term "3- to 12-membered heterocycloalkenyl" by itself or in combination with other terms refers to a partially unsaturated cyclic group consisting of 3 to 12 ring atoms containing at least one carbon-carbon double bond, wherein 1, 2, 3, or 4 ring atoms are heteroatoms independently selected from O, S, and N, and the rest are carbon atoms, wherein nitrogen atoms are optionally quaternized, and carbon, nitrogen, and sulfur heteroatoms may be optionally oxidized (i.e., C(=O), NO, and S(O)ₚ, p is 1 or 2). The 3- to 12-membered heterocycloalkenyl includes monocyclic, bicyclic, and tricyclic systems, wherein the bicyclic and tricyclic systems include a spiro ring, a fused ring, and a bridged ring, and any ring in this system is non-aromatic. In addition, with regard to the "3- to 12-membered heterocycloalkenyl", a heteroatom may occupy the connection position of the heterocycloalkenyl with the rest of the molecule. The 3- to 12-membered heterocycloalkenyl includes 3- to 10-membered, 3- to 8-membered, 3- to 6-membered, 3- to 5-membered, 4- to 6-membered, 4- to 5-membered, 5- to 6-membered, 4-membered, 5-membered, and 6-membered heterocycloalkenyl, etc. Examples of 3- to 12-membered heterocycloalkenyl include, but are not limited to,

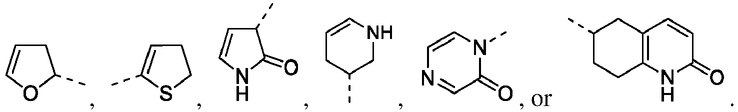

Unless otherwise specified, the term "5- to 6-membered heterocycloalkenyl" by itself or in combination with other terms refers to a partially unsaturated cyclic group consisting of 5 to 6 ring atoms containing at least one carbon-carbon double bond, wherein 1, 2, 3, or 4 ring atoms are heteroatoms independently selected from O, S, and N, and the rest are carbon atoms, wherein nitrogen atoms are optionally quaternized, and carbon, nitrogen, and sulfur heteroatoms may be optionally oxidized (i.e., C(=O), NO, and S(O)ₚ, p is 1 or 2). The 5- to 6-membered heterocycloalkenyl includes monocyclic and bicyclic systems, wherein the bicyclic system includes a spiro ring, a fused ring, and a bridged ring, and any ring in this system is non-aromatic. In addition, with regard to the "5- to 6-membered heterocycloalkenyl", a heteroatom may occupy the connection position of the heterocycloalkenyl with the rest of the molecule. The 5- to 6-membered heterocycloalkenyl includes 5-membered and 6-membered heterocycloalkenyl, etc. Examples of 5- to 6-membered heterocycloalkenyl include, but are not limited to,

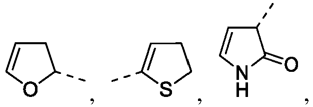

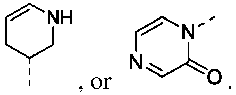

Unless otherwise specified, the terms "C₆₋₁₂ aromatic ring" and "C₆₋₁₂ aryl" in the present disclosure can be used interchangeably, and the term "C₆₋₁₂ aromatic ring" or "C₆₋₁₂ aryl" refers to a cyclic hydrocarbon group consisting of 6 to 12 carbon atoms with a conjugated π-electron system, which may be a monocyclic, fused bicyclic, or fused tricyclic system, in which each ring is aromatic. C₆₋₁₂ aryl may be monovalent, divalent, or multivalent, and the C₆₋₁₂ aryl includes C₆₋₁₀, C₆₋₉, C₆₋₈, C₁₂, C₁₀, and C₆ aryl, etc. Examples of C₆₋₁₂ aryl include, but are not limited to, phenyl, naphthyl (including 1-naphthyl and 2-naphthyl, etc.).

Unless otherwise specified, the terms "5- to 12-membered heteroaromatic ring" and "5- to 12-membered heteroaryl" in the present disclosure can be used interchangeably, and the term "5- to 12-membered heteroaryl" refers to a cyclic group consisting of 5 to 12 ring atoms with a conjugated π-electron system, where 1, 2, 3, or 4 ring atoms are heteroatoms independently selected from O, S, and N, and the rest are carbon atoms. The 5- to 12-membered heteroaryl may be a monocyclic, fused bicyclic, or fused tricyclic system in which each ring is aromatic, and in which the nitrogen atom is optionally quaternized, and the nitrogen and sulfur heteroatoms may be optionally oxidized (i.e., NO and S(O)ₚ, where p is 1 or 2). The 5- to 12-membered heteroaryl may be attached to the rest of the molecule through a heteroatom or a carbon atom. The 5- to 12-membered heteroaryl includes 5- to 10-membered, 5- to 8-membered, 5- to 7-membered, 5- to 6-membered, 5-membered, and 6-membered heteroaryl, etc. Examples of the 5- to 12-membered heteroaryl include, but are not limited to, pyrrolyl (including *N*-pyrrolyl, 2-pyrrolyl, and 3-pyrrolyl, etc.), pyrazolyl (including 2-pyrazolyl and 3-pyrazolyl, etc.), imidazolyl (including *N*-imidazolyl, 2-imidazolyl, 4-imidazolyl, and 5-imidazolyl, etc.), oxazolyl (including 2-oxazolyl, 4-oxazolyl, and 5-oxazolyl, etc.), triazolyl (1*H*-1,2,3-triazolyl, 2*H*-1,2,3-triazolyl, 1*H*-1,2,4-triazolyl, and 4*H*-1,2,4-triazolyl, etc.), tetrazolyl, isoxazolyl (3-isoxazolyl, 4-isoxazolyl, and 5-isoxazolyl, etc.), thiazolyl (including 2-thiazolyl, 4-thiazolyl, and 5-thiazolyl, etc.), furyl (including 2-furyl and 3-furyl, etc.), thienyl (including 2-thienyl and 3-thienyl, etc.), pyridyl (including 2-pyridyl, 3-pyridyl, and 4-pyridyl, etc.), pyrazinyl, pyrimidinyl (including 2-pyrimidinyl and 4-pyrimidinyl, etc.), benzothiazolyl (including 5-benzothiazolyl, etc.), purinyl, benzimidazolyl (including 2-benzimidazolyl, etc.), benzoxazolyl, indolyl (including 5-indolyl, etc.), isoquinolinyl (including 1-isoquinolyl and 5-isoquinolinyl, etc.), quinoxalinyl (including 2-quinoxalinyl and 5-quinoxalinyl, etc.), or quinolinyl (including 3-quinolinyl and 6-quinolinyl, etc.).

Unless otherwise specified, the terms "5- to 6-membered heteroaromatic ring" and "5-to 6-membered heteroaryl" in the present disclosure can be used interchangeably, and the term "5- to 6-membered heteroaryl" refers to a monocyclic group consisting of 5 to 6 ring atoms with a conjugated π-electron system, wherein 1, 2, 3, or 4 ring atoms are heteroatoms independently selected from O, S, and N, and the rest are carbon atoms. Herein, the nitrogen atom is optionally quaternized, the nitrogen and sulfur heteroatoms may be optionally oxidized (i.e., NO and S(O)ₚ, wherein p is 1 or 2). The 5- to 6-membered heteroaryl may be attached to the rest of the molecule through a heteroatom or a carbon atom. The 5- to 6-membered heteroaryl includes 5-membered and 6-membered heteroaryl. Examples of the 5- to 6-membered heteroaryl include, but are not limited to, pyrrolyl (including *N*-pyrrolyl, 2-pyrrolyl, and 3-pyrrolyl, etc.), pyrazolyl (including 2-pyrazolyl and 3-pyrazolyl, etc.), imidazolyl (including *N*-imidazolyl, 2-imidazolyl, 4-imidazolyl, and 5-imidazolyl, etc.), oxazolyl (including 2-oxazolyl, 4-oxazolyl, and 5-oxazolyl, etc.), triazolyl (1*H*-1,2,3-triazolyl, 2*H-*1,2,3-triazolyl, 1*H*-1,2,4-triazolyl, and 4*H*-1, 2,4-triazolyl, etc.), tetrazolyl, isoxazolyl (3-isoxazolyl, 4-isoxazolyl, and 5-isoxazolyl, etc.), thiazolyl (including 2-thiazolyl, 4-thiazolyl, and 5-thiazolyl, etc.), furyl (including 2-furyl and 3-furyl, etc.), thienyl (including 2-thienyl and 3-thienyl, etc.), pyridyl (including 2-pyridyl, 3-pyridyl, and 4-pyridyl, etc.), pyrazinyl, or pyrimidinyl (including 2-pyrimidinyl and 4-pyrimidinyl, etc.).

The term "leaving group" refers to a functional group or atom which can be substituted by another functional group or atom through a substitution reaction (such as nucleophilic substitution reaction). For example, representative leaving groups include triflate; chlorine, bromine, and iodine; sulfonate group, such as mesylate, tosylate, p-bromobenzenesulfonate, p-toluenesulfonate, etc.; acyloxy, such as acetoxy, trifluoroacetoxy, etc.

The term "protecting group" includes, but is not limited to, "amino protecting group", "hydroxyl protecting group", or "mercapto protecting group". The term "amino protecting group" refers to a protecting group suitable for preventing the side reactions occurring at the nitrogen of an amino. Representative amino protecting groups include, but are not limited to: formyl; acyl, such as alkanoyl (e.g., acetyl, trichloroacetyl, or trifluoroacetyl); alkoxycarbonyl, such as tert-butoxycarbonyl (Boc); arylmethoxycarbonyl such as benzyloxycarbonyl (Cbz) and 9-fluorenylmethoxycarbonyl (Fmoc); arylmethyl, such as benzyl (Bn), trityl (Tr), 1,1-bis-(4'-methoxyphenyl)methyl; silyl, such as trimethylsilyl (TMS) and tert-butyldimethylsilyl (TBS). The term "hydroxyl protecting group" refers to a protecting group suitable for preventing the side reactions of hydroxyl. Representative hydroxyl protecting groups include, but are not limited to: alkyl, such as methyl, ethyl, and tert-butyl; acyl, such as alkanoyl (e.g., acetyl); arylmethyl, such as benzyl (Bn), p-methoxybenzyl (PMB), 9-fluorenylmethyl (Fm), and diphenylmethyl (benzhydryl, DPM); silyl, such as trimethylsilyl (TMS) and tert-butyl dimethylsilyl (TBS).

The compounds of the present disclosure can be prepared by a variety of synthetic methods known to those skilled in the art, including the specific embodiments listed below, the embodiments formed by their combination with other chemical synthesis methods, and equivalent alternatives known to those skilled in the art, preferred embodiments include but are not limited to the examples of the present disclosure.

The structure of the compounds of the present disclosure can be confirmed by conventional methods known to those skilled in the art, and if the present disclosure involves an absolute configuration of a compound, then the absolute configuration can be confirmed by means of conventional techniques in the art. For example, in the case of single crystal X-ray diffraction (SXRD), the absolute configuration can be confirmed by collecting diffraction intensity data from the cultured single crystal using a Bruker D8 venture diffractometer with CuKα radiation as the light source and scanning mode: ϕ/ω scan, and after collecting the relevant data, the crystal structure can be further analyzed by direct method (Shelxs97), so that the absolute configuration can be confirmed.

The solvents used in the present disclosure are commercially available.

The following abbreviations are used in the present disclosure: Alloc stands for allyloxycarbonyl; SEM stands for trimethylsilylethoxymethyl; OTs stands for 4-toluenesulfonyl; Boc stands for tert-butoxycarbonyl; DCM stands for dichloromethane; DIEA stands for *N*,*N*-diisopropylethylamine; MeI stands for iodomethane; PE stands for petroleum ether; EA stands for ethyl acetate; THF stands for tetrahydrofuran; EtOH stands for ethanol; MeOH stands for methanol; Boc₂O stands for di-tert-butyl dicarbonate; NH₄Cl stands for ammonium chloride; T₃P stands for propylphosphonic anhydride; Pd/C stands for palladium/carbon catalyst; TMSN₃ stands for azidotrimethylsilane; NCS stands for N-chlorosuccinimide; HBr stands for hydrobromic acid; AcOH stands for acetic acid; HATU stands for *O*-(7-azabenzotriazol-1-yl)-*N*,*N*,*N*',*N*'-tetramethyluronium hexafluorophosphate; DBU stands for 1,8-diazabicyclo[5.4.0]undec-7-ene; FA stands for formic acid; ACN stands for acetonitrile; TLC stands for thin-layer chromatography; HPLC stands for high performance liquid chromatography; pre-HPLC stands for preparative high performance liquid chromatography; LCMS stands for liquid chromatography-mass chromatography. DMSO stands for dimethyl sulfoxide; DMSO-*d*₆ stands for deuterated dimethyl sulfoxide; CD₃OD stands for deuterated methanol; CDCl₃ stands for deuterated chloroform; D₂O stands for deuterium water.

The compounds of the present disclosure are named according to the conventional naming principles in the art or by ChemDraw^{®} software, and the commercially available compounds use the supplier catalog names.

### DETAILED DESCRIPTION OF THE PREFERRED EMBODIMENT

The present disclosure is described in detail by the examples below, but it does not mean that there are any adverse restrictions on the present disclosure. The compounds of the present disclosure can be prepared by a variety of synthetic methods known to those skilled in the art, including the specific embodiments listed below, the embodiments formed by their combination with other chemical synthesis methods, and equivalent alternatives known to those skilled in the art, preferred embodiments include but are not limited to the examples of the present disclosure. It will be apparent to those skilled in the art that various variations and improvements can be made to specific embodiments of the present disclosure without departing from the spirit and scope of the present disclosure.

### Intermediate A

### Synthetic Route:

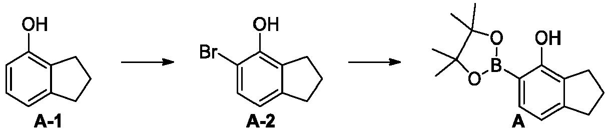

### Step 1

Intermediate **A-1** (1.50 g, 11.2 mmol) was dissolved in dichloromethane (30 mL), and *N*-bromosuccinimide (1.99 g, 11.2 mmol) was added thereto at 0°C, and the reaction mixture was stirred at 25°C for 12 hours. The reaction mixture was added with water (10 mL) and extracted with dichloromethane (20 mL × 3), and the organic phase was dried over anhydrous sodium sulfate, filtered, and concentrated under reduced pressure. The crude product was separated by silica gel column chromatography (petroleum ether/ethyl acetate, 100/1 to 5/1, V/V) to obtain intermediate **A-2**. ¹H NMR (400 MHz, CDCl₃) δ 7.23 (d, *J*=8.0 Hz, 1H), 6.71 (d, *J*=8.0 Hz, 1H), 5.46 (s, 1H), 2.95-2.87 (m, 4H), 2.15-2.08 (m, 2H).

### Step 2

Intermediate **A-2** (940 mg, 4.41 mmol) was dissolved in dioxane (10 mL), and bis(pinacolato)diboron (1.34 g, 5.29 mmol), [1,1'-bis(diphenylphosphino)ferrocene]dichloropalladium(II) (323 mg, 441 µmol), and potassium acetate (1.30 g, 13.2 mmol) were added thereto. The system was replaced with nitrogen three times, and the reaction mixture was stirred at 100°C for 12 hours. The reaction mixture was directly concentrated under reduced pressure, and the residue was separated by silica gel column chromatography (petroleum ether/ethyl acetate, 100/1 to 10/1, V/V) to obtain intermediate **A**. ¹H NMR (400 MHz, CDCl₃) δ 7.91 (s, 1H), 7.44 (d, *J*=7.6 Hz, 1H), 6.81 (d, *J*=7.6 Hz, 1H), 2.94-2.87 (m, 4H), 2.12-2.04 (m, 2H), 1.36 (s, 12H).

### Example 1

### Synthetic Route:

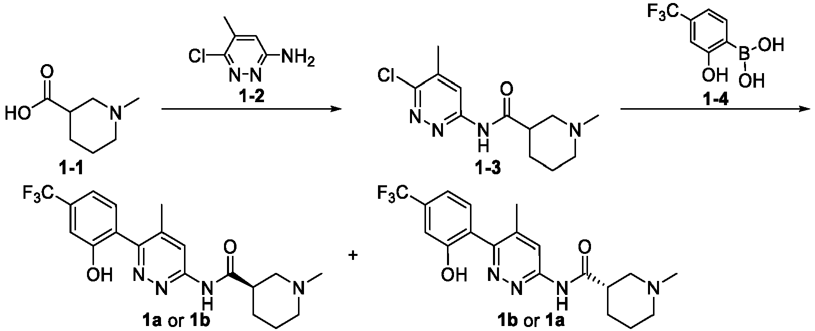

### Step 1

Compound **1-1** (350 mg, 2.44 mmol) and compound **1-2** (349 mg, 2.44 mmol) were dissolved in dichloromethane (2 mL), and propylphosphonic anhydride (4.65 g, 7.31 mmol, 50% ethyl acetate solution) and triethylamine (740 mg, 7.31 mmol) were added thereto. The reaction mixture was stirred at 20°C for 12 hours. The reaction mixture was diluted with ethyl acetate (50 mL), washed with water (10 mL), and the organic phase was dried over anhydrous sodium sulfate, filtered, and concentrated under reduced pressure. The crude product was separated by preparative high performance liquid chromatography (separation column: Welch Xtimate C18 150 × 25 mm × 5 µm; mobile phase: 10 mmol ammonium bicarbonate aqueous solution-acetonitrile; gradient: acetonitrile: 13% to 43%, 9 min) to obtain compound **1-3.** MS-ESI calculated for [M+H]⁺ 269, found 269.

### Step 2

Compound **1-3** (150 mg, 558 µmol) and compound **1-4** (149 mg, 725 µmol) were dissolved in 1,4-dioxane (4 mL) and water (1 mL), and then the reaction mixture was added with potassium carbonate (231 mg, 1.67 mmol) and [1,1'-bis(diphenylphosphino)ferrocene]dichloropalladium(II) (41 mg, 56 µmol). The reaction mixture was heated to 85°C and reacted for 10 hours under nitrogen atmosphere. The reaction mixture was concentrated under reduced pressure, and the crude product was separated by silica gel column chromatography (dichloromethane/methanol, 20/1 to 5/1, V/V) to obtain the crude product, which was separated by SFC (separation column: Phenomenex-Cellulose-2 250 mm × 30 mm × 10 µm; mobile phase: supercritical CO₂-a solution of 0.1% ammonia water in ethanol; gradient: ethanol: 30% to 30%) to obtain compound **1a** (the first peak) and compound **1b** (the second peak). The e.e. values were then measured by SFC (chromatographic column: Cellulose 2 150 mm × 4.6 mm × 5 µm; mobile phase: supercritical CO₂-a solution of 0.05% diethylamine in ethanol; gradient: a solution of 0.05% diethylamine in ethanol: 5% to 40 %).

Compound **1a**: e.e.% = 99.3%, RT = 3.879 min. ¹H NMR (400 MHz, CD₃OD) δ 8.38 (s, 1H), 7.44 (d, *J*=8.0 Hz, 1H), 7.27 (d, *J*=8.0 Hz, 1H), 7.21 (s, 1H), 2.96-2.90 (m, 1H), 2.82-2.73 (m, 2H), 2.46-2.38 (m, 1H), 2.36 (s, 3H), 2.28 (s, 3H), 2.25-2.19 (m, 1H), 2.01-1.93 (m, 1H), 1.87-1.81 (m, 1H), 1.74-1.59 (m, 2H). MS-ESI calculated for [M+H]⁺ 395, found 395.

Compound **1b**: e.e.% = 98.24%, RT = 4.687 min. ¹H NMR (400 MHz, CD₃OD) δ 8.38 (s, 1H), 7.44 (d, *J*=8.0 Hz, 1H), 7.26 (d, *J*=8.0 Hz, 1H), 7.21 (s, 1H), 2.94-2.90 (m, 1H), 2.82-2.71 (m, 2H), 2.46-2.38 (m, 1H), 2.35 (s, 3H), 2.27 (s, 3H), 2.25-2.19 (m, 1H), 2.01-1.93 (m, 1H), 1.87-1.81 (m, 1H), 1.74-1.59 (m, 2H). MS-ESI calculated for [M+H]⁺ 395, found 395.

### Example 2

### Synthetic Route:

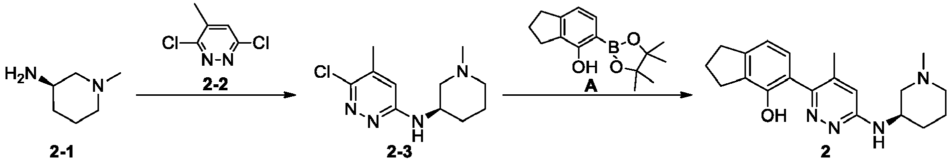

### Step 1

Compound **2-1** (504 mg, 4.42 mmol) and compound **2-2** (600 mg, 3.68 mmol) were dissolved in *N*-methylpyrrolidone (0.5 mL), and then *N,N*-diisopropylethylamine (1.92 mL, 11.04 mmol) was added thereto. The reaction mixture was heated to 180°C under microwave irradiation and reacted for 2 hours. The reaction mixture was concentrated under reduced pressure, diluted with ethyl acetate (50 mL × 2), washed with water (20 mL × 2), and the organic phase was dried over anhydrous sodium sulfate, filtered, and concentrated under reduced pressure. The crude product was separated by silica gel column chromatography (dichloromethane/methanol, 20/1 to 5/1, V/V) to obtain the crude product, which was separated by SFC (separation column: DAICEL CHIRALPAK AS 250 mm × 30 mm × 10 µm; mobile phase: supercritical CO₂-a solution of 0.1% ammonia water in ethanol; gradient: ethanol: 15% to 15%) to obtain compound **2-3**. ¹H NMR (400MHz, CDCl₃) δ 6.53 (s, 1H), 5.17 (s, 1H), 4.04 (s, 1H), 2.68-2.33 (m, 3H), 2.27 (s, 6H), 2.22-2.06 (m, 1H), 1.84-1.53 (m, 3H), 1.86-1.51 (m, 1H). MS-ESI calculated for [M+H]⁺ 241, found 241. MS-ESI calculated for [M+H]⁺ 241, found 241.

### Step 2

Compound **2-3** (77 mg, 320 µmol) and intermediate **A** (100 mg, 384 µmol) were dissolved in 1,4-dioxane (2 mL) and water (0.5 mL), and then the reaction mixture was added with potassium carbonate (132 mg, 960 mmol) and [1,1'-bis(diphenylphosphino)ferrocene]dichloropalladium(II) (23 mg, 32 µmol). The reaction mixture was heated to 90°C and reacted for 10 hours under nitrogen atmosphere. The reaction mixture was concentrated under reduced pressure, and the crude product was separated by silica gel column chromatography (dichloromethane/methanol, 20/1 to 5/1, V/V) to obtain the crude product of compound **2,** which was then separated by preparative high performance liquid chromatography (separation column: Phenomenex Gemini-NX 80 × 40 mm × 3 µm; mobile phase: 0.04% ammonia solution-acetonitrile; gradient: acetonitrile: 20% to 50%, 8 min) to obtain compound **2**. ¹H NMR (400 MHz, CDCl₃) δ 11.54 (s, 1H), 7.22 (d, *J*=8.0 Hz, 1H), 6.88-6.79 (m, 2H), 7.21 (s, 1H), 4.52 (s, 1H), 3.04-2.93 (m, 5H), 2.89-2.74 (m, 2H), 2.69-2.48 (m, 2H), 2.43 (s, 3H), 2.19-2.05 (m, 4H), 1.83-1.66 (m, 3H). MS-ESI calculated for [M+H]⁺ 339, found 339.

### Example 3

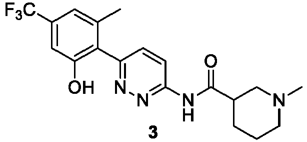

### Synthetic Route:

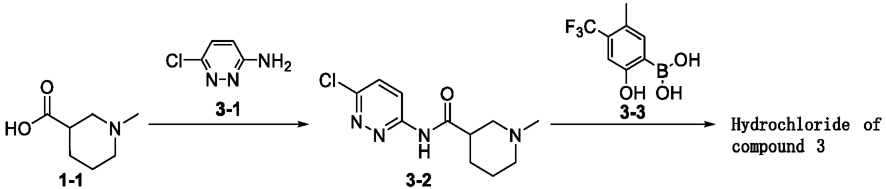

### Step 1

Compound **1-1** (500 mg, 3,86 mmol) and compound **3-1** (553 mg, 2.44 mmol) were dissolved in dichloromethane (2 mL), and propylphosphonic anhydride (7.37 g, 6.89 mmol, 50% ethyl acetate solution) and triethylamine (1.17 g, 11.58 mmol) were added thereto. The reaction mixture was stirred at 20°C for 12 hours. The reaction mixture was added with water (10 mL) and 1M NaOH aqueous solution (8 mL), extracted with ethyl acetate (30 mL × 3), and the organic phase was washed with saturated brine (10 mL), dried over anhydrous sodium sulfate, filtered, and concentrated under reduced pressure. The crude product was subjected to preparative high performance liquid chromatography (separation column: Welch Xtimate C18 150 × 25 mm × 5 µm; mobile phase: 10 mmol ammonium bicarbonate aqueous solution-acetonitrile; gradient: acetonitrile: 16% to 46%, 9 min) to obtain compound **3-2.** MS-ESI calculated for [M+H]⁺ 255, found 255.

### Step 2

Compound **3-2** (50 mg, 196 µmol) and compound **3-3** (48 mg, 216 µmol) were dissolved in 1,4-dioxane (2 mL) and water (0.4 mL), and then the reaction mixture was added with potassium carbonate (81 mg, 588 µmol) and [1,1'-bis(diphenylphosphino)ferrocene]dichloropalladium(II) (14 mg, 20 µmol). The reaction mixture was heated to 85°C and reacted for 10 hours under nitrogen atmosphere. The reaction mixture was concentrated under reduced pressure, and the crude product was separated by silica gel column chromatography (dichloromethane/methanol, 20/1 to 5/1, V/V) to obtain the crude product, which was separated by preparative high performance liquid chromatography (separation column: Phenomenex Synergi C18 150 × 30 mm × 4 µm; mobile phase: 0.05% hydrochloric acid aqueous solution-acetonitrile; gradient: acetonitrile: 22% to 52%, 10 min) to obtain the hydrochloride of compound **3**. ¹H NMR (400 MHz, CD₃OD) δ 8.97-8.85 (m, 1H), 8.31-8.15 (m, 1H), 7.20 (s, 1H), 7.11 (s, 1H), 3.86-3.68 (m, 1H), 3.61-3.42 (m, 1H), 3.35 (s, 1H), 3.28-3.17 (m, 2H), 3.10-2.98 (m, 1H), 2.94 (d, *J*=2.0 Hz, 3H), 2.26 (d, *J*=3.0 Hz, 4H), 2.15-1.94 (m, 2H), 1.92-1.68 (m, 1H). MS-ESI calculated for [M+H]⁺ 395, found 395.

### Example 4

### Synthetic Route:

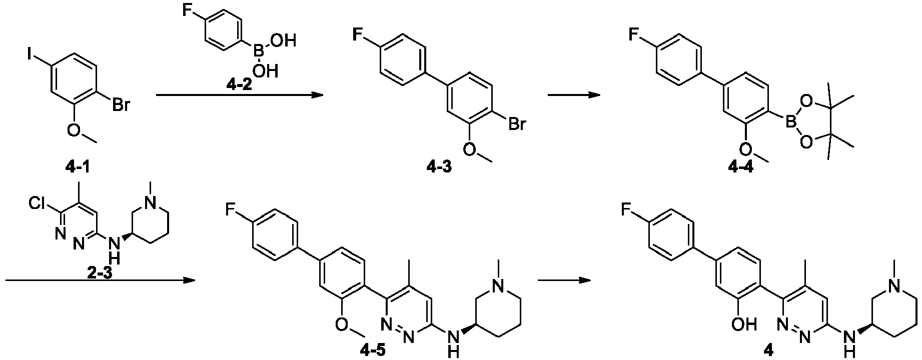

### Step 1

Compound **4-1** (2.46 g, 7.86 mmol), compound **4-2** (1.00 g, 7.15 mmol), tetrakis(triphenylphosphine)palladium (826 mg, 715 µmol), and potassium carbonate (2.96 g, 21.4 mmol) were dissolved in water (10 mL) and dioxane (30 mL), and the reaction mixture was stirred at 90°C for 12 hours under nitrogen atmosphere. The reaction mixture was concentrated under reduced pressure, and the crude product was purified by silica gel column chromatography (petroleum ether/ethyl acetate = 20/1 to 5/1) to obtain compound **4-3**. ¹H NMR (400MHz, CD₃OD) 7.67-7.63 (m, 2H), 7.59-7.57 (m, 1H), 7.20-7.15 (m, 3H), 7.09-7.06 (m, 1H), 3.95 (s, 3H).

### Step 2

Compound **4-3** (50.0 mg, 178 µmol), bis(pinacolato)diboron (90.3 mg, 356 µmol), potassium acetate (52.4 mg, 534 µmol), and [1,1'-bis(diphenylphosphino)ferrocene]dichloropalladium(II) dichloromethane complex (14.5 mg, 17.8 µmol) were dissolved in dioxane (3 mL), and the reaction mixture was stirred at 100°C for 16 hours under nitrogen atmosphere. The reaction mixture was concentrated under reduced pressure to obtain compound **4-4.**

### Step 3

Compound **4-4** (60.0 mg, 183 µmol), compound **2-3** (35.8 mg, 146 µmol), [1,1'-bis(diphenylphosphino)ferrocene]dichloropalladium(II) (13.4 mg, 18.3 µmol), and potassium carbonate (75.8 mg, 549 µmol) were dissolved in water (0.5 mL) and dioxane (3 mL), and the reaction mixture was stirred at 90°C for 12 hours under nitrogen atmosphere. The reaction mixture was concentrated under reduced pressure and the crude product was purified by thin-layer chromatography (dichloromethane/methanol, 5/1, V/V) to obtain compound **4-5.** MS-ESI calculated for [M+H]⁺ 407, found 407.

### Step 4

Compound **4-5** (53.0 mg, 109 µmol) was dissolved in anhydrous dichloromethane (5 mL), and the reaction mixture was cooled to 0°C, and then boron tribromide (136 mg, 542 µmol) was added to the reaction mixture. The reaction mixture was warmed to 20°C and stirred for 12 hours. The reaction mixture was quenched with methanol (5 mL) and concentrated under reduced pressure, then ammonia water (1 mL) was added thereto, and the reaction mixture was concentrated under reduced pressure. The crude product was separated by preparative high performance liquid chromatography (chromatographic column: Phenomenex Gemini-NX C18 75 × 30 mm × 3 µm; mobile phase: 10 mmol/L ammonium bicarbonate aqueous solution-acetonitrile; gradient: acetonitrile: 29% to 59%, 6.5 min) to obtain compound **4**. ¹H NMR (400MHz, CD₃OD) 7.68-7.65 (m, 2H), 7.32-7.30 (m, 1H), 7.22-7.18 (m, 3H), 7.16-7.14 (m, 1H), 6.79 (s, 1H), 4.18-4.11 (m, 1H), 3.11-3.07 (m, 1H), 2.74-2.72 (m, 1H), 2.33 (s, 3H), 2.28 (m, 1H), 2.21 (m, 3H), 2.02 (m, 2H), 1.87-1.82 (m, 1H), 1.77-1.69 (m, 1H), 1.46-1.40 (m, 1H). MS-ESI calculated for [M+H]⁺ 393, found 393.

### Example 5

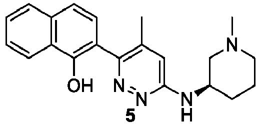

### Synthetic Route:

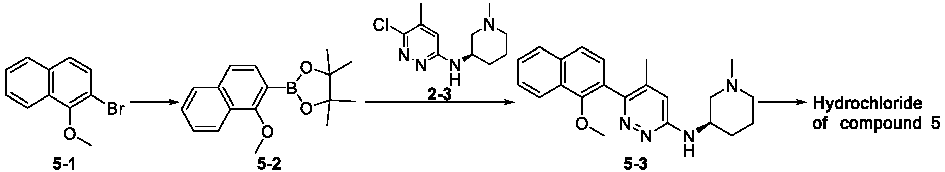

### Step 1

Compound **5-1** (50.0 mg, 211 µmol), [1,1'-bis(diphenylphosphino)ferrocene]dichloropalladium(II) dichloromethane complex (17.2 mg, 21.1 µmol), bis(pinacolato)diboron (107 mg, 422 µmol), and potassium acetate (41.4 mg, 422 µmol) were dissolved in dioxane (3 mL), and the reaction mixture was stirred at 90°C for 12 hours under nitrogen atmosphere. The reaction mixture was concentrated under reduced pressure to obtain compound **5-2,** which was directly used in the next step without purification.

### Step 2

Compound **5-2** (60.0 mg, 211 µmol), compound **2-3** (41.4 mg, 169 µmol), [1,1'-bis(diphenylphosphino)ferrocene]dichloropalladium(II) (15.5 mg, 21.1 µmol), and potassium carbonate (87.6 mg, 634 µmol) were dissolved in water (0.5 mL) and dioxane (3 mL), and the reaction mixture was stirred at 90°C for 12 hours under nitrogen atmosphere. The reaction mixture was concentrated under reduced pressure, and the crude product was purified by thin-layer chromatography (dichloromethane/methanol, 5/1, V/V) to obtain compound **5-3.** MS-ESI calculated for [M+H]⁺ 363, found 363.

### Step 3

Compound **5-3** (32.0 mg, 71.5 µmol) was dissolved in dry dichloromethane (5 mL), and the reaction mixture was cooled to 0°C in an ice-water bath, and then boron tribromide (89.5 mg, 357 µmol) was slowly added to the reaction mixture. The reaction mixture was warmed to 20°C, and stirred at 20°C for 12 hours. The reaction mixture was quenched with methanol (5 mL) and concentrated under reduced pressure, then ammonia water (1 mL) was added thereto, and the reaction mixture was concentrated under reduced pressure. The crude product was separated by preparative high performance liquid chromatography (chromatographic column: Phenomenex Luna C18 75 × 30 mm × 3 µm; 0.05% hydrochloric acid aqueous solution-acetonitrile; gradient: acetonitrile: 8% to 28%, 6 min) to obtain the hydrochloride of compound **5**. ¹H NMR (400MHz, CD₃OD) δ 8.35-8.33 (m, 1H), 7.98-7.96 (m, 1H), 7.71-7.59 (m, 4H), 7.41-7.38 (m, 1H), 4.41-4.32 (m, 1H), 3.90-3.84 (m, 1H), 3.60-3.56 (m, 1H), 3.17-3.03 (m, 1H), 3.03-2.96 (m, 3H), 2.93-2.92 (m, 1H), 2.38-2.37 (m, 3H), 2.31-2.28 (m, 1H), 2.18-2.02 (m, 2H), 1.76-1.66 (m, 1H). MS-ESI calculated for [M+H]⁺ 349, found 349.

### Example 6

### Synthetic Route:

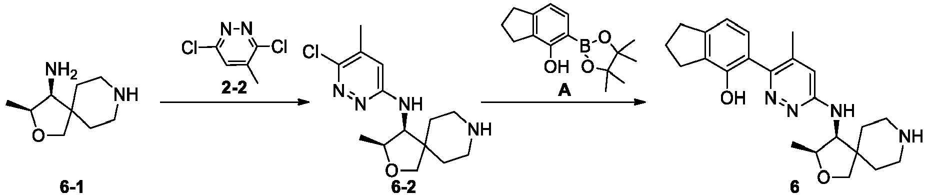

### Step 1

Compound **6-1** (300 mg, 1.76 mmol) was dissolved in N-methylpyrrolidone (3 mL), and compound **2-2** (345 mg, 2.11 mmol) and *N*,*N*-diisopropylethylamine (683 mg, 5.29 mmol) were added thereto. The reaction mixture was stirred under microwave irradiation at 180°C for 2 hours, added with water (10 mL), and extracted with ethyl acetate (20 mL × 4). The organic phases were combined, washed with saturated brine (20 mL × 3), dried over anhydrous sodium sulfate, filtered, and concentrated under reduced pressure. The crude product was separated by silica gel column chromatography (dichloromethane/methanol, 10/1 to 5/1, V/V) to obtain the crude product, which was separated by SFC (separation column: DAICEL CHIRALPAK AS 250 mm × 30 mm × 10 µm; mobile phase: supercritical CO₂-0.1% ammonia solution-ethanol; gradient: a solution of 0.1% ammonia water in ethanol: 20% to 20%) to obtain compound **6-2.** MS-ESI calculated for [M+H]⁺ 297, found 297.

### Step 2

Compound **6-2** (75.0 mg, 253 µmol) was dissolved in dioxane (3 mL) and water (0.6 mL), and intermediate **A** (78.9 mg, 303 µmol), [1,1'-bis(diphenylphosphino)ferrocene]dichloropalladium(II) (18.5 mg, 25.3 µmol), and potassium carbonate (87.3 mg, 632 µmol) were added thereto. The reaction mixture was stirred at 100°C for 12 hours under nitrogen atmosphere. The reaction mixture was concentrated under reduced pressure, separated by silica gel column chromatography (dichloromethane/methanol, 100/0 to 10/1, V/V) to obtain the crude product, which was separated by SFC (separation column: DAICEL CHIRALPAK AD 250 mm × 30 mm × 10 µm; mobile phase: supercritical CO₂-a solution of 0.1% ammonia water in ethanol; gradient: a solution of 0.1% ammonia water in ethanol: 55% to 55%) to obtain compound **6.** The e.e. values were then measured by SFC (chromatographic column: Chiralpak AS-3 250 mm × 30 mm × 10 µm; mobile phase: supercritical CO₂-a solution of 0.05% diethylamine in ethanol; gradient: a solution of 0.05% diethylamine in ethanol: 5% to 40 %). e.e.%=100%, RT=2.417 min, ¹H NMR (400 MHz, CD₃OD) δ 7.19 (s, 1H), 7.04 (d, *J*=7.6 Hz, 1H), 6.86 (d, *J*=7.6 Hz, 1H), 4.30-4.24 (m, 1H), 4.09-4.03 (m, 2H), 3.90 (d, *J*=8.8 Hz, 1H), 3.76 (d, *J*=8.8 Hz, 1H), 3.43-3.37 (m, 1H), 3.05 (d, *J*=4.4 Hz, 1H), 2.98-2.90 (m, 4H), 2.24 (s, 3H), 2.18-2.10 (m, 2H), 1.91-1.80 (m, 2H), 1.75-1.68 (m, 2H), 1.24 (d, *J*=6.4 Hz, 3H). MS-ESI calculated for [M+H]⁺ 395, found 395.

### Example 7

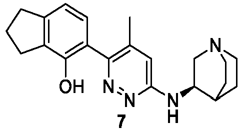

### Synthetic Route:

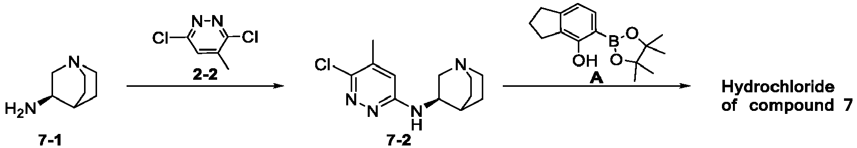

### Step 1

Compound **7-1** (100 mg, 1.19 mmol) was dissolved in 1,4-dioxane (8 mL), and then the reaction mixture was added with compound **2-2** (194 mg, 1.19 mmol), tris(dibenzylideneacetone)dipalladium(0) (145 mg, 158 µmol), 4,5-bis(diphenylphosphino)-9,9-dimethylxanthene (183 mg, 317 mmol), and cesium carbonate (645 mg, 1.98 mmol). The reaction mixture was heated to 100°C and reacted for 12 hours under nitrogen atmosphere. The reaction mixture was concentrated under reduced pressure, and the crude product was separated by preparative high performance liquid chromatography (separation column: Phenomenex Genimi NX C18 150 × 40 mm × 5 µm; mobile phase: 0.05% hydrochloric acid aqueous solution-acetonitrile; gradient: acetonitrile: 1% to 25%, 10 min) to obtain the hydrochloride of compound **7-2.** MS-ESI calculated for [M+H]⁺ 253, found 253.

### Step 2

The hydrochloride of compound **7-2** (100 mg, 396 µmol) and intermediate **A** (206 mg, 791 µmol) were dissolved in 1,4-dioxane (4 mL) and water (0.8 mL), and then the reaction mixture was added with [1,1'-bis(diphenylphosphino)ferrocene]dichloropalladium(II) (29 mg, 40 µmol) and potassium carbonate (191 mg, 1.38 mmol). The reaction mixture was heated to 100°C and reacted for 12 hours under nitrogen atmosphere. The reaction mixture was concentrated under reduced pressure, and the crude product was separated by preparative high performance liquid chromatography (separation column: Phenomenex Genimi NX C18 150 × 40 mm × 5 µm; mobile phase: 0.05% hydrochloric acid aqueous solution-acetonitrile; gradient: acetonitrile: 1% to 25%, 10 min) to obtain the hydrochloride of compound **7**. ¹H NMR (400 MHz, CD₃OD) δ 7.45 (s, 1H), 7.09 (d, *J*=7.8 Hz, 1H), 6.95 (d, *J*=7.8 Hz, 1H), 4.46-4.32 (m, 1H), 3.92-3.87 (m, 1H), 3.54-3.33 (m, 4H), 3.27 (d, *J*=3.8 Hz, 1H), 3.00-2.97 (m, 2H), 32.94-2.90 (m, 2H), 2.46-2.45 (m, 1H), 2.39-2.32 (m, 1H), 2.30 (s, 3H), 2.19-2.10 (m, 4H), 2.05-1.86 (m, 1H). MS-ESI calculated for [M+H]⁺ 351, found 351.

### Example 8

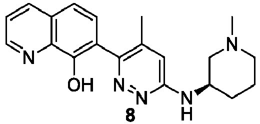

### Synthetic Route:

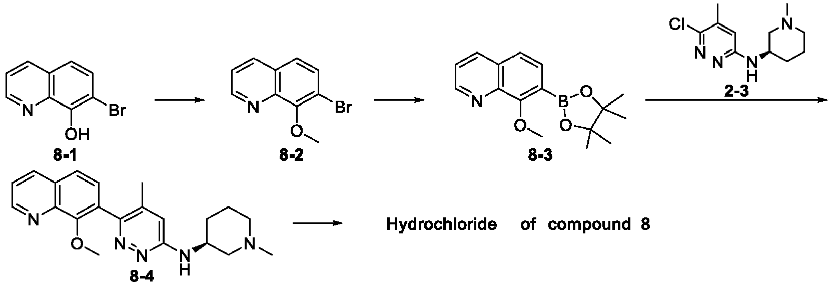

### Step 1

Compound **8-1** (1.00 g, 4.46 mmol) and iodomethane (1.27 g, 8.93 mmol) were dissolved in acetonitrile (10 mL), and the reaction mixture was added with potassium carbonate (1.85 g, 13.4 mmol), stirred at 40°C for 12 hours. The reaction mixture was quenched with saturated sodium bicarbonate solution (10 mL). The reaction mixture was concentrated under reduced pressure and the crude product was purified by silica gel column chromatography (dichloromethane/methanol, 50/1, V/V) to obtain compound **8-2.** MS-ESI calculated for [M+H]⁺ 240, found 240.

### Step 2

Compound **8-2** (500 mg, 2.10 mmol), potassium acetate (412 mg, 4.20 mmol), bis(pinacolato)diboron (1.07 g, 4.20 mmol), and [1,1'-bis(diphenylphosphino)ferrocene]dichloropalladium(II) dichloromethane complex (172 mg, 210 µmol) were dissolved in dioxane (10 mL), and the reaction mixture was stirred at 90°C for 3 hours under nitrogen atmosphere. The reaction mixture was concentrated under reduced pressure, and the crude product was purified by silica gel column chromatography (petroleum ether/ethyl acetate, 100/0 to 20/1, V/V) to obtain compound **8-3**. ¹H NMR (400MHz, CD₃OD) δ 8.90-8.80 (m, 1H), 8.37-8.34 (m, 1H), 7.80-7.78 (m, 1H), 7.68-7.66 (m, 1H), 7.60-7.57 (m, 1H), 3.08 (s, 3H), 1.43 (s, 12H). MS-ESI calculated for [M+H]⁺ 286, found 286.

### Step 3

Compound **8-3** (50.0 mg, 175 µmol), compound **2-3** (46.4 mg, 193 µmol), tetrakis(triphenylphosphine)palladium (20.3 mg, 17.5 µmol), and cesium carbonate (171 mg, 526 µmol) were dissolved in water (0.3 mL) and dioxane (3 mL), and the reaction mixture was stirred at 90°C for 12 hours under nitrogen atmosphere. The reaction mixture was concentrated under reduced pressure and the crude product was purified by thin-layer chromatography (dichloromethane/methanol, 5/1, V/V) to obtain compound **8-4.** MS-ESI calculated for [M+H]⁺ 364, found 364.

### Step 4

Compound **8-4** (70.0 mg, 155 µmol) was dissolved in anhydrous dichloromethane (5 mL), and the reaction mixture was cooled to 0°C in an ice-water bath, and then boron tribromide (194 mg, 774 µmol) was slowly added to the reaction mixture. The reaction mixture was stirred at 20°C for 12 hours. The reaction mixture was quenched with methanol (5 mL), concentrated under reduced pressure, then added with ammonia water (1 mL), and concentrated under reduced pressure. The crude product was separated by preparative high performance liquid chromatography (chromatographic column: Phenomenex Luna C18 75 × 30 mm × 3 µm; 0.05% hydrochloric acid aqueous solution-acetonitrile; gradient: acetonitrile: 0% to 15%, 6 min) to obtain the hydrochloride of compound **8**. ¹H NMR (400MHz, CD₃OD) δ 9.14-9.13 (m, 1H), 9.05-9.03 (m, 1H), 8.10-8.07 (m, 1H), 7.92-7.89 (m, 1H), 7.80-7.76 (m, 1H), 7.73-7.66 (m, 1H), 4.45-4.33 (m, 1H), 3.86-3.83 (m, 1H), 3.59-3.56 (m, 1H), 3.41-3.35 (m, 1H), 3.16-3.05 (m, 1H), 2.98-2.93 (m, 3H), 2.36 (s, 3H), 2.34-2.29 (m, 1H), 2.16-2.06 (m, 2H), 1.99-1.68 (m, 1H). MS-ESI calculated for [M+H]⁺ 350, found 350.

### Example 9

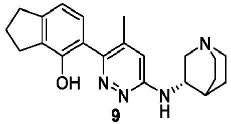

### Synthetic Route:

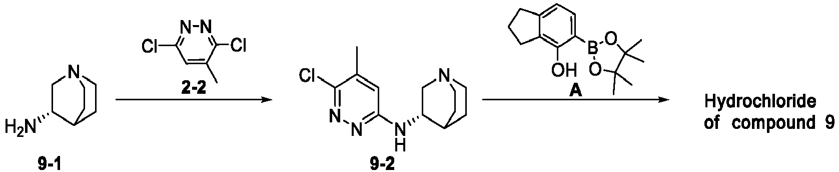

### Step 1

Compound **9-1** (200 mg, 1.58 mmol) was dissolved in 1,4-dioxane (8 mL), and then the reaction mixture was added with compound **2-2** (387 mg, 2.38 mmol), tris(dibenzylideneacetone)dipalladium(0) (290 mg, 317 µmol), 4,5-bis(diphenylphosphino)-9,9-dimethylxanthene (367 mg, 634 mmol), and cesium carbonate (1.29 g, 3.96 mmol). The reaction mixture was heated to 100°C and reacted for 12 hours under nitrogen atmosphere. The reaction mixture was concentrated under reduced pressure, and the crude product was separated by preparative high performance liquid chromatography (separation column: Phenomenex Genimi NX C18 150 × 40 mm × 5 µm; mobile phase: 0.05% hydrochloric acid aqueous solution-acetonitrile; gradient: acetonitrile: 1% to 25%, 10 min) to obtain the hydrochloride of compound **9-2.** MS-ESI calculated for [M+H]⁺ 253, found 253.

### Step 2

The hydrochloride of compound **9-2** (100 mg, 396 µmol) and intermediate **A** (206 mg, 791 µmol) were dissolved in 1,4-dioxane (4 mL) and water (0.8 mL), and then the reaction mixture was added with [1,1'-bis(diphenylphosphino)ferrocene]dichloropalladium(II) (29 mg, 40 µmol) and potassium carbonate (191 mg, 1.38 mmol). The reaction mixture was heated to 100°C and reacted for 12 hours under nitrogen atmosphere. The reaction mixture was concentrated under reduced pressure, and the crude product was separated by preparative high performance liquid chromatography (separation column: Phenomenex Genimi NX C18 150 × 40 mm × 5 µm; mobile phase: 0.05% hydrochloric acid aqueous solution-acetonitrile; gradient: acetonitrile: 1% to 25%, 10 min) to obtain the hydrochloride of compound **9**. ¹H NMR (400 MHz, CD₃OD) δ 7.63 (s, 1H), 7.12 (d, *J*=7.6 Hz, 1H), 6.98 (d, *J*=7.6 Hz, 1H), 4.39 (s, 1H), 3.92-3.86 (m, 1H), 3.55-3.36 (m, 4H), 3.27 (d, *J*=3.8 Hz, 1H), 3.01-2.98 (m, 2H), 2.95-2.91 (m, 2H), 2.46 (s, 1H), 2.34 (s, 4H), 2.23-2.06 (m, 4H), 1.99 (s, 1H). MS-ESI calculated for [M+H]⁺ 351, found 351.

### Example 10

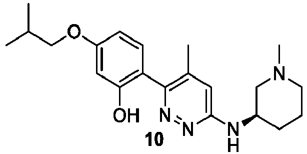

### Synthetic Route:

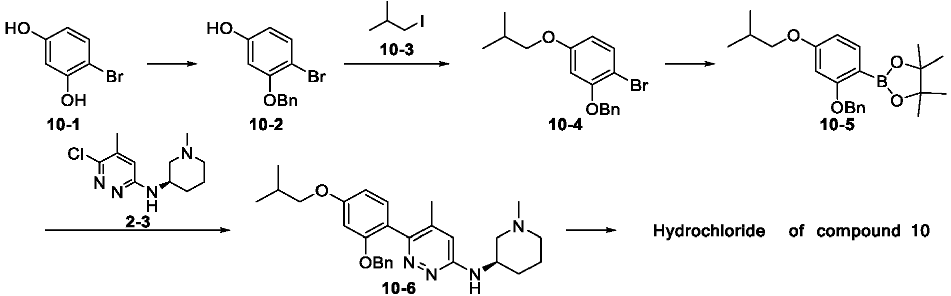

### Step 1

Compound **10-1** (3.00 g, 15.9 mmol) was dissolved in *N*,*N*-dimethylformamide (30 mL), cooled to 0°C in an ice-water bath, and the reaction mixture was added with sodium hydride (1.14 g, 28.6 mmol, purity: 60%), stirred for one hour, then added with benzyl bromide (3.26 g, 19.1 mmol), and stirred at 20°C for 12 hours. The reaction mixture was quenched with saturated ammonium chloride (20 mL), extracted with ethyl acetate (20 mL × 3), and the organic phase was washed with hydrochloric acid solution (1 mol/L, 20 mL × 2) and saturated brine (20 mL × 2), dried over anhydrous sodium sulfate, and filtered. The filtrate was concentrated under reduced pressure, and the crude product was purified by thin-layer chromatography (petroleum ether/dichloromethane, 0/1, V/V) to obtain compound **10-2**. ¹H NMR (400MHz, DMSO-*d*₆) δ 9.72 (m, 1H), 7.48-7.40 (m, 4H), 7.36-7.31 (m, 2H), 6.59-6.58 (m, 1H), 6.36-6.33 (m, 1H), 5.14 (s, 2H). MS-ESI calculated for [M+H]⁺ 280, found 280.

### Step 2

Compound **10-2** (433 mg, 1.45 mmol) was dissolved in acetonitrile (10 mL), and the reaction mixture was added with cesium carbonate (1.42 g, 4.36 mmol) and compound **10-3** (535 mg, 2.91 mmol), stirred at 75°C for 12 hours. The reaction mixture was concentrated under reduced pressure, and the crude product was purified by column chromatography (petroleum ether/ethyl acetate, 20/1 to 10/1, V/V) to obtain compound **10-4.** MS-ESI calculated for [M+H]⁺ 336, found 336.

### Step 3

Compound **10-4** (330 mg, 984 µmol), 2-dicyclohexylphosphino-2',6'-dimethoxybiphenyl (80.8 mg, 197 µmol), and tris(dibenzylideneacetone)dipalladium (90.1 mg, 98.4 µmol) were dissolved in dioxane (5 mL). The reaction mixture was added with triethylamine (299 mg, 2.95 mmol) and pinacolborane (252 mg, 1.97 mmol), stirred at 75°C for 12 hours under nitrogen atmosphere. The reaction mixture was concentrated under reduced pressure to obtain compound **10-5.** MS-ESI calculated for [M+H]⁺ 383, found 383.

### Step 4

Compound **10-5** (300 mg, 784 µmol), compound **2-3** (208 mg, 863 µmol), sodium bicarbonate (198 mg, 2.35 mmol), and tetrakis(triphenylphosphine)palladium (90.7 mg, 78.5 µmol) were dissolved in water (0.3 mL) and dioxane (3 mL), and the reaction mixture was stirred at 104°C for 12 hours under nitrogen atmosphere. The reaction mixture was concentrated under reduced pressure and the crude product was purified by thin-layer chromatography (dichloromethane/methanol, 5/1, V/V) to obtain compound **10-6.** MS-ESI calculated for [M+H]⁺ 461, found 461.

### Step 5

Compound **10-6** (25 mg, 54.3 µmol) was dissolved in ethyl acetate (5 mL), and the reaction mixture was added with palladium/carbon (54.3 µmol, purity: 10%) and acetic acid (326 µg, 5.43 µmol), replaced with hydrogen three times, and the reaction mixture was stirred at 90°C and a pressure of 15 Psi for 12 hours. The reaction mixture was filtered, and the filtrate was concentrated under reduced pressure. The crude product was separated by preparative high performance liquid chromatography (chromatographic column: Phenomenex Luna C18 75 × 30 mm × 3 µm; 0.05% hydrochloric acid aqueous solution-acetonitrile; gradient: acetonitrile: 13% to 33%, 6 min) to obtain the hydrochloride of compound **10**. ¹H NMR (400MHz, CD₃OD) δ 7.65-7.49 (m, 1H), 7.31-7.24 (m, 1H), 6.67 (dd, *J*=2.2, 8.6 Hz, 1H), 6.59 (d, *J*=2.2 Hz, 1H), 4.41-4.21 (m, 1H), 3.86-3.78 (m, 3H), 3.56 (d, *J*=6.4 Hz, 1H), 3.17-3.00 (m, 1H), 3.21-2.78 (m, 4H), 2.39-2.35 (m, 3H), 2.32-2.20 (m, 1H), 2.17-1.96 (m, 3H), 1.73-1.63 (m, 1H), 1.07 (d, J=6.4 Hz, 6H). MS-ESI calculated for [M+H]⁺ 371, found 371.

### Example 11

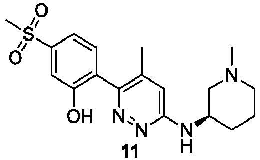

### Synthetic Route:

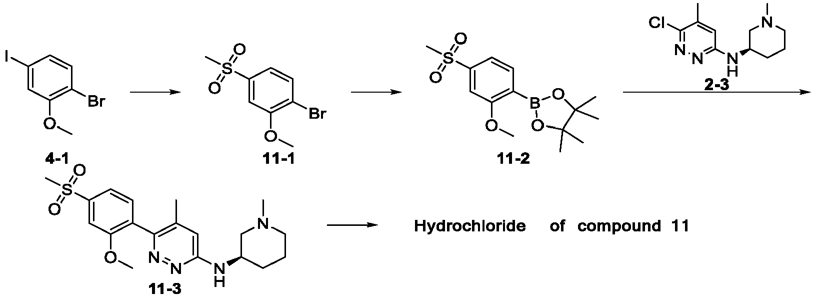

### Step 1

Compound **4-1** (1.5 g, 4.79 mmol) was dissolved in dimethyl sulfoxide (8 mL), and then the reaction mixture was added with potassium metabisulfite (2.13 g, 9.59 mmol), tetrabutylammonium bromide (1.70 g, 5.27 mmol), palladium acetate (54 mg, 240 µmol), triphenylphosphine (189 mg, 719 µmol), o-phenanthroline (129 mg, 719 µmol), and sodium formate (569 µL, 11 mmol). The reaction mixture was replaced with nitrogen for 10 minutes, and then heated to 70°C and reacted for 3 hours under nitrogen atmosphere. Then the reaction mixture was cooled to 20°C, and the system was added with iodomethane (0.6 mL, 9.59 mmol), and continued to stir for 18 hours. The reaction mixture was added with water (30 mL) and extracted with ethyl acetate (20 mL × 3), and the organic phase was dried over anhydrous sodium sulfate, filtered, and concentrated under reduced pressure. The crude product was separated by silica gel column chromatography (petroleum ether/ethyl acetate, 100/1 to 1/1, V/V) to obtain compound **11-1**. ¹H NMR (400 MHz, CDCl₃) δ 7.70 (d, *J*=8.0 Hz, 1H), 7.28-7.22 (m, 1H), 7.14-7.12 (m, 1H), 3.97 (s, 3H), 3.49 (s, 3H).

### Step 2

Compound **11-1** (900 mg, 3.39 mmol) was dissolved in 1,4-dioxane (10 mL), then the reaction mixture was added with bis(pinacolato)diboron (1.29 g, 5.09 mmol), [1,1'-bis(diphenylphosphino)ferrocene]dichloropalladium(II) (248 mg, 339 µmol), and potassium carbonate (1.0 g, 10.2 mmol). The reaction mixture was heated to 100°C and reacted for 12 hours under nitrogen atmosphere. The reaction mixture was added with water (20 mL) and extracted with ethyl acetate (20 mL × 3), and the organic phase was dried over anhydrous sodium sulfate, filtered, and concentrated under reduced pressure. The crude product was separated by silica gel column chromatography (petroleum ether/ethyl acetate, 100/1 to 1/1, V/V) to obtain compound **11-2**. ¹H NMR (400 MHz, CDCl₃) δ 7.83 (d, *J*=7.8 Hz, 1H), 7.50 (dd, *J*=1.4, 7.8 Hz, 1H), 7.37 (d, *J*=1.4 Hz, 1H), 3.91 (s, 3H), 3.04 (s, 3H), 1.37 (s, 12H).

### Step 3

Compound **11-2** (200 mg, 640 µmol) and compound **2-3** (154 mg, 640 µmol) were dissolved in 1,4-dioxane (4 mL) and water (0.8 mL), and then the reaction mixture was added with [1,1'-bis(diphenylphosphino)ferrocene]dichloropalladium(II) (47 mg, 64 µmol) and potassium carbonate (310 mg, 2.24 mmol). The reaction mixture was heated to 100°C and reacted for 12 hours under nitrogen atmosphere. The reaction mixture was concentrated under reduced pressure, and the crude product was separated by preparative high performance liquid chromatography (separation column: Phenomenex Genimi NX C18 150 × 40 mm × 5 µm; mobile phase: 0.05% hydrochloric acid aqueous solution-acetonitrile; gradient: acetonitrile: 5% to 35%, 10 min) to obtain the hydrochloride of compound **11-3.** MS-ESI calculated for [M+H]⁺ 391, found 391.

### Step 4

The hydrochloride of compound **11-3** (45 mg, 97 µmol) was dissolved in dichloromethane (5 mL), cooled to 0°C in an ice-water bath, and then the reaction mixture was added with boron tribromide (28 µL, 291 µmol), reacted at 0°C for 2 hours under nitrogen atmosphere. The reaction mixture was quenched with water (5 mL), and concentrated under reduced pressure. The crude product was separated by preparative high performance liquid chromatography (separation column: Phenomenex Genimi NX C18 150 × 40 mm × 5 µm; mobile phase: 0.05% hydrochloric acid aqueous solution-acetonitrile; gradient: acetonitrile: 1% to 30%, 10 min) to obtain the hydrochloride of compound **11**. ¹H NMR (400 MHz, CD₃OD) δ 7.74-7.45 (m, 4H), 4.47-4.27 (m, 1H), 3.91-3.76 (m, 1H), 3.57 (d, *J*=11.2 Hz, 1H), 3.20 (s, 3H), 3.00-2.91 (m, 3H), 2.35 (s, 3H), 2.28 (d, *J*=10.8 Hz, 1H), 2.20 - 2.08 (m, 1H), 1.80-1.63 (m, 1H), 1.22 (s, 3H). MS-ESI calculated for [M+H]⁺ 377, found 377.

### Example 12

### Synthetic Route:

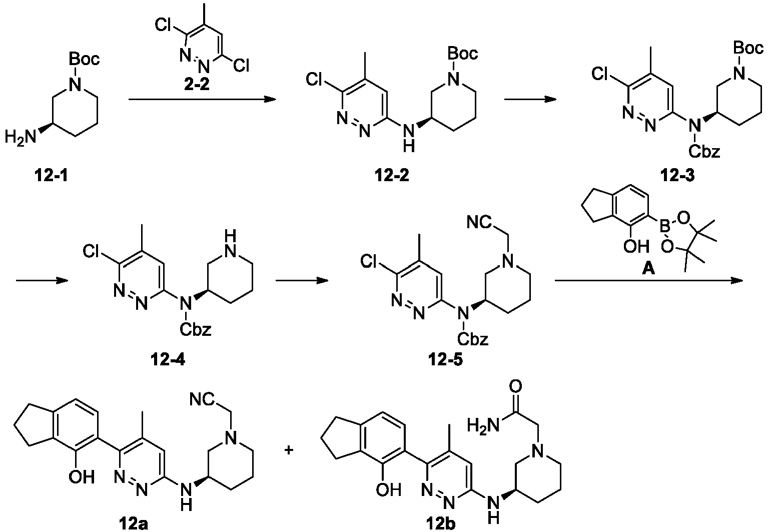

### Step 1

Compound **12-1** (12.5 g, 62.6 mmol) was dissolved in 1,4-dioxane (200 mL), and then the reaction mixture was added with compound 2-2 (8.50 g, 52.2 mmol), tris(dibenzylideneacetone)dipalladium (4.78 g, 5.21 mmol), 4,5-bis(diphenylphosphino)-9,9-dimethylxanthene (6.03 g, 10.43 mmol), and cesium carbonate (42.5 g, 130 mmol). The reaction mixture was stirred at 90°C for 12 hours under nitrogen atmosphere. The reaction mixture was directly concentrated under reduced pressure, and the crude product was separated by preparative high performance liquid chromatography (separation column: Phenomenex Genimi NX C18 150 × 40 mm × 5 µm; mobile phase: 10 mmol/L ammonium bicarbonate aqueous solution-acetonitrile; gradient: acetonitrile: 30% to 60%, 10 min) to obtain compound **12-2**. ¹H NMR (400MHz, CDCl₃) δ 6.56 (s, 1H), 4.84 (s, 1H), 3.87 (s, 1H), 3.72 (d, *J*=13.6 Hz, 1H), 3.52-3.43 (m, 1H), 3.32 (d, *J*=9.2 Hz, 2H), 2.29 (s, 3H), 1.99 - 1.94 (m, 1H), 1.74-1.66 (m, 2H), 1.58 (s, 1H), 1.44 (s, 9H). MS-ESI calculated for [M+H]⁺ 327, found 327.

### Step 2

Compound **12-2** (2.10 g, 6.43 mmol) was dissolved in tetrahydrofuran (20 mL), and sodium hydride (386 mg, 9.64 mmol, purity: 60%) was added thereto at 0°C, and then benzyl chloroformate (2.19 g, 12.9 mmol) was added thereto. The reaction mixture was stirred at 60°C for 12 hours. The reaction mixture was added with water (10 mL) and extracted with ethyl acetate (30 mL × 3), and the organic phase was dried over anhydrous sodium sulfate, filtered, and concentrated under reduced pressure. The crude product was separated by silica gel column chromatography (petroleum ether/ethyl acetate, 100/1 to 3/1, V/V) to obtain compound **12-3.** MS-ESI calculated for [M-56+H]⁺ 405, found 405.

### Step 3

Compound **12-3** (600 mg, 1.30 mmol) was dissolved in ethyl acetate (5 mL), and hydrogen chloride-ethyl acetate (4 mol/L, 10 mL) was added thereto, and the reaction mixture was stirred at 25°C for 1 hour. The reaction mixture was concentrated under reduced pressure to obtain the hydrochloride of compound **12-4,** which was directly used in the next step without purification. MS-ESI calculated for [M+H]⁺ 361, found 361.

### Step 4

The hydrochloride of compound **12-4** (200 mg, 503 µmol) was dissolved in acetonitrile (10 mL), then bromoacetonitrile (121 mg, 1.01 mmol) and potassium carbonate (278 mg, 2.01 mmol) were added thereto, and the reaction mixture was stirred at 25°C for 12 hours. The reaction mixture was concentrated under reduced pressure. The crude product was separated by silica gel column chromatography (petroleum ether/ethyl acetate, 100/1 to 1/1, V/V) to obtain compound **12-5.** MS-ESI calculated for [M+H]⁺ 400, found 400.

### Step 5

Compound **12-5** (190 mg, 475 µmol) was dissolved in dioxane (2 mL) and water (0.4 mL), and intermediate **A** (136 mg, 523 µmol), [1,1'-bis(diphenylphosphino)ferrocene]dichloropalladium(II) (34.8 mg, 47.5 µmol), and potassium carbonate (164 mg, 1.19 mmol) were added thereto. The reaction mixture was stirred at 100°C for 12 hours under nitrogen atmosphere. The reaction mixture was concentrated under reduced pressure, and the crude product was separated by silica gel column chromatography (petroleum ether/ethyl acetate, 100/1 to 0/1, V/V) to obtain the crude product, which was separated by high performance liquid chromatography (chromatographic column: Phenomenex Genimi NX C18 150 × 40 mm × 5 µm; mobile phase: 0.05% hydrochloric acid aqueous solution-acetonitrile; gradient: acetonitrile: 10% to 40%, 10 min) to obtain the hydrochloride of compounds **12a** and **12b.** The hydrochloride of compounds **12a** and **12b** was separated by preparative high performance liquid chromatography (chromatographic column: Phenomenex Gemini-NX 80 × 30 mm × 3 µm; mobile phase: 10 mmol/L ammonium bicarbonate aqueous solution-acetonitrile; gradient: acetonitrile: 38% to 68%, 9 min) to obtain compounds **12a** and **12b.**

Compound **12a**: ¹H NMR (400 MHz, CD₃OD) δ 7.04 (d, *J*=7.2 Hz, 1H), 6.85 (d, *J*=7.2 Hz, 1H), 6.81 (s, 1H), 4.20-4.14 (m, 1H), 3.71 (s, 2H), 3.08-3.06 (m, 1H), 2.98-2.89 (m, 4H), 2.73-2.71 (m, 1H), 2.48-2.44 (m, 1H), 2.36-2.31 (m, 1H), 2.18 (s, 3H), 2.15-2.10 (m, 2H), 1.98-1.95 (m, 1H), 1.92-1.87 (m, 1H), 1.77-1.69 (m, 1H), 1.49-1.47 (m, 1H). MS-ESI calculated for [M+H]⁺ 364, found 364.

Compound **12b**: ¹H NMR (400 MHz, CD₃OD) δ 7.02 (d, *J*=7.6 Hz, 1H), 6.83 (d, *J*=7.6 Hz, 1H), 6.81 (s, 1H), 4.19-4.15 (m, 1H), 3.01 (d, *J*=4.0 Hz, 2H), 2.96-2.88 (m, 5H), 2.57-2.54 (m, 1H), 2.47-2.44 (m, 1H), 2.36-2.33 (m, 1H), 2.17 (s, 3H), 2.15-2.08 (m, 2H), 1.86-1.81 (m, 2H), 1.71-1.68 (m, 1H), 1.58-1.52 (m, 1H). MS-ESI calculated for [M+H]⁺ 382, found 382.

### Example 13

### Synthetic Route:

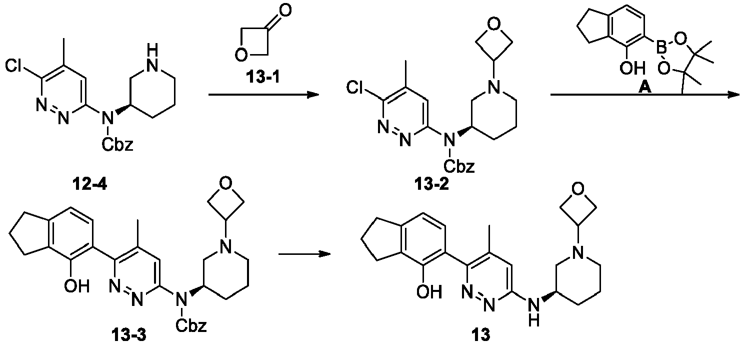

### Step 1

The hydrochloride of compound **12-4** (200 mg, 503 µmol) was dissolved in methanol (5 mL) and dichloromethane (5 mL), and compound **13-1** (72.6 mg, 1.01 mmol), acetic acid (60.5 mg, 1.01 mmol), triethylamine (50.9 mg, 503 µmol), and sodium triacetoxyborohydride (320 mg, 1.51 mmol) were added thereto, and the reaction mixture was stirred at 25°C for 12 hours. The pH of the reaction mixture was neutralized to 8 with saturated sodium bicarbonate solution, and the reaction mixture was extracted with ethyl acetate (10 mL × 3). The organic phase was dried over anhydrous sodium sulfate, filtered, and concentrated under reduced pressure. The crude product was separated by silica gel column chromatography (petroleum ether/ethyl acetate, 100/1 to 0/1, V/V) to obtain compound **13-2.** MS-ESI calculated for [M-56+H]⁺ 361, found 361.

### Step 2

Compound **13-2** (46.0 mg, 110 µmol) was dissolved in dioxane (2 mL) and water (0.4 mL), and intermediate **A** (31.6 mg, 121 µmol), [1,1'-bis(diphenylphosphino)ferrocene]dichloropalladium(II) (8.07 mg, 11.0 µmol), and potassium carbonate (38.1 mg, 276 µmol) were added thereto. The reaction mixture was stirred at 100°C for 12 hours under nitrogen atmosphere. The reaction mixture was concentrated under reduced pressure, and the crude product was separated by silica gel column chromatography (petroleum ether/ethyl acetate, 100/1 to 0/1, V/V) to obtain compound **13-3.** MS-ESI calculated for [M+H]⁺ 515, found 515.

### Step 3

Compound **13-3** (50.0 mg, 97.2 µmol) was dissolved in tetrahydrofuran (4 mL), and the mixture was added with wet palladium/carbon (1.00 mg, purity: 10%), replaced with hydrogen three times. The reaction mixture was stirred at 25°C and a pressure of 15 Psi for 12 hours. The reaction mixture was filtered, and the filtrate was directly concentrated under reduced pressure, and the crude product was separated by preparative high performance liquid chromatography (chromatographic column: Phenomenex Gemini-NX 80 × 30 mm × 3 µm; mobile phase: 10 mmol/L ammonium bicarbonate aqueous solution-acetonitrile; gradient: acetonitrile: 32% to 62%, 9 min) to obtain compound **13**. ¹H NMR (400 MHz, CD₃OD) δ 7.03 (d, *J*=7.6 Hz, 1H), 6.85 (d, *J*=7.6 Hz, 1H), 6.81 (s, 1H), 4.71-4.68 (m, 3H), 4.64-4.61 (m, 2H), 4.17-4.13 (m, 1H), 3.56-3.53 (m, 1H), 2.98-2.89 (m, 5H), 2.55 (s, 1H), 2.18 (s, 3H), 2.15-2.12 (m, 2H), 2.01 (s, 2H), 1.86-1.83 (m, 1H), 1.76-1.68 (m, 1H), 1.51 (s, 1H). MS-ESI calculated for [M+H]⁺ 381, found 381.

### Example 14

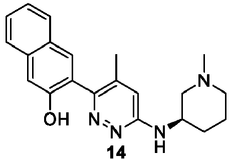

### Synthetic Route:

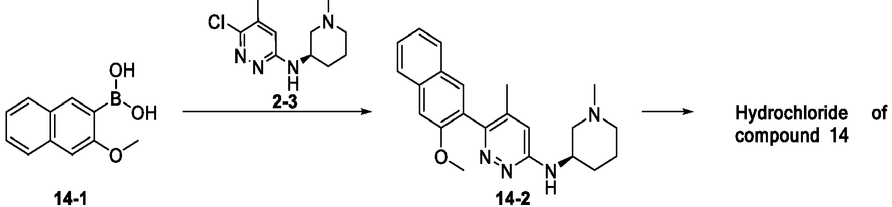

### Step 1

Compound **14-1** (260 mg, 250 µmol) and compound **2-3** (100 mg, 498 µmol) were dissolved in 1,4-dioxane (5 mL) and water (1 mL), and then the reaction mixture was added with [1,1'-bis(diphenylphosphino)ferrocene]dichloropalladium(II) (18 mg, 25 µmol) and potassium carbonate (103 mg, 0.75 mmol). The reaction mixture was heated to 110°C and reacted for 12 hours under nitrogen atmosphere. The reaction mixture was concentrated under reduced pressure, and the crude product was separated by preparative high performance liquid chromatography (separation column: Phenomenex Genimi NX C18 150 × 40 mm × 5 µm; mobile phase: 0.05% hydrochloric acid aqueous solution-acetonitrile; gradient: acetonitrile: 5% to 35%, 10 min) to obtain the hydrochloride of compound **14-2.** MS-ESI calculated for [M+H]⁺ 363, found 363.

### Step 2

The hydrochloride of compound **14-2** (45 mg, 103 µmol) was dissolved in dichloromethane (3 mL), cooled to 0°C in an ice-water bath, and then the reaction mixture was added with boron tribromide (30 µL, 310 µmol), reacted at 20°C for 2 hours under nitrogen atmosphere. The reaction mixture was quenched with water (5 mL), and concentrated under reduced pressure. The crude product was separated by preparative high performance liquid chromatography (separation column: Phenomenex Genimi NX C18 150 × 40 mm × 5 µm; mobile phase: 0.05% hydrochloric acid aqueous solution-acetonitrile; gradient: acetonitrile 1% to 30%, 10 min) to obtain the hydrochloride of compound **14**. ¹H NMR (400 MHz, CD₃OD) δ 8.04-7.94 (m, 1H), 7.90 (d, *J*=8.0 Hz, 1H), 7.79 (d, *J*=8.0 Hz, 1H), 7.74-7.52 (m, 2H), 7.47-7.32 (m, 2H), 4.52-4.22 (m, 1H), 3.97-3.73 (m, 1H), 3.59-3.56 (m, 1H), 3.24-3.02 (m, 2H), 3.00-2.85 (m, 3H), 2.46-2.34 (m, 3H), 2.33-1.94 (m, 3H), 1.78-1.68 (m, 1H). MS-ESI calculated for [M+H]⁺ 349, found 349.

### Example 15

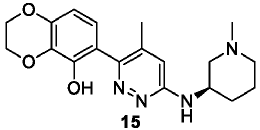

### Synthetic Route:

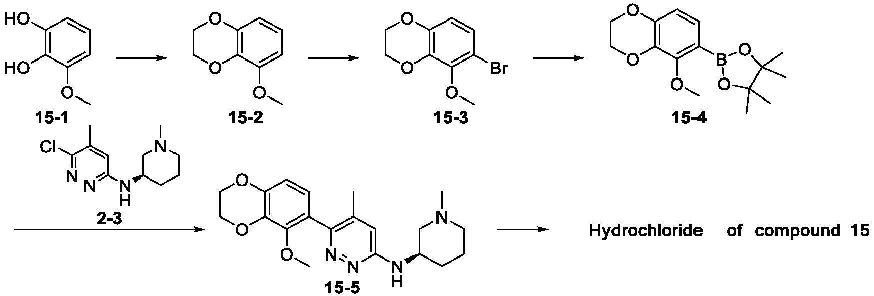

### Step 1

Compound **15-1** (5 g, 35.7 mmol) was dissolved in N,N-dimethylformamide (30 mL), and then the reaction mixture was added with 1,2-dibromoethane (6.7 g, 35.7 mmol) and potassium carbonate (19.7 g, 143 mmol). The reaction mixture was heated to 60°C and reacted for 6 hours under nitrogen atmosphere. The reaction mixture was added with water (50 mL) and extracted with ethyl acetate (30 mL × 3), and the organic phase was dried over anhydrous sodium sulfate, filtered, and concentrated under reduced pressure. The crude product was separated by silica gel column chromatography (petroleum ether/ethyl acetate, 100/1 to 1/1, V/V) to obtain compound **15-2**. ¹H NMR (400 MHz, CDCl₃) δ 6.86-6.71 (m, 1H), 6.60-6.44 (m, 2H), 4.39-4.30 (m, 2H), 4.28-4.26 (m, 2H), 3.95-3.81 (m, 3H).

### Step 2

Compound **15-2** (5 g, 30 mmol) was dissolved in dichloromethane (10 mL), cooled to 0°C in an ice-water bath, and then the reaction mixture was added with N-bromosuccinimide (5.36 g, 30 mmol). The reaction mixture was reacted at 20°C for 2 hours under nitrogen atmosphere. The reaction mixture was added with water (30 mL) and extracted with ethyl acetate (20 mL × 3), and the organic phase was dried over anhydrous sodium sulfate, filtered, and concentrated under reduced pressure. The crude product was separated by silica gel column chromatography (petroleum ether/ethyl acetate, 100/1 to 1/1, V/V) to obtain compound **15-3**. ¹H NMR (400 MHz, CDCl₃) δ 6.98 (d, *J*=9.0 Hz, 1H), 6.57 (d, *J*=9.0 Hz, 1H), 4.34-4.30 (m, 2H), 4.28-4.24 (m, 2H), 3.94-3.84 (m, 3H).

### Step 3

Compound **15-3** (760 mg, 3.10 mmol) was dissolved in 1,4-dioxane (10 mL), then the reaction mixture was added with bis(pinacolato)diboron (1.18 g, 4.65 mmol), [1,1'-bis(diphenylphosphino)ferrocene]dichloropalladium(II) (226 mg, 310 µmol), and potassium carbonate (0.91 g, 9.30 mmol). The reaction mixture was heated to 100°C and reacted for 12 hours under nitrogen atmosphere. The reaction mixture was added with water (10 mL) and extracted with ethyl acetate (20 mL × 3), and the organic phase was dried over anhydrous sodium sulfate, filtered, and concentrated under reduced pressure. The crude product was separated by silica gel column chromatography (petroleum ether/ethyl acetate, 100/1 to 1/1, V/V) to obtain compound **15-4**. ¹H NMR (400 MHz, CDCl₃) δ 7.18 (d, *J*=8.0 Hz, 1H), 6.64 (d, *J*=8.0 Hz, 1H), 4.31-4.21 (m, 4H), 3.85 (s, 3H), 1.38-1.30 (m, 12H).

### Step 4

Compound **15-4** (218 mg, 748 µmol) and compound **2-3** (60 mg, 249 µmol) were dissolved in 1,4-dioxane (5 mL) and water (1 mL), then the reaction mixture was added with [1,1'-bis(diphenylphosphino)ferrocene]dichloropalladium(II) (18 mg, 25 µmol) and potassium carbonate (120 mg, 0.87 mmol). The reaction mixture was heated to 100°C and reacted for 12 hours under nitrogen atmosphere. The reaction mixture was concentrated under reduced pressure, and the crude product was separated by preparative high performance liquid chromatography (separation column: Phenomenex Genimi NX C18 150 × 40 mm × 5 µm; mobile phase: 0.05% hydrochloric acid aqueous solution-acetonitrile; gradient: acetonitrile: 5% to 35%, 10 min) to obtain the hydrochloride of compound **15-5.** MS-ESI calculated for [M+H]⁺ 371, found 371.

### Step 5

The hydrochloride of compound **15-5** (45 mg, 97 µmol) was dissolved in dichloromethane (5 mL), cooled to 0°C in an ice-water bath, and then the reaction mixture was added with boron tribromide (28 µL, 291 µmol), reacted at 0°C for 2 hours under nitrogen atmosphere. The reaction mixture was quenched with water (5 mL), and concentrated under reduced pressure. The crude product was separated by preparative high performance liquid chromatography (separation column: Phenomenex Genimi NX C18 150 × 40 mm × 5 µm; mobile phase: 0.05% hydrochloric acid aqueous solution-acetonitrile; gradient: acetonitrile: 1% to 30%, 10 min) to obtain the hydrochloride of compound **15**. ¹H NMR (400 MHz, CD₃OD) δ 7.78-7.42 (m, 1H), 6.85 (s, 1H), 6.61 (d, *J*=6.8 Hz, 1H), 4.37 (s, 5H), 3.93-3.75 (m, 1H), 3.56 (d, *J*=10.4 Hz, 1H), 3.22-3.00 (m, 1H), 2.90 (s, 4H), 2.45-2.29 (m, 3H), 2.25 (d, *J*=11.2 Hz, 1H), 2.29-1.87 (m, 2H), 1.72-1.69 (m, 1H). MS-ESI calculated for [M+H]⁺ 357, found 357.

### Example 16

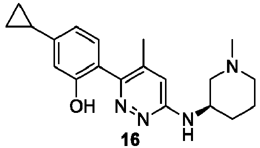

### Synthetic Route:

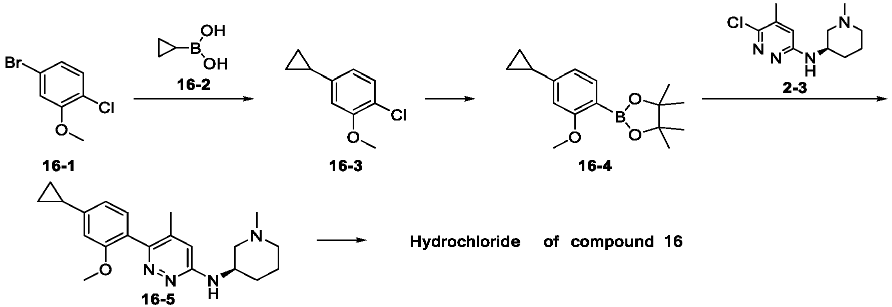

### Step 1

Compound **16-1** (3.00 g, 13.5 mmol), compound **16-2** (1.19 g, 13.8 mmol), tricyclohexylphosphine (950 mg, 3.39 mmol), and cesium carbonate (8.83 g, 27.1 mmol) were dissolved in toluene (100 mL) and water (10.0 mL), and the reaction mixture was added with palladium acetate (304 mg, 1.35 mmol) under nitrogen atmosphere, and the reaction was stirred at 80°C for 12 hours. The reaction mixture was filtered, and the filtrate was concentrated under reduced pressure. The crude product was purified by silica gel column chromatography (petroleum ether/ethyl acetate, 20/1, V/V) to obtain compound **16-3**. ¹H NMR (400 MHz, CDCl₃) δ 7.24 (d, *J*=8.0 Hz, 1H), 6.69 (d, *J*=4.0 Hz, 1H), 6.62-6.60 (m, 1H), 3.91 (s, 3H), 1.93-1.86 (m, 1H), 1.02-0.97 (m, 2H), 0.72-0.68 (m, 2H).

### Step 2

Compound **16-3** (1.00 g, 5.48 mmol), bis(pinacolato)diboron (1.46 g, 5.75 mmol), and potassium acetate (1.07 g, 10.9 mmol) were dissolved in 1,4-dioxane (20.0 mL), and the reaction mixture was added with bis(tri-tert-butylphosphine)palladium(0) (280 mg, 0.547 mmol) under nitrogen atmosphere, and the reaction was stirred at 115°C for 12 hours. The reaction mixture was filtered, and the filtrate was concentrated under reduced pressure. The residue was purified by silica gel column chromatography (petroleum ether/ethyl acetate, 20/1, V/V) to obtain compound **16-4**. ¹H NMR (400 MHz, CDCl₃) δ 7.50 (d, *J*=8.0 Hz, 1H), 6.57-6.52 (m, 2H), 3.75 (s, 3H), 1.84-1.77 (m, 1H), 1.26 (s, 12H), 0.92-0.88 (m, 2H), 0.68-0.64 (m, 2H).

### Step 3

Compound **16-4** (171 mg, 0.623 mmol), compound **2-3** (100 mg, 0.415 mmol), and potassium phosphate (176 mg, 0.831 mmol) were dissolved in 1,4-dioxane (5 mL) and water (0.5 mL), and the reaction mixture was added with 1,1'-bis(di-tert-butylphosphino)ferrocene palladium dichloride-dichloromethane (33.9 mg, 41.5 µmol) under nitrogen atmosphere, and the reaction was stirred at 115°C for 12 hours. The reaction mixture was filtered, and the filtrate was concentrated under reduced pressure. The residue was purified by thin-layer chromatography (dichloromethane/methanol/triethylamine, 5/1/0.002, V/V/V) to obtain compound **16-5.** MS-ESI calculated for [M+H]⁺ 353, found 353.

### Step 4

Compound **16-5** (60.0 mg, 166 µmol) was dissolved in anhydrous dichloromethane (3.00 mL), and boron tribromide (125 mg, 499 µmol) was slowly added dropwise thereto at 0°C. The reaction mixture was stirred and reacted at 20°C for 1 hour. The reaction mixture was quenched with water (3.00 mL) and then concentrated under reduced pressure, and the residue was purified by preparative high performance liquid chromatography (YMC Triart 30 × 150 mm × 7 µm; mobile phase: 0.05% hydrochloric acid aqueous solution-acetonitrile; gradient: acetonitrile: 12% to 32%, 7 min) to obtain the hydrochloride of compound **16.** ¹H NMR (400 MHz, CD₃OD) δ 7.47 (s, 1H), 7.23-7.20 (m, 1H), 6.80-6.78 (m, 1H), 6.73 (s, 1H), 4.34-4.24 (m, 1H), 3.85-3.79 (m, 1H), 3.56-3.53 (m, 1H), 3.05-2.99 (m, 1H), 2.94-2.88 (m, 3H), 2.35-2.33 (m, 3H), 2.26-2.23 (m, 1H), 2.12-1.99 (m, 1H), 1.97-1.91 (m, 3H), 1.71-1.61 (m, 1H), 1.08-1.03 (m, 2H), 0.77-0.73 (m, 2H). MS-ESI calculated for [M+H]⁺ 339, found 339.

### Example 17

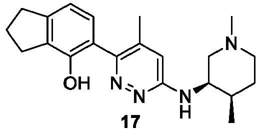

### Synthetic Route:

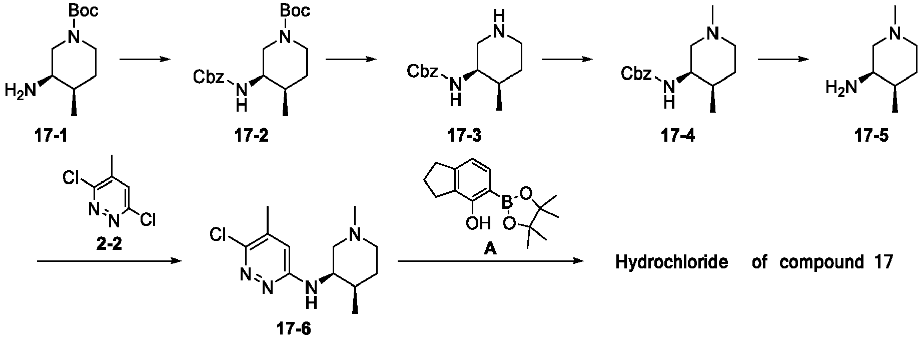

### Step 1

Compound **17-1** (1.00 g, 4.67 mmol) was dissolved in dichloromethane (10 mL). Benzyl chloroformate (1.19 g, 7.00 mmol), triethylamine (1.42 g, 14.0 mmol), and 4-dimethylaminopyridine (57.0 mg, 467 µmol) were added thereto, and the reaction mixture was stirred at 25°C for 12 hours. The reaction mixture was added with water (20 mL) and extracted with dichloromethane (20 mL × 3), and the organic phases were combined, dried over anhydrous sodium sulfate, filtered, and concentrated under reduced pressure. The crude product was separated by silica gel column chromatography (petroleum ether/ethyl acetate, 100/1 to 3/1, V/V) to obtain compound **17-2.** MS-ESI calculated for [M-56+H]⁺ 293, found 293.

### Step 2

Compound **17-2** (1.15 g, 3.30 mmol) was dissolved in ethyl acetate (5 mL), and hydrogen chloride-ethyl acetate (4 mol/L, 10 mL) was added thereto, and the reaction mixture was stirred at 25°C for 1 hour. The reaction mixture was directly concentrated to obtain the hydrochloride of compound **17-3,** which was directly used in the next step without purification. MS-ESI calculated for [M+H]⁺ 249, found 249.

### Step 3

The hydrochloride of compound **17-3** (820 mg, 3.30 mmol) was dissolved in dichloromethane (10 mL) and methanol (10 mL), and formaldehyde aqueous solution(804 mg, 9.91 mmol, purity: 37%), acetic acid (397 mg, 6.60 mmol), triethylamine (334 mg, 3.30 mmol), and sodium triacetoxyborohydride (2.10 g, 9.91 mmol) were added thereto, and the reaction mixture was stirred at 25°C for 12 hours. The reaction mixture was directly concentrated under reduced pressure. The crude product was separated by silica gel column chromatography (dichloromethane/methanol, 100/1 to 10/1, V/V) to obtain compound **17-4.** MS-ESI calculated for [M+H]⁺ 263, found 263.

### Step 4

Compound **17-4** (600 mg, 2.29 mmol) was dissolved in tetrahydrofuran (3 mL), and the mixture was added with wet palladium/carbon (4.00 mg, purity: 10%), replaced with hydrogen three times. The reaction mixture was stirred at 25°C under hydrogen (15 Psi) atmosphere for 12 hours. The reaction mixture was filtered, and the filtrate was directly concentrated under reduced pressure to obtain compound **17-5**. ¹H NMR (400 MHz, CD₃OD) δ 2.92-2.89 (m, 1H), 2.64 (s, 2H), 2.32-2.28 (m, 1H), 2.25 (s, 3H), 2.12-2.04 (m, 1H), 1.80-1.73 (m, 1H), 1.56-1.50 (m, 2H), 0.96 (d, *J*=6.8 Hz, 3H).

### Step 5

Compound **17-5** (220 mg, 1.72 mmol) was dissolved in 1,4-dioxane (5 mL), and then the reaction mixture was added with compound **2-2** (364 mg, 2.23 mmol), tris(dibenzylideneacetone)dipalladium (157 mg, 172 µmol), 4,5-bis(diphenylphosphino)-9,9-dimethylxanthene (199 mg, 343 µmol), and cesium carbonate (1.68 g, 5.15 mmol). The system was replaced with nitrogen three times, and the reaction mixture was stirred at 110°C for 12 hours. The reaction mixture was directly concentrated under reduced pressure, and the residue was separated by silica gel column chromatography (dichloromethane/methanol, 100/1 to 10/1, V/V) to obtain compound **17-6.** MS-ESI calculated for [M+H]⁺ 255, found 255.

### Step 6

Compound **17-6** (80.0 mg, 314 µmol) was dissolved in dioxane (2 mL) and water (0.4 mL), and intermediate **A** (40.8 mg, 157 µmol), [1,1'-bis(diphenylphosphino)ferrocene]dichloropalladium(II) (23.0 mg, 31.4 µmol), and potassium carbonate (109 mg, 785 µmol) were added thereto. The system was replaced with nitrogen three times, and the reaction mixture was stirred at 100°C for 12 hours. The reaction mixture was directly concentrated under reduced pressure, and the crude product was separated by preparative high performance liquid chromatography (chromatographic column: Xtimate C18 150 × 40 mm × 5 µm; mobile phase: 0.05% hydrochloric acid aqueous solution-acetonitrile; gradient: acetonitrile: 5% to 35%, 10 min) to obtain the hydrochloride of compound **17**. ¹H NMR (400 MHz, CD₃OD) δ 7.80 (s, 1H), 7.14 (d, *J*=7.6 Hz, 1H), 7.00 (d, *J*=7.6 Hz, 1H), 4.37 (s, 1H), 3.84 (d, *J*=13.2 Hz, 1H), 3.58 (d, *J*=13.2 Hz, 1H), 3.30-3.22 (m, 1H), 3.18-3.12 (m, 1H), 3.04-2.94 (m, 5H), 2.89 (s, 3H), 2.36 (s, 3H), 2.20-2.14 (m, 3H), 1.97-1.90 (m, 1H), 1.21-1.17 (m, 3H). MS-ESI calculated for [M+H]⁺ 353, found 353.

### Example 18

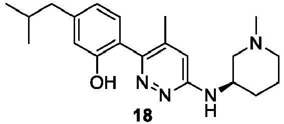

### Synthetic Route:

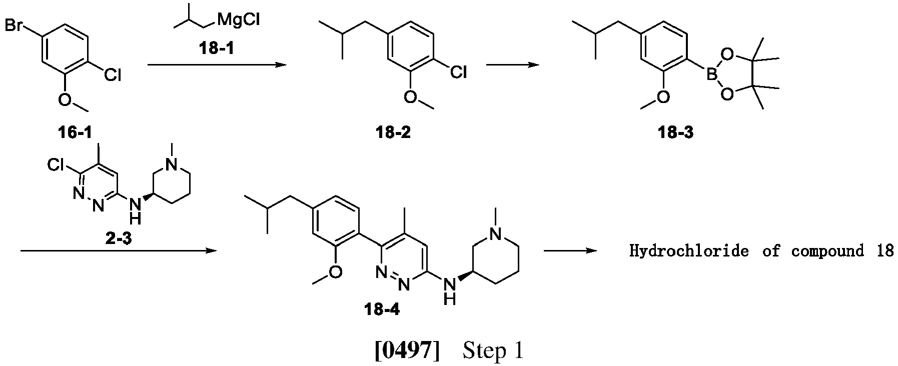

Compound **16-1** (3.00 g, 13.5 mmol) and 1,1'-bis(di-tert-butylphosphino)ferrocene palladium dichloride-dichloromethane (1.11 g, 1.35 mmol) were dissolved in anhydrous tetrahydrofuran (90.0 mL), and compound **18-1** (18.0 mL, 36.0 mmol, 2 mol/L tetrahydrofuran solution) was added dropwise thereto under nitrogen atmosphere. The reaction mixture was stirred at 60°C for 12 hours under nitrogen atmosphere. The reaction mixture was quenched with saturated ammonium chloride aqueous solution (50 mL), extracted with ethyl acetate (50 mL × 2), and the combined organic phases were washed with saturated brine (100 mL × 1), dried over anhydrous sodium sulfate, filtered. The filtrate was concentrated under reduced pressure, and the residue was purified by silica gel column chromatography (petroleum ether/ethyl acetate, 20/1, V/V) to obtain compound **18-2**. ¹H NMR (400 MHz, CDCl₃) δ 7.26 (d, *J*=8.0 Hz, 1H), 6.73-6.70 (m, 2H), 3.91 (s, 3H), 2.47 (d, *J*=7.2 Hz, 2H), 1.93-1.83 (m, 1H), 0.93 (d, *J*=6.8 Hz, 6H).

### Step 2

Compound **18-2** (100 mg, 0.503 mmol), bis(pinacolato)diboron (134 mg, 0.528 mmol), 2-dicyclohexylphosphino-2',4',6'-triisopropylbiphenyl (48.0 mg, 0.101 mmol), and sodium acetate (50.0 mg, 0.604 mmol) were dissolved in 1,4-dioxane (3 mL), and the reaction mixture was added with bis(dibenzylideneacetone)palladium (29.0 mg, 50.3 µmol) under nitrogen atmosphere, and the reaction was stirred at 80°C for 12 hours. The reaction mixture was filtered, and the filtrate was concentrated under reduced pressure. The residue was purified by thin-layer chromatography (petroleum ether/ethyl acetate, 20/1, V/V) to obtain compound **18-3**. ¹H NMR (400 MHz, CDCl₃) δ 7.61 (d, *J*=8.0 Hz, 1H), 6.76 (d, *J*=8.0 Hz, 1H), 6.66 (s, 1H), 3.85 (s, 3H), 2.49 (d, *J*=7.2 Hz, 2H), 1.94-1.84 (m, 1H), 1.37 (s, 12H), 0.92 (d, *J*=6.8 Hz, 6H).

### Step 3

Compound **2-3** (50.0 mg, 0.208 mmol), compound **18-3** (167 mg, 0.575 mmol), and potassium phosphate (88.2 mg, 0.415 mmol) were dissolved in 1,4-dioxane (5 mL) and water (0.5 mL), and the reaction mixture was added with 1,1'-bis(di-tert-butylphosphino)ferrocene palladium dichloride-dichloromethane (17.0 mg, 20.8 µmol) under nitrogen atmosphere, and the reaction was stirred at 110°C for 12 hours. The reaction mixture was filtered, and the filtrate was concentrated under reduced pressure. The crude product was purified by thin-layer chromatography (dichloromethane/methanol/triethylamine, 5/1/0.002, V/V/V) to obtain compound **18-4.** MS-ESI calculated for [M+H]⁺ 369, found 369.

### Step 4

Compound **18-4** (78.0 mg, 154 µmol) was dissolved in anhydrous dichloromethane (3.00 mL), and boron tribromide (116 mg, 461 µmol) was slowly added dropwise thereto at 0°C. The reaction mixture was stirred and reacted at 25°C for 1 hour. The reaction mixture was quenched with methanol (3.00 mL) at 0°C, and then concentrated under reduced pressure. The crude product was purified by preparative high performance liquid chromatography (YMC Triart 30 × 150 mm × 7 µm; mobile phase: 0.05% hydrochloric acid aqueous solution-acetonitrile; gradient: acetonitrile: 23% to 43%, 9 min) to obtain the hydrochloride of compound **18**. ¹H NMR (400 MHz, CD₃OD) δ 7.53 (s, 1H), 7.29-7.27 (m, 1H), 6.92-6.88 (m, 2H), 4.37-4.29 (m, 1H), 3.87-3.81 (m, 1H), 3.58-3.55 (m, 1H), 3.17-3.04 (m, 1H), 2.96-2.90 (m, 4H), 2.54 (d, *J*=8.0 Hz, 2H), 2.37-2.36 (m, 3H), 2.28-2.25 (m, 1H), 2.13-2.01 (m, 1H), 1.97-1.91 (m, 2H), 1.74-1.64 (m, 1H), 0.97 (d, *J*=6.4 Hz, 6H). MS-ESI calculated for [M+H]⁺ 355, found 355.

### Example 19

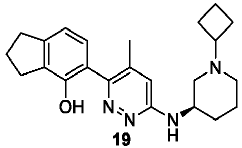

### Synthetic Route:

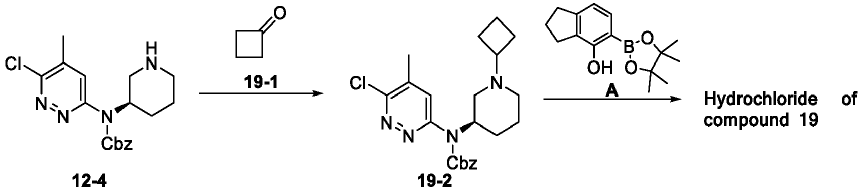

### Step 1

Compound **12-4** (550 mg, 1.38 mmol) was dissolved in anhydrous tetrahydrofuran (4 mL), and the reaction mixture was added with compound 19-1 (0.21 mL, 2.77 mmol) under nitrogen atmosphere. The reaction mixture was stirred and reacted at 70°C for 12 hours. Then the reaction system was cooled to 0°C in an ice-water bath, and the system was added with sodium triacetoxyborohydride (880 mg, 4.15 mmol), heated to 70°C and stirred for 1 hour. The reaction mixture was quenched with water (10 mL), extracted with ethyl acetate (10 mL × 3), and the combined organic phases were washed with saturated brine (20 mL × 1), dried over anhydrous sodium sulfate, filtered. The filtrate was concentrated under reduced pressure, and the residue was purified by silica gel column chromatography (dichloromethane/methanol, 5/1 to 0/1, V/V) to obtain compound **19-2.** MS-ESI calculated for [M+H]⁺ 415, found 415.

### Step 2

Compound **19-2** (101 mg, 0.36 mmol) and intermediate **A** (103 mg, 0.40 mmol) were dissolved in 1,4-dioxane (4 mL) and water (0.8 mL), and the reaction mixture was added with [1,1'-bis(diphenylphosphino)ferrocene]dichloropalladium(II) (26 mg, 36 µmol) and potassium carbonate (125 mg, 0.90 mmol) under nitrogen atmosphere. The reaction mixture was stirred and reacted at 100°C for 12 hours. The reaction mixture was directly concentrated under reduced pressure, and the crude product was separated by preparative high performance liquid chromatography (chromatographic column: Xtimate C18 150 × 40 mm × 5 µm; mobile phase: 0.05% hydrochloric acid aqueous solution-acetonitrile; gradient: acetonitrile: 10% to 30%, 6 min) to obtain the hydrochloride of compound **19**. ¹H NMR (400MHz, DMSO-*d*₆) δ 10.75 (s, 1H), 9.43 (s, 1H), 6.99 (d, *J*=7.6 Hz, 1H), 6.79 (d, *J*=7.6 Hz, 2H), 4.71-4.15 (m, 1H), 3.66 (s, 1H), 2.96-2.76 (m, 4H), 2.33 (s, 2H), 2.17 (s, 2H), 2.09 (br s, 3H), 2.06-1.99 (m, 2H), 1.84-1.60 (m, 2H), 1.52 (s, 1H). MS-ESI calculated for [M+H]⁺ 379, found 379.

### Example 20

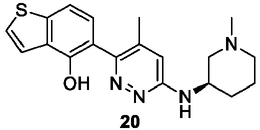

### Synthetic Route:

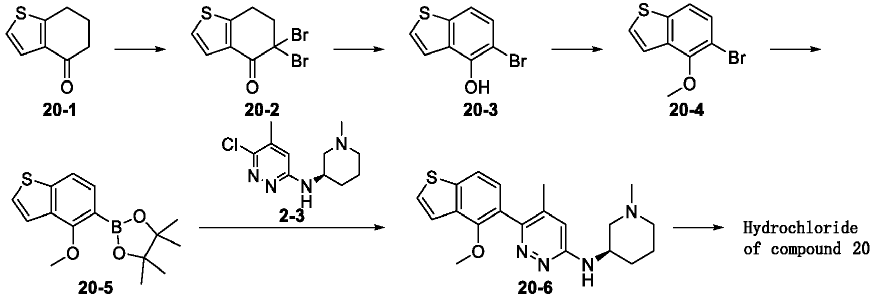

### Step 1

To ethyl acetate (8 mL) was added copper bromide (2.81 g, 12.5 mmol), and the suspension was stirred at 80°C for 10 minutes. Compound **20-1** (478 mg, 3.14 mmol) was dissolved in chloroform (8 mL), then the mixture was added into the above suspension, and the reaction mixture was stirred and reacted at 80°C for 12 hours. The reaction mixture was concentrated under reduced pressure, and the residue was diluted with ethyl acetate (50 mL) and then filtered. The filtrate was washed with saturated sodium bicarbonate aqueous solution (50 mL × 1), and the organic phase was dried over anhydrous sodium sulfate, filtered, and concentrated under reduced pressure to obtain compound **20-2**. ¹H NMR (400 MHz, CDCl₃) δ 7.50 (d, *J*=5.2 Hz, 1H), 7.19 (d, *J*=5.2 Hz, 1H), 3.18 (s, 4H). MS-ESI calculated for [M+H]⁺ 310, 311, 312, found 310, 311, 312.

### Step 2

Compound **20-2** (950 mg, 3.06 mmol) and lithium carbonate (1.36 g, 18.4 mmol) were dissolved in N,N-dimethylformamide (10.0 mL), and the mixture was stirred at 100°C for 6 hours. The reaction mixture was filtered, then the pH of the filtrate was adjusted to 1 with hydrochloric acid aqueous solution (1.00 mol/L), and the mixture was extracted with ethyl acetate (50 mL × 2). The combined organic phases were sequentially washed with water (60 mL × 3) and saturated brine (100 mL × 1), dried over anhydrous sodium sulfate, filtered, and concentrated under reduced pressure to obtain compound **20-3**. ¹H NMR (400 MHz, CDCl₃) δ 7.43 (d, *J*=4.0 Hz, 1H), 7.33-7.32 (m, 1H), 7.31-7.30 (m, 1H), 7.27-7.25 (m, 1H), 5.79 (s, 1H).

### Step 3

Compound **20-3** (770 mg, 3.36 mmol) was dissolved in acetonitrile (10 mL), and the reaction mixture was added with potassium carbonate (929 mg, 6.72 mmol) and dimethyl sulfate (530 mg, 4.20 mmol), and the reaction was stirred at 60°C for 12 hours under nitrogen atmosphere. The reaction mixture was filtered, and the filtrate was concentrated under reduced pressure. The residue was dissolved in methanol (10 mL), and sodium hydroxide (1.0 g) was added thereto, and the mixture was stirred at room temperature for 1 hour to degrade excess dimethyl sulfate. The reaction mixture was filtered, and the filtrate was concentrated under reduced pressure. The residue was dissolved in dichloromethane (50 mL), sequentially washed with water (50 mL × 1) and saturated brine (50 mL × 1), dried over anhydrous sodium sulfate, filtered, concentrated under reduced pressure, and the residue was purified by thin-layer chromatography (petroleum ether/ethyl acetate, 5/1, V/V) to obtain compound **20-4**. ¹H NMR (400 MHz, CDCl₃) δ 7.57-7.51 (m, 2H), 7.49-7.45 (m, 2H), 4.03 (s, 3H).

### Step 4

Compound **20-4** (445 mg, 1.83 mmol), bis(pinacolato)diboron (558 mg, 2.20 mmol), and potassium acetate (359 mg, 3.66 mmol) were dissolved in 1,4-dioxane (10.0 mL), and the reaction mixture was added with bis(triphenylphosphine)palladium(II) chloride (128 mg, 183 µmol) under nitrogen atmosphere, and the reaction was stirred at 90°C for 12 hours. The reaction mixture was filtered, and the filtrate was concentrated under reduced pressure. The residue was purified by silica gel column chromatography (10/1, petroleum ether/ethyl acetate, Rf = 0.30) to obtain compound **20-5**. ¹H NMR (400 MHz, CDCl₃) δ 7.61-7.59 (m, 1H), 7.55-7.53 (m, 1H), 7.43-7.41 (m, 1H), 7.29-7.27 (m, 1H), 3.93 (s, 3H), 1.31 (s, 12H). MS-ESI calculated for [M+H]⁺ 291, found 291.

### Step 5

Compound **20-5** (70.0 mg, 0.291 mmol), compound **2-3** (110 mg, 0.378 mmol), and potassium phosphate (123 mg, 0.581 mmol) were dissolved in 1,4-dioxane (5 mL) and water (0.5 mL), and the reaction mixture was added with 1,1'-bis(di-tert-butylphosphino)ferrocene palladium dichloride-dichloromethane (23.8 mg, 29.1 µmol) under nitrogen atmosphere, and the reaction was stirred at 110°C for 12 hours. The reaction mixture was filtered, and the filtrate was concentrated under reduced pressure. The residue was purified by thin-layer chromatography (dichloromethane/methanol/triethylamine, 5/1/0.002, V/V) to obtain compound **20-6.** MS-ESI calculated for [M+H]⁺ 369, found 369.

### Step 6

Compound **20-6** (110 mg, 289 µmol) was dissolved in anhydrous dichloromethane (5.00 mL), and boron tribromide (217 mg, 866 µmol) was slowly added dropwise thereto at 0°C. The reaction mixture was stirred and reacted at 25°C for 12 hours. The reaction mixture was quenched with methanol (3.00 mL) at 0°C, and then concentrated under reduced pressure. The residue was purified by preparative high performance liquid chromatography (YMC Triart 30 × 150 mm × 7 µm; mobile phase A: 0.05% hydrochloric acid aqueous solution; mobile phase B: acetonitrile; B%: 9% to 29%, 10 min) to obtain the hydrochloride of compound **20**. ¹H NMR (400 MHz, CD₃OD) δ 7.61-7.59 (m, 1H), 7.56-7.55 (m, 2H), 7.52-7.29 (m, 1H), 7.22-7.19 (m, 1H), 4.27-4.21 (m, 1H), 3.78-3.71 (m, 1H), 3.46-3.43 (m, 1H), 3.07-2.93 (m, 2H), 2.85-2.80 (m, 3H), 2.26-2.24 (m, 3H), 2.17-2.09 (m, 1H), 2.04-1.92 (m, 2H), 1.88-1.56 (m, 1H). MS-ESI calculated for [M+H]⁺ 355, found 355.

### Example 21

### Synthetic Route:

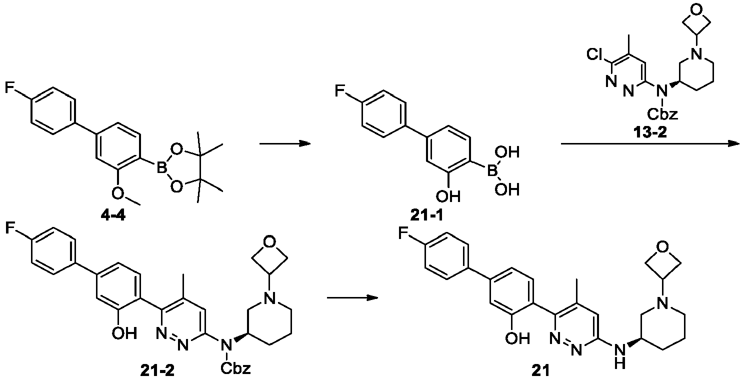

### Step 1

Compound **4-4** (600 mg, 1.83 mmol) was dissolved in dichloromethane (10 mL), cooled to 0°C in an ice-water bath, and boron tribromide (0.53 mL, 5.48 mmol) was added thereto. The reaction mixture was stirred at 0°C for 0.5 hours. The reaction mixture was quenched with water (10 mL), extracted with ethyl acetate (20 mL × 3), and the combined organic phases were dried over anhydrous sodium sulfate, filtered, and concentrated under reduced pressure. The crude product was separated by silica gel column chromatography (dichloromethane/methanol, 100/1 to 10/1, V/V) to obtain compound **21-1**. ¹H NMR (400MHz, CD₃OD) δ 7.59-7.51 (m, 3H), 7.13-7.08 (m, 2H), 7.03 - 7.01 (m, 1H), 6.94 (d, J=1.6 Hz, 1H).

### Step 2

Compound **21-1** (173 mg, 0.53 mmol) was dissolved in 1,4-dioxane (2 mL) and water (0.5 mL). Compound **13-2** (200 mg, 0.48 mmol), [1,1'-bis(diphenylphosphino)ferrocene]dichloropalladium(II) (35 mg, 48 µmol), and potassium carbonate (166 mg, 1.20 mmol) were added thereto. The system was replaced with nitrogen three times, and the reaction mixture was stirred at 100°C for 12 hours. The reaction mixture was directly concentrated under reduced pressure, and the residue was separated by thin-layer chromatography (dichloromethane/methanol, 100/0 to 10/1, V/V) to obtain the crude product of compound **21-2,** which was separated by preparative high performance liquid chromatography (chromatographic column: Phenomenx C18 80× 40 mm× 3 µm; mobile phase: 0.05% ammonia solution-acetonitrile; gradient: acetonitrile: 43% to 73%, 8 min) to obtain compound **21-2.** MS-ESI calculated for [M+H]⁺ 569, found 569.

### Step 3

Compound **21-2** (260 mg, 0.46 mmol) was dissolved in anhydrous tetrahydrofuran (20 mL), and the mixture was added with wet palladium/carbon (80 mg, purity: 10%), replaced with hydrogen three times. The reaction mixture was stirred at 25°C and a pressure of 15 Psi for 12 hours. The reaction mixture was filtered, and the filtrate was directly concentrated under reduced pressure to obtain the crude product of compound **21.** The crude product was separated by preparative high performance liquid chromatography (chromatographic column: Phenomenex C18 80 × 40 mm × 3 µm; mobile phase: 0.05% ammonia solution-acetonitrile; gradient: acetonitrile: 39% to 69%, 8 min) to obtain compound **21**. ¹H NMR (400 MHz, CD₃OD) δ 7.72-7.61 (m, 2H), 7.61-7.50 (m, 1H), 7.30 (d, *J*=7.8 Hz, 1H), 7.23-7.15 (m, 2H), 7.14 (d, *J*=1.8 Hz, 1H), 6.80 (s, 1H), 4.74-4.65 (m, 3H), 4.62 (t, *J*=6.4 Hz, 1H), 4.18-4.14 (m, 1H), 3.57-3.50 (m, 1H), 2.93 (d, *J*=9.2 Hz, 1H), 2.54 (s, 1H), 2.19 (s, 3H), 2.10 (s, 1H), 1.99 (s, 2H), 1.86 - 1.82 (m, 1H), 1.77-1.62 (m, 1H), 1.55-1.45 (m, 1H).

### Example 22

### Synthetic Route:

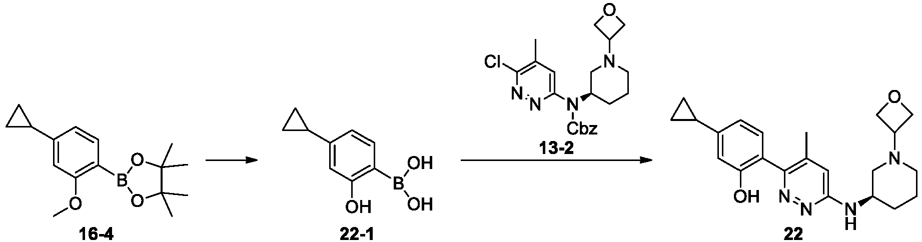

### Step 1

Compound **16-4** (500 mg, 1.82 mmol) was dissolved in dichloromethane (5 mL), cooled to 0°C in an ice-water bath, and boron tribromide (0.35 mL, 3.65 mmol) was added thereto. The reaction mixture was stirred at 0°C for 0.5 hours. The reaction mixture was quenched with water (10 mL), extracted with ethyl acetate (20 mL × 3), and the combined organic phases were dried over anhydrous sodium sulfate, filtered, and concentrated under reduced pressure. The crude product was separated by silica gel column chromatography (dichloromethane/methanol, 100/1 to 10/1, V/V) to obtain compound **22-1**. ¹H NMR (400 MHz, CDCl₃) δ 7.82-7.59 (m, 1H), 6.96-6.82 (m, 1H), 6.71-6.62 (m, 1H), 1.96-1.82 (m, 1H), 1.09-0.97 (m, 2H), 0.80-0.75 (m, 2H).

### Step 2

Compound **22-1** (195 mg, 1.09 mmol) was dissolved in 1,4-dioxane (10 mL) and water (2 mL). Compound **13-2** (380 mg, 911 µmol), [1,1'-bis(diphenylphosphino)ferrocene]dichloropalladium(II) (66.7 mg, 91.2 µmol), and potassium carbonate (315 mg, 2.28 mmol) were added thereto. The system was replaced with nitrogen three times, and the reaction mixture was stirred at 100°C for 12 hours. The reaction mixture was directly concentrated under reduced pressure, and the residue was separated by thin-layer chromatography (dichloromethane/methanol, 100/0 to 10/1, V/V) to obtain the crude product of compound **22.** The crude product was separated by preparative high performance liquid chromatography (chromatographic column: Phenomenx C18 80× 40 mm× 3 µm; mobile phase: 0.05% ammonia solution-acetonitrile; gradient: acetonitrile: 33% to 63%, 8 min) to obtain compound **22**. ¹H NMR (400 MHz, CD₃OD) δ 7.08 (d, *J*=8.0 Hz, 1H), 6.77 (s, 1H), 6.66 (d, *J*=8.0 Hz, 1H), 6.61 (s, 1H), 4.69-4.59 (m, 4H), 4.14-4.10 (m, 1H), 3.55-3.49 (m, 1H), 2.92-2.90 (m, 1H), 2.55-2.53 (m, 1H), 2.13 (s, 3H), 2.09-2.03 (m, 1H), 1.97 (s, 2H), 1.90-1.80 (m, 2H), 1.73-1.65 (m, 1H), 1.50-1.42 (m, 1H), 0.99-0.95 (m, 2H), 0.71-0.67 (m, 2H). MS-ESI calculated for [M+H]⁺ 381, found 381.

### Example 23

### Synthetic Route:

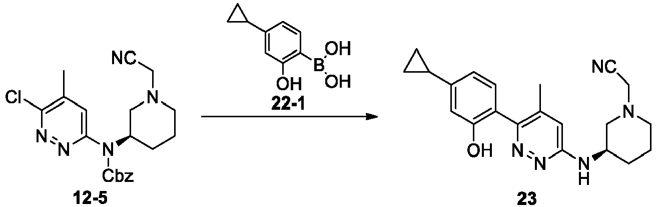

Compound **12-5** (160 mg, 400 µmol) was dissolved in 1,4-dioxane (2 mL) and water (0.4 mL). Compound **22-1** (85.5 mg, 480 µmol), [1,1'-bis(diphenylphosphino)ferrocene]dichloropalladium(II) (29.3 mg, 40.0 µmol), and potassium carbonate (138 mg, 1.00 mmol) were added thereto. The system was replaced with nitrogen three times, and the reaction mixture was stirred at 100°C for 12 hours. The reaction mixture was directly concentrated under reduced pressure, and the residue was separated by thin-layer chromatography (dichloromethane/methanol, 100/0 to 10/1, V/V) to obtain the crude product of compound **23.** The crude product was separated by preparative high performance liquid chromatography (chromatographic column: Phenomenx C18 80× 40 mm× 3 µm; mobile phase: 0.05% ammonia solution-acetonitrile; gradient: acetonitrile: 37% to 67%, 8 min) to obtain compound **23**. ¹H NMR (400 MHz, CD3OD) δ 7.08 (d, *J*=8.0 Hz, 1H), 6.76 (s, 1H), 6.67 (d, *J*=8.0 Hz, 1H), 6.61 (s, 1H), 4.17-4.13 (m, 1H), 3.69 (s, 2H), 3.07-3.04 (m, 1H), 2.71-2.68 (m, 1H), 2.44 (t, *J*=8.8 Hz, 1H), 2.31 (t, *J*=8.8 Hz, 1H), 2.14 (s, 3H), 1.94-1.85 (m, 3H), 1.75-1.67 (m, 1H), 1.47- 1.41 (m, 1H), 1.00-0.95 (m, 2H), 0.71-0.67 (m, 2H). MS-ESI calculated for [M+H]⁺ 364, found 364.

### Example 24

### Synthetic Route:

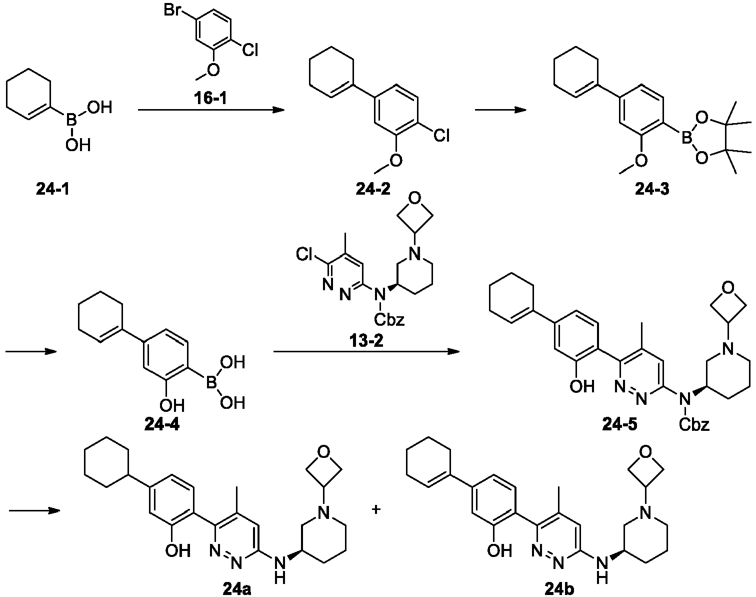

### Step 1

Compound **16-1** (2.81 g, 12.7 mmol) and compound **24-1** (2.0 g, 15.9 mmol) were dissolved in 1,4-dioxane (50 mL) and water (10 mL), and the reaction mixture was added with [1,1'-bis(diphenylphosphino)ferrocene]dichloropalladium(II) (2.32 g, 2.77 mmol) and potassium carbonate (6.58 g, 47.6 mmol) under nitrogen atmosphere. The reaction mixture was stirred and reacted at 100°C for 12 hours. The reaction mixture was directly concentrated under reduced pressure, and the crude product was separated by silica gel column chromatography (petroleum ether/ethyl acetate, 100/1 to 10/1, V/V) to obtain compound **24-2.** ¹H NMR (400 MHz, CD₃OD) δ 7.26 (d, *J*=8.0 Hz, 1H), 6.97-6.81 (m, 2H), 6.12-6.01 (m, 1H), 3.96-3.84 (m, 3H), 2.44-2.30 (m, 2H), 2.26-2.11 (m, 2H), 1.83-1.72 (m, 2H), 1.70-1.59 (m, 2H).

### Step 2

Compound **24-2** (1.0 g, 4.49 mmol), bis(pinacolato)diboron (1.71 g, 6.74 mmol), 2-dicyclohexylphosphino-2',4',6'-triisopropylbiphenyl (428 mg, 0.90 mmol), and sodium acetate (737 mg, 8.98 mmol) were dissolved in 1,4-dioxane (10 mL), and the reaction mixture was added with bis(dibenzylideneacetone)palladium (258 mg, 0.45 mmol) under nitrogen atmosphere, and the reaction was stirred at 115°C for 12 hours. The reaction mixture was directly concentrated under reduced pressure, and the crude product was separated by silica gel column chromatography (petroleum ether/ethyl acetate, 100/1 to 10/1, V/V) to obtain compound **24-3**. ¹H NMR (400 MHz, CDCl₃) δ 7.67-7.60 (m, 1H), 6.96 (dd, *J*=1.2, 7.8 Hz, 1H), 6.87 (d, *J*=1.2 Hz, 1H), 6.18 - 6.13 (m, 1H), 3.89-3.84 (m, 3H), 2.49-2.36 (m, 2H), 2.23-2.21 (m, 2H), 1.85-1.72 (m, 2H), 1.71-1.59 (m, 2H), 1.39-1.31 (m, 12H).

### Step 3

Compound **24-3** (1.5 g, 4.77 mmol) was dissolved in dichloromethane (100 mL), cooled to 0°C in an ice-water bath, and boron tribromide (1.38 mL, 14.3 mmol) was added thereto. The reaction mixture was stirred at 0°C for 0.5 hours. The reaction mixture was quenched with water (30 mL), extracted with ethyl acetate (300 mL × 3), and the combined organic phases were dried over anhydrous sodium sulfate, filtered, and concentrated under reduced pressure. The crude product was separated by silica gel column chromatography (dichloromethane/methanol, 100/1 to 10/1, V/V) to obtain compound **24-4**. ¹H NMR (400MHz, CD₃OD) δ 6.93-6.87 (m, 1H), 6.82-6.77 (m, 1H), 6.73-6.67 (m, 1H), 6.15 (s, 1H), 2.45-2.31 (m, 2H), 2.27-2.14 (m, 2H), 1.85-1.74 (m, 2H), 1.72-1.58 (m, 2H)

### Step 4

Compound **24-4** (157 mg, 0.72 µmol) and compound **13-2** (200 mg, 0.48 mmol) were dissolved in 1,4-dioxane (8 mL) and water (1.6 mL), and then the reaction mixture was added with [1,1'-bis(diphenylphosphino)ferrocene]dichloropalladium(II) (35 mg, 48 µmol) and potassium carbonate (199 mg, 1.44 mmol). The reaction mixture was heated to 100°C and reacted for 12 hours under nitrogen atmosphere. The reaction mixture was concentrated under reduced pressure, and the crude product was separated by silica gel column chromatography (dichloromethane/methanol, 100/1 to 10/1, V/V) to obtain compound **24-5.** MS-ESI calculated for [M+H]⁺ 555, found 555.

### Step 5

Compound **24-5** (200 mg, 0.36 mmol) was dissolved in anhydrous ethanol (20 mL), and the mixture was added with wet palladium/carbon (80 mg, purity: 10%), replaced with hydrogen three times. The reaction mixture was stirred at 25°C and a pressure of 15 Psi for 12 hours. The reaction mixture was filtered, and the filtrate was directly concentrated under reduced pressure to obtain the crude products of compound **24a** and compound **24b.** The crude products were separated by preparative high performance liquid chromatography (chromatographic column: Phenomenex C18 80 × 40 mm × 3 µm; mobile phase: 0.05% ammonia solution-acetonitrile; gradient: acetonitrile: 43% to 73%, 8 min) to obtain compound **24a** and compound **24b**.

Compound **24a**: ¹H NMR (400 MHz, CD₃OD) δ 7.13 (d, *J*=7.8 Hz, 1H), 6.82 (d, *J*=8.0 Hz, 1H), 6.78 (s, 2H), 4.73-4.66 (m, 3H), 4.66-4.57 (m, 1H), 4.22-4.09 (m, 1H), 3.61-3.50 (m, 1H), 2.94 (d, *J*=9.2 Hz, 1H), 2.67-2.41 (m, 2H), 2.17 (s, 4H), 2.02 (s, 2H), 1.89 (s, 5H), 1.80-1.65 (m, 2H), 1.55-1.39 (m, 5H), 1.32 (d, *J*=10.0 Hz, 1H). MS-ESI calculated for [M+H]⁺ 423, found 423.

Compound **24b:** ¹H NMR (400 MHz, CD₃OD) δ7.17 (d, *J*=8.0 Hz, 1H), 7.01 (d, *J*=7.8 Hz, 1H), 6.96 (s, 1H), 6.89 (s, 1H), 6.19 (s, 1H), 4.76-4.67 (m, 3H), 4.67-4.59 (m, 2H), 4.20-4.06 (m, 1H), 3.62 (t, *J*=6.4 Hz, 1H), 2.98 (d, *J*=8.8 Hz, 1H), 2.62 (s, 1H), 2.43 (d, *J*=1.8 Hz, 2H), 2.25 (d, *J*=3.6 Hz, 2H), 2.19 (s, 3H), 2.07- 2.03 (m, 2H), 1.91-1.79 (m, 3H), 1.78-1.65 (m, 3H), 1.53 (d, *J*=10.4 Hz, 1H). MS-ESI calculated for [M+H]⁺ 421, found 421.

### Example 25

### Synthetic Route:

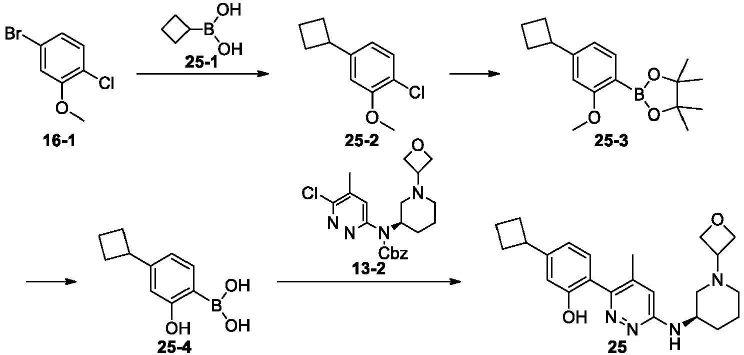

### Step 1

Compound **16-1** (3.80 g, 17.2 mmol), compound **25-1** (1.80 g, 18.0 mmol), and cesium carbonate (11.2 g, 34.3 mmol) were dissolved in toluene (50 mL) and water (5 mL), and the reaction mixture was added with 1,1'-bis(di-tert-butylphosphino)ferrocene palladium dichloride-dichloromethane (1.40 g, 1.72 mmol) under nitrogen atmosphere, and the reaction was stirred at 110°C for 12 hours. The reaction mixture was filtered, and the filtrate was concentrated under reduced pressure. The residue was purified by silica gel column chromatography (20/1, petroleum ether/ethyl acetate, Rf = 0.55) to obtain compound **25-2.** ¹H NMR (400 MHz, CDCl₃) δ 7.30-7.28 (m, 1H), 6.79-6.77 (m, 2H), 3.93 (s, 3H), 3.58-3.50 (m, 1H), 2.41-2.34 (m, 2H), 2.20-2.10 (m, 2H), 2.08-1.98 (m, 1H), 1.91-1.84 (m, 1H).

### Step 2

Compound **25-2** (300 mg, 1.53 mmol), bis(pinacolato)diboron (426 mg, 1.68 mmol), 2-dicyclohexylphosphino-2',4',6'-triisopropylbiphenyl (145 mg, 0.305 mmol), and sodium acetate (150 mg, 1.83 mmol) were dissolved in 1,4-dioxane (6.00 mL), and the reaction mixture was added with bis(dibenzylideneacetone)palladium (87.7 mg, 152 µmol) under nitrogen atmosphere, and the reaction was stirred at 115°C for 12 hours. The reaction mixture was filtered, and the filtrate was concentrated under reduced pressure. The residue was purified by silica gel column chromatography (20/1, petroleum ether/ethyl acetate, Rf = 0.25) to obtain compound **25-3**. ¹H NMR (400 MHz, CDCl₃) δ 7.64-7.63 (m, 1H), 6.83 (d, *J*=8.0 Hz, 1H), 6.71 (s, 1H), 3.86 (s, 3H), 3.61-3.52 (m, 1H), 2.40-2.32 (m, 2H), 2.22-2.12 (m, 2H), 2.09-2.02 (m, 1H), 1.91-1.83 (m, 1H), 1.36 (s, 12H).

### Step 3

Compound **25-3** (230 mg, 798 µmol) was dissolved in anhydrous dichloromethane (5.00 mL), and boron tribromide (400 mg, 1.60 mmol) was slowly added dropwise thereto at 0°C. The reaction mixture was stirred and reacted at 0°C for 0.5 hours. The reaction mixture was quenched with water (30.0 mL) at 0°C, extracted with dichloromethane (30 mL × 2). The combined organic phases were dried over anhydrous sodium sulfate, filtered, and concentrated under reduced pressure, and the residue was purified by thin-layer chromatography (dichloromethane/methanol, 20/1, V/V) to obtain compound **25-4**. ¹H NMR (400 MHz, CD₃OD) δ 7.50 (br s, 1H), 6.72-6.70 (m, 1H), 6.64 (s, 1H), 3.54-3.45 (m, 1H), 2.36-2.31 (m, 2H), 2.19-2.09 (m, 2H), 2.08-2.00 (m, 1H), 1.90-1.82 (m, 1H).

### Step 4

Compound **25-4** (60.0 mg, 0.312 mmol), compound **13-2** (100 mg, 0.240 mmol), and potassium carbonate (66.3 mg, 0.479 mmol) were dissolved in 1,4-dioxane (5 mL) and water (0.5 mL), and the reaction mixture was added with 1,1'-bis(di-t-butylphosphino)ferrocene palladium dichloride (17.6 mg, 24.0 µmol) under nitrogen atmosphere, and the reaction was stirred at 110°C for 12 hours. The reaction mixture was filtered, and the filtrate was concentrated under reduced pressure, and the residue was purified by preparative high performance liquid chromatography (Waters Xbridge 150 × 25 mm × 5 µm; mobile phase A: 10 mmol/L ammonium bicarbonate aqueous solution; mobile phase B: acetonitrile; B%: 40% to 70%, 10 min) to obtain compound **25**. ¹H NMR (400 MHz, CD₃OD) δ 7.14 (d, *J*=8.0 Hz, 1H), 6.82 (d, *J*=8.0 Hz, 1H), 6.79 (s, 2H), 4.71-4.68 (m, 2H), 4.64-4.62 (m, 4H), 4.18-4.12 (m, 1H), 3.60-3.52 (m, 2H), 2.94-2.92 (m, 1H), 2.59-2.56 (m, 1H), 2.41-2.34 (m, 2H), 2.21-2.18 (m, 1H), 2.16 (s, 3H), 2.13-2.08 (m, 1H), 2.06-2.00 (m, 2H), 1.93-1.83 (m, 2H), 1.76-1.68 (m, 1H), 1.54-1.45 (m, 1H). MS-ESI calculated for [M+H]⁺ 395, found 395.

### Example 26

### Synthetic Route:

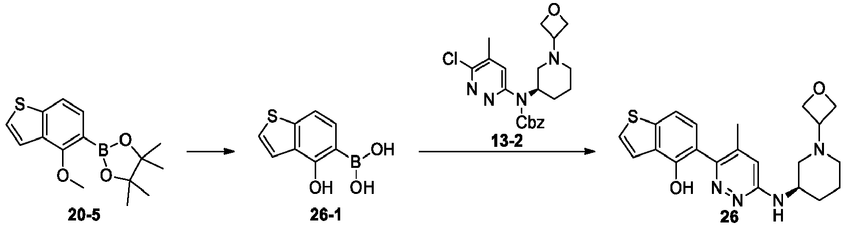

### Step 1

Compound **20-5** (763 mg, 2.63 mmol) was dissolved in anhydrous dichloromethane (8.00 mL), and boron tribromide (1.32 g, 5.26 mmol) was slowly added dropwise thereto at 0°C. The reaction mixture was stirred and reacted at 0°C for 0.5 hours. The reaction mixture was quenched with water (30.0 mL) at 0°C, extracted with dichloromethane (30 mL × 2). The combined organic phases were dried over anhydrous sodium sulfate, filtered, and concentrated under reduced pressure, and the residue was purified by silica gel column chromatography (10/1, dichloromethane/methanol, Rf = 0.40) to obtain compound **26-1**. ¹H NMR (400 MHz, CD₃OD) δ 7.58-7.52 (m, 1H), 7.51-7.49 (m, 1H), 7.40-7.35 (m, 2H).

### Step 2

Compound **13-2** (120 mg, 0.288 mmol), compound **26-1** (100 mg, 0.515 mmol), and potassium carbonate (119 mg, 0.863 mmol) were dissolved in 1,4-dioxane (6 mL) and water (0.6 mL), and the reaction mixture was added with 1,1'-bis(di-t-butylphosphino)ferrocene palladium dichloride (21.1 mg, 28.8 µmol) under nitrogen atmosphere, and the reaction was stirred at 110°C for 12 hours. The reaction mixture was filtered, and the filtrate was concentrated under reduced pressure, and the residue was purified by preparative high performance liquid chromatography (Waters Xbridge 150 × 25 mm × 5 µm; mobile phase A: 0.05% ammonia solution; mobile phase B: acetonitrile; B%: 22% to 50%, 9 min) to obtain compound **26**. ¹H NMR (400 MHz, CD₃OD) δ 7.61 (d, *J*=4.0 Hz, 1H), 7.51-7.49 (m, 2H), 7.26 (d, *J*=8.0 Hz, 1H), 6.86 (s, 1H), 4.72-4.69 (m, 2H), 4.65-4.62 (m, 2H), 4.21-4.14 (m, 1H), 3.57-3.54 (m, 1H), 2.95-2.91 (m, 1H), 2.60-2.56 (m, 1H), 2.22 (s, 3H), 2.18-2.10 (m, 1H), 2.06-2.00 (m, 2H), 1.89-1.83 (m, 1H), 1.77-1.69 (m, 1H), 1.56-1.47 (m, 1H). MS-ESI calculated for [M+H]⁺ 397, found 397.

### Example 27

### Synthetic Route:

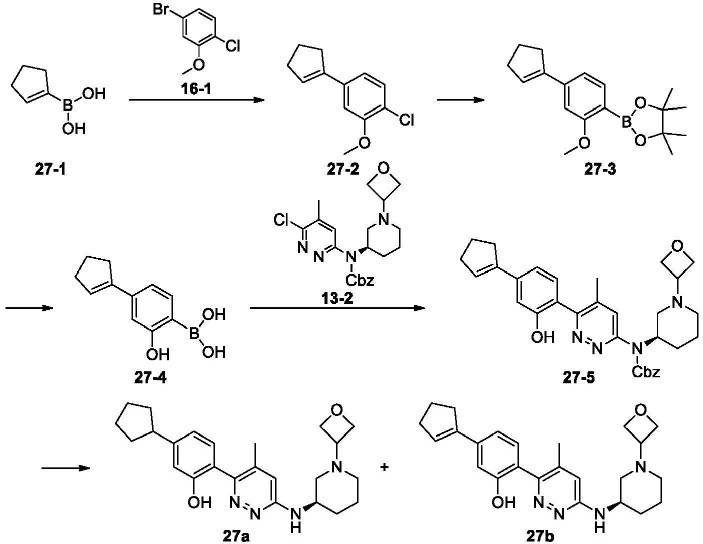

### Step 1

Compound **27-1** (1.82 g, 16.3 mmol) was dissolved in 1,4-dioxane (20 mL) and water (4 mL). Compound **16-1** (3 g, 13.6 mmol), [1,1'-bis(diphenylphosphino)ferrocene]dichloropalladium(II) (1.98 g, 2.71 mmol), and potassium carbonate (5.62 g, 40.6 mmol) were added thereto. The system was replaced with nitrogen three times, and the reaction mixture was stirred at 100°C for 12 hours. The reaction mixture was directly concentrated under reduced pressure, and the crude product was separated by silica gel column chromatography (petroleum ether/ethyl acetate, 100/1 to 10/1, V/V) to obtain compound **27-2**. ¹H NMR (400 MHz, CDCl₃) δ 7.29 (d, *J*=8.4 Hz, 1H), 7.00 (s, 1H), 6.97 (d, *J*=8.4 Hz, 1H), 6.19-6.18 (m, 1H), 3.93 (s, 3H), 2.72-2.68 (m, 2H), 2.57-2.52 (m, 2H), 2.08-2.00 (m, 2H).

### Step 2

Compound **27-2** (2.00 g, 9.58 mmol) was dissolved in 1,4-dioxane (30 mL), and bis(pinacolato)diboron (3.65 g, 14.4 mmol), sodium acetate (1.57 g, 19.2 mmol), 2-dicyclohexylphosphino-2',4',6'-triisopropylbiphenyl (914 mg, 1.92 mmol), and tris(dibenzylideneacetone)dipalladium (878 mg, 958 µmol) were added thereto. The system was replaced with nitrogen three times, and the reaction mixture was stirred at 115°C for 12 hours. The reaction mixture was directly concentrated under reduced pressure, and the crude product was separated by silica gel column chromatography (petroleum ether/ethyl acetate, 100/1 to 10/1, V/V) to obtain compound **27-3**. ¹H NMR (400 MHz, CDCl₃) δ 7.43 (d, *J*=2.4 Hz, 1H), 7.03-7.01 (m, 1H), 6.93 (s, 1H), 6.26-6.25 (m, 1H), 3.86 (s, 3H), 2.74-2.70 (m, 2H), 2.56-2.52 (m, 2H), 2.07-2.01 (m, 2H), 1.36 (s, 12H).

### Step 3

Compound **27-3** (2.50 g, 8.33 mmol) was dissolved in dichloromethane (30 mL), and boron tribromide (4.17 g, 16.7 mmol) was added thereto at 0°C. The reaction mixture was stirred at 0°C for 0.5 hours. The reaction mixture was added with water (10 mL) and extracted with ethyl acetate (20 mL × 3), and the organic phases were combined, dried over anhydrous sodium sulfate, filtered, and concentrated under reduced pressure. The crude product was separated by silica gel column chromatography (dichloromethane/methanol, 100/1 to 10/1, V/V) to obtain compound **27-4**. ¹H NMR (400 MHz, CD₃OD) δ 7.53 (d, *J*=7.6 Hz, 1H), 6.95 (d, *J*=7.6 Hz, 1H), 6.82 (s, 1H), 6.22 (s, 1H), 2.69-2.65 (m, 2H), 2.54-2.49 (m, 2H), 2.05-2.00 (m, 2H).

### Step 4

Compound **27-4** (294 mg, 1.44 mmol) was dissolved in 1,4-dioxane (10 mL) and water (2 mL). Compound **13-2** (500 mg, 1.20 mmol), [1,1'-bis(diphenylphosphino)ferrocene]dichloropalladium(II) (87.8 mg, 120 µmol), and potassium carbonate (414 mg, 3.00 mmol) were added thereto. The system was replaced with nitrogen three times, and the reaction mixture was stirred at 100°C for 12 hours. The reaction mixture was directly concentrated under reduced pressure, and the residue was separated by thin-layer chromatography (petroleum ether/ethyl acetate, 100/1 to 0/1, V/V) to obtain compound **27-5.** MS-ESI calculated for [M+H]⁺ 541, found 541.

### Step 5

Compound **27-5** (360 mg, 666 µmol) was dissolved in tetrahydrofuran (10 mL), and the mixture was added with wet palladium/carbon (100 mg, purity: 10%), replaced with hydrogen three times. The reaction mixture was stirred at 25°C and a pressure of 15 Psi for 12 hours. The reaction mixture was filtered, and the filtrate was directly concentrated under reduced pressure to obtain the crude products of compound **27a** and compound **27b**. The crude products were separated by preparative high performance liquid chromatography (chromatographic column: Phenomenex C18 80 × 40 mm × 3 µm; mobile phase: 0.05% ammonia solution-acetonitrile; gradient: acetonitrile: 39% to 69%, 8 min) to obtain compound **27a** and compound **27b**.

Compound **27a**: ¹H NMR (400 MHz, CD₃OD) δ 7.11 (d, *J*=8.0 Hz, 1H), 6.83 (dd, *J*=1.6, 8.0 Hz, 1H), 6.80 (d, *J*=1.2, 1H),6.76 (d, *J*=1.2, 1H), 4.69-4.65 (m, 3H), 4.63-4.59 (m, 1H), 4.15-4.11 (m, 1H), 3.56-3.49 (m, 1H), 3.04-2.95 (m, 1H), 2.91-2.89 (m, 1H), 2.55-2.53 (m, 1H), 2.14 (s, 3H), 2.11-2.04 (m, 3H), 2.00-1.95 (m, 2H), 1.88-1.78 (m, 3H), 1.76-1.59 (m, 5H), 1.49-1.46 (m, 1H). MS-ESI calculated for [M+H]⁺ 409, found 409.

Compound **27b**: ¹H NMR (400 MHz, CD₃OD) δ 7.16 (d, *J*=8.0 Hz, 1H), 7.06 (dd, *J*=1.2, 8.0 Hz, 1H), 6.99 (d, *J*=1.2 Hz, 1H), 6.77 (s, 1H), 6.24-6.22 (m, 1H), 4.70-4.66 (m, 4H), 4.17-4.11 (m, 1H), 3.56-3.50 (m, 1H), 2.93-2.89 (m, 1H), 2.73-2.69 (m, 2H), 2.57-2.52 (m, 3H), 2.15 (s, 3H), 2.08-1.98 (m, 5H), 1.85-1.81 (m, 1H), 1.72-1.62 (m, 1H), 1.49-1.47 (m, 1H). MS-ESI calculated for [M+H]⁺ 407, found 407.

### Example 28

### Synthetic Route:

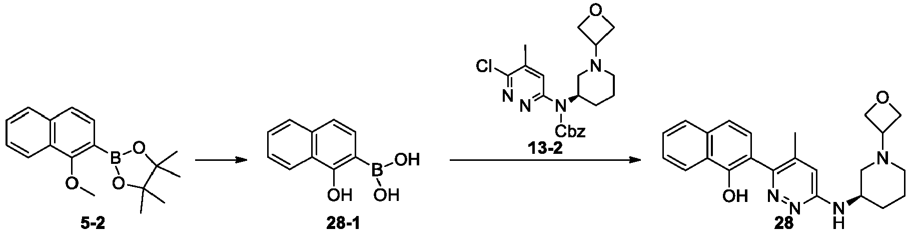

### Step 1

Compound **5-2** (0.5 g, 1.76 mmol) was dissolved in dichloromethane (100 mL), cooled to 0°C in an ice-water bath, and boron tribromide (0.34 mL, 3.52 mmol) was added thereto. The reaction mixture was stirred at 0°C for 0.5 hours. The reaction mixture was quenched with water (10 mL), extracted with ethyl acetate (20 mL × 3), and the combined organic phases were dried over anhydrous sodium sulfate, filtered, and concentrated under reduced pressure. The crude product was separated by silica gel column chromatography (dichloromethane/methanol, 100/1 to 10/1, V/V) to obtain compound **28-1.** ¹H NMR (400MHz, DMSO-*d*₆) δ 8.24-7.99 (m, 1H), 7.89-7.71 (m, 2H), 7.69-7.50 (m, 1H), 7.48-7.29 (m, 2H).

### Step 2

Compound **28-1** (100 mg, 0.53 µmol) and compound **13-2** (266 mg, 0.64 mmol) were dissolved in 1,4-dioxane (10 mL) and water (2 mL), and then the reaction mixture was added with [1,1'-bis(diphenylphosphino)ferrocene]dichloropalladium(II) (78 mg, 106 µmol) and potassium carbonate (220 mg, 1.60 mmol). The reaction mixture was heated to 100°C and reacted for 12 hours under nitrogen atmosphere. The reaction mixture was concentrated under reduced pressure, and the crude product was separated by preparative high performance liquid chromatography (chromatographic column: Phenomenex C18 80 × 40 mm × 3 µm; mobile phase: 0.05% ammonia solution-acetonitrile; gradient: acetonitrile: 35% to 65%, 8 min) to obtain compound **28.** ¹H NMR (400MHz, CD₃OD) δ 8.41-8.26 (m, 1H), 7.89-7.77 (m, 1H), 7.53-7.47 (m, 2H), 7.46-7.35 (m, 2H), 6.88 (s, 1H), 4.75-4.67 (m, 3H), 4.65-4.59 (m, 1H), 4.25-4.14 (m, 1H), 3.58-3.52 (m, 1H), 2.93 (d, J=8.0 Hz, 1H), 2.56 (s, 1H), 2.28 (s, 3H), 2.12 (s, 1H), 2.07-1.93 (m, 2H), 1.87 - 1.84 (m, 1H), 1.77 - 1.69 (m, 1H), 1.52-1.51 (m, 1H). MS-ESI calculated for [M+H]⁺ 391, found 391.

### Example 29

### Synthetic Route:

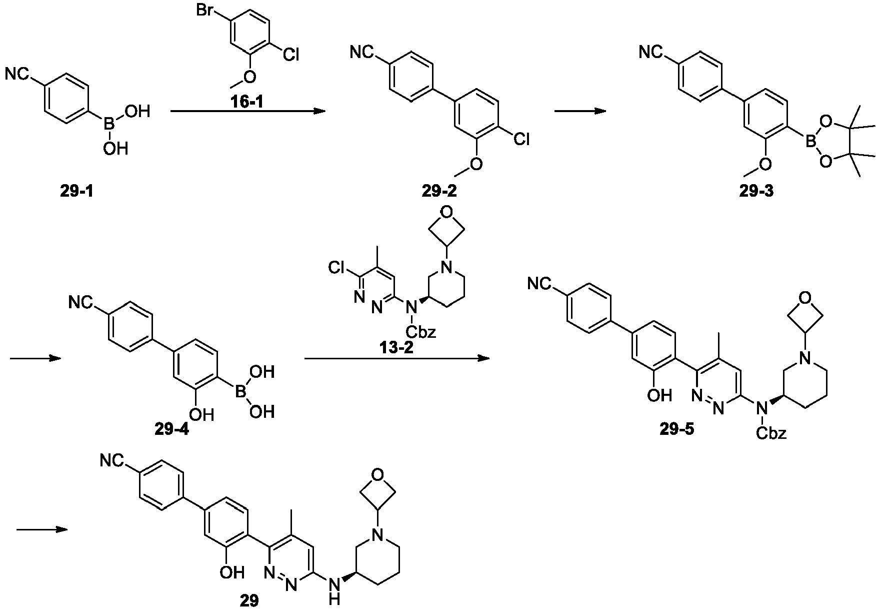

### Step 1

Compound **29-1** (2.0 g, 13.6 mmol) was dissolved in 1,4-dioxane (40 mL) and water (8 mL). Compound **16-1** (3.0 g, 13.6 mmol), [1,1'-bis(diphenylphosphino)ferrocene]dichloropalladium(II) (1.99 g, 2.72 mmol), and potassium carbonate (5.64 g, 40.8 mmol) were added thereto. The system was replaced with nitrogen three times, and the reaction mixture was stirred at 100°C for 12 hours. The reaction mixture was directly concentrated under reduced pressure, and the crude product was separated by silica gel column chromatography (petroleum ether/ethyl acetate, 100/1 to 10/1, V/V) to obtain compound **29-2.** ¹H NMR (400 MHz, CDCl₃) δ 7.82-7.72 (m, 2H), 7.71-7.59 (m, 2H), 7.47 (d, *J*=7.8 Hz, 1H), 7.15-7.06 (m, 2H), 3.99 (s, 3H).

### Step 2

Compound **29-2** (2.00 g, 8.21 mmol) was dissolved in 1,4-dioxane (20 mL), and bis(pinacolato)diboron (3.13 g, 12.3 mmol), sodium acetate (1.35 g, 16.4 mmol), 2-dicyclohexylphosphino-2',4',6'-triisopropylbiphenyl (783 mg, 1.64 mmol), and tris(dibenzylideneacetone)dipalladium (472 mg, 821 µmol) were added thereto. The system was replaced with nitrogen three times, and the reaction mixture was stirred at 115°C for 12 hours. The reaction mixture was directly concentrated under reduced pressure, and the crude product was separated by silica gel column chromatography (petroleum ether/ethyl acetate, 100/1 to 10/1, V/V) to obtain compound **29-3.** ¹H NMR (400 MHz, CDC13) δ 7.80 (d, *J*=7.6 Hz, 1H), 7.76-7.67 (m, 4H), 7.19 - 7.17 (m, 1H), 7.04 (d, J=1.2 Hz, 1H), 3.93 (s, 3H), 1.44-1.36 (m, 12H).

### Step 3

Compound **29-3** (2.50 g, 7.46 mmol) was dissolved in dichloromethane (30 mL), and boron tribromide (2.16 mL, 22.4 mmol) was added thereto at 0°C. The reaction mixture was stirred at 0°C for 0.5 hours. The reaction mixture was added with water (40 mL) and extracted with ethyl acetate (40 mL × 3), and the organic phases were combined, dried over anhydrous sodium sulfate, filtered, and concentrated under reduced pressure. The crude product was separated by silica gel column chromatography (dichloromethane/methanol, 100/1 to 10/1, V/V) to obtain compound **29-4.** ¹H NMR (400MHz, DMSO-*d*₆) δ 7.93-7.83 (m, 4H), 7.77 (d, J=7.6 Hz, 1H), 7.2 (dd, *J*=1.6, 8.0 Hz, 1H), 7.13-7.08 (m, 1H).

### Step 4

Compound **29-4** (104 mg, 0.44 mmol) was dissolved in 1,4-dioxane (10 mL) and water (2 mL). Compound **13-2** (219 mg, 0.53 mmol), [1,1'-bis(diphenylphosphino)ferrocene]dichloropalladium(II) (64 mg, 88 µmol), and potassium carbonate (182 mg, 1.32 mmol) were added thereto. The system was replaced with nitrogen three times, and the reaction mixture was stirred at 100°C for 12 hours. The reaction mixture was directly concentrated under reduced pressure, and the residue was separated by thin-layer chromatography (petroleum ether/ethyl acetate, 100/1 to 0/1, V/V) to obtain compound **29-5.** MS-ESI calculated for [M+H]⁺ 590, found 590.

### Step 5

Compound **29-5** (263 mg, 0.46 mmol) was dissolved in anhydrous tetrahydrofuran (20 mL), and the mixture was added with wet palladium/carbon (80 mg, purity: 10%), replaced with hydrogen three times. The reaction mixture was stirred at 25°C and a pressure of 15 Psi for 12 hours. The reaction mixture was filtered, and the filtrate was directly concentrated under reduced pressure to obtain the crude product of compound **21.** The crude product was separated by preparative high performance liquid chromatography (chromatographic column: Phenomenex C18 80 × 40 mm × 3 µm; mobile phase: 0.05% ammonia solution-acetonitrile; gradient: acetonitrile: 34% to 64%, 8 min) to obtain compound **29.** ¹H NMR (400MHz, CD₃OD) δ 7.84 (d, *J*=1.0 Hz, 4H), 7.42-7.35 (m, 1H), 7.31-7.27 (m, 1H), 7.23 (d, *J*=1.6 Hz, 1H), 6.82 (s, 1H), 4.75-4.66 (m, 3H), 4.66-4.54 (m, 1H), 4.24-4.10 (m, 1H), 3.58 - 3.52 (m, 1H), 2.94 (d, *J*=9.2 Hz, 1H), 2.56 (s, 1H), 2.20 (s, 3H), 2.12 (s, 1H), 2.01 (s, 2H), 1.84 (d, *J*=3.8 Hz, 1H), 1.78-1.65 (m, 1H), 1.52 (s, 1H). MS-ESI calculated for [M+H]⁺ 442, found 442.

### Example 30

### Synthetic Route:

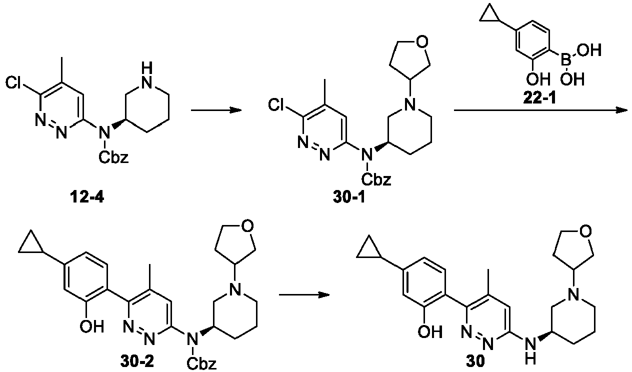

### Step 1

The hydrochloride of compound **12-4** (300 mg, 755 µmol) was dissolved in tetrahydrofuran (10 mL), and tetrahydrofuran-3-one (195 mg, 2.27 mmol) was added thereto. The reaction mixture was stirred at 70°C for 12 hours. Then sodium triacetoxyborohydride (320 mg, 1.51 mmol) was added thereto at 0°C, and the reaction mixture was stirred at 25°C for 1 hour. The pH of the reaction mixture was neutralized to 8 with saturated sodium bicarbonate solution, and the reaction mixture was extracted with ethyl acetate (10 mL × 3). The organic phases were combined, dried over anhydrous sodium sulfate, filtered, and concentrated under reduced pressure. The crude product was separated by silica gel column chromatography (petroleum ether/ethyl acetate, 100/1 to 0/1, V/V) to obtain compound **30-1.** MS-ESI calculated for [M+H]⁺ 431, found 431.

### Step 2

Compound **30-1** (160 mg, 371 µmol) was dissolved in dioxane (2 mL) and water (0.4 mL). Compound **22-1** (79.3 mg, 446 µmol), [1,1'-bis(diphenylphosphino)ferrocene]dichloropalladium(II) (27.2 mg, 37.1 µmol), and potassium carbonate (128 mg, 928 µmol) were added thereto. The system was replaced with nitrogen three times, and the reaction mixture was stirred at 100°C for 12 hours. The reaction mixture was directly concentrated under reduced pressure, and the residue was separated by thin-layer chromatography (petroleum ether/ethyl acetate, 100/1 to 0/1, V/V) to obtain compound **30-2.** MS-ESI calculated for [M+H]⁺ 529, found 529.

### Step 3

Compound **30-2** (120 mg, 227 µmol) was dissolved in tetrahydrofuran (5 mL), and the mixture was added with wet palladium/carbon (50.0 mg, purity: 10%), replaced with hydrogen three times. The reaction mixture was stirred at 25°C and a pressure of 15 Psi for 12 hours. The reaction mixture was filtered, and the filtrate was directly concentrated under reduced pressure to obtain the crude product of compound **1.** The crude product was separated by preparative high performance liquid chromatography (chromatographic column: Phenomenex C18 80 × 40 mm × 3 µm; mobile phase: 0.05% ammonia solution-acetonitrile; gradient: acetonitrile: 35% to 65%, 8 min) to obtain compound **30.** ¹H NMR (400 MHz, CD₃OD) δ 7.07 (d, *J*=7.6 Hz, 1H), 6.74 (s, 1H), 6.66 (dd, *J*=1.6, 7.6 Hz, 1H), 6.61 (d, *J*=1.6 Hz, 1H), 4.11-4.07 (m, 1H), 3.95-3.83 (m, 2H), 3.77-3.58 (m, 3H), 3.16-2.97 (m, 2H), 2.79-2.62 (m, 1H), 2.29-2.19 (m, 1H), 2.13 (s, 3H), 2.10-2.04 (m, 2H), 2.00-1.97 (m, 1H), 1.91-1.84 (m, 2H), 1.82-1.76 (m, 1H), 1.72-1.63 (m, 1H), 1.03-0.91 (m, 2H), 0.70-0.66 (m, 2H). MS-ESI calculated for [M+H]⁺ 395, found 395.

### Example 31

### Synthetic Route:

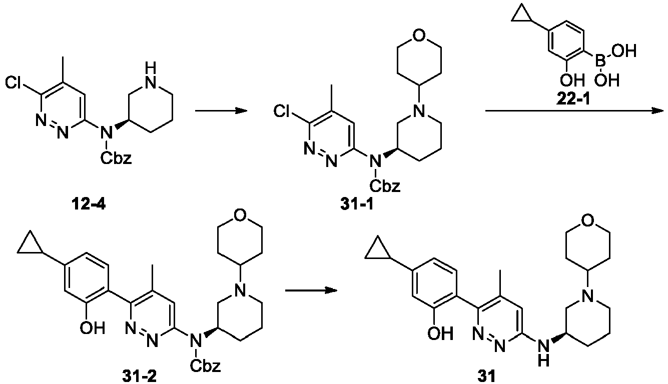

### Step 1

The hydrochloride of compound **12-4** (300 mg, 755 µmol) was dissolved in tetrahydrofuran (10 mL), and tetrahydropyran-4-one (227 mg, 2.27 mmol) was added thereto. The reaction mixture was stirred at 70°C for 12 hours. Then sodium triacetoxyborohydride (320 mg, 1.51 mmol) was added thereto at 0°C, and the reaction mixture was stirred at 25°C for 1 hour. The pH of the reaction mixture was neutralized to 8 with saturated sodium bicarbonate solution, and the reaction mixture was extracted with ethyl acetate (10 mL × 3). The organic phases were combined, dried over anhydrous sodium sulfate, filtered, and concentrated under reduced pressure. The crude product was separated by silica gel column chromatography (petroleum ether/ethyl acetate, 100/1 to 0/1, V/V) to obtain compound **31-1.** MS-ESI calculated for [M+H]⁺ 445, found 445.

### Step 2

Compound **31-1** (200 mg, 449 µmol) was dissolved in dioxane (2 mL) and water (0.4 mL). Compound **22-1** (96.0 mg, 539 µmol), [1,1'-bis(diphenylphosphino)ferrocene]dichloropalladium(II) (32.9 mg, 45.0 µmol), and potassium carbonate (155 mg, 1.12 mmol) were added thereto. The system was replaced with nitrogen three times, and the reaction mixture was stirred at 100°C for 12 hours. The reaction mixture was directly concentrated under reduced pressure, and the residue was separated by thin-layer chromatography (petroleum ether/ethyl acetate, 100/1 to 0/1, V/V) to obtain compound **31-2.** MS-ESI calculated for [M+H]⁺ 543, found 543.

### Step 3

Compound **31-2** (74.0 mg, 136 µmol) was dissolved in tetrahydrofuran (5 mL), and the mixture was added with wet palladium/carbon (50.0 mg, purity: 10%), replaced with hydrogen three times. The reaction mixture was stirred at 25°C and a pressure of 15 Psi for 12 hours. The reaction mixture was filtered, and the filtrate was directly concentrated under reduced pressure to obtain the crude product of compound **2.** The crude product was separated by preparative high performance liquid chromatography (chromatographic column: Phenomenex C18 80 × 40 mm × 3 µm; mobile phase: 0.05% ammonia solution-acetonitrile; gradient: acetonitrile: 38% to 68%, 8 min) to obtain compound **31.** ¹H NMR (400 MHz, CD₃OD) δ 7.08 (d, *J*=8.0 Hz, 1H), 6.74 (d, *J*=1.6 Hz, 1H), 6.67 (dd, *J*=1.6, 8.0 Hz, 1H), 6.61 (d, *J*=1.6 Hz, 1H), 4.11-4.06 (m, 1H), 4.00-3.97 (m, 2H), 3.43-3.37 (m, 2H), 3.20-3.13 (m, 1H), 2.85-2.82 (m, 1H), 2.61-2.54 (m, 1H), 2.36 (t, *J*=9.6 Hz, 1H), 2.23 (t, *J*=9.6 Hz, 1H), 2.13 (s, 3H), 2.03-1.98 (m, 1H), 1.91-1.86 (m, 1H), 1.85-1.79 (m, 3H), 1.72-1.54 (m, 3H), 1.45-1.41 (m, 1H), 0.99-0.93 (m, 2H), 0.71-0.67 (m, 2H). MS-ESI calculated for [M+H]⁺ 409, found 409.

### Example 32

### Synthetic Route:

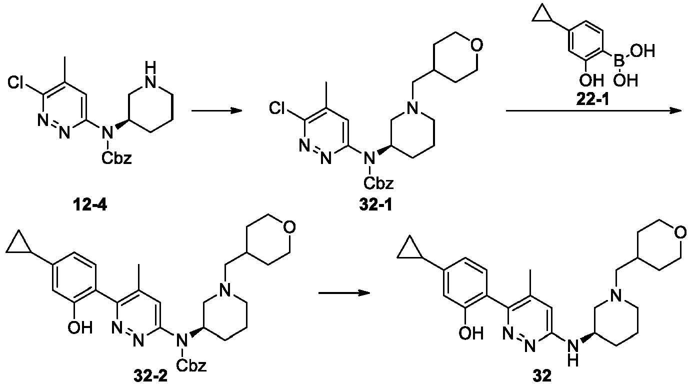

### Step 1

The hydrochloride of compound **12-4** (500 mg, 1.26 mmol) was dissolved in tetrahydrofuran (8 mL), and tetrahydropyran-4-one (287 mg, 2.52 mmol) was added thereto. The reaction mixture was stirred at 70°C for 3 hours. Then sodium triacetoxyborohydride (533 mg, 2.52 mmol) was added thereto at 0°C, and the reaction mixture was stirred at 25°C for 1 hour. The pH of the reaction mixture was neutralized to 8 with saturated sodium bicarbonate solution, and the reaction mixture was extracted with ethyl acetate (10 mL × 3). The organic phases were combined, dried over anhydrous sodium sulfate, filtered, and concentrated under reduced pressure. The crude product was separated by silica gel column chromatography (petroleum ether/ethyl acetate, 100/1 to 0/1, V/V) to obtain compound **32-1.** MS-ESI calculated for [M+H]⁺ 459, found 459.

### Step 2

Compound **32-1** (400 mg, 872 µmol) was dissolved in dioxane (5 mL) and water (1 mL), and intermediate **C** (186 mg, 1.05 µmol), [1,1'-bis(diphenylphosphino)ferrocene]dichloropalladium(II) (63.8 mg, 87.2 µmol), and potassium carbonate (301 mg, 2.18 mmol) were added thereto. The system was replaced with nitrogen three times, and the reaction mixture was stirred at 100°C for 12 hours. The reaction mixture was directly concentrated under reduced pressure, and the residue was separated by thin-layer chromatography (petroleum ether/ethyl acetate, 100/1 to 0/1, V/V) to obtain compound **32-2.** MS-ESI calculated for [M+H]⁺ 543, found 543.

### Step 3

Compound **32-2** (200 mg, 359 µmol) was dissolved in tetrahydrofuran (5 mL), and the mixture was added with wet palladium/carbon (50.0 mg, purity: 10%), replaced with hydrogen three times. The reaction mixture was stirred at 65°C and a pressure of 15 Psi for 12 hours. The reaction mixture was filtered, and the filtrate was directly concentrated under reduced pressure to obtain the crude product of compound **3.** The crude product was separated by preparative high performance liquid chromatography (chromatographic column: Phenomenex C18 80 × 40 mm × 3 µm; mobile phase: 0.05% ammonia solution-acetonitrile; gradient: acetonitrile: 43% to 73%, 8 min) to obtain compound **32.** ¹H NMR (400 MHz, CD₃OD) δ 7.08 (d, *J*=8.0 Hz, 1H), 6.75 (s, 1H), 6.66 (dd, *J*=1.6, 8.0 Hz, 1H), 6.61 (s, 1H), 4.10-4.06 (m, 1H), 3.93-3.90 (m, 2H), 3.44-3.37 (m, 2H), 2.94 (s, 1H), 2.62 (s, 1H), 2.23-2.13 (m, 7H), 1.91-1.64 (m, 7H), 1.44-1.42 (m, 1H), 1.29-1.19 (m, 2H), 0.99-0.95 (m, 2H), 0.75-0.67 (m, 2H). MS-ESI calculated for [M+H]⁺ 423, found 423.

### Example 33

### Synthetic Route:

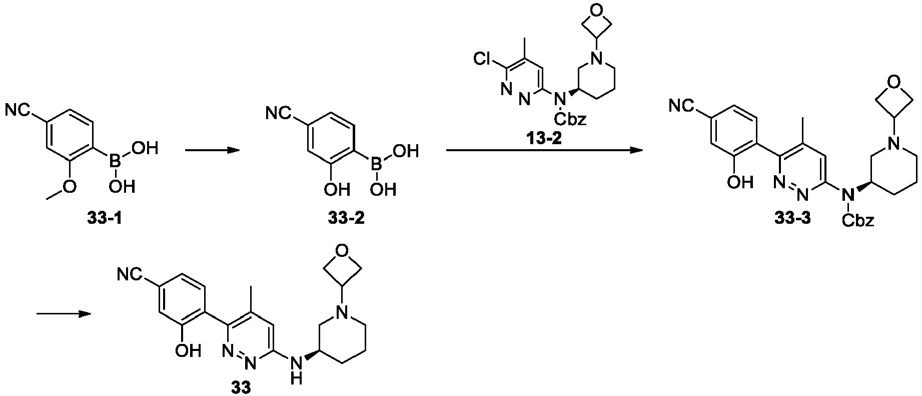

### Step 1

Compound **33-1** (1.00 g, 5.65 mmol) was dissolved in dichloromethane (8 mL), and boron tribromide (2.83 g, 11.3 mmol) was added thereto at 0°C. The reaction mixture was stirred at 0°C for 0.5 hours. The reaction mixture was quenched with water (10 mL), extracted with dichloromethane (20 mL × 3), and the organic phases were combined, dried over anhydrous sodium sulfate, filtered, and concentrated under reduced pressure. The crude product was separated by silica gel column chromatography (dichloromethane/methanol, 100/1 to 10/1, V/V) to obtain compound **33-2.** ¹H NMR (400 MHz, CDCl₃) δ 7.64 (s, 1H), 7.14 (dd, *J*=1.2, 7.6 Hz, 1H), 7.05 (s, 1H).

### Step 2

Compound **33-2** (164 mg, 1.01 mmol) was dissolved in dioxane (5 mL) and water (1 mL). Compound **13-2** (350 mg, 840 µmol), [1,1'-bis(diphenylphosphino)ferrocene]dichloropalladium(II) (61.4 mg, 84.0 µmol), and potassium carbonate (290 mg, 2.10 mmol) were added thereto. The system was replaced with nitrogen three times, and the reaction mixture was stirred at 100°C for 12 hours. The reaction mixture was directly concentrated under reduced pressure, and the residue was separated by silica gel column chromatography (petroleum ether/ethyl acetate, 100/1 to 0/1, V/V) to obtain compound **33-3.** MS-ESI calculated for [M+H]⁺ 500, found 500.

### Step 3

Compound **33-3** (400 mg, 801 µmol) was dissolved in tetrahydrofuran (10 mL), and the mixture was added with wet palladium/carbon (40.0 mg, purity: 10%), replaced with hydrogen three times. The reaction mixture was stirred at 25°C and a pressure of 15 Psi for 12 hours. The reaction mixture was filtered, and the filtrate was directly concentrated under reduced pressure to obtain the crude product of compound **33.** The crude product was separated by preparative high performance liquid chromatography (chromatographic column: Phenomenex C18 80 × 40 mm × 3 µm; mobile phase: 0.05% ammonia solution-acetonitrile; gradient: acetonitrile: 15% to 45%, 8 min) to obtain compound **33.** ¹H NMR (400 MHz, CD₃OD) δ 7.31 (d, *J*=7.2 Hz, 1H), 7.14-7.11 (m, 2H), 6.77 (s, 1H), 4.69-4.65 (m, 3H), 4.61 (t, *J*=6.4 Hz, 1H), 4.16-4.12 (m, 1H), 3.55-3.49 (m, 1H), 2.92-2.90 (m, 1H), 2.53 (s, 1H), 2.13 (s, 3H), 2.08-1.94 (m, 3H), 1.84-1.81 (m, 1H), 1.74-1.65 (m, 1H), 1.48-1.41 (m, 1H). MS-ESI calculated for [M+H]⁺ 366, found 366.

### Example 34

### Synthetic Route:

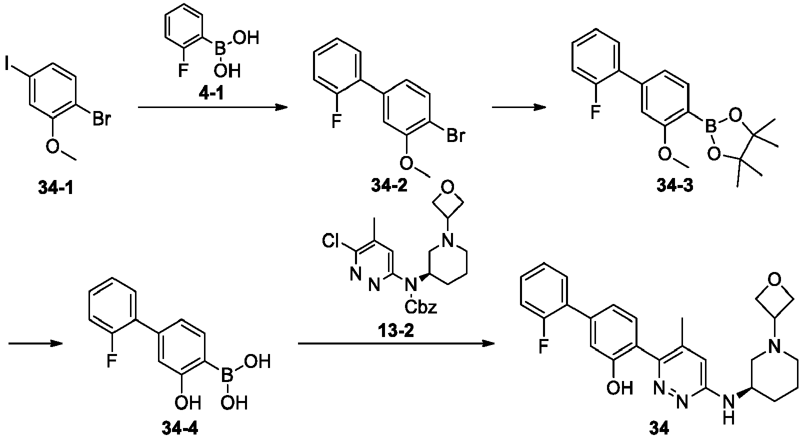

### Step 1

Compound **34-1** (89.4 mg, 0.639 mmol), compound **4-1** (200 mg, 0.639 mmol), potassium phosphate (271 mg, 1.28 mmol), and [1,1'-bis(diphenylphosphino)ferrocene]dichloropalladium(II) dichloromethane complex (52.2 mg, 63.9 µmol) were dissolved in 1,4-dioxane (5 mL) and water (0.5 mL), and the reaction mixture was stirred at 80°C for 12 hours under nitrogen atmosphere. The reaction mixture was filtered, and the filtrate was concentrated under reduced pressure. The crude product was purified by thin-layer chromatography (petroleum ether/ethyl acetate, 20/1, V/V) to obtain compound **34-2.** ¹H NMR (400MHz, CDCl₃) δ 7.53 (d, *J*=8.0 Hz, 1H), 7.38-7.33 (m, 1H), 7.30-7.24 (m, 1H), 7.16-7.12 (m, 1H), 7.11-7.06 (m, 1H), 7.01-7.00 (m, 1H), 6.95-6.93 (m, 1H), 3.87 (s, 3H).

### Step 2

Compound **34-2** (163 mg, 580 µmol), bis(pinacolato)diboron (177 mg, 696 µmol), potassium acetate (114 mg, 1.16 mmol), and bis(triphenylphosphine)palladium(II) chloride (40.7 mg, 58.0 µmol) were dissolved in 1,4-dioxane (5 mL), and the system was replaced with nitrogen three times. The reaction mixture was stirred at 90°C for 12 hours. The reaction mixture was filtered, and the filtrate was concentrated under reduced pressure. The crude product was purified by thin-layer chromatography (petroleum ether/ethyl acetate, 10/1, V/V0) to obtain compound **34-3.** ¹H NMR (400 MHz, CDCl₃) δ 7.78 (d, *J*=7.6 Hz, 1H), 7.50-7.46 (m, 1H), 7.38-7.32 (m, 1H), 7.25-7.21 (m, 1H), 7.19-7.14 (m, 2H), 7.07 (s, 1H), 3.90 (s, 3H), 1.39 (s, 12H).

### Step 3

Compound **34-3** (144 mg, 439 µmol) was dissolved in dichloromethane (5 mL), and boron tribromide (84.6 µL, 878 µmol) was added dropwise thereto at 0°C. The reaction mixture was stirred at 0°C for 0.5 hours. The reaction mixture was quenched with ice water (30 mL), extracted with dichloromethane (30 mL × 2), and the combined organic phases were dried over anhydrous sodium sulfate, filtered, and concentrated under reduced pressure. The crude product was purified by thin-layer chromatography (dichloromethane/methanol, 20/1, V/V) to obtain compound **34-4.** ¹H NMR (400 MHz, CD₃OD) δ 7.69 (d, *J*=4.4 Hz, 1H), 7.50-7.46 (m, 1H), 7.38-7.36 (m, 1H), 7.27 -7.23 (m, 1H), 7.21-7.16 (m, 1H), 7.04-6.98 (m, 2H).

### Step 4

Compound **34-4** (100 mg, 0.431 mmol), compound **13-2** (120 mg, 0.288 mmol), potassium carbonate (119 mg, 0.864 mmol), and [1,1'-bis(diphenylphosphino)ferrocene]dichloropalladium(II) (21.1 mg, 28.8 µmol) were dissolved in 1,4-dioxane (5 mL) and water (1 mL), and the reaction mixture was stirred at 110°C for 12 hours under nitrogen atmosphere. The reaction mixture was filtered, and the filtrate was concentrated under reduced pressure, and the residue was purified by thin-layer chromatography (dichloromethane/methanol, 10/1, V/V) and preparative high performance liquid chromatography (Waters Xbridge 150 × 25 mm × 5 µm; mobile phase A: 10 mmol/L ammonium bicarbonate aqueous solution; mobile phase B: acetonitrile; B%: 37% to 67%, 10 min) to obtain compound **34.** ¹H NMR (400 MHz, CD₃OD) δ 7.55-7.51 (m, 1H), 7.42-7.37 (m, 1H), 7.33-7.31 (m, 1H), 7.30-7.26 (m, 1H), 7.24-7.19 (m, 1H), 7.16-7.14 (m, 2H), 6.82 (d, *J*=0.8 Hz, 1H), 4.72-4.69 (m, 3H), 4.65-4.59 (m, 1H), 4.21-4.14 (m, 1H), 3.59-3.52 (m, 1H), 2.96-2.93 (m, 1H), 2.58-2.56 (m, 1H), 2.22 (d, *J*=0.8 Hz, 3H), 2.14-2.10 (m, 1H), 2.03-2.01 (m, 2H), 1.88-1.83 (m, 1H), 1.77-1.67 (m, 1H), 1.55-1.45 (m, 1H). MS-ESI calculated for [M+H]⁺ 435, found 435.

### Example 35

### Synthetic Route:

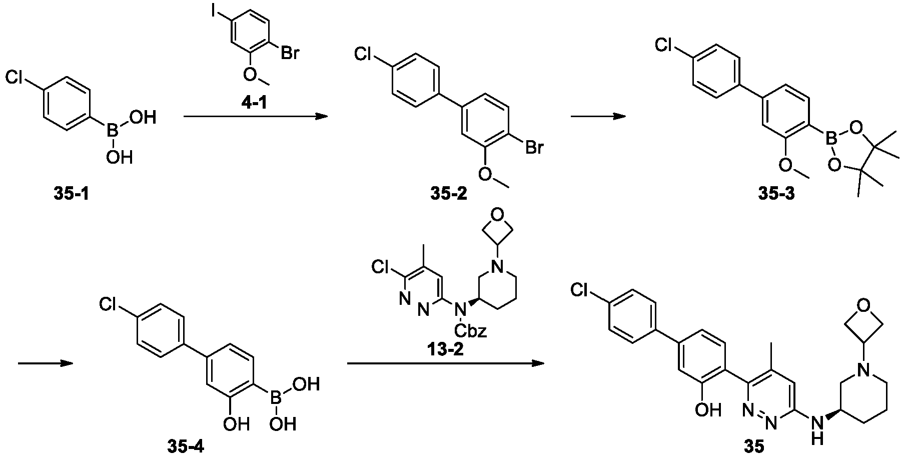

### Step 1

Compound **35-1** (999 mg, 6.39 mmol), compound **4-1** (2 g, 6.39 mmol), potassium phosphate (2.71 g, 12.8 mmol), and [1,1'-bis(diphenylphosphino)ferrocene]dichloropalladium(II) dichloromethane complex (522 mg, 0.639 mmol) were dissolved in 1,4-dioxane (20 mL) and water (2 mL), and the reaction mixture was stirred at 80°C for 12 hours under nitrogen atmosphere. The reaction mixture was filtered, and the filtrate was concentrated under reduced pressure. The crude product was purified by silica gel column chromatography (petroleum ether/ethyl acetate, 20/1, V/V) to obtain compound **35-2.** ¹H NMR (400MHz, CDCl₃) δ 7.61 (d, *J*=8.4 Hz, 1H), 7.52-7.50 (m, 2H), 7.45-7.42 (m, 2H), 7.06-7.02 (m, 2H), 3.99 (s, 3H).

### Step 2

Compound **35-2** (1.78 g, 5.98 mmol), bis(pinacolato)diboron (1.82 g, 7.18 mmol), potassium acetate (1.17 g, 12.0 mmol), andbis(triphenylphosphine)palladium(II) chloride (420 mg, 0.598 mmol) were dissolved in 1,4-dioxane (30 mL), and the reaction mixture was stirred at 90°C for 12 hours under nitrogen atmosphere. The reaction mixture was filtered, and the filtrate was concentrated under reduced pressure. The crude product was purified by silica gel column chromatography (petroleum ether/ethyl acetate, 10/1, V/V) to obtain compound **35-3.** ¹H NMR (400 MHz, CDCl₃) δ 7.77 (d, *J*=7.6 Hz, 1H), 7.56-7.54 (m, 2H), 7.44-7.42 (m, 2H), 7.17-7.14 (m, 1H), 7.03 (d, *J*=1.2 Hz, 1H), 3.93 (s, 3H), 1.39 (s, 12H).

### Step 3

Compound **35-3** (800 mg, 2.32 mmol) was dissolved in dichloromethane (8 mL), and boron tribromide (447 µL, 4.64 mmol) was added dropwise thereto at 0°C. The reaction mixture was stirred at 0°C for 0.5 hours. The reaction mixture was quenched with ice water (30 mL), extracted with dichloromethane (30 mL × 2), and the combined organic phases were dried over anhydrous sodium sulfate, filtered, and the filtrate was concentrated under reduced pressure. The crude product was purified by thin-layer chromatography (dichloromethane/methanol, 20/1, V/V) to obtain compound **35-4.** ¹H NMR (400 MHz, CD₃OD) δ 7.71-7.67 (m, 1H), 7.64-7.58 (m, 2H), 7.46-7.42 (m, 2H), 7.11 -7.09 (m, 1H), 7.02 (d, *J*=1.6 Hz, 1H).

### Step 4

Compound **35-4** (100 mg, 0.402 mmol), compound **13-2** (150 mg, 0.360 mmol), potassium carbonate (149 mg, 1.08 mmol), and [1,1'-bis(diphenylphosphino)ferrocene]dichloropalladium(II) (26.3 mg, 36.0 µmol) were dissolved in 1,4-dioxane (6 mL) and water (1 mL), and the reaction mixture was stirred at 110°C for 12 hours under nitrogen atmosphere. The reaction mixture was filtered, and the filtrate was concentrated under reduced pressure, and the residue was separated by thin-layer chromatography (dichloromethane/methanol, 10/1, V/V) to obtain the crude product. Then the crude product was purified by preparative high performance liquid chromatography (Waters Xbridge 150 × 25 mm × 5 µm; mobile phase A: 10 mmol/L ammonium bicarbonate aqueous solution; mobile phase B: acetonitrile; B%: 42% to 72%, 7 min) to obtain compound **35.** ¹H NMR (400 MHz, CD₃OD) δ 7.66-7.64 (m, 2H), 7.48-7.46 (m, 2H), 7.33-7.31 (m, 1H), 7.23-7.21 (m, 1H), 7.16 (d, *J*=0.8 Hz, 1H), 6.81 (s, 1H), 4.72-4.69 (m, 3H), 4.65-4.62 (m, 1H), 4.21-4.14 (m, 1H), 3.57-3.52 (m, 1H), 2.96-2.92 (m, 1H), 2.58-2.56 (m, 1H), 2.20 (s, 3H), 2.13-2.11 (m, 1H), 2.06-1.94 (m, 2H), 1.88-1.82 (m, 1H), 1.77-1.65 (m, 1H), 1.56-1.46 (m, 1H). MS-ESI calculated for [M+H]⁺ 451, found 451.

### Example 36

### Synthetic Route:

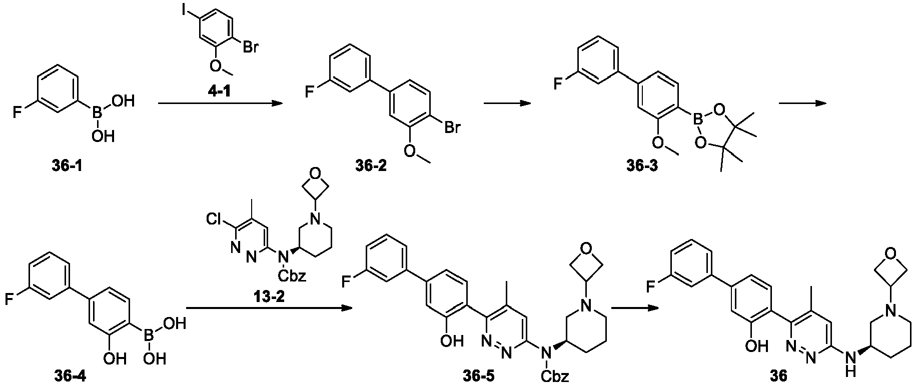

### Step 1

Compound **36-1** (447 mg, 3.20 mmol) was dissolved in 1,4-dioxane (15 mL) and water (1.5 mL). Compound **4-1** (1.0 g, 3.20 mmol), [1,1'-bis(diphenylphosphino)ferrocene]dichloropalladium(II) dichloromethane complex (261 mg, 0.32 mmol), and potassium phosphate (1.36 g, 6.40 mmol) were added thereto. The system was replaced with nitrogen three times, and the reaction mixture was stirred at 80°C for 12 hours. The reaction mixture was directly concentrated under reduced pressure, and the crude product was separated by silica gel column chromatography (petroleum ether/ethyl acetate, 100/1 to 10/1, V/V) to obtain compound **36-2.** ¹H NMR (400MHz, CDCl₃) δ 7.62 (d, *J*=8.0 Hz, 1H), 7.49-7.39 (m, 1H), 7.38-7.33 (m, 1H), 7.30-7.26 (m, 1H), 7.13-7.03 (m, 3H), 3.99 (s, 3H).

### Step 2

Compound **36-2** (925 mg, 3.29 mmol), bis(pinacolato)diboron (1.0 g, 3.95 mmol), potassium acetate (646 mg, 6.58 mmol), and [1,1'-bis(diphenylphosphino)ferrocene]dichloropalladium(II) dichloromethane complex (231 mg, 0.33 mmol) were dissolved in dioxane (15 mL), and the reaction mixture was stirred at 90°C for 12 hours under nitrogen atmosphere. The reaction mixture was concentrated under reduced pressure to obtain compound **36-3.** ¹H NMR (400MHz, CDCl₃) δ 7.76-7.72 (m, 1H), 7.68 (d, *J*=7.6 Hz, 1H), 7.52 (d, *J*=8.0 Hz, 1H), 7.11-7.05 (m, 3H), 6.87-6.82 (m, 1H), 3.89 (s, 3H), 1.29 (s, 12H).

### Step 3

Compound **36-3** (674 mg, 2.05 mmol) was dissolved in dichloromethane (10 mL), and boron tribromide (0.4 mL, 4.11 mmol) was added thereto at 0°C. The reaction mixture was stirred at 0°C for 0.5 hours. The reaction mixture was added with water (20 mL) and extracted with ethyl acetate (20 mL × 3), and the organic phases were combined, dried over anhydrous sodium sulfate, filtered, and concentrated under reduced pressure. The crude product was separated by silica gel column chromatography (dichloromethane/methanol, 100/1 to 10/1, V/V) to obtain compound **36-4.** ¹H NMR (400MHz, CD₃OD) δ 7.71 (s, 1H), 7.51-7.42 (m, 2H), 7.39-7.31 (m, 1H), 7.13 (dd, *J*=1.6, 7.8 Hz, 1H), 7.11-7.06 (m, 1H), 7.05 (d, *J*=1.6 Hz, 1H).

### Step 4

Compound **36-4** (114 mg, 0.49 mmol) was dissolved in 1,4-dioxane (4 mL) and water (0.4 mL). Compound **13-2** (157 mg, 0.38 mmol), [1,1'-bis(diphenylphosphino)ferrocene]dichloropalladium(II) (28 mg, 38 µmol), and potassium carbonate (104 mg, 0.76 mmol) were added thereto. The system was replaced with nitrogen three times, and the reaction mixture was stirred at 110°C for 12 hours. The reaction mixture was directly concentrated under reduced pressure, and the residue was separated by thin-layer chromatography (petroleum ether/ethyl acetate, 100/1 to 0/1, V/V) to obtain compound **36-5.** MS-ESI calculated for [M+H]⁺ 569, found 569.

### Step 5

Compound **36-5** (200 mg, 359 µmol) was dissolved in methanol (1 mL), and the mixture was added with wet palladium/carbon (5.0 mg, purity: 10%), replaced with hydrogen three times. The reaction mixture was stirred at 80°C and a pressure of 15 Psi for 24 hours. The reaction mixture was filtered, and the filtrate was directly concentrated under reduced pressure to obtain the crude product of compound **3.** The crude product was separated by preparative high performance liquid chromatography (chromatographic column: Waters Xbridge 150 × 25 mm × 5 µm; mobile phase: 10 mmol/L ammonium bicarbonate aqueous solution-acetonitrile; gradient: acetonitrile: 38% to 68%, 10 min) to obtain compound **36.** ¹H NMR (400 MHz, CD₃OD) δ 7.53-7.45 (m, 2H), 7.40-7.38 (m, 1H), 7.33 (d, *J*=8.0 Hz, 1H), - 7.24 (dd, *J*=1.8, 8.0 Hz, 1H), 7.18 (d, *J*=1.6 Hz, 1H), 7.13-7.08 (m, 1H), 6.82 (s, 1H), 4.73-4.66 (m, 3H), 4.65-4.58 (m, 1H), 4.18 (s, 1H), 3.65-3.50 (m, 1H), 2.94 (s, 1H), 2.56 (s, 1H), 2.21 (s, 3H), 2.12 (s, 1H), 2.00 (s, 2H), 1.90-1.80 (m, 1H), 1.78-1.65 (m, 1H), 1.55-1.40 (m, 1H). MS-ESI calculated for [M+H]⁺ 435, found 435.

### Example 37

### Synthetic Route:

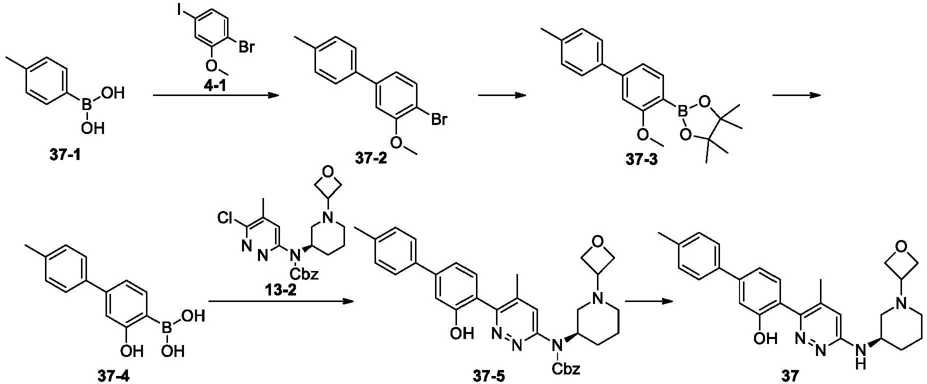

### Step 1

Compound **37-1** (434 mg, 3.20 mmol) was dissolved in 1,4-dioxane (15 mL) and water (1.5 mL). Compound **4-1** (1.0 g, 3.20 mmol), [1,1'-bis(diphenylphosphino)ferrocene]dichloropalladium(II) dichloromethane complex (261 mg, 0.32 mmol), and potassium phosphate (1.36 g, 6.40 mmol) were added thereto. The system was replaced with nitrogen three times, and the reaction mixture was stirred at 80°C for 12 hours. The reaction mixture was directly concentrated under reduced pressure, and the crude product was separated by silica gel column chromatography (petroleum ether/ethyl acetate, 100/1 to 10/1, V/V) to obtain compound **37-2.** ¹H NMR (400 MHz, CDCl₃) δ 7.48 (d, *J*=8.1 Hz, 1H), 7.41-7.35 (m, 2H), 7.20-7.13 (m, 2H), 6.99 (d, *J*=2.0 Hz, 1H), 6.95 (dd, *J*=2.1, 8.1 Hz, 1H), 3.86 (s, 3H), 2.31 (s, 3H).

### Step 2

Compound **37-2** (200 mg, 0.72 mmol), bis(pinacolato)diboron (220 mg, 0.87 mmol), potassium acetate (141 mg, 1.44 mmol), and [1,1'-bis(diphenylphosphino)ferrocene]dichloropalladium(II) dichloromethane complex (50 mg, 0.07 mmol) were dissolved in dioxane (5 mL), and the reaction mixture was stirred at 90°C for 12 hours under nitrogen atmosphere. The reaction mixture was concentrated under reduced pressure to obtain compound **37-3.** ¹H NMR (400MHz, CDCl₃) δ = 7.76 (d, *J*=7.6 Hz, 1H), 7.52 (d, *J*=8.2 Hz, 2H), 7.26 (s, 2H), 7.18 (dd, *J*=1.4, 7.6 Hz, 1H), 7.07 (d, *J*=1.2 Hz, 1H), 3.92 (s, 3H), 2.42 (s, 3H), 1.38 (s, 12H).

### Step 3

Compound **37-3** (200 mg, 0.62 mmol) was dissolved in dichloromethane (2 mL), and boron tribromide (0.1 mL, 1.23 mmol) was added thereto at 0°C. The reaction mixture was stirred at 0°C for 0.5 hours. The reaction mixture was added with water (20 mL) and extracted with ethyl acetate (20 mL × 3), and the organic phases were combined, dried over anhydrous sodium sulfate, filtered, and concentrated under reduced pressure. The crude product was separated by silica gel column chromatography (dichloromethane/methanol, 100/1 to 10/1, V/V) to obtain compound **37-4.**

### Step 4

Compound **37-4** (120 mg, 0.53 mmol) was dissolved in 1,4-dioxane (4 mL) and water (0.4 mL). Compound **13-2** (146 mg, 0.35 mmol), [1,1'-bis(diphenylphosphino)ferrocene]dichloropalladium(II) (26 mg, 35 µmol), and potassium carbonate (97 mg, 0.7 mmol) were added thereto. The system was replaced with nitrogen three times, and the reaction mixture was stirred at 110°C for 12 hours. The reaction mixture was directly concentrated under reduced pressure, and the residue was separated by thin-layer chromatography (petroleum ether/ethyl acetate, 100/1 to 0/1, V/V) to obtain compound **37-5.** MS-ESI calculated for [M+H]⁺ 565, found 565.

### Step 5

Compound **37-5** (90 mg, 159 µmol) was dissolved in methanol (1 mL), and the mixture was added with wet palladium/carbon (5.0 mg, purity: 10%), replaced with hydrogen three times. The reaction mixture was stirred at 80°C and a pressure of 15 Psi for 24 hours. The reaction mixture was filtered, and the filtrate was directly concentrated under reduced pressure to obtain the crude product of compound **3.** The crude product was separated by preparative high performance liquid chromatography (chromatographic column: Waters Xbridge 150 × 25 mm × 5 µm; mobile phase: 0.05% ammonia solution-acetonitrile; gradient: acetonitrile: 33% to 63%, 9 min) to obtain compound **37.** ¹H NMR (400 MHz, CD₃OD) δ 7.54 (d, *J*=8.0 Hz, 2H), 7.29 (t, *J*=7.0 Hz, 3H), 7.23-7.19 (m, 1H), 7.16 (d, *J*=1.6 Hz, 1H), 6.81 (s, 1H), 4.74-4.64 (m, 4H), 4.18 (d, *J*=4.6 Hz, 1H), 3.55 (t, *J*=6.6 Hz, 1H), 2.98-2.90 (m, 1H), 2.62-2.52 (m, 1H), 2.40 (s, 3H), 2.21 (s, 3H), 2.18-2.08 (m, 1H), 2.05-1.95 (m, 2H), 1.92-1.80 (m, 1H), 1.78-1.68 (m, 1H), 1.58-1.46 (m, 1H). MS-ESI calculated for [M+H]⁺ 431, found 431.

### Example 38

### Synthetic Route:

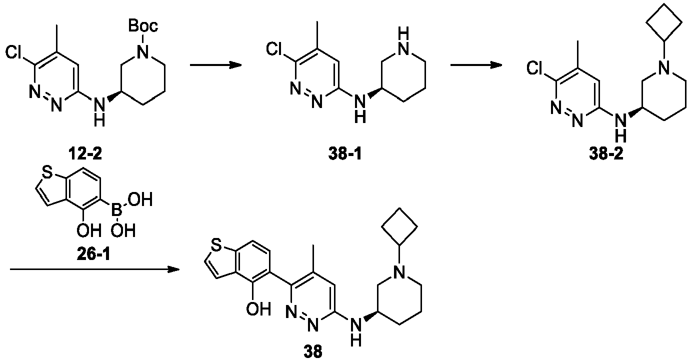

### Step 1

Compound **12-2** (1.7 g, 5.20 mmol) was dissolved in ethyl acetate (5 mL), then 4 M hydrogen chloride/ethyl acetate solution (10 mL) was added thereto, and the reaction mixture was stirred at 25°C for 1 hour. The reaction mixture was directly concentrated to obtain the hydrochloride of compound **38-1.** ¹H NMR (400 MHz, CD₃OD) δ 7.56 (s, 1H), 4.25-4.18 (m, 1H), 3.62-3.58 (m, 1H), 3.40-3.35 (m, 1H), 3.18-3.10 (m, 2H), 2.49 (s, 3H), 2.26-2.22 (m, 1H), 2.18-2.10 (m, 1H), 2.04-1.93 (m, 1H), 1.86-1.76 (m, 1H). MS-ESI calculated for [M+H]⁺ 227, found 227.

### Step 2

The hydrochloride of compound **38-1** (250 mg, 950 µmol) was dissolved in tetrahydrofuran (5 mL), and cyclobutanone (133 mg, 1.90 mmol) was added thereto. The reaction mixture was stirred at 70°C for 12 hours. Then sodium triacetoxyborohydride (403 mg, 1.90 mmol) was added thereto at 0°C, and the reaction mixture was stirred at 25°C for 0.5 hours. The pH of the reaction mixture was neutralized to 8 with saturated sodium bicarbonate solution, and the reaction mixture was extracted with ethyl acetate (10 mL × 3). The organic phases were combined, dried over anhydrous sodium sulfate, filtered, and concentrated under reduced pressure. The crude product was separated by silica gel column chromatography (dichloromethane/methanol, 100/1 to 10/1, V/V) to obtain compound **38-2.** MS-ESI calculated for [M+H]⁺ 281, found 281.

### Step 3

Compound **38-2** (60.0 mg, 214 µmol) was dissolved in dioxane (3 mL) and water (0.6 mL), and intermediate **26-1** (50.0 mg, 256 µmol), [1,1'-bis(diphenylphosphino)ferrocene]dichloropalladium(II) (15.6 mg, 21.4 µmol), and potassium carbonate (73.8 mg, 534 µmol) were added thereto. The system was replaced with nitrogen three times, and the reaction mixture was stirred at 100°C for 12 hours. The reaction mixture was directly concentrated under reduced pressure, and the residue was separated by thin-layer chromatography (dichloromethane/methanol, 100/0 to 10/1, V/V) to obtain the crude product of compound **38.** The crude product was separated by preparative high performance liquid chromatography (chromatographic column: Phenomenx C18 80× 40 mm× 3 µm; mobile phase: 0.05% ammonia solution-acetonitrile; gradient: acetonitrile: 45% to 75%, 8 min) to obtain compound **38.** MS-ESI calculated for [M+H]⁺ 395, found 395. ¹H NMR (400 MHz, CD₃OD) δ 7.59 (d, *J*=5.6 Hz, 1H), 7.50-7.49 (m, 2H), 7.24 (d, *J*=8.4 Hz, 1H), 6.81 (s, 1H), 4.59 (s, 1H), 4.20-4.13 (m, 1H), 3.22 (s, 1H), 3.03 (s, 1H), 2.86 (s, 1H), 2.20 (s, 3H), 2.13-1.87 (m, 7H), 1.80-1.71 (m, 3H), 1.49-1.47 (m, 1H).

### Example 39

### Synthetic Route:

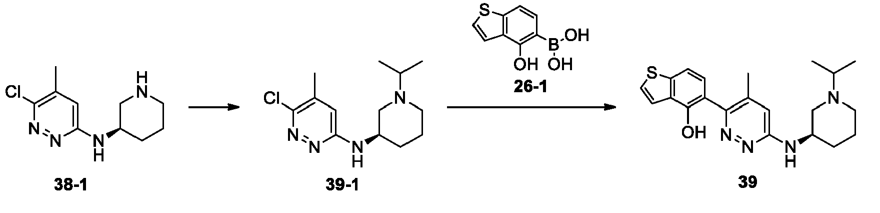

### Step 1

The hydrochloride of compound **38-1** (250 mg, 950 µmol) was dissolved in tetrahydrofuran (5 mL), and acetone (110 mg, 1.90 mmol) was added thereto. The reaction mixture was stirred at 70°C for 12 hours. Then sodium triacetoxyborohydride (403 mg, 1.90 mmol) was added thereto at 0°C, and the reaction mixture was stirred at 25°C for 0.5 hours. The pH of the reaction mixture was neutralized to 8 with saturated sodium bicarbonate solution, and the reaction mixture was extracted with ethyl acetate (10 mL × 3). The organic phases were combined, dried over anhydrous sodium sulfate, filtered, and concentrated under reduced pressure. The crude product was separated by silica gel column chromatography (dichloromethane/methanol, 100/1 to 10/1, V/V) to obtain compound **39-1.** MS-ESI calculated for [M+H]⁺ 269, found 269.

### Step 2

Compound **39-1** (60.0 mg, 223 µmol) was dissolved in dioxane (3 mL) and water (0.6 mL), and intermediate **26-1** (47.6 mg, 246 µmol), [1,1'-bis(diphenylphosphino)ferrocene]dichloropalladium(II) (16.3 mg, 22.3 µmol), and potassium carbonate (77.1 mg, 558 µmol) were added thereto. The system was replaced with nitrogen three times, and the reaction mixture was stirred at 100°C for 12 hours. The reaction mixture was directly concentrated under reduced pressure, and the residue was separated by thin-layer chromatography (dichloromethane/methanol, 100/0 to 10/1, V/V) to obtain the crude product of compound **39.** The crude product was separated by preparative high performance liquid chromatography (chromatographic column: Phenomenx C18 80× 40 mm× 3 µm; mobile phase: 0.05% ammonia solution-acetonitrile; gradient: acetonitrile: 44% to 74%, 8 min) to obtain compound **39.** ¹H NMR (400 MHz, CD₃OD) δ7.59 (d, *J*=5.6 Hz, 1H), 7.50-7.49 (m, 2H), 7.24 (d, *J*=8.0 Hz, 1H), 6.83 (s, 1H), 4.59 (s, 1H), 4.21-4.16 (m, 1H), 3.03-2.94 (m, 2H), 2.55-2.43 (m, 2H), 2.20 (s, 3H), 2.08-2.06 (m, 1H), 1.94-1.90 (m, 1H), 1.81-1.72 (m, 1H), 1.54-1.45 (m, 1H), 1.18 (d, *J*=1.6 Hz, 3H), 1.16 (d, *J*=1.6 Hz, 3H). MS-ESI calculated for [M+H]⁺ 383, found 383.

### Example 40

### Synthetic Route:

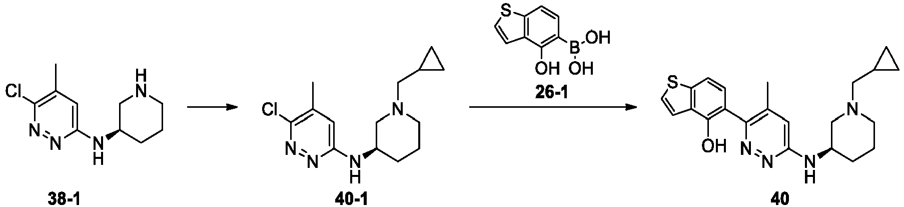

### Step 1

The hydrochloride of compound **38-1** (300 mg, 1.14 mmol) was dissolved in tetrahydrofuran (5 mL), and cyclopropanecarboxaldehyde (160 mg, 2.28 mmol) was added thereto. The reaction mixture was stirred at 70°C for 12 hours. Then sodium triacetoxyborohydride (483 mg, 2.28 mmol) was added thereto at 0°C, and the reaction mixture was stirred at 25°C for 0.5 hours. The pH of the reaction mixture was neutralized to 8 with saturated sodium bicarbonate solution, and the reaction mixture was extracted with ethyl acetate (10 mL × 3). The organic phases were combined, dried over anhydrous sodium sulfate, filtered, and concentrated under reduced pressure. The crude product was separated by silica gel column chromatography (dichloromethane/methanol, 100/1 to 10/1, V/V) to obtain compound **40-1.** MS-ESI calculated for [M+H]⁺ 281, found 281.

### Step 2

Compound **40-1** (60.0 mg, 214 µmol) was dissolved in dioxane (2 mL) and water (0.4 mL), and intermediate **26-1** (45.6 mg, 235 µmol), [1,1'-bis(diphenylphosphino)ferrocene]dichloropalladium(II) (15.6 mg, 21.4 µmol), and potassium carbonate (73.8 mg, 534 µmol) were added thereto. The system was replaced with nitrogen three times, and the reaction mixture was stirred at 100°C for 12 hours. The reaction mixture was directly concentrated under reduced pressure, and the residue was separated by thin-layer chromatography (dichloromethane/methanol, 100/0 to 10/1, V/V) to obtain the crude product of compound **40.** The crude product was separated by preparative high performance liquid chromatography (chromatographic column: Phenomenx C18 80× 40 mm× 3 µm; mobile phase: 0.05% ammonia solution-acetonitrile; gradient: acetonitrile: 44% to 74%, 8 min) to obtain compound **40.** ¹H NMR (400 MHz, CD3OD) δ 7.59 (d, *J*=5.6 Hz, 1H), 7.50-7.49 (m, 2H), 7.24 (d, *J*=8.0 Hz, 1H), 6.83 (s, 1H), 4.24-4.20 (m, 1H), 4.60 (s, 2H), 3.44 (br s, 1H), 3.09 (br s, 1H), 2.56-2.37 (m, 2H), 2.20 (s, 3H), 2.11-2.08 (m, 1H), 1.94-1.91 (m, 1H), 1.82-1.76 (m, 1H), 1.52-1.50 (m, 1H), 0.99-0.97 (m, 1H), 0.64-0.59 (m, 2H), 0.26-0.22 (m, 2H). MS-ESI calculated for [M+H]⁺ 395, found 395.

### Example 41

### Synthetic Route:

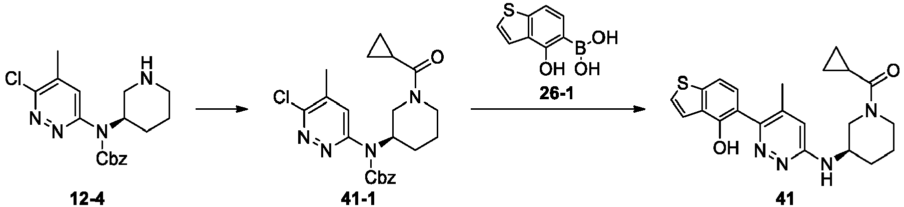

### Step 1

The hydrochloride of compound **12-4** (250 mg, 629 µmol) was dissolved in dichloromethane (5 mL). Triethylamine (191 mg, 1.89 mmol) and cyclopropanecarbonyl chloride (132 mg, 1.26 mmol) were added thereto, and the reaction mixture was stirred at 25°C for 1 hour. The pH of the reaction mixture was neutralized to 8 with saturated sodium bicarbonate solution, and the reaction mixture was extracted with ethyl acetate (10 mL × 3). The organic phases were combined, dried over anhydrous sodium sulfate, filtered, and concentrated under reduced pressure. The crude product was separated by silica gel column chromatography (petroleum ether/ethyl acetate, 100/1 to 0/1, V/V) to obtain compound **41-1.** MS-ESI calculated for [M+H]⁺ 429, found 429.

### Step 2

Compound **41-1** (120 mg, 280 µmol) was dissolved in dioxane (2 mL) and water (0.4 mL), and intermediate **26-1** (59.7 mg, 308 µmol), [1,1'-bis(diphenylphosphino)ferrocene]dichloropalladium(II) (20.5 mg, 28.0 µmol), and potassium carbonate (96.7 mg, 699 µmol) were added thereto. The system was replaced with nitrogen three times, and the reaction mixture was stirred at 100°C for 12 hours. The reaction mixture was directly concentrated under reduced pressure, and the residue was separated by thin-layer chromatography (dichloromethane/methanol, 100/0 to 10/1, V/V) to obtain the crude product of compound **41.** The crude product was separated by preparative high performance liquid chromatography (chromatographic column: Phenomenx C18 80× 40 mm× 3 µm; mobile phase: 0.05% ammonia solution-acetonitrile; gradient: acetonitrile: 28% to 58%, 8 min) to obtain compound **41.** ¹H NMR (400 MHz, CD₃OD) δ 7.59 (d, *J*=5.2 Hz, 1H), 7.50-7.46 (m, 2H), 7.26 (d, *J*=8.2 Hz, 1H), 6.95 (s, 1H), 4.06-3.90 (m, 3H), 3.47-3.36 (m, 1H), 2.24 (s, 3H), 2.19-2.13 (m, 1H), 1.93-1.88 (m, 2H), 1.81-1.72 (m, 1H), 1.70-1.61 (m, 1H), 1.29 (s, 1H), 0.92-0.88 (m, 2H), 0.81-0.79 (m, 2H). MS-ESI calculated for [M+H]⁺ 409, found 409.

### Example 42

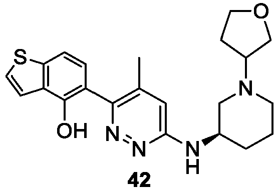

### Synthetic Route:

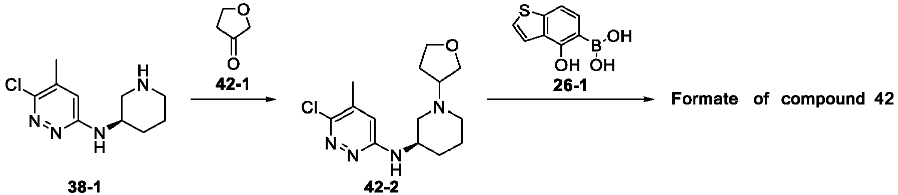

### Step 1

The hydrochloride of compound **38-1** (240 mg, 912 µmol) and compound **42-1** (120 mg, 1.39 mmol) were dissolved in tetrahydrofuran (6 mL). The reaction mixture was stirred at 70°C for 12 hours, and cooled to 0°C, and sodium triacetoxyborohydride (387 mg, 1.82 mmol) was added thereto. The reaction mixture was stirred at 25°C for 1 hour and then at 70°C for 12 hours. The reaction mixture was filtered, and the filtrate was concentrated under reduced pressure. The crude product was separated by silica gel column chromatography (dichloromethane/methanol, 20/1, V/V) to obtain compound **42-2.** MS-ESI calculated for [M+H]⁺ 297, found 297.

### Step 2

Compound **42-2** (80 mg, 0.149 mmol), compound **26-1** (70 mg, 0.361 mmol), potassium carbonate (61.8 mg, 0.447 mmol), and [1,1'-bis(diphenylphosphino)ferrocene]dichloropalladium(II) (21.8 mg, 29.8 µmol) were dissolved in N,N-dimethylformamide (5 mL) and water (1 mL), and the reaction mixture was stirred and reacted at 110°C for 12 hours under nitrogen atmosphere. The reaction mixture was filtered, and the filtrate was concentrated under reduced pressure, and the residue was purified by preparative high performance liquid chromatography (Phenomenex C18 75 × 30 mm × 3 µm; mobile phase A: 0.225% formic acid aqueous solution; mobile phase B: acetonitrile; B%: 2% to 32%, 2 min) to obtain the formate of compound **42.** ¹H NMR (400 MHz, CD₃OD) δ 8.43 (s, 1H), 7.62 (d, *J*=5.6 Hz, 1H), 7.54-7.52 (m, 2H), 7.25 (d, *J*=8.0 Hz, 1H), 6.89 (s, 1H), 4.27-4.22 (m, 1H), 4.04-3.98 (m, 1H), 3.95-3.84 (m, 2H), 3.78-3.72 (m, 1H), 3.57-3.38 (m, 2H), 3.12-2.98 (m, 1H), 2.74-2.68 (m, 1H), 2.65-2.58 (m, 1H), 2.29-2.25 (m, 1H), 2.22 (s, 3H), 2.11-2.03 (m, 2H), 2.02-1.96 (m, 1H), 1.87-1.78 (m, 1H), 1.66-1.58 (m, 1H). MS-ESI calculated for [M+H]⁺ 411, found 411.

### Example 43

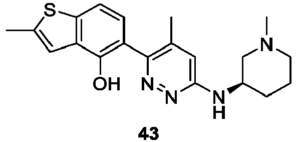

### Synthetic Route:

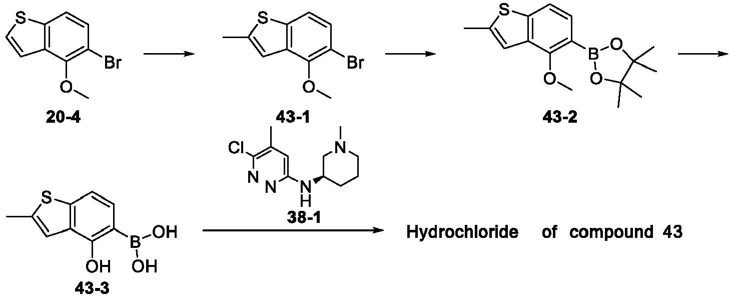

### Step 1

Compound **20-4** (4.19 g, 24.7 mmol) was dissolved in tetrahydrofuran (50 mL), and a mixed solution of lithium diisopropylamide 2M tetrahydrofuran in n-heptane (17.2 mL, 34.5 mmol) was added thereto at 0°C, and the reaction mixture was stirred at 0°C for 0.5 hours. The reaction mixture was added with iodomethane (23.4 g, 74.0 mmol), stirred at 25°C for 12 hours. The reaction mixture was added with water (50 mL) and extracted with ethyl acetate (50 mL × 3), and the organic phases were combined, dried over anhydrous sodium sulfate, filtered, and concentrated under reduced pressure. The crude product was separated by silica gel column chromatography (petroleum ether/ethyl acetate, 100/1 to 20/1, V/V) to obtain compound **43-1.** ¹H NMR (400MHz, CDCl₃) δ 7.41-7.32 (m, 2H), 7.10 (s, 1H), 3.97 (s, 3H), 2.61 (d, *J*=1.0 Hz, 3H).

### Step 2

Compound **43-1** (3.7 g, 14.4 mmol) was dissolved in dioxane (100 mL), and bis(pinacolato)diboron (5.48 g, 21.6 mmol), potassium acetate (2.82 g, 28.8 mmol), and [1,1'-bis(diphenylphosphino)ferrocene]dichloropalladium(II) (1.01 g, 1.44 mmol) were added thereto. The system was replaced with nitrogen three times, and the reaction mixture was stirred at 100°C for 12 hours. The reaction mixture was directly concentrated under reduced pressure, and the residue was separated by silica gel column chromatography (petroleum ether/ethyl acetate, 100/1 to 10/1, V/V) to obtain compound **43-2.** ¹H NMR (400MHz, CDCl₃) δ 7.59 (d, *J*=8.4 Hz, 1H), 7.51-7.47 (m, 1H), 7.13 (d, *J*=1.0 Hz, 1H), 3.74-3.69 (m, 3H), 2.58 (d, *J*=1.2 Hz, 3H), 1.38 (s, 12H).

### Step 3

Compound **43-2** (3.5 g, 11.5 mmol) was dissolved in dichloromethane (20 mL), and boron tribromide (2.22 mL, 23 mmol) was added thereto at 0°C. The reaction mixture was stirred at 0°C for 0.5 hours. The reaction mixture was added with water (30 mL) and extracted with dichloromethane (30 mL × 3), and the organic phases were combined, dried over anhydrous sodium sulfate, filtered, and concentrated under reduced pressure. To the crude product was added dichloromethane (10 mL), and a solid was precipitated, and the mixture was filtered to obtain compound **43-3.** ¹H NMR (400MHz, DMSO-*d*₆) δ 7.64-7.50 (m, 2H), 7.21 (s, 1H), 2.58 (d, *J*=1.2 Hz, 3H).

### Step 4

Compound **43-3** (200 mg, 961 µmol) was dissolved in dioxane (10 mL) and water (2 mL), and intermediate **38-1** (231 mg, 961 µmol), [1,1'-bis(diphenylphosphino)ferrocene]dichloropalladium(II) (140 mg, 192 µmol), and potassium carbonate (132 mg, 961 µmol) were added thereto. The system was replaced with nitrogen three times, and the reaction mixture was stirred at 100°C for 12 hours. The reaction mixture was directly concentrated under reduced pressure, and the crude product was separated by preparative high performance liquid chromatography (chromatographic column: Xtimate C18 150 × 40 mm × 5 µm; mobile phase: 0.05% hydrochloric acid aqueous solution-acetonitrile; gradient: acetonitrile: 20% to 50%, 6 min) to obtain the hydrochloride of compound **43.** ¹H NMR (400 MHz, CD₃OD) δ 7.78-7.48 (m, 2H), 7.36 (s, 1H), 7.22 (d, J=8.0 Hz, 1H), 4.49-4.28 (m, 1H), 3.95-3.79 (m, 1H), 3.57 (d, 7=11.3 Hz, 1H), 3.33 (s, 2H), 3.20-3.01 (m, 1H), 3.00-2.88 (m, 3H), 2.64 (s, 3H), 2.42-2.33 (m, 3H), 2.27 (d, 7=11.8 Hz, 1H), 2.19-1.95 (m, 1H), 1.80-1.61 (m, 1H). MS-ESI calculated for [M+H]⁺ 369, found 369.

### Example 44

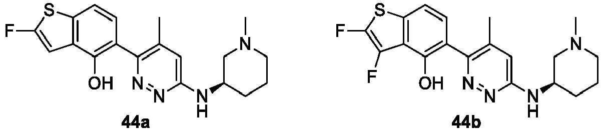

### Synthetic Route:

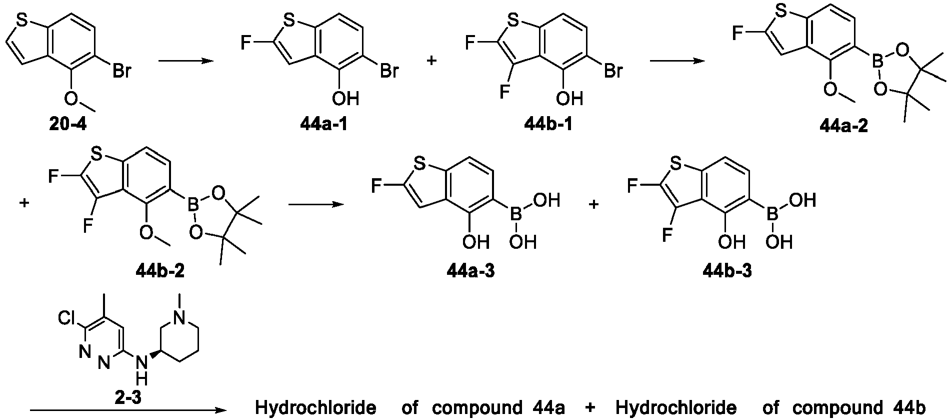

### Step 1

Compound **20-4** (6.00 g, 24.7 mmol) was dissolved in tetrahydrofuran (80 mL), and a mixed solution of lithium diisopropylamide 2M tetrahydrofuran in n-heptane (18.5 mL) was added thereto at 0°C, and the reaction mixture was stirred at 0°C for 0.5 hours. The reaction mixture was added with N-fluorobenzenesulfonimide (23.4 g, 74.0 mmol), stirred at 25°C for 4 hours. The reaction mixture was added with water (40 mL) and extracted with ethyl acetate (40 mL × 3), and the organic phases were combined, dried over anhydrous sodium sulfate, filtered, and concentrated under reduced pressure. The crude product was separated by silica gel column chromatography (petroleum ether/ethyl acetate, 100/1 to 10/1, V/V) to obtain a mixture of intermediates **44a-1** and **44b-1.**

### Step 2

The mixture of **44a-1** (2.90 g, 7.11 mmol, purity: 64%) and **44b-1** (2.90 g, 3.32 mmol, purity: 32%) were dissolved in dioxane (30 mL), and bis(pinacolato)diboron (2.71 g, 10.7 mmol), potassium acetate (1.40 g, 14.2 mmol), and [1,1'-bis(diphenylphosphino)ferrocene]dichloropalladium(II) (499 mg, 711 µmol) were added thereto. The system was replaced with nitrogen three times, and the reaction mixture was stirred at 100°C for 12 hours. The reaction mixture was directly concentrated under reduced pressure, and the residue was separated by silica gel column chromatography (petroleum ether/ethyl acetate, 100/1 to 10/1, V/V) to obtain a mixture of **44a-2** and **44b-2.**

### Step 3

The mixture of **44a-2** and **44b-2** (500 mg, 1.62 mmol) was dissolved in dichloromethane (10 mL), and boron tribromide (610 mg, 2.43 mmol) was added thereto at 0°C. The reaction mixture was stirred at 0°C for 0.5 hours. The reaction mixture was added with water (10 mL) and extracted with dichloromethane (20 mL × 3), and the organic phases were combined, dried over anhydrous sodium sulfate, filtered, and concentrated under reduced pressure. To the crude product was added dichloromethane (6 mL), and a solid was precipitated, and the mixture was filtered to obtain a mixture of **44a-3** and **44b-3.**

### Step 4

Intermediate **2-3** (80.0 mg, 332 µmol) was dissolved in dioxane (2 mL) and water (0.4 mL), and the mixture of **44a-3** and **44b-3** (77.5 mg, 366 µmol), [1,1'-bis(diphenylphosphino)ferrocene]dichloropalladium(II) (24.3 mg, 33.2 µmol), and potassium carbonate (115 mg, 831 µmol) were added thereto. The system was replaced with nitrogen three times, and the reaction mixture was stirred at 100°C for 12 hours. The reaction mixture was directly concentrated under reduced pressure, and the residue was separated by thin-layer chromatography (dichloromethane/methanol, 100/0 to 10/1, V/V) to obtain the crude products of compounds **44a** and **44b.** The crude products were separated by preparative high performance liquid chromatography (chromatographic column: Xtimate C18 150 × 40 mm × 5 µm; mobile phase: 0.05% hydrochloric acid aqueous solution-acetonitrile; gradient: acetonitrile: 12% to 30%, 6 min) to obtain compounds **44a** and **44b.**

Compound **44a:** ¹H NMR (400 MHz, CD₃OD) δ 7.69-7.57 (m, 1H), 7.52 (d, *J*=7.6 Hz, 1H), 7.30 (d, J=7.6 Hz, 1H), 7.16-7.15 (m, 1H), 4.38-4.34 (m, 1H), 3.86-3.80 (m, 1H), 3.56-3.53 (m 1H), 3.16-3.02 (m, 1H), 2.95-2.89 (m, 3H), 2.35-2.33 (m, 3H), 2.27-2.24 (m, 1H), 2.15-2.11 (m, 1H), 2.08-1.95 (m, 2H), 1.74-1.69 (m, 1H). MS-ESI calculated for [M+H]⁺ 373, found 373.

Compound **44b:** ¹H NMR (400 MHz, CD₃OD) δ 7.69-7.58 (m, 1H), 7.54 (d, *J*=7.2 Hz, 1H), 7.35 (d, *J*=7.2 Hz, 1H), 4.39-4.34 (m, 1H), 3.86-3.80 (m, 1H), 3.57-3.54 (m, 1H), 3.16-3.02 (m, 1H), 2.95-2.90 (m, 3H), 2.34-2.33 (m, 3H), 2.28-2.25 (m, 1H), 2.16-2.12 (m, 1H), 2.06-1.95 (m, 2H), 1.75-1.66 (m, 1H). MS-ESI calculated for [M+H]⁺ 391, found 391.

### Example 45

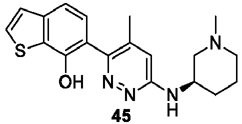

### Synthetic Route:

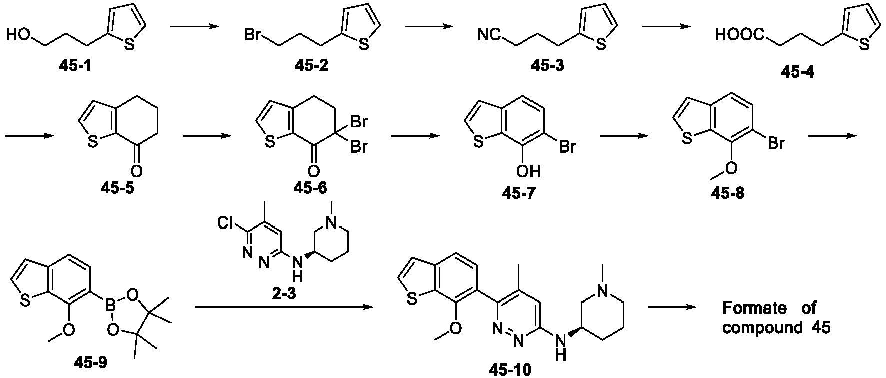

### Step 1

Compound **45-1** (4.6 g, 32.3 mmol) and triphenylphosphine (9.33 g, 35.6 mmol) were dissolved in dichloromethane (150 mL), cooled to 0°C, and carbon tetrabromide (11.8 g, 35.6 mmol) was added thereto. The reaction mixture was stirred at 25°C for 12 hours. The reaction mixture was concentrated under reduced pressure, and the residue was slurried with petroleum ether (200 mL) at 25°C for 1 hour, filtered, and the filtrate was concentrated under reduced pressure. The crude product was purified by silica gel column chromatography (petroleum ether/ethyl acetate, 1/0, V/V) to obtain compound **45-2.** ¹H NMR (400 MHz, CDCl₃) δ 7.17 (d, *J*=5.2 Hz, 1H), 6.97-6.95 (m, 1H), 6.87-6.85 (m, 1H), 3.48-3.45 (m, 2H), 3.06-3.03 (m, 2H), 2.27-2.20 (m, 2H).

### Step 2

Compound **45-2** (3 g, 14.6 mmol) was dissolved in dimethyl sulfoxide (30 mL), and sodium cyanide (1.22 g, 24.9 mmol) was added thereto. The reaction mixture was stirred at 70°C for 3 hours under nitrogen atmosphere. The reaction mixture was poured into water (50 mL) and extracted with ethyl acetate (30 mL × 2). The combined organic phases were sequentially washed with water (50 mL × 3) and saturated brine (50 mL × 1), dried over anhydrous sodium sulfate, filtered, and the filtrate was concentrated under reduced pressure to obtain compound **45-3.** ¹H NMR (400 MHz, CDCl₃) δ -7.18 (dd, *J*=5.2, 0.8 Hz, 1H), 6.96-6.94 (m, 1H), 6.86-6.85 (m, 1H), 3.04-3.00 (m, 2H), 2.40-2.37 (m, 2H), 2.08-2.00 (m, 2H).

### Step 3

Compound **45-3** (1.64 g, 10.8 mmol) and potassium hydroxide (3.53 g, 62.9 mmol) were dissolved in ethanol (15 mL) and water (15 mL), and the reaction mixture was stirred at 100°C for 24 hours. The reaction mixture was concentrated under reduced pressure to remove ethanol, and the pH of the residue was adjusted to 4 with hydrochloric acid aqueous solution (6.00 mol/L) at 0°C, and then the mixture was extracted with ethyl acetate (30 mL ×2). The combined organic phases were washed with saturated brine (50 mL ×1), dried over anhydrous sodium sulfate, filtered, and the filtrate was concentrated under reduced pressure to obtain compound **45-4.** ¹H NMR (400 MHz, CDCl₃) δ 7.15-7.13 (m, 1H), 6.94-6.92 (m, 1H), 6.82-6.81 (m, 1H), 2.94-2.90 (m, 2H), 2.45-2.41 (m, 2H), 2.07-2.00 (m, 2H).

### Step 4

Compound **45-4** (2 g, 11.8 mmol) was dissolved in acetic anhydride (2 mL) and phosphoric acid (0.04 mL), and the reaction mixture was stirred at 120°C for 2.5 hours. The reaction mixture was cooled to 0°C, quenched with water (20 mL), extracted with dichloromethane (30 mL × 2), and the combined organic phases were sequentially washed with sodium hydroxide aqueous solution (1N, 50 mL × 1) and saturated brine (50 mL × 2), dried over anhydrous sodium sulfate, filtered, and the filtrate was concentrated under reduced pressure. The crude product was purified by silica gel column chromatography (petroleum ether/ethyl acetate, 5/1, V/V) to obtain compound **45-5.** ¹H NMR (400 MHz, CDCl₃) δ 7.39 (d, J=5.2 Hz, 1H), 7.06 (d, J=5.2 Hz, 1H), 3.06-3.03 (m, 2H), 2.58-2.55 (m, 2H), 2.25-2.19 (m, 2H).

### Step 5

To ethyl acetate (30 mL) was added copper bromide (9.10 g, 40.7 mmol), and the suspension was stirred at 80°C for 10 minutes. Compound **45-5** (1.55 g, 10.2 mmol) was dissolved in chloroform (30 mL), then the mixture was added into the above suspension, and the reaction mixture was stirred and reacted at 80°C for 24 hours. The reaction mixture was concentrated under reduced pressure, and the residue was diluted with ethyl acetate (100 mL) and then filtered. The filtrate was washed with saturated sodium bicarbonate aqueous solution (100 mL × 1), and the organic phase was dried over anhydrous sodium sulfate, filtered, and the filtrate was concentrated under reduced pressure to obtain compound **45-6.** ¹H NMR (400 MHz, CDCl₃) δ 7.41 (d, *J*=5.2 Hz, 1H), 7.10 (d, *J*=5.2 Hz, 1H), 3.09 (s, 4H). MS-ESI calculated for [M+H]⁺ 311, found 311.

### Step 6

Compound **45-6** (2.63 g, 8.48 mmol) and lithium carbonate (3.76 g, 50.9 mmol) were dissolved in N,N-dimethylformamide (30.0 mL), and the reaction mixture was stirred at 100°C for 6 hours. The reaction mixture was filtered, and the pH of the filtrate was adjusted to 1 with hydrochloric acid aqueous solution (1.00 mol/L), and the mixture was extracted with ethyl acetate (100 mL × 2). The combined organic phases were sequentially washed with water (100 mL × 3) and saturated brine (100 mL × 1), dried over anhydrous sodium sulfate, filtered, and the filtrate was concentrated under reduced pressure to obtain compound **45-7.** ¹H NMR (400 MHz, CDCl₃) δ 7.51 (d, *J*=5.2 Hz, 1H), 7.40-7.38 (m, 2H), 7.35-7.33 (m, 1H), 5.90 (s, 1H). MS-ESI calculated for [M+H]⁺ 230, found 230.

### Step 7

Compound **45-7** (1.91 g, 8.34 mmol) was dissolved in acetone (20 mL), and the reaction mixture was added with potassium carbonate (2.30 g, 16.7 mmol) and methyl iodide (2.37 g, 16.7 mmol), stirred at 40°C for 12 hours. The reaction mixture was filtered, and the filtrate was concentrated under reduced pressure. The residue was purified by silica gel column chromatography (petroleum ether/ethyl acetate, 5/1, V/V) to obtain compound **45-8.** ¹H NMR (400 MHz, CDCl₃) δ 7.44-7.39 (m, 2H), 7.37-7.35 (m, 2H), 3.93 (s, 3H).

### Step 8

Compound **45-8** (1.64 g, 6.75 mmol), bis(pinacolato)diboron (2.23 g, 8.77 mmol), potassium acetate (1.32 g, 13.5 mmol), and bis(triphenylphosphine)palladium(II) chloride (473 mg, 675 µmol) were dissolved in 1,4-dioxane (40.0 mL), and the reaction mixture was stirred at 90°C for 12 hours under nitrogen atmosphere. The reaction mixture was filtered, and the filtrate was concentrated under reduced pressure. The residue was purified by silica gel column chromatography (petroleum ether/ethyl acetate, 10/1, V/V) to obtain compound **45-9.** ¹H NMR (400 MHz, CDCl₃) δ 7.69-7.67 (m, 1H), 7.63-7.61 (m, 1H), 7.50 (d, *J*=5.6 Hz, 1H), 7.36 (d, *J*=5.6 Hz, 1H), 4.01 (s, 3H), 1.40 (s, 12H). MS-ESI calculated for [M+H]⁺ 291, found 291.

### Step 9

Compound 2-3 (100 mg, 0.415 mmol), compound **45-9** (180 mg, 0.620 mmol), potassium carbonate (172 mg, 1.25 mmol), and [1,1'-bis(diphenylphosphino)ferrocene]dichloropalladium(II) dichloromethane complex (33.9 mg, 41.5 µmol) were dissolved in 1,4-dioxane (5 mL) and water (0.5 mL), and the reaction mixture was stirred at 100°C for 12 hours under nitrogen atmosphere. The reaction mixture was filtered, and the filtrate was concentrated under reduced pressure. The residue was purified by thin-layer chromatography (dichloromethane/methanol/triethylamine, 5/1/0.02, V/V) to obtain compound **45-10.** MS-ESI calculated for [M+H]⁺ 369, found 369.

### Step 10

Compound **45-10** (120 mg, 317 µmol) was dissolved in anhydrous dichloromethane (5.0 mL), and boron tribromide (91.6 µL, 950 µmol) was slowly added dropwise thereto at 0°C. The reaction mixture was stirred and reacted at 25°C for 1 hour. The reaction mixture was quenched with methanol (3.00 mL) at 0°C, and then concentrated under reduced pressure. The residue was purified by preparative high performance liquid chromatography (Phenomenex Synergi C18 150 × 25 mm × 10 µm; mobile phase A: 0.225% formic acid aqueous solution; mobile phase B: acetonitrile; B%: 0% to 26%, 10 min) to obtain the formate of compound **45.** ¹H NMR (400 MHz, CD₃OD) δ 8.45 (s, 1H), 7.64-7.62 (m, 1H), 7.56-7.54 (m, 2H), 7.24 (d, *J*=8.4 Hz, 1H), 6.90 (d, *J*=0.8 Hz, 1H), 4.45-4.39 (m, 1H), 3.77-3.71 (m, 1H), 3.24-3.22 (m, 1H), 3.02-2.98 (m, 1H), 2.80 (s, 3H), 2.21 (d, *J*=0.4 Hz, 3H), 2.17-2.08 (m, 2H), 1.97-1.89 (m, 1H), 1.71-1.63 (m, 1H). MS-ESI calculated for [M+H]⁺ 355, found 355.

### Example 46

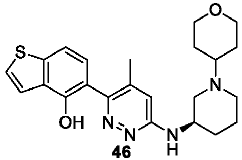

### Synthetic Route:

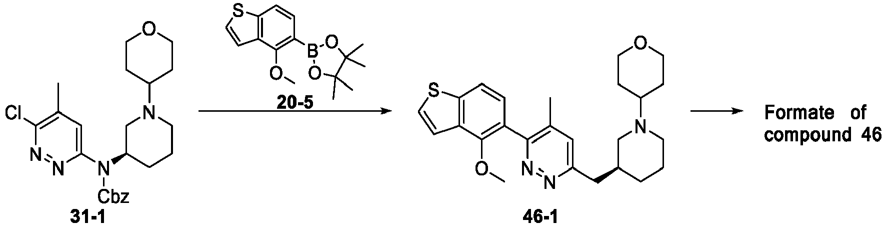

### Step 1

Compound **31-1** (140 mg, 0.315 mmol), compound **20-5** (183 mg, 0.629 mmol), potassium carbonate (130 mg, 0.944 mmol), and [1,1'-bis(diphenylphosphino)ferrocene]dichloropalladium(II) dichloromethane complex (25.7 mg, 31.5 µmol) were dissolved in 1,4-dioxane (5 mL) and water (0.5 mL), and the reaction mixture was stirred at 100°C for 12 hours under nitrogen atmosphere. The reaction mixture was filtered, and the filtrate was concentrated under reduced pressure. The residue was purified by thin-layer chromatography (dichloromethane/methanol, 10/1, V/V) to obtain compound **46-1.** MS-ESI calculated for [M+H]⁺ 439, found 439.

### Step 2

Compound **46-1** (110 mg, 243 µmol) was dissolved in anhydrous dichloromethane (6.00 mL), and boron tribromide (70.1 µL, 728 µmol) was slowly added dropwise thereto at 0°C. The reaction mixture was stirred and reacted at 25°C for 1 hour. The reaction mixture was quenched with methanol (3.00 mL) at 0°C, and then concentrated under reduced pressure. The residue was purified by preparative high performance liquid chromatography (Phenomenex Synergi C18 150 × 25 mm × 10 µm; mobile phase A: 0.225% formic acid aqueous solution; mobile phase B: acetonitrile; B%: 0% to 30%, 10 min) to obtain the formate of compound **46.** ¹H NMR (400 MHz, CD₃OD) δ 8.48 (s, 1H), 7.63-7.62 (m, 1H), 7.55-7.53 (m, 2H), 7.24 (d, *J*=8.0 Hz, 1H), 6.88 (d, *J*=0.8 Hz, 1H), 4.39-4.32 (m, 1H), 4.08-4.04 (m, 2H), 3.78-3.76 (m, 1H), 3.47-3.40 (m, 2H), 3.30-3.26 (m, 1H), 2.99-2.93 (m, 1H), 2.83 (s, 1H), 2.22 (s, 3H), 2.19-2.00 (m, 4H), 1.97-1.80 (m, 2H), 1.79-1.65 (m, 3H). MS-ESI calculated for [M+H]⁺ 425, found 425.

### Example 47

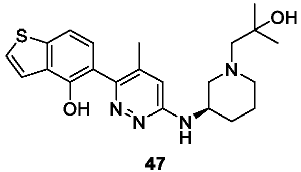

### Synthetic Route:

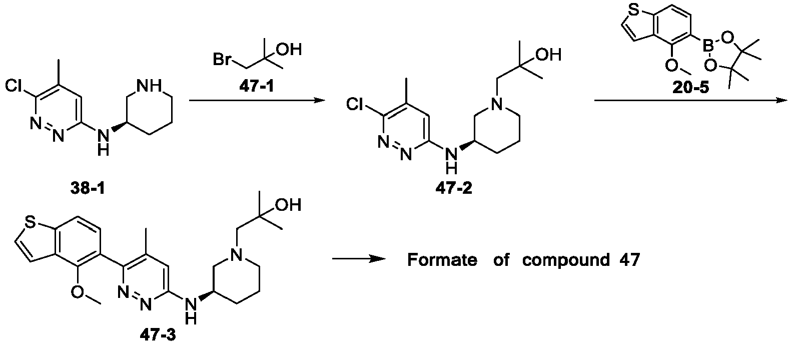

### Step 1

The hydrochloride of compound **38-1** (200 mg, 760 µmol), compound **47-1** (233 mg, 1.52 mmol), and potassium carbonate (315 mg, 2.28 mmol) were dissolved in N,N-dimethylformamide (5 mL), reacted at 60°C for 12 hours, and the reaction mixture was filtered. The filtrate was concentrated under reduced pressure, and the residue was purified by silica gel column chromatography (dichloromethane/methanol, 10/1, V/V) to obtain compound **47-2.** MS-ESI for compound **47-2,** calculated for [M+H]⁺ 299, found 299.

### Step 2

Compound **47-2** (600 mg, 2.01 mmol), compound **20-5** (1.17 g, 4.02 mmol), and potassium carbonate (833 mg, 6.02 mmol) were dissolved in dioxane (10 mL) and water (1 mL), and the reaction mixture was added with [1,1'-bis(diphenylphosphino)ferrocene]dichloropalladium(II) (147 mg, 201 µmol) under nitrogen atmosphere, and the reaction was stirred at 100°C for 12 hours. The reaction mixture was filtered, and the filtrate was concentrated under reduced pressure. The residue was purified by silica gel column chromatography (dichloromethane/methanol, 10/1, V/V) to obtain compound **47-3.** MS-ESI for compound **47-3,** calculated for [M+H]⁺ 427, found 427.

### Step 3

Compound **47-3** (662 mg, 1.55 mmol) was dissolved in dichloromethane (15 mL), and boron tribromide (1.17 g, 4.66 mmol) was slowly added dropwise thereto at 0°C. The reaction mixture was stirred and reacted at 25°C for 1 hour, quenched with methanol (10 mL) at 0°C, and then concentrated under reduced pressure. The residue was purified by preparative high performance liquid chromatography (C18 column 75 × 30 mm × 3 µm; mobile phase A: 0.225% formic acid aqueous solution; mobile phase B: acetonitrile; B%: 0% to 30%, 7 min) to obtain the formate of compound **47.** Formate of compound **47** ¹H NMR (400 MHz, CD₃OD) δ 8.403 (s, 1H), 7.62 (d, *J*=5.6 Hz, 1H), 7.54-7.52 (m, 2H), 7.24 (d, *J*=8.4 Hz, 1H), 6.89 (s, 1H), 4.45-4.38 (m, 1H), 3.79-3.76 (m, 1H), 3.43-3.40 (m, 1H), 3.34-3.33 (m, 3H), 3.04-3.03 (m, 1H), 2.92 -2 .87 (m, 1H), 2.22 (s, 3H), 2.15-2.10 (m, 1H), 2.04-2.00 (m, 1H), 1.71-1.61 (m, 1H), 1.33 (d, *J*=4.4 Hz, 6H). MS-ESI for the formate of compound **47,** calculated for [M+H]⁺ 413, found 413.

### Example 48

### Synthetic Route:

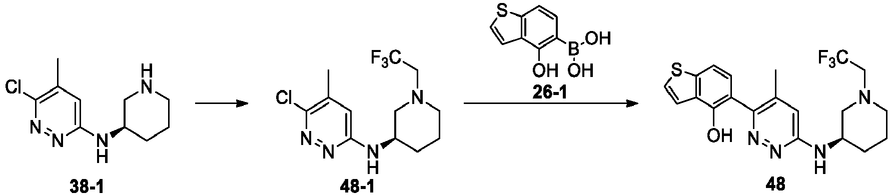

### Step 1

The hydrochloride of compound **38-1** (300 mg, 1.14 mmol) was dissolved in toluene (4 mL), and trifluoroethyl trifluoromethanesulfonate (529 mg, 2.28 mmol) and triethylamine (346 mg, 3.42 mmol) were added thereto. The reaction mixture was stirred at 100°C for 12 hours. The reaction mixture was added with water (10 mL) and extracted with ethyl acetate (30 mL × 3), and the organic phases were combined, dried over anhydrous sodium sulfate, filtered, and concentrated under reduced pressure. The crude product was separated by silica gel column chromatography (dichloromethane/methanol, 100/1 to 10/1, V/V) to obtain compound **48-1.** MS-ESI calculated for [M+H]⁺ 309, found 309.

### Step 2

Compound **48-1** (100 mg, 324 µmol) was dissolved in dioxane (6 mL) and water (1.2 mL). Intermediate **26-1** (75.4 mg, 389 µmol), [1,1'-bis(diphenylphosphino)ferrocene]dichloropalladium(II) (23.7 mg, 32.4 µmol), and potassium carbonate (112 mg, 810 µmol) were added thereto. The system was replaced with nitrogen three times, and the reaction mixture was stirred at 100°C for 12 hours. The reaction mixture was directly concentrated under reduced pressure, and the residue was separated by thin-layer chromatography (dichloromethane/methanol, 100/0 to 10/1, V/V) to obtain the crude product of compound **48.** The crude product was separated by preparative high performance liquid chromatography (chromatographic column: Phenomenx C18 80× 40 mm× 3 µm; mobile phase: 0.05% ammonia solution-acetonitrile; gradient: acetonitrile: 52% to 82%, 8 min) to obtain compound **48.** ¹H NMR (400 MHz, CD₃OD) δ 7.58 (d, *J*=5.6 Hz, 1H), 7.48 (d, *J*=5.6 Hz, 2H), 7.25 (d, *J*=8.0 Hz, 1H), 6.83 (s, 1H), 4.14-4.10 (m, 1H), 3.18-3.09 (m, 3H), 2.84-2.82 (m, 1H), 2.56 (t, *J*=9.2 Hz, 1H), 2.45 (t, *J*=9.2 Hz, 1H), 2.20 (s, 3H), 1.96-1.93 (m, 1H), 1.82-1.79 (m, 1H), 1.72-1.64 (m, 1H), 1.50-1.42 (m, 1H). MS-ESI calculated for [M+H]⁺ 423, found 423.

### Example 49

### Synthetic Route:

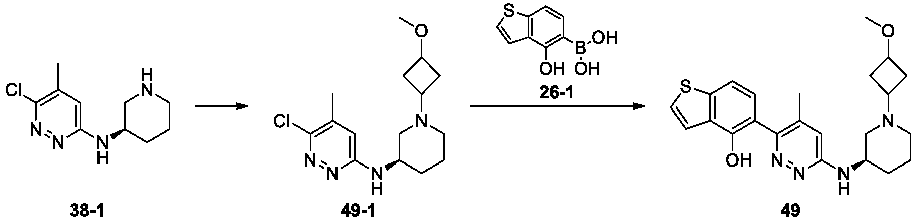

### Step 1

The hydrochloride of compound **38-1** (200 mg, 760 µmol) was dissolved in tetrahydrofuran (5 mL), and 3-methoxycyclobutan-1-one (152 mg, 1.52 mmol) was added thereto. The reaction mixture was stirred at 70°C for 12 hours. Then sodium triacetoxyborohydride (322 mg, 1.52 mmol) was added thereto at 0°C, and the reaction mixture was stirred at 25°C for 0.5 hours. The pH of the reaction mixture was neutralized to 8 with saturated sodium bicarbonate solution, and the reaction mixture was extracted with ethyl acetate (10 mL × 3). The organic phases were combined, dried over anhydrous sodium sulfate, filtered, and concentrated under reduced pressure. The crude product was separated by silica gel column chromatography (dichloromethane/methanol, 100/1 to 10/1, V/V) to obtain compound **49-1.** MS-ESI calculated for [M+H]⁺ 311, found 311.

### Step 2

Compound **49-1** (140 mg, 450 µmol) was dissolved in dioxane (3 mL) and water (0.6 mL), and intermediate **26-1** (105 mg, 541 µmol), [1,1'-bis(diphenylphosphino)ferrocene]dichloropalladium(II) (33.0 mg, 45.0 µmol), and potassium carbonate (156 mg, 1.13 mmol) were added thereto. The system was replaced with nitrogen three times, and the reaction mixture was stirred at 100°C for 12 hours. The reaction mixture was directly concentrated under reduced pressure, and the residue was separated by thin-layer chromatography (dichloromethane/methanol, 100/0 to 10/1, V/V) to obtain the crude product of compound **49.** The crude product was separated by preparative high performance liquid chromatography (chromatographic column: Phenomenx C18 80× 40 mm× 3 µm; mobile phase: 0.05% ammonia solution-acetonitrile; gradient: acetonitrile: 40% to 70%, 8 min) to obtain compound **49.** ¹H NMR (400 MHz, CD₃OD) δ 7.59 (d, *J*=5.6 Hz, 1H), 7.49-7.47 (m, 2H), 7.24 (d, *J*=8.4 Hz, 1H), 6.81 (s, 1H), 4.14-4.09 (m, 1H), 3.69-3.62 (m, 1H), 3.24-3.23 (m, 3H), 3.05-2.95 (m, 1H), 2.66-2.64 (m, 1H), 2.51-2.36 (m, 3H), 2.20 (s, 3H), 2.11-1.94 (m, 3H), 1.83-1.64 (m, 4H), 1.44-1.42 (m, 1H). MS-ESI calculated for [M+H]⁺ 425, found 425.

### Example 50

### Synthetic Route:

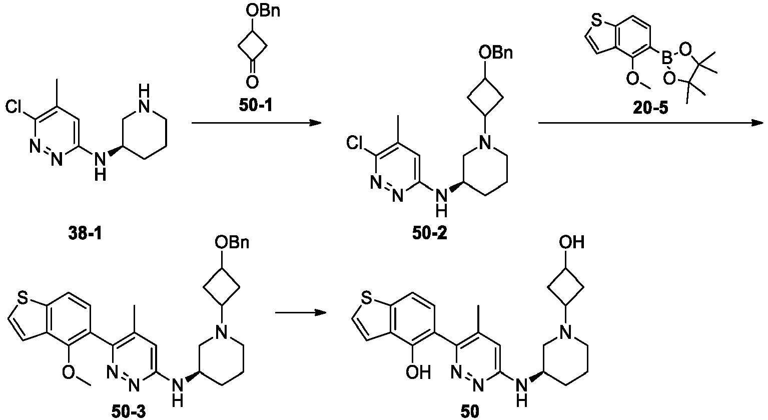

### Step 1

The hydrochloride of compound **38-1** (600 mg, 2.28 mmol) and compound **50-1** (2.01 g, 11.4 mmol) were dissolved in tetrahydrofuran (3 mL) and methanol (6 mL). The reaction mixture was stirred at 70°C for 12 hours, and cooled to 25°C, and sodium triacetoxyborohydride (2.42 g, 11.4 mmol) was added thereto. The reaction mixture was stirred at 70°C for 24 hours. The reaction mixture was filtered, and the filtrate was concentrated under reduced pressure. The crude product was purified by silica gel column chromatography (dichloromethane/methanol, 20/1, V/V) to obtain compound **50-2.** MS-ESI calculated for [M+H]⁺ 387, found 387.

### Step 2

Compound **50-2** (170 mg, 0.439 mmol), compound **20-5** (661 mg, 2.24 mmol), potassium carbonate (182 mg, 1.32 mmol), and [1,1'-bis(diphenylphosphino)ferrocene]dichloropalladium(II) (32.2 mg, 43.9 µmol) were dissolved in 1,4-dioxane (6 mL) and water (0.6 mL), and the reaction mixture was stirred at 110°C for 12 hours under nitrogen atmosphere. The reaction mixture was filtered, and the filtrate was concentrated under reduced pressure. The residue was separated by silica gel column chromatography (dichloromethane/methanol, 20/1, V/V) to obtain compound **50-3.** MS-ESI calculated for [M+H]⁺ 515, found 515.

### Step 3

Compound **50-3** (83 mg, 143 µmol) was dissolved in anhydrous dichloromethane (5.00 mL), and boron tribromide (41.3 µL, 429 µmol) was slowly added dropwise thereto at 0°C. The reaction mixture was stirred and reacted at 25°C for 12 hours. The reaction mixture was quenched with methanol (3.00 mL) at 0°C and then concentrated under reduced pressure, and the residue was purified by preparative high performance liquid chromatography (Waters Xbridge 150 × 25 mm × 5 µm; mobile phase A: 10 mmol/L ammonium bicarbonate aqueous solution; mobile phase B: acetonitrile; B%: 37% to 57%, 8 min) to obtain compound **50.** ¹H NMR (400 MHz, CD₃OD) δ 7.62-7.60 (m, 1H), 7.53-7.51 (m, 2H), 7.25 (d, *J*=8.4 Hz, 1H), 6.85 (s, 1H), 4.60 (s, 1H), 4.27-4.21 (m, 1H), 4.04-3.97 (m, 1H), 3.02-2.97 (m, 1H), 2.81-2.69 (m, 1H), 2.63-2.60 (m, 2H), 2.52-2.33 (m, 2H), 2.22 (s, 3H), 2.12-2.09 (m, 1H), 1.99-1.89 (m, 3H), 1.85-1.75 (m, 1H), 1.61-1.53 (m, 1H). MS-ESI calculated for [M+H]⁺ 411, found 411.

### Example 51

### Synthetic Route:

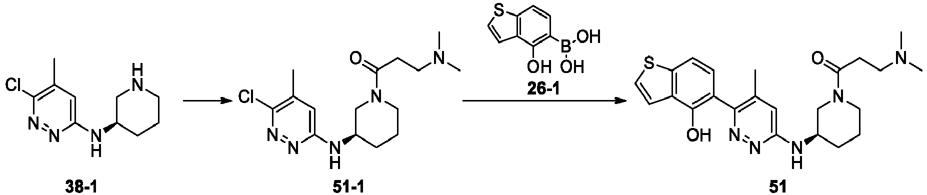

### Step 1

The hydrochloride of compound **38-1** (200 mg, 760 µmol) was dissolved in ethyl acetate (5 mL), and 3-dimethylaminopropionic acid hydrochloride (233 mg, 1.52 mmol), triethylamine (385 mg, 3.80 mmol), andpropylphosphonic anhydride (1.21 g, 1.90 mmol, 50% ethyl acetate solution) were added thereto. The reaction mixture was stirred at 25°C for 12 hours. The pH of the reaction mixture was neutralized to 8 with saturated sodium bicarbonate solution, and the reaction mixture was extracted with ethyl acetate (10 mL × 5). The organic phases were combined, dried over anhydrous sodium sulfate, filtered, and concentrated under reduced pressure. The crude product was separated by silica gel column chromatography (dichloromethane/methanol, 100/1 to 10/1, V/V) to obtain compound **51-1.** MS-ESI calculated for [M+H]⁺ 326, found 326.

### Step 2

Compound **51-1** (100 mg, 307 µmol) was dissolved in dioxane (3 mL) and water (0.6 mL), and intermediate **26-1** (71.5 mg, 368 µmol), [1,1'-bis(diphenylphosphino)ferrocene]dichloropalladium(II) (22.5 mg, 30.7 µmol), and potassium carbonate (106 mg, 767 µmol) were added thereto. The system was replaced with nitrogen three times, and the reaction mixture was stirred at 100°C for 12 hours. The reaction mixture was directly concentrated under reduced pressure, and the residue was separated by silica gel column chromatography (dichloromethane/methanol, 100/0 to 10/1, V/V) to obtain the crude product of compound **51.** The crude product was separated by high performance liquid chromatography (chromatographic column: Phenomenx C18 80× 40 mm× 3 µm; mobile phase: 0.05% ammonia solution-acetonitrile; gradient: acetonitrile: 29% to 59%, 8 min) to obtain compound **51.** ¹H NMR (400 MHz, CD₃OD) δ 7.60-7.58 (m, 1H), 7.46-7.43 (m, 2H), 7.24-7.21 (m, 1H), 6.86-6.84 (m, 1H), 4.17-3.90 (m, 2H), 3.72-3.69 (m, 1H), 3.58-3.37 (m, 2H), 2.67-2.46 (m, 4H), 2.30 (s, 2H), 2.21 (s, 3H), 2.20 (s, 4H), 2.15-2.08 (m, 1H), 1.94-1.75 (m, 2H), 1.69-1.60 (m, 1H). MS-ESI calculated for [M+H]⁺ 440, found 440.

### Example 52

### Synthetic Route:

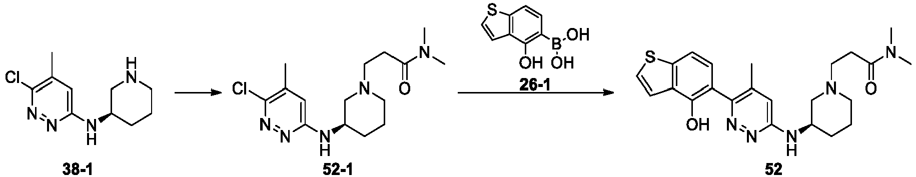

### Step 1

The hydrochloride of compound **38-1** (200 mg, 760 µmol) was dissolved in tetrahydrofuran (5 mL), and *N,N*-dimethylacrylamide (151 mg, 1.52 mmol), p-toluenesulfonic acid monohydrate (145 mg, 760 µmol), and triethylamine (76.9 mg, 760 µmol) were added thereto. The reaction mixture was stirred at 70°C for 12 hours. The reaction mixture was added with water (10 mL) and extracted with ethyl acetate (30 mL × 3), and the organic phases were combined, dried over anhydrous sodium sulfate, filtered, and concentrated under reduced pressure. The crude product was separated by silica gel column chromatography (dichloromethane/methanol, 100/1 to 10/1, V/V) to obtain compound **52-1.** MS-ESI calculated for [M+H]⁺ 326, found 326.

### Step 2

Compound **52-1** (100 mg, 307 µmol) was dissolved in dioxane (3 mL) and water (0.6 mL), and intermediate **26-1** (71.5 mg, 368 µmol), [1,1'-bis(diphenylphosphino)ferrocene]dichloropalladium(II) (22.5 mg, 30.7 µmol), and potassium carbonate (106 mg, 767 µmol) were added thereto. The system was replaced with nitrogen three times, and the reaction mixture was stirred at 100°C for 12 hours. The reaction mixture was directly concentrated under reduced pressure, and the residue was separated by silica gel column chromatography (dichloromethane/methanol, 100/0 to 10/1, V/V) to obtain the crude product of compound **52.** The crude product was separated by preparative high performance liquid chromatography (chromatographic column: Phenomenx C18 80× 40 mm× 3 µm; mobile phase: 0.05% ammonia solution-acetonitrile; gradient: acetonitrile: 29% to 59%, 8 min) to obtain compound **52.** ¹H NMR (400 MHz, CD₃OD) δ 7.59 (d, 7=6.0 Hz, 1H), 7.48-7.46 (m, 2H), 7.24 (d, *J*=8.4 Hz, 1H), 6.82 (s, 1H), 4.15-4.10 (m, 1H), 3.09 (s, 4H), 2.93 (s, 3H), 2.74-2.70 (m, 3H), 2.64-2.60 (m, 2H), 2.30-2.27 (m, 1H), 2.21 (s, 3H), 2.16-2.13 (m, 1H), 2.03-1.97 (m, 1H), 1.85-1.81 (m, 1H), 1.74-1.65 (m, 1H), 1.47-1.43 (m, 1H). MS-ESI calculated for [M+H]⁺ 440, found 440.

### Example 53

### Synthetic Route:

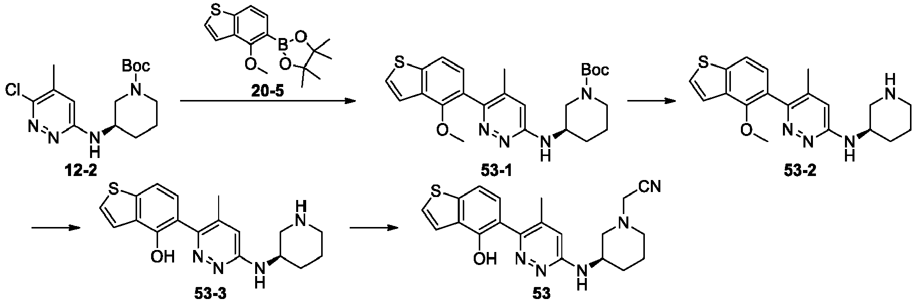

### Step 1

Compound **12-2** (200 mg, 612 µmol), compound **20-5** (887.90 mg, 3.06 mmol), and potassium carbonate (253.73 mg, 1.84 mmol) were dissolved in dioxane (5 mL) and water (0.5 mL), and the reaction mixture was added with [1,1'-bis(diphenylphosphino)ferrocene]dichloropalladium(II) (44.8 mg, 61.2 µmol) under nitrogen atmosphere, and the reaction was stirred at 110°C for 12 hours. The reaction mixture was filtered, and the filtrate was concentrated under reduced pressure. The residue was purified by silica gel column chromatography (petroleum ether/ethyl acetate, 50/1 to 1/1, V/V) to obtain compound **53-1.** MS-ESI calculated for [M+H]⁺ 455, found 455.

### Step 2

Compound **53-1** (178 mg, 392 µmol) was dissolved in hydrogen chloride/ethyl acetate (4 mol/L, 5 mL), and stirred and reacted at 25°C for 1 hour. The reaction mixture was concentrated under reduced pressure to obtain the hydrochloride of compound **53-2.** MS-ESI calculated for [M+H]⁺ 355, found 355.

### Step 3

The hydrochloride of compound **53-2** (181 mg, 511 mmol) was dissolved in dichloromethane (5 mL), and boron tribromide (383 mg, 1.53 mmol) was slowly added dropwise thereto at 0°C. The reaction mixture was stirred and reacted at 25°C for 1 hour, quenched with ammonia water (3 mL) at 0°C, and then concentrated under reduced pressure to obtain compound 53-3. MS-ESI calculated for [M+H]⁺ 341, found 341.

### Step 4

Compound 53-3 (85 mg, 250 µmol), compound bromoacetonitrile (30.0 mg, 250 µmol), and potassium carbonate (104 mg, 749 µmol) were dissolved in N,N-dimethylformamide (5 mL), and the mixture was stirred and reacted at 0°C for 2 hours. The reaction mixture was filtered and purified by preparative high performance liquid chromatography (C18 column 150×25 mm×5 µm; mobile phase: 10 mmol/L ammonium bicarbonate aqueous solution-acetonitrile; gradient: acetonitrile: 34% to 64%, 8 min) to obtain compound **53.** ¹H NMR (400 MHz, DMSO-*d*₆) δ 7.66 (d, *J*=3.2 Hz, 2H), 7.52 (d, *J*=8.0 Hz, 1H), 7.24 (d, *J*=8.0 Hz, 1H), 6.73 (d, *J*=8.0 Hz, 2H), 4.11 (s, 1H), 3.78 (s, 2H), 3.01 (d, *J*=8.0 Hz, 1H), 2.69-2.66 (m, 1H), 2.67-2.22 (m, 1H), 2.11-2.08 (m, 3H), 1.92-1.89 (m, 1H), 1.80-1.77 (m, 1H), 1.60-1.58 (m, 1H), 1.33-1.31 (m, 1H). MS-ESI calculated for [M+H]⁺ 380, found 380.

### Example 54

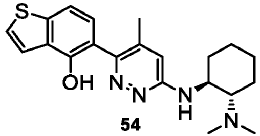

### Synthetic Route:

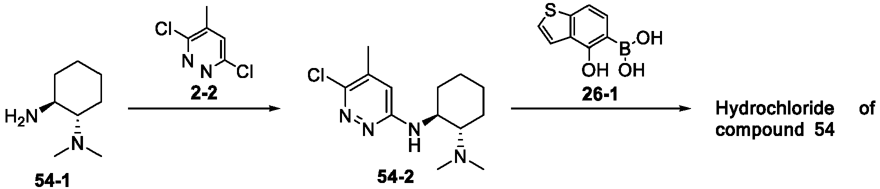

### Step 1

Compound **54-1** (480 mg, 3.37 mmol) was dissolved in 1,4-dioxane (2 mL), and then compound **2-2** (500 mg, 3.07 mmol), palladium acetate (68.9 mg, 307 µmol), 4,5-bis(diphenylphosphino)-9,9-dimethylxanthene (355 mg, 613 µmol), and cesium carbonate (2.50 g, 7.67 mmol) were added thereto. The system was replaced with nitrogen three times, and the reaction mixture was stirred at 100°C for 12 hours. The reaction mixture was directly concentrated under reduced pressure, and the residue was separated by silica gel column chromatography (dichloromethane/methanol, 100/1 to 10/1, V/V) to obtain compound **54-2.** MS-ESI calculated for [M+H]⁺ 269, found 269.

### Step 2

Compound **54-2** (200 mg, 744 µmol) was dissolved in dioxane (2 mL) and water (0.4 mL). Intermediate **26-1** (144 mg, 744 µmol), [1,1'-bis(diphenylphosphino)ferrocene]dichloropalladium(II) (54.5 mg, 74.4 µmol), and potassium carbonate (257 mg, 1.86 mmol) were added thereto. The system was replaced with nitrogen three times, and the reaction mixture was stirred at 100°C for 12 hours. The reaction mixture was directly concentrated under reduced pressure, and the residue was separated by silica gel column chromatography (dichloromethane/methanol, 100/0 to 10/1, V/V) to obtain the crude product of compound **54.** The crude product was separated by preparative high performance liquid chromatography (chromatographic column: Phenomenx C18 150× 40 mm× 5 µm; mobile phase: 0.05% hydrochloric acid aqueous solution-acetonitrile; gradient: acetonitrile: 1% to 30%, 10 min) to obtain the hydrochloride of compound **54.** ¹H NMR (400 MHz, CD₃OD) δ 7.71-7.66 (m, 3H), 7.61 (s, 1H), 7.32 (d, *J*=8.0 Hz, 1H), 4.33 (td, *J*=12.0, 3.6 Hz, 1H), 3.45 (td, *J*=12.0, 3.6 Hz, 1H), 2.97 (s, 3H), 2.86 (s, 3H), 2.37 (s, 3H), 2.25-2.22(m, 2H), 2.04-1.99 (m, 1H), 1.87-1.86 (m, 1H), 1.70-1.54 (m, 2H), 1.51-1.43 (m, 2H). MS-ESI calculated for [M+H]⁺ 383, found 383.

### Example 55

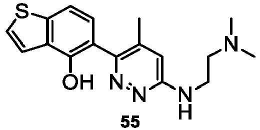

### Synthetic Route:

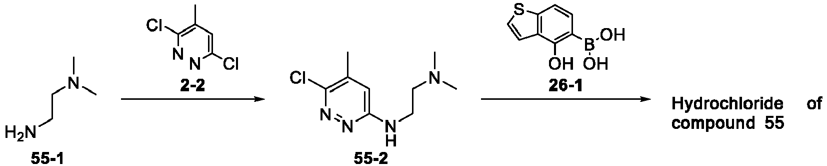

### Step 1

Compound **55-1** (324 mg, 3.68 mmol) was dissolved in 1,4-dioxane (10 mL), and compound **2-2** (500 mg, 3.07 mmol), palladium acetate (68.9 mg, 307 µmol), 4,5-bis(diphenylphosphino)-9,9-dimethylxanthene (355 mg, 613 µmol), and cesium carbonate (2.50 g, 7.67 mmol) were added thereto. The system was replaced with nitrogen three times, and the reaction mixture was stirred at 100°C for 12 hours. The reaction mixture was directly concentrated under reduced pressure, and the residue was separated by silica gel column chromatography (dichloromethane/methanol, 100/1 to 10/1, V/V) to obtain compound **55-2.** MS-ESI calculated for [M+H]⁺ 215, found 215.

### Step 2

Compound **55-2** (150 mg, 699 µmol) was dissolved in dioxane (2 mL) and water (0.4 mL). Intermediate **26-1** (149 mg, 769 µmol), [1,1'-bis(diphenylphosphino)ferrocene]dichloropalladium(II) (51.1 mg, 69.9 µmol), and potassium carbonate (241 mg, 1.75 mmol) were added thereto. The system was replaced with nitrogen three times, and the reaction mixture was stirred at 100°C for 12 hours. The reaction mixture was directly concentrated under reduced pressure, and the residue was separated by silica gel column chromatography (dichloromethane/methanol, 100/0 to 10/1, V/V) to obtain the crude product of compound **55.** The crude product was separated by preparative high performance liquid chromatography (chromatographic column: Phenomenx C18 150× 40 mm× 5 µm; mobile phase: 0.05% hydrochloric acid aqueous solution-acetonitrile; gradient: acetonitrile: 5% to 35%, 10 min) to obtain the hydrochloride of compound **55.** ¹H NMR (400 MHz, CD₃OD) δ 7.71-7.70 (m, 1H), 7.68-7.63 (m, 3H), 7.32 (d, *J*=8.4 Hz, 1H), 3.92 (t, *J*=5.4 Hz, 2H), 3.52 (t, *J*=5.4 Hz, 2H), 3.01 (s, 6H), 2.36 (s, 3H). MS-ESI calculated for [M+H]⁺ 329, found 329.

### Example 56

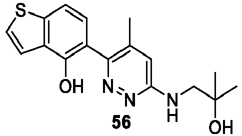

### Synthetic Route:

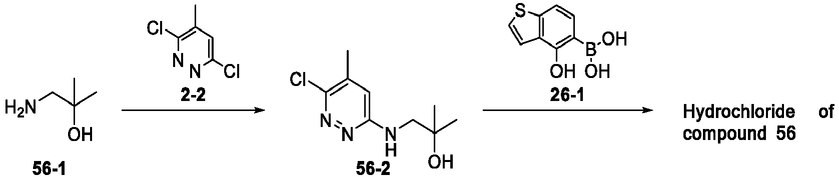

### Step 1

Compound **56-1** (273 mg, 3.07 mmol) was dissolved in 1,4-dioxane (5 mL), and compound **2-2** (500 mg, 3.07 mmol), palladium acetate (68.9 mg, 307 µmol), 4,5-bis(diphenylphosphino)-9,9-dimethylxanthene (355 mg, 613 µmol), and cesium carbonate (2.50 g, 7.67 mmol) were added thereto. The system was replaced with nitrogen three times, and stirred at 100°C for 12 hours. The reaction mixture was directly concentrated under reduced pressure, and the residue was separated by silica gel column chromatography (dichloromethane/methanol, 100/1 to 10/1, V/V) to obtain compound **56-2.** MS-ESI calculated for [M+H]⁺ 216, found 216.

### Step 2

Compound **56-2** (120 mg, 556 µmol) was dissolved in dioxane (2 mL) and water (0.4 mL). Intermediate **26-1** (118 mg, 612 µmol), [1,1'-bis(diphenylphosphino)ferrocene]dichloropalladium(II) (40.7 mg, 55.6 µmol), and potassium carbonate (192 mg, 1.39 mmol) were added thereto. The system was replaced with nitrogen three times, and stirred at 100°C for 2 hours. The reaction mixture was directly concentrated under reduced pressure, and the residue was separated by silica gel column chromatography (dichloromethane/methanol, 100/0 to 10/1, V/V) to obtain the crude product of compound **56.** The crude product was separated by high performance liquid chromatography (chromatographic column: Phenomenx C18 150× 40 mm× 5 µm; mobile phase: 0.05% hydrochloric acid aqueous solution-acetonitrile; gradient: acetonitrile: 5% to 35%, 10 min) to obtain the hydrochloride of compound **56.** ¹H NMR (400 MHz, CD₃OD) δ 7.65-7.64 (m, 1H), 7.60-7.53 (m, 3H), 7.22 (d, *J*=8.4 Hz, 1H), 3.48 (s, 2H), 2.30 (s, 3H), 1.34 (s, 6H). MS-ESI calculated for [M+H]⁺ 330, found 330.

### Example 57

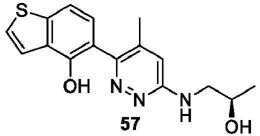

### Synthetic Route:

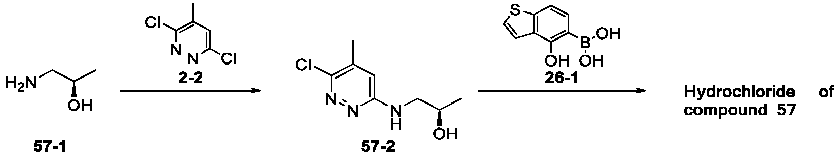

### Step 1

Compound **57-1** (253 mg, 3.37 mmol) was dissolved in 1,4-dioxane (2 mL), and compound **2-2** (500 mg, 3.07 mmol), palladium acetate (68.9 mg, 307 µmol), 4,5-bis(diphenylphosphino)-9,9-dimethylxanthene (355 mg, 613 µmol), and cesium carbonate (2.50 g, 7.67 mmol) were added thereto. The system was replaced with nitrogen three times, and the reaction mixture was stirred at 100°C for 12 hours. The reaction mixture was directly concentrated under reduced pressure, and the residue was separated by silica gel column chromatography (dichloromethane/methanol, 100/1 to 10/1, V/V) to obtain compound **57-2.** MS-ESI calculated for [M+H]⁺ 202, found 202.

### Step 2

Compound **57-2** (150 mg, 744 µmol) was dissolved in dioxane (2 mL) and water (0.4 mL). Intermediate **26-1** (144 mg, 744 µmol), [1,1'-bis(diphenylphosphino)ferrocene]dichloropalladium(II) (54.4 mg, 74.4 µmol), and potassium carbonate (257 mg, 1.86 mmol) were added thereto. The system was replaced with nitrogen three times, and the reaction mixture was stirred at 100°C for 12 hours. The reaction mixture was directly concentrated under reduced pressure, and the residue was separated by silica gel column chromatography (dichloromethane/methanol, 100/0 to 10/1, V/V) to obtain the crude product of compound **57.** The crude product was separated by preparative high performance liquid chromatography (chromatographic column: Xtimate C18 150× 40 mm× 5 µm; mobile phase: 0.05% hydrochloric acid aqueous solution-acetonitrile; gradient: acetonitrile: 5% to 35%, 10 min) to obtain the hydrochloride of compound **57.** ¹H NMR (400 MHz, CD₃OD) δ 7.64 (d, *J*=5.6 Hz, 1H), 7.60-7.57 (m, 2H), 7.49 (s, 1H), 7.22 (d, *J*=8.4 Hz, 1H), 4.12-4.04 (m, 1H), 3.54-3.50 (m, 1H), 3.42-3.37 (m, 1H), 2.30 (s, 3H), 1.29 (d, *J*=6.4 Hz, 3H). MS-ESI calculated for [M+H]⁺ 316, found 316.

### Example 58

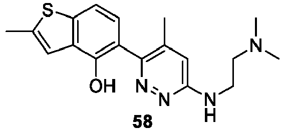

### Synthetic Route:

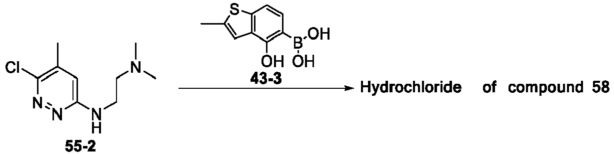

Compound **55-2** (250 mg, 780 µmol) was dissolved in dioxane (2 mL) and water (0.4 mL). Intermediate **43-3** (162 mg, 780 µmol), [1,1'-bis(diphenylphosphino)ferrocene]dichloropalladium(II) (57.1 mg, 78.0 µmol), and potassium carbonate (270 mg, 1.95 mmol) were added thereto. The system was replaced with nitrogen three times, and stirred at 100°C for 12 hours. The reaction mixture was directly concentrated under reduced pressure, and the residue was separated by thin-layer chromatography (dichloromethane/methanol, 100/0 to 10/1, V/V) to obtain the crude product of compound **58.** The crude product was separated by preparative high performance liquid chromatography (chromatographic column: Phenomenx C18 150× 40 mm× 5 µm; mobile phase: 0.05% hydrochloric acid aqueous solution-acetonitrile; gradient: acetonitrile: 7% to 37%, 10 min) to obtain the hydrochloride of compound **58.** ¹H NMR (400 MHz, CD₃OD) δ 7.62 (s, 1H), 7.52 (d, *J*=8.4 Hz, 1H), 7.34 (s, 1H), 7.21 (d, *J*=8.4 Hz, 1H), 3.91 (t, J=6.0 Hz, 2H), 3.51 (t, *J*=6.0 Hz, 2H), 3.01 (s, 6H), 2.62 (s, 3H), 2.35 (s, 3H). MS-ESI calculated for [M+H]⁺ 343, found 343.

### Example 59

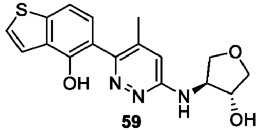

### Synthetic Route:

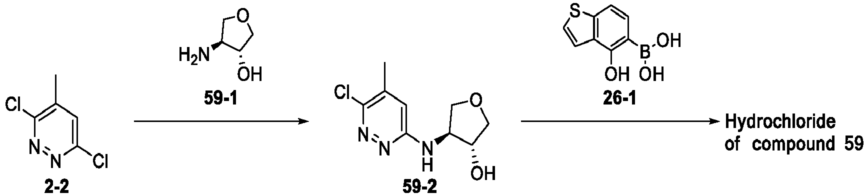

### Step 1

Compound **2-2** (1.2 g, 7.36 mmol), compound **59-1** (889 mg, 8.62 mmol), 4,5-bis(diphenylphosphino)-9,9-dimethylxanthene (852 mg, 1.47 mmol), cesium carbonate (4.80 g, 14.7 mmol), and tris(dibenzylideneacetone)dipalladium (674 mg, 0.736 mmol) were dissolved in 1,4-dioxane (30 mL), and the reaction mixture was stirred at 90°C for 12 hours under nitrogen atmosphere. The reaction mixture was filtered, and the filtrate was concentrated under reduced pressure, and the residue was first purified by silica gel column chromatography (petroleum ether/ethyl acetate, 100/1 to 1/5, V/V), and then purified by thin-layer chromatography (dichloromethane /methanol, 20/1, V/V) to obtain compound **59-2.** ¹H NMR (400 MHz, CDCl₃) δ 6.62 (s, 1H), 4.96 (d, J=3.2 Hz, 1H), 4.42-4.40 (m, 1H), 4.26-4.24 (m, 1H), 4.22-4.17 (m, 2H), 4.08-4.04 (m, 1H), 3.74-3.68 (m, 2H), 2.24 (s, 3H). MS-ESI calculated for [M+H]⁺ 230, found 230.

### Step 2

Compound **59-2** (100 mg, 0.435 mmol), compound **26-1** (170 mg, 0.876 mmol), potassium carbonate (181 mg, 1.31 mmol), and [1,1'-bis(diphenylphosphino)ferrocene]dichloropalladium(II) dichloromethane complex (35.6 mg, 43.5 µmol) were dissolved in 1,4-dioxane (5 mL) and water (0.5 mL), and the reaction mixture was stirred at 100°C for 12 hours under nitrogen atmosphere. The reaction mixture was filtered, and the filtrate was concentrated under reduced pressure, and the residue was purified by preparative high performance liquid chromatography (3_Phenomenex Luna C18 75 × 30 mm × 3 µm; mobile phase: 0.05% hydrochloric acid aqueous solution-acetonitrile; gradient: acetonitrile 13% to 33%, 6 min) to obtain the hydrochloride of compound **59.** ¹H NMR (400 MHz, CD₃OD) δ 7.70-7.68 (m, 1H), 7.66-7.64 (m, 2H), 7.52 (s, 1H), 7.30-7.28 (m, 1H), 4.41 (s, 1H), 4.28 (s, 1H), 4.24-4.21 (m, 1H), 4.14-4.10 (m, 1H), 3.89 (d, *J*=8.4 Hz, 1H), 3.78 (d, *J*=9.6 Hz, 1H), 2.35 (s, 3H). MS-ESI calculated for [M+H]⁺ 344, found 344.

### Example 60

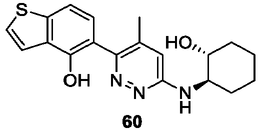

### Synthetic Route:

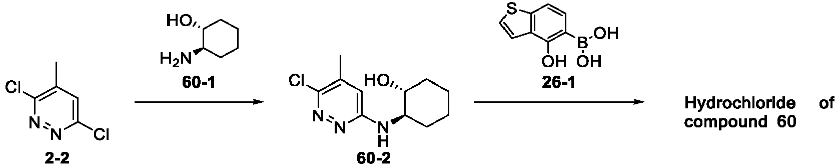

### Step 1

Compound **2-2** (500 mg, 3.07 mmol) and compound **60-1** (500 mg, 3.30 mmol) were dissolved in N-methylpyrrolidone (10 mL), and *N,N-*diisopropylethylamine (1.60 mL, 9.20 mmol) was added thereto. The reaction mixture was heated to 220°C under microwave irradiation and reacted for 6 hours. The reaction mixture was diluted with ethyl acetate (100 mL), sequentially washed with water (80 mL × 3) and saturated brine (80 mL × 1), and the organic phase was dried over anhydrous sodium sulfate, filtered. The filtrate was concentrated under reduced pressure, and the residue was purified by thin-layer chromatography (dichloromethane/methanol, 20/1, V/V) to obtain compound **60-2.** ¹H NMR (400MHz, CDCl₃) δ 6.62 (s, 1H), 4.95 (d, *J*=7.2Hz, 1H), 3.65-3.56 (m, 1H), 3.48 - 3.42(m, 1H), 2.28 (s, 3H), 2.14-2.05 (m, 2H), 1.81-1.71 (m, 2H), 1.43-1.24 (m, 4H). MS-ESI calculated for [M+H]⁺ 242, found 242.

### Step 2

Compound **60-2** (100 mg, 0.414 mmol), compound **26-1** (161 mg, 0.830 mmol), potassium carbonate (172 mg, 1.24 mmol), and [1,1'-bis(diphenylphosphino)ferrocene]dichloropalladium(II) dichloromethane complex (33.8 mg, 41.4 µmol) were dissolved in 1,4-dioxane (6 mL) and water (0.6 mL), and the reaction mixture was stirred and reacted at 100°C for 12 hours under nitrogen atmosphere. The reaction mixture was filtered, and the filtrate was concentrated under reduced pressure, and the residue was purified by preparative high performance liquid chromatography (3_Phenomenex Luna C18 75 × 30 mm × 3 µm; mobile phase: 0.05% hydrochloric acid aqueous solution-acetonitrile; gradient: acetonitrile 20% to 40%, 6 min) to obtain the hydrochloride of compound **60.** ¹H NMR (400 MHz, CD₃OD) δ 7.66-7.64 (m, 1H), 7.61-7.58 (m, 2H), 7.41 (s, 1H), 7.24-7.22 (m, 1H), 3.69-3.65 (m, 1H), 3.57-3.51 (m, 1H), 2.31 (s, 3H), 2.12-2.09 (m, 2H), 1.84-1.82 (m, 2H), 1.51-1.38 (m, 4H). MS-ESI calculated for [M+H]⁺ 356, found 356.

### Example 61

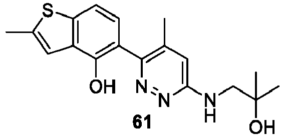

### Synthetic Route:

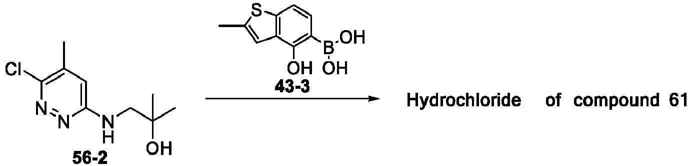

Compound **56-2** (100 mg, 464 µmol) was dissolved in dioxane (2 mL) and water (0.4 mL). Intermediate **43-3** (106 mg, 510 µmol), [1,1'-bis(diphenylphosphino)ferrocene]dichloropalladium(II) (33.9 mg, 46.4 µmol), and potassium carbonate (160 mg, 1.16 mmol) were added thereto. The system was replaced with nitrogen three times, and the reaction mixture was stirred at 100°C for 12 hours. The reaction mixture was directly concentrated under reduced pressure, and the residue was separated by silica gel column chromatography (dichloromethane/methanol, 100/0 to 10/1, V/V) to obtain the crude product of compound **61.** The crude product was separated by preparative high performance liquid chromatography (chromatographic column: Phenomenx C18 150× 40 mm× 5 µm; mobile phase: 0.05% hydrochloric acid aqueous solution-acetonitrile; gradient: acetonitrile: 7% to 37%, 10 min) to obtain the hydrochloride of compound **61.** ¹H NMR (400 MHz, CD₃OD) δ 7.50 (s, 1H), 7.44 (d, *J*=8.0 Hz, 1H), 7.28 (s, 1H), 7.12 (d, *J*=8.0 Hz, 1H), 3.46 (s, 2H), 2.60 (s, 3H), 2.30 (s, 3H), 1.33 (s, 6H). MS-ESI calculated for [M+H]⁺ 344, found 344.

### Example 62

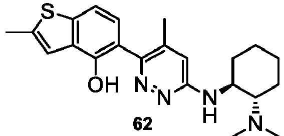

### Synthetic Route:

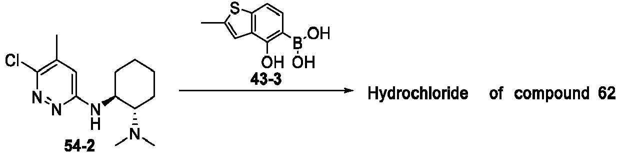

Compound **54-2** (300 mg, 692 µmol) was dissolved in dioxane (2 mL) and water (0.4 mL). Intermediate **43-3** (432 mg, 2.08 mmol), [1,1'-bis(diphenylphosphino)ferrocene]dichloropalladium(II) (50.6 mg, 69.2 µmol), and potassium carbonate (239 mg, 1.73 mmol) were added thereto. The system was replaced with nitrogen three times, and the reaction mixture was stirred at 100°C for 12 hours. The reaction mixture was directly concentrated under reduced pressure, and the residue was separated by silica gel column chromatography (dichloromethane/methanol, 100/0 to 10/1, V/V) to obtain the crude product of compound **62.** The crude product was separated by preparative high performance liquid chromatography (chromatographic column: Phenomenx C18 150× 40 mm× 5 µm; mobile phase: 0.05% hydrochloric acid aqueous solution-acetonitrile; gradient: acetonitrile: 5% to 35%, 10 min) to obtain the hydrochloride of compound **62.** ¹H NMR (400 MHz, CD₃OD) δ 7.60 (s, 1H), 7.53 (d, *J*=8.4 Hz, 1H), 7.34 (s, 1H), 7.22 (d, *J*=8.4 Hz, 1H), 4.33 (dt, *J*=4.0, 10.8 Hz, 1H), 3.45 (dt, *J*=4.0, 10.8 Hz, 1H), 2.91 (s, 6H), 2.62 (s, 3H), 2.36 (s, 3H), 2.23 (s, 2H), 2.04-1.99 (m, 1H), 1.87-1.86 (m, 1H), 1.70-1.57 (m, 2H), 1.53-1.46 (m, 2H). MS-ESI calculated for [M+H]⁺ 397, found 397.

### Example 63

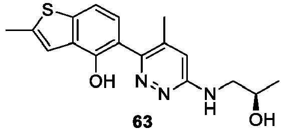

### Synthetic Route:

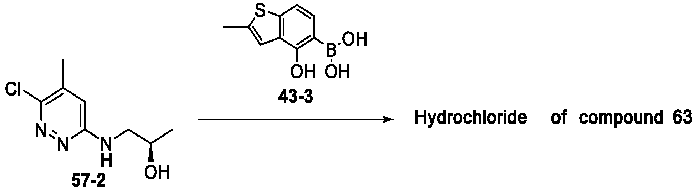

Compound **57-2** (150 mg, 744 µmol) was dissolved in dioxane (2 mL) and water (0.4 mL). Intermediate **43-3** (310 mg, 1.49 mmol), [1,1'-bis(diphenylphosphino)ferrocene]dichloropalladium(II) (54.4 mg, 74.4 µmol), and potassium carbonate (257 mg, 1.86 mmol) were added thereto. The system was replaced with nitrogen three times, and the reaction mixture was stirred at 100°C for 12 hours. The reaction mixture was directly concentrated under reduced pressure, and the residue was separated by silica gel column chromatography (dichloromethane/methanol, 100/0 to 10/1, V/V) to obtain the crude product of compound **63.** The crude product was separated by preparative high performance liquid chromatography (chromatographic column: Phenomenx C18 150× 40 mm× 5 µm; mobile phase: 0.05% hydrochloric acid aqueous solution-acetonitrile; gradient: acetonitrile: 5% to 35%, 10 min) to obtain the hydrochloride of compound **63.** ¹H NMR (400 MHz, CD₃OD) δ 7.46-7.44 (m, 2H), 7.28 (s, 1H), 7.12 (d, *J*=8.4 Hz, 1H), 4.09-4.05 (m, 1H), 3.53-3.49 (m, 1H), 3.41-3.36 (m, 1H), 2.61 (s, 3H), 2.30 (s, 3H), 1.28 (d, *J*=6.4 Hz, 3H). MS-ESI calculated for [M+H]⁺ 330, found 330.

### Example 64

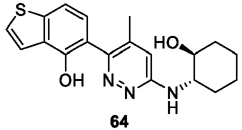

### Synthetic Route:

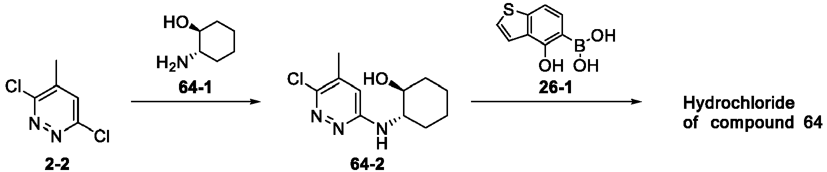

### Step 1

Compound **2-2** (470 mg, 2.88 mmol) and compound **64-1** (470 mg, 3.10 mmol) were dissolved in *N*-methylpyrrolidone (10 mL), and *N,N*-diisopropylethylamine (1.51 mL, 8.65 mmol) was added thereto. The reaction mixture was heated to 220°C under microwave irradiation and reacted for 3 hours. The reaction mixture was diluted with ethyl acetate (100 mL), sequentially washed with water (80 mL × 3) and saturated brine (80 mL × 1), and the organic phase was dried over anhydrous sodium sulfate, filtered. The filtrate was concentrated under reduced pressure, and the residue was purified by thin-layer chromatography (dichloromethane/methanol, 20/1, V/V) to obtain compound **64-2.** ¹H NMR (400MHz, CDCl₃) δ 6.63 (s, 1H), 5.11 (d, J=7.2 Hz, 1H), 3.64-3.58 (m, 1H), 3.49-3.43 (m, 1H), 2.28 (s, 3H), 2.12-2.09 (m, 2H), 1.79-1.73 (m, 2H), 1.47-1.23 (m, 4H). MS-ESI calculated for [M+H]⁺ 242, found 242.

### Step 2

Compound **64-2** (90 mg, 0.372 mmol), compound **26-1** (145 mg, 0.748 mmol), potassium carbonate (154 mg, 1.12 mmol), and [1,1'-bis(diphenylphosphino)ferrocene]dichloropalladium(II) dichloromethane complex (30.4 mg, 37.2 µmol) were dissolved in 1,4-dioxane (6 mL) and water (0.6 mL), and the reaction mixture was stirred at 100°C for 12 hours under nitrogen atmosphere. The reaction mixture was filtered, and the filtrate was concentrated under reduced pressure, and the residue was purified by preparative high performance liquid chromatography (3_Phenomenex Luna C18 75 × 30 mm × 3 µm; mobile phase: 0.05% hydrochloric acid aqueous solution-acetonitrile; gradient: acetonitrile 19% to 39%, 6 min) to obtain the hydrochloride of compound **64.** ¹H NMR (400 MHz, CD₃OD) δ 7.66-7.64 (m, 1H), 7.62-7.58 (m, 2H), 7.39-7.37 (m, 1H), 7.24-7.22 (m, 1H), 3.70-3.67 (m, 1H), 3.57-3.51 (m, 1H), 2.31 (s, 3H), 2.12-2.09 (m, 2H), 1.85-1.83 (m, 2H), 1.53-1.31 (m, 4H). MS-ESI calculated for [M+H]⁺ 356, found 356.

### Example 65

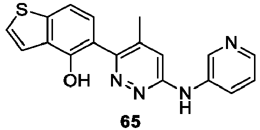

### Synthetic Route:

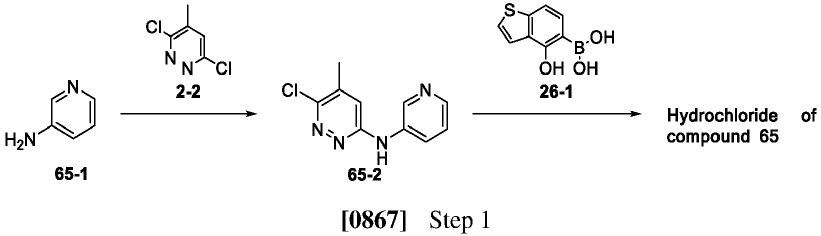

Compound **65-1** (318 mg, 3.37 mmol) was dissolved in 1,4-dioxane (20 mL), and then compound **2-2** (500 mg, 3.07 mmol), tris(dibenzylideneacetone)dipalladium (281 mg, 307 µmol), 4,5-bis(diphenylphosphino)-9,9-dimethylxanthene (355 mg, 613 µmol), and cesium carbonate (2.50 g, 7.67 mmol) were added thereto. The system was replaced with nitrogen three times, and the reaction mixture was stirred at 100°C for 12 hours. The reaction mixture was directly concentrated under reduced pressure, and the residue was separated by silica gel column chromatography (dichloromethane/methanol, 100/1 to 10/1, V/V) to obtain compound **65-2.** MS-ESI calculated for [M+H]⁺ 221, found 221.

### Step 2

Compound **65-2** (150 mg, 680 µmol) was dissolved in dioxane (2 mL) and water (0.4 mL). Intermediate **26-1** (145 mg, 748 µmol), [1,1'-bis(diphenylphosphino)ferrocene]dichloropalladium(II) (50.0 mg, 68.0 µmol), and potassium carbonate (235 mg, 1.70 mmol) were added thereto. The system was replaced with nitrogen three times, and the reaction mixture was stirred at 100°C for 12 hours. The reaction mixture was directly concentrated under reduced pressure, and the residue was separated by silica gel column chromatography (dichloromethane/methanol, 100/0 to 10/1, V/V) to obtain the crude product of compound **65.** The crude product was separated by preparative high performance liquid chromatography (chromatographic column: Phenomenx C18 150× 40 mm× 5 µm; mobile phase: 0.05% hydrochloric acid aqueous solution-acetonitrile; gradient: acetonitrile: 10% to 30%, 10 min) to obtain the hydrochloride of compound **65.** ¹H NMR (400 MHz, CD₃OD) δ 9.49 (s, 1H), 8.80 (d, *J*=8.4 Hz, 1H), 8.61 (d, *J*=5.4 Hz, 1H), 8.13 (dd, *J*=5.4, 8.4 Hz, 1H), 8.02 (s, 1H), 7.74-7.69 (m, 3H), 7.40 (d, *J*=8.4 Hz, 1H), 2.51 (s, 3H). MS-ESI calculated for [M+H]⁺ 335, found 335.

### Example 66

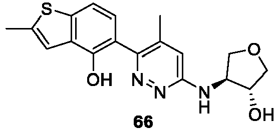

### Synthetic Route:

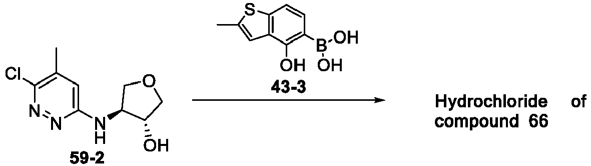

### Step 1

Compound **59-2** (70 mg, 0.305 mmol), compound **43-3** (200 mg, 0.961 mmol), potassium carbonate (126 mg, 0.914 mmol), and [1,1'-bis(diphenylphosphino)ferrocene]dichloropalladium(II) dichloromethane complex (24.9 mg, 30.5 µmol) were dissolved in 1,4-dioxane (6 mL) and water (0.6 mL), and the reaction mixture was stirred at 100°C for 12 hours under nitrogen atmosphere. The reaction mixture was filtered, and the filtrate was concentrated under reduced pressure, and the residue was purified by preparative high performance liquid chromatography (3_Phenomenex Luna C18 75 × 30 mm × 3 µm; mobile phase: 0.05% hydrochloric acid aqueous solution-acetonitrile; gradient: acetonitrile 17% to 37%, 6 min) to obtain the hydrochloride of compound **66.** ¹H NMR (400 MHz, CD₃OD) δ 7.52-7.50 (m, 2H), 7.33 (s, 1H), 7.20-7.18 (m, 1H), 4.40-4.39 (m, 1H), 4.28-4.27 (m, 1H), 4.24-4.20 (m, 1H), 4.13-4.10 (m, 1H), 3.90-3.87 (m, 1H), 3.79-3.76 (m, 1H), 2.63 (s, 3H), 2.34 (s, 3H). MS-ESI calculated for [M+H]⁺ 358, found 358.

### Example 67

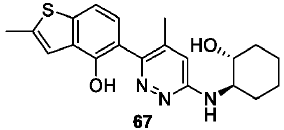

### Synthetic Route:

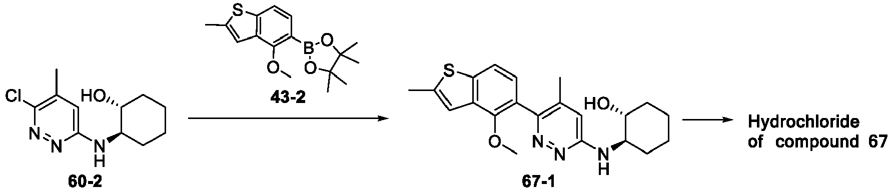

### Step 1

Compound **60-2** (120 mg, 0.496 mmol), compound **43-2** (454 mg, 1.49 mmol), potassium carbonate (206 mg, 1.49 mmol), and [1,1'-bis(diphenylphosphino)ferrocene]dichloropalladium(II) dichloromethane complex (40.5 mg, 49.6 µmol) were dissolved in 1,4-dioxane (6 mL) and water (0.6 mL), and the reaction mixture was stirred and reacted at 100°C for 12 hours under nitrogen atmosphere. The reaction mixture was filtered, and the filtrate was concentrated under reduced pressure. The residue was purified by thin-layer chromatography (dichloromethane/methanol, 20/1, V/V) to obtain compound **67-1.** MS-ESI calculated for [M+H]⁺ 384, found 384.

### Step 2

Compound **67-1** (125 mg, 202 µmol) was dissolved in anhydrous dichloromethane (5.00 mL), and boron tribromide (58.5 µL, 607 µmol) was slowly added dropwise thereto at 0°C. The reaction mixture was stirred and reacted at 25°C for 12 hours. The reaction mixture was quenched with methanol (5.00 mL) at 0°C, and then concentrated under reduced pressure. The residue was purified by preparative high performance liquid chromatography (3_Phenomenex Luna C18 75 × 30 mm × 3 µm; mobile phase: 0.05% hydrochloric acid aqueous solution-acetonitrile; gradient: acetonitrile: 23% to 43%, 31 min) to obtain the hydrochloride of compound **67.** ¹H NMR (400 MHz, CD₃OD) δ 7.45 (d, *J*=8.4 Hz, 1H), 7.39 (s, 1H), 7.29 (s, 1H), 7.13 (d, *J*=8.0 Hz, 1H), 3.69-3.64 (m, 1H), 3.57-3.51 (m, 1H), 2.62 (s, 3H), 2.30 (s, 3H), 2.12-2.09 (m, 2H), 1.84-1.82 (m, 2H), 1.54-1.38 (m, 4H). MS-ESI calculated for [M+H]⁺ 370, found 370.

### Example 68

### Synthetic Route:

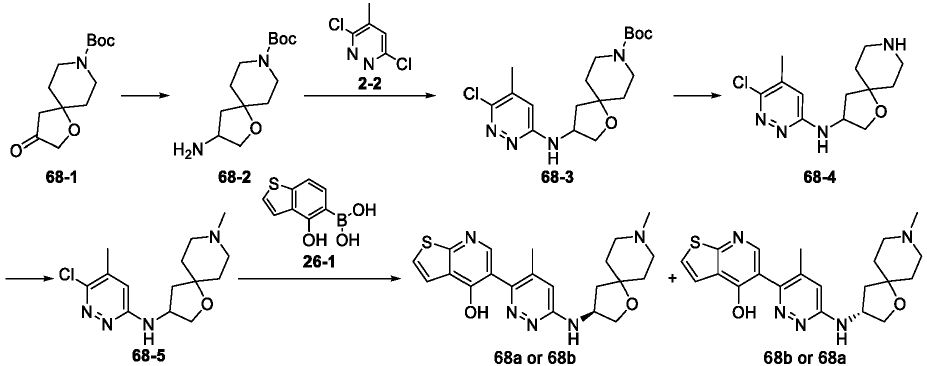

### Step 1

Compound **68-1** (5 g, 19.6 mmol) was dissolved in anhydrous methanol (80 mL), and ammonia gas (5.67 g, 333 mmol) was introduced thereto at 0°C for 20 minutes. Titanium tetraisopropoxide (11.1 g, 39.2 mmol) was added thereto, stirred at 20°C for 12 hours, and the reaction mixture was cooled to 0°C, added with sodium borohydride (1.23 g, 32.5 mmol), stirred and reacted at 0°C for 3 hours. The reaction was quenched by saturated potassium carbonate solution (10 mL) at 0°C, filtered and concentrated, and the crude product was purified by silica gel column chromatography (dichloromethane/methanol, 100/1 to 30/1, V/V) to obtain compound **68-2.** ¹H NMR (400 MHz, CD₃OD) δ 3.96-3.93 (m, 1H), 3.60-3.54 (m, 3H), 3.51-3.48 (m, 1H), 2.14-2.09 (m, 1H), 1.69-1.65 (m, 2H), 1.63-1.58 (m, 1H), 1.55-1.50 (m, 2H), 1.45 (s, 9H). MS-ESI calculated for [M-Boc+H]⁺ 201, found 201.

### Step 2

Compound **2-2** (600 mg, 3.68 mmol), compound **68-2** (1.08 g, 4.21 mmol), 4,5-bis(diphenylphosphino)-9,9-dimethylxanthene (426 mg, 0.736 mmol), cesium carbonate (2.40 g, 7.36 mmol), and palladium acetate (83 mg, 0.370 mmol) were dissolved in 1,4-dioxane (20 mL), and the reaction mixture was stirred and reacted at 90°C for 12 hours under nitrogen atmosphere. The reaction mixture was filtered, and the filtrate was concentrated under reduced pressure. The residue was purified by silica gel column chromatography (dichloromethane/methanol, 100/1 to 20/1, V/V) to obtain compound **68-3.** ¹H NMR (400 MHz, CDCl₃) δ 6.44 (d, *J*=0.8 Hz, 1H), 4.68 (s, 1H), 4.51 (s, 1H), 4.08-4.05 (m, 1H), 3.71-3.68 (m, 1H), 3.55-3.52 (m, 2H), 3.30-3.23 (m, 2H), 2.22 (s, 3H), 2.19-2.15 (m, 1H), 1.68-1.63 (m, 2H), 1.61-1.57 (m, 1H), 1.48-1.44 (m, 1H), 1.39 (s, 9H). MS-ESI calculated for [M+H]⁺ 383, found 383.

### Step 3

Compound **68-3** (220 mg, 0.574 mmol) was dissolved in ethyl acetate (5 mL), and hydrogen chloride-ethyl acetate (4 mol/L, 5 mL) was added thereto, and the reaction mixture was stirred at 25°C for 1 hour. The reaction mixture was concentrated under reduced pressure to obtain the hydrochloride of compound **68-4,** which was directly used in the next step without purification. ¹H NMR (400 MHz, CD₃OD) δ 7.48 (s, 1H), 4.46-4.41 (m, 1H), 4.19-4.15 (m, 1H), 4.01-3.98 (m, 1H), 3.31-3.29 (m, 4H), 2.48 (d, *J*=1.2 Hz, 3H), 2.44-2.39 (m, 1H), 2.06-2.01 (m, 4H), 1.95--1.87 (m, 1H). MS-ESI calculated for [M+H]⁺ 283, found 283.

### Step 4

The hydrochloride of compound **68-4** (185 mg, 0.579 mmol) and 37% formaldehyde aqueous solution (216 µL, 2.90 mmol) were dissolved in dichloromethane (4 mL) and methanol (2 mL). The reaction mixture was stirred at 25°C for 1 hour, and sodium triacetoxyborohydride (185 mg, 0.873 mmol) was added thereto. The reaction mixture was stirred at 25°C for 12 hours. The reaction mixture was concentrated under reduced pressure, and the crude product was layered in saturated sodium carbonate aqueous solution (20 mL) and ethyl acetate (20 mL). The aqueous phase was extracted with ethyl acetate (20 mL × 1), and the combined organic phases were dried over anhydrous sodium sulfate, filtered, and the filtrate was concentrated under reduced pressure to obtain compound **68-5,** which was directly used in the next step without purification. MS-ESI calculated for [M+H]⁺ 297, found 297.

### Step 5

Compound **68-5** (143 mg, 0.482 mmol), compound **26-1** (187 mg, 0.964 mmol), potassium carbonate (200 mg, 1.45 mmol), and [1,1'-bis(diphenylphosphino)ferrocene]dichloropalladium(II) dichloromethane complex (39.3 mg, 48.2 µmol) were dissolved in 1,4-dioxane (6 mL) and water (0.6 mL), and the reaction mixture was stirred and reacted at 100°C for 12 hours under nitrogen atmosphere. The reaction mixture was filtered, and the filtrate was concentrated under reduced pressure, and the residue was purified by preparative high performance liquid chromatography (3_Phenomenex Luna C18 75 × 30 mm × 3 µm; mobile phase: 0.05% hydrochloric acid aqueous solution-acetonitrile; gradient: acetonitrile 1% to 30%, 6 min), and then purified by preparative high performance liquid chromatography (DAICEL CHIRALPAK IE 250 × 30 mm × 3 µm; mobile phase: n-hexane; gradient: 0.1% ammonia water-ethanol solution 85% to 85%, 15 min) to obtain compounds **68a** and **68b.** The e.e. % values were then measured by SFC (Chiralpak IE-3 50 mm × 4.6 mm × 3 µm; mobile phase: phase A was n-hexane, phase B was ethanol solution containing 0.05% diethylamine; gradient: phase B 70% to 70%).

Compound **68a:** e.e.% = 100%, RT = 2.030 min. ¹H NMR (400 MHz, CD₃OD) δ 7.61 (d, *J*=5.6 Hz, 1H), 7.34 (d, *J*=5.6 Hz, 1H), 7.29 (d, *J*=8.4 Hz, 1H), 7.17 (d, *J*=8.4 Hz, 1H), 6.81 (s, 1H), 4.65--4.59 (m, 1H), 4.23--4.19 (m, 1H), 3.79--3.76 (m, 1H), 2.55--2.47 (m, 4H), 2.34--2.32 (m, 1H), 2.30 (s, 3H), 2.23 (s, 3H), 1.93--1.78 (m, 3H), 1.76--1.69 (m, 1H). MS-ESI calculated for [M+H]⁺ 411, found 411.

Compound 68b: e.e.% = 99.6%, RT = 5.604 min. ¹H NMR (400 MHz, CD₃OD) δ 7.61 (d, *J*=5.6 Hz, 1H), 7.50-7.48 (m, 2H), 7.26 (d, *J*=8.0 Hz, 1H), 6.85 (s, 1H), 4.66--4.60 (m, 1H), 4.23--4.19 (m, 1H), 3.80--3.76 (m, 1H), 2.54--2.44 (m, 4H), 2.34--2.32 (m, 1H), 2.30 (s, 3H), 2.23 (s, 3H), 1.92--1.80 (m, 3H), 1.77--1.70 (m, 1H). MS-ESI calculated for [M+H]⁺ 411, found 411.

### Example 69

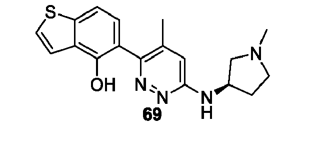

### Synthetic Route:

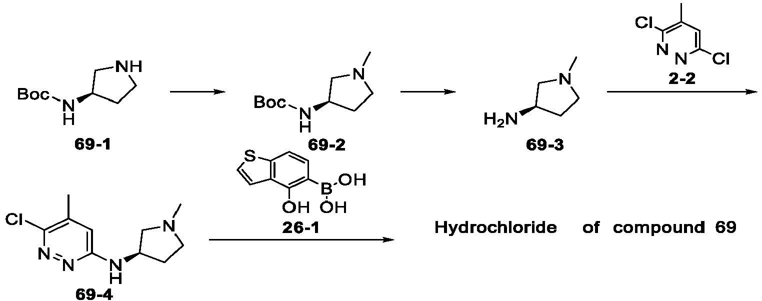

### Step 1

Compound **69-1** (800 mg, 4.30 mmol) was dissolved in tetrahydrofuran (5 mL), and formaldehyde aqueous solution (3.49 g, 42.95 mmol, purity: 37%) was added thereto. The reaction mixture was stirred at 70°C for 12 hours. Then sodium triacetoxyborohydride (1.82 g, 8.59 mmol) was added thereto at 0°C, and the reaction mixture was stirred at 25°C for 0.5 hours. The pH of the reaction mixture was neutralized to 8 with saturated sodium bicarbonate solution, and the reaction mixture was directly concentrated under reduced pressure. The crude product was separated by silica gel column chromatography (dichloromethane/methanol, 100/1 to 10/1, V/V) to obtain compound **69-2.** ¹H NMR (400 MHz, CD₃OD) δ 4.25-4.18 (m, 1H), 3.30-3.25 (m, 2H), 3.08-3.01 (m, 2H), 2.72 (s, 3H), 2.40-2.31 (m, 1H), 1.90-1.87 (m, 1H), 1.44 (s, 9H).

### Step 2

Compound **69-2** (700 mg, 3.50 mmol) was dissolved in ethyl acetate (5 mL) and methanol (5 mL), and hydrogen chloride-ethyl acetate (4 mol/L, 5 mL) was added thereto, and the reaction mixture was stirred at 25°C for 2 hours. The reaction mixture was directly concentrated to obtain the hydrochloride of compound **69-3,** which was directly used in the next step without purification. MS-ESI calculated for [M+H]⁺ 101, found 101.

### Step 3

The hydrochloride of compound **69-3** (436 mg, 2.52 mmol) was dissolved in 1,4-dioxane (10 mL), and then compound **2-2** (410 mg, 2.52 mmol), tris(dibenzylideneacetone)dipalladium (231 mg, 252 µmol), 4,5-bis(diphenylphosphino)-9,9-dimethylxanthene (292 mg, 504 µmol), and cesium carbonate (3.69 g, 11.3 mmol) were added thereto. The system was replaced with nitrogen three times, and stirred at 100°C for 12 hours. The reaction mixture was directly concentrated under reduced pressure, and the residue was separated by silica gel column chromatography (dichloromethane/methanol, 100/1 to 10/1, V/V) to obtain compound **69-4.** MS-ESI calculated for [M+H]⁺ 227, found 227.

### Step 4

Compound **69-4** (200 mg, 335 µmol) was dissolved in dioxane (2 mL) and water (0.4 mL). Intermediate **26-1** (97.6 mg, 503 µmol), [1,1'-bis(diphenylphosphino)ferrocene]dichloropalladium(II) (24.5 mg, 33.5 µmol), and potassium carbonate (116 mg, 838 µmol) were added thereto. The system was replaced with nitrogen three times, and the reaction mixture was stirred at 100°C for 12 hours. The reaction mixture was directly concentrated under reduced pressure, and the residue was separated by silica gel column chromatography (dichloromethane/methanol, 100/0 to 10/1, V/V) to obtain the crude product of compound **69.** The crude product was separated by preparative high performance liquid chromatography (chromatographic column: Phenomenx C18 150× 40 mm× 5 µm; mobile phase: 0.05% hydrochloric acid aqueous solution-acetonitrile; gradient: acetonitrile: 1% to 30%, 10 min) to obtain the hydrochloride of compound **69.** ¹H NMR (400 MHz, CD₃OD) δ 7.72-7.63 (m, 4H), 7.30 (d, *J*=7.6 Hz, 1H), 4.73-4.69 (m, 1H), 4.17-3.82 (m, 2H), 3.52-3.44 (m, 1H), 3.28-3.22 (m, 1H), 3.06-3.01 (m, 3H), 2.82-2.48 (m, 1H), 2.36 (s, 3H), 2.28-2.19 (m, 1H). MS-ESI calculated for [M+H]⁺ 341, found 341.

### Example 70

### Synthetic Route:

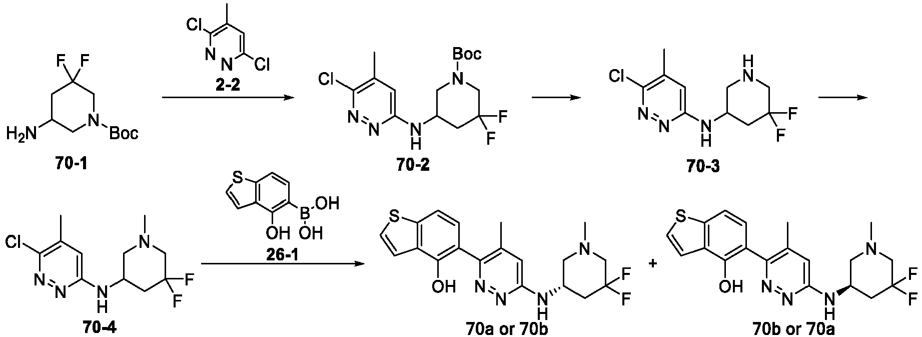

### Step 1

Compound **70-1** (1.00 g, 4.23 mmol) was dissolved in 1,4-dioxane (15 mL), and then compound **2-2** (897 mg, 5.50 mmol), tris(dibenzylideneacetone)dipalladium (388 mg, 423 µmol), 4,5-bis(diphenylphosphino)-9,9-dimethylxanthene (490 mg, 847 µmol), and cesium carbonate (3.45 g, 10.6 mmol) were added thereto. The system was replaced with nitrogen three times, and stirred at 100°C for 12 hours. The reaction mixture was directly concentrated under reduced pressure, and the residue was separated by silica gel column chromatography (dichloromethane/methanol, 100/1 to 10/1, V/V) to obtain compound **70-2.** MS-ESI calculated for [M+H]⁺ 363, found 363.

### Step 2

Compound **70-2** (680 mg, 1.87 mmol) was dissolved in ethyl acetate (5 mL), and hydrogen chloride-ethyl acetate (4 mol/L, 15 mL) was added thereto, and the reaction mixture was stirred at 25°C for 1 hour. The reaction mixture was directly concentrated to obtain the hydrochloride of compound **70-3,** which was directly used in the next step without purification. MS-ESI calculated for [M+H]⁺ 263, found 263.

### Step 3

Compound **70-3** (560 mg, 2.13 mmol) was dissolved in tetrahydrofuran (3 mL), and formaldehyde aqueous solution (1.73 g, 21.3 mmol, purity: 37%) was added thereto. The reaction mixture was stirred at 70°C for 2 hours. Then sodium triacetoxyborohydride (904 mg, 4.26 mmol) was added thereto at 0°C, and the reaction mixture was stirred at 25°C for 0.5 hours. The pH of the reaction mixture was neutralized to 8 with saturated sodium bicarbonate solution, and the reaction mixture was directly concentrated under reduced pressure. The crude product was separated by silica gel column chromatography (dichloromethane/methanol, 100/1 to 10/1, V/V) to obtain compound **70-4.** MS-ESI calculated for [M+H]⁺ 277, found 277.

### Step 4

Compound **70-4** (220 mg, 795 µmol) was dissolved in dioxane (3 mL) and water (0.6 mL). Intermediate **26-1** (216 mg, 1.11 mmol), [1,1'-bis(diphenylphosphino)ferrocene]dichloropalladium(II) (58.2 mg, 79.5 µmol), and potassium carbonate (275 mg, 1.99 mmol) were added thereto. The system was replaced with nitrogen three times, and the reaction mixture was stirred at 100°C for 12 hours. The reaction mixture was directly concentrated under reduced pressure, and the residue was purified by silica gel column chromatography (dichloromethane/methanol, 100/0 to 10/1, V/V). The crude product was purified by preparative high performance liquid chromatography (chromatographic column: Phenomenx C18 150× 40 mm× 5 µm; mobile phase: 0.05% hydrochloric acid aqueous solution-acetonitrile; gradient: acetonitrile: 1% to 30%, 10 min), and then separated by SFC (separation column: DAICEL CHIRALCEL OJ 250 mm × 30 mm × 10 µm; mobile phase: 0.1% ammonia water-ethanol; gradient: ethanol 30% to 30%) to obtain compounds **70a** and **70b.** The e.e. % values were then measured by SFC (Chiralcel OJ-3 100 mm × 4.6 mm × 3µm; mobile phase: phase A was supercritical CO₂, phase B was ethanol solution containing 0.05% diethylamine; gradient: phase B 5% to 40%).

**Compound 70a:** e.e.% = 96.1%, RT = 3.508 min. ¹H NMR (400 MHz, CD₃OD) δ 7.59 (d, *J*=5.6 Hz, 1H), 7.50-7.48 (m, 2H), 7.24 (d, *J*=8.4 Hz, 1H), 6.83 (s, 1H), 4.44 (s, 1H), 3.02-3.00 (m, 1H), 2.89-2.81 (m, 1H), 2.61-2.52 (m, 1H), 2.45-2.42 (m, 1H), 2.39 (s, 3H), 2.31-2.26 (m, 1H), 2.20 (s, 3H), 2.03-1.90 (m, 1H). MS-ESI calculated for [M+H]⁺ 391, found 391.

**Compound 70b:** e.e.% = 87.2%, RT = 3.647 min. ¹H NMR (400 MHz, CD₃OD) δ 7.60 (d, *J*=5.6 Hz, 1H), 7.52-7.51 (m, 2H), 7.33-7.24 (m, 1H), 6.94 (s, 1H), 4.43 (s, 1H), 3.04-3.02 (m, 1H), 2.92-2.84 (m, 1H), 2.65-2.55 (m, 1H), 2.48-2.44 (m, 1H), 2.41 (s, 3H), 2.34-2.29 (m, 1H), 2.22 (s, 3H), 2.04-1.94 (m, 1H). MS-ESI calculated for [M+H]⁺ 391, found 391.

### Example 71

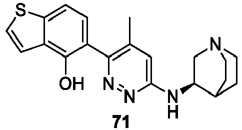

### Synthetic Route:

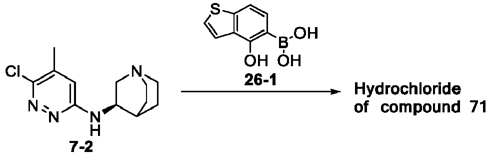

Compound **7-2** (521 mg, 2.06 mmol), compound **26-1** (200 mg, 1.03 mmol), potassium carbonate (138 mg, 2.06 mmol), and [1,1'-bis(diphenylphosphino)ferrocene]dichloropalladium(II) dichloromethane complex (151 mg, 206 µmol) were dissolved in 1,4-dioxane (10 mL) and water (2 mL), and the reaction mixture was stirred at 100°C for 12 hours under nitrogen atmosphere. The reaction mixture was filtered, and the filtrate was concentrated under reduced pressure, and the residue was purified by preparative high performance liquid chromatography (Xtimate C18 150 ×40 mm × 5 µm; mobile phase: 0.05% hydrochloric acid aqueous solution-acetonitrile; gradient: acetonitrile 20% to 50%, 10 min) to obtain the hydrochloride of compound **71.** ¹H NMR (400 MHz, CD₃OD) δ 7.86-7.60 (m, 4H), 7.31 (d, *J*=8.3 Hz, 1H), 4.54-4.31 (m, 1H), 3.92 (t, *J*=10.8 Hz, 1H), 3.57-3.36 (m, 6H), 2.50 (d, *J*=2.8 Hz, 1H), 2.38 (s, 3H), 2.15 (d, *J*=6.9 Hz, 2H), 2.02 (s, 1H). MS-ESI calculated for [M+H]⁺ 367, found 367.

### Example 72

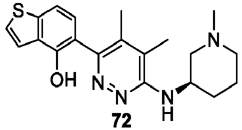

### Synthetic Route:

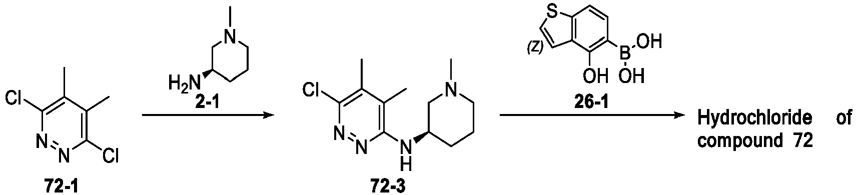

### Step 1

Compound **72-1** (1.00 g, 5.65 mmol) was dissolved in 1,4-dioxane (5 mL), and compound **2-1** (1.29 g, 11.3 mmol), palladium acetate (127 mg, 565 µmol), 4,5-bis(diphenylphosphino)-9,9-dimethylxanthene (654 mg, 1.13 mmol), and cesium carbonate (4.60 g, 14.1 mmol) were added thereto. The system was replaced with nitrogen three times, and the reaction mixture was stirred at 100°C for 12 hours. The reaction mixture was directly concentrated under reduced pressure, and the residue was separated by silica gel column chromatography (dichloromethane/methanol, 100/1 to 10/1, V/V) to obtain compound **72-3.** MS-ESI calculated for [M+H]⁺ 255, found 255.

### Step 2

Compound **72-3** (200 mg, 785 µmol) was dissolved in dioxane (20 mL) and water (4 mL), and intermediate **26-1** (228 mg, 1.18 mmol), [1,1'-bis(diphenylphosphino)ferrocene]dichloropalladium(II) (57.4 mg, 78.5 µmol), and potassium carbonate (271 mg, 1.96 mmol) were added thereto. The system was replaced with nitrogen three times, and the reaction mixture was stirred at 100°C for 4 hours. The reaction mixture was directly concentrated under reduced pressure, and the residue was separated by silica gel column chromatography (dichloromethane/methanol, 100/0 to 10/1, V/V) to obtain the crude product of compound **72.** The crude product was separated by preparative high performance liquid chromatography (chromatographic column: Phenomenx C18 150× 40 mm× 5 µm; mobile phase: 0.05% hydrochloric acid aqueous solution-acetonitrile; gradient: acetonitrile: 5% to 35%, 10 min) to obtain the hydrochloride of compound **72.** ¹H NMR (400 MHz, CD₃OD) δ 7.71-7.66 (m, 3H), 7.29 (d, *J*=7.2 Hz, 1H), 4.55 (s, 1H), 3.85-3.78 (m, 1H), 3.57-3.54 (m, 1H), 3.16-3.01 (m, 2H), 2.95-2.87 (m, 3H), 2.62-2.47 (m, 3H), 2.32 (s, 3H), 2.26-2.12 (m, 2H), 2.04-1.80 (m, 2H). MS-ESI calculated for [M+H]⁺ 369, found 369.

### Example 73

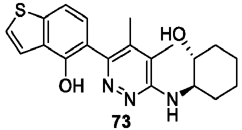

### Synthetic Route:

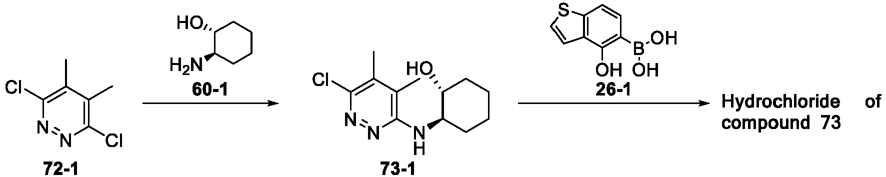

### Step 1

Compound **72-1** (1.00 g, 5.65 mmol) was dissolved in 1,4-dioxane (2 mL), and then compound **60-1** (976 mg, 8.47 mmol), tris(dibenzylideneacetone)dipalladium (517 mg, 565 µmol), 4,5-bis(diphenylphosphino)-9,9-dimethylxanthene (654 mg, 1.13 mmol), and cesium carbonate (4.60 g, 14.12 mmol) were added thereto. The system was replaced with nitrogen three times, and the reaction mixture was stirred at 100°C for 12 hours. The reaction mixture was directly concentrated under reduced pressure, and the residue was separated by silica gel column chromatography (dichloromethane/methanol, 100/1 to 10/1, V/V) to obtain compound **73-1.** MS-ESI calculated for [M+H]⁺ 256, found 256.

### Step 2

Compound **73-1** (187 mg, 731 µmol) was dissolved in dioxane (5 mL) and water (1 mL), and intermediate **26-1** (199 mg, 1.02 mmol), [1,1'-bis(diphenylphosphino)ferrocene]dichloropalladium(II) (53.5 mg, 73.1 µmol), and potassium carbonate (253 mg, 1.83 mmol) were added thereto. The system was replaced with nitrogen three times, and the reaction mixture was stirred at 90°C for 4 hours. The reaction mixture was directly concentrated under reduced pressure, and the residue was separated by silica gel column chromatography (dichloromethane/methanol, 100/0 to 10/1, V/V) to obtain the crude product of compound **73.** The crude product was then separated by preparative high performance liquid chromatography (chromatographic column: Phenomenx C18 150× 40 mm× 5 µm; mobile phase: 0.05% hydrochloric acid aqueous solution-acetonitrile; gradient: acetonitrile: 5% to 35%, 10 min) to obtain the hydrochloride of compound **73.** ¹H NMR (400 MHz, CD₃OD) δ 7.66-7.60 (m, 3H), 7.24-7.21 (d, *J*=8.4 Hz, 1H), 3.91-3.85 (m, 1H), 3.72-3.65 (m, 1H), 2.41 (s, 3H), 2.27 (s, 3H), 2.11-2.10 (m, 2H), 1.82-1.81 (m, 2H), 1.56-1.37 (m, 4H). MS-ESI calculated for [M+H]⁺ 370, found 370.

### Example 74

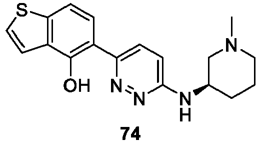

### Synthetic Route:

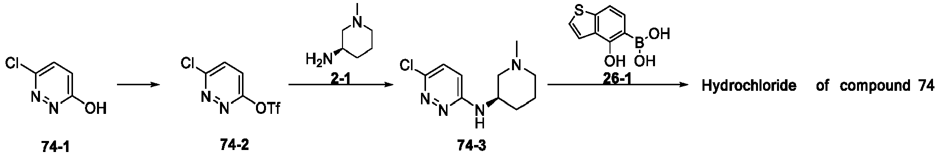

### Step 1

Compound **74-1** (5.00 g, 38.3 mmol) was dissolved in dichloromethane (50.0 mL), and trifluoromethanesulfonic anhydride (8.22 mL, 49.8 mmol) and pyridine (6.18 mL, 76.6 mmol) were added dropwise thereto at 0°C. The reaction mixture was stirred and reacted at 25°C for 12 hours. The reaction mixture was concentrated under reduced pressure, and the residue was purified by silica gel column chromatography (petroleum ether/ethyl acetate, 100/0 to 1/1, V/V) to obtain compound **74-2.** MS-ESI calculated for [M+H]⁺ 263, found 263.

### Step 2

Compound **74-2** (500 mg, 1.90 mmol), the hydrochloride of compound **2-1** (429 mg, 2.85 mmol), and *N,N*-diisopropylethylamine (1.32 mL, 7.60 mmol) were dissolved in *N,N-*dimethylformamide (5.00 mL), and the reaction mixture was stirred at 100°C for 1 hour. The reaction mixture was dried under reduced pressure with an oil pump, and the residue was diluted with dichloromethane (20.0 mL). The mixture was eluted by silica gel column chromatography (dichloromethane/methanol, 100/0 to 1/1, V/V), and the filtrate was collected and evaporated to dryness by rotary evaporation to obtain compound **74-3.** MS-ESI calculated for [M+H]⁺ 227, found 227.

### Step 3

Compound **74-3** (430 mg, 1.90 mmol), compound **26-1** (740 mg, 3.81 mmol), potassium carbonate (786 mg, 5.69 mmol), and [1,1'-bis(diphenylphosphino)ferrocene]dichloropalladium(II) dichloromethane complex (155 mg, 190 µmol) were dissolved in 1,4-dioxane (30.0 mL) and water (3.00 mL), and the reaction mixture was stirred at 100°C for 12 hours under nitrogen atmosphere. The reaction mixture was filtered, and the filtrate was concentrated under reduced pressure, and the residue was purified by preparative high performance liquid chromatography (chromatographic column: Phenomenex Luna C18 75 × 30 mm × 3 µm; mobile phase: 0.05% hydrochloric acid aqueous solution-acetonitrile; gradient: acetonitrile 20% to 44%, 9 min) to obtain the hydrochloride of compound **74.** ¹H NMR (400 MHz, CD₃OD) δ 8.42-8.36 (m, 1H), 7.72-7.71 (m, 1H), 7.65-7.57 (m, 4H), 4.39-4.32 (m, 1H), 3.89-3.85 (m, 1H), 3.57-3.54 (m, 1H), 3.07-3.00 (m, 1H), 2.96 (s, 2H), 2.90 (s, 1H), 2.30-2.26 (m, 1H), 2.18-2.13 (m, 1H), 2.03-2.00 (m, 2H), 1.73-1.62 (m, 1H). MS-ESI calculated for [M+H]⁺ 341, found 341.

### Example 75

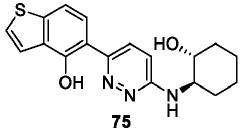

### Synthetic Route:

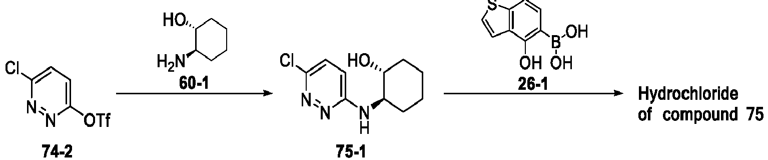

### Step 1

Compound **74-2** (500 mg, 1.90 mmol), compound **60-1** (329 mg, 2.86 mmol), and N,N-diisopropylethylamine (0.663 mL, 3.81 mmol) were dissolved in *N,N*-dimethylformamide (5.00 mL), and the reaction mixture was stirred and reacted at 100°C for 1 hour. The reaction was quenched with water (5.00 mL), and the reaction mixture was extracted with ethyl acetate (5.00 mL × 3), then washed with water (5.00 mL × 2) and saturated brine (5.00 mL × 1), and the organic phase was dried over anhydrous sodium sulfate, filtered. The filtrate was concentrated under reduced pressure, and the residue was purified by silica gel column chromatography (dichloromethane/methanol, 20/1, V/V) to obtain compound **75-1.** MS-ESI calculated for [M+H]⁺ 228, found 228.

### Step 2

Compound **75-1** (67.0 mg, 0.294 mmol), compound **26-1** (115 mg, 0.591 mmol), potassium carbonate (122 mg, 0.883 mmol), and [1,1'-bis(diphenylphosphino)ferrocene]dichloropalladium(II) dichloromethane complex (24.0 mg, 29.4 µmol) were dissolved in 1,4-dioxane (4.00 mL) and water (0.40 mL), and the reaction mixture was stirred at 100°C for 12 hours under nitrogen atmosphere. The reaction mixture was filtered, and the filtrate was concentrated under reduced pressure, and the residue was purified by preparative high performance liquid chromatography (chromatographic column: Phenomenex Luna C18 75 × 30 mm × 3 µm; mobile phase: 0.05% hydrochloric acid aqueous solution-acetonitrile; gradient: acetonitrile 20% to 42%, 9 min) to obtain the hydrochloride of compound **75.** ¹H NMR (400 MHz, CD₃OD) δ 8.36 (d, *J*=10.0 Hz, 1H), 7.72 (d, *J*=8.4 Hz, 1H), 7.64 (d, *J*=4.8 Hz, 1H), 7.55-7.53 (m, 2H), 7.49 (d, *J*=10.0 Hz, 1H), 3.71-3.65 (m, 1H), 3.55-3.52 (m, 1H), 2.13-2.07 (m, 2H), 1.84-1.81 (m, 2H), 1.48-1.41 (m, 4H). MS-ESI calculated for [M+H]⁺ 342, found 342.

### Example 76

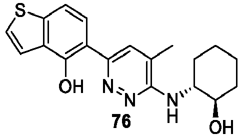

### Synthetic Route:

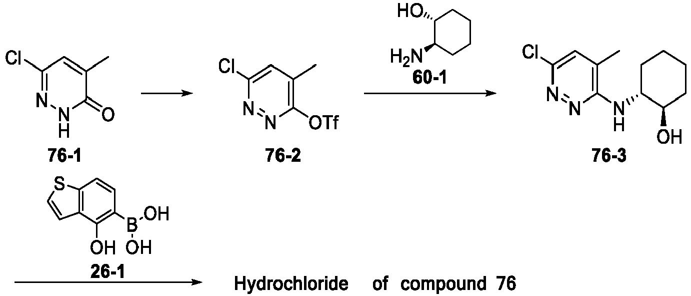

### Step 1

Compound **76-1** (4.00 g, 27.7 mmol) was dissolved in dichloromethane (40.0 mL), and trifluoromethanesulfonic anhydride (5.94 mL, 36.0 mmol) and pyridine (4.47 mL, 55.3 mmol) were added dropwise thereto at 0°C. The reaction mixture was stirred and reacted at 25°C for 12 hours. The reaction mixture was concentrated under reduced pressure, and the residue was purified by silica gel column chromatography (1/3, petroleum ether/ethyl acetate, Rf = 0.36) to obtain compound **76-2.** ¹H NMR (400 MHz, CDCl₃) δ 7.57 (s, 1H), 2.45 (s, 3H). MS-ESI calculated for [M+H]⁺ 277, found 277.

### Step 2

Compound **76-2** (500 mg, 1.77 mmol), compound **60-1** (305 mg, 2.65 mmol), and *N,N*-diisopropylethylamine (0.615 mL, 3.53 mmol) were dissolved in dimethylformamide (0.500 mL), and the reaction mixture was stirred and reacted at 100°C for 1 hour. The reaction was quenched with water (1.00 mL), and the reaction mixture was extracted with ethyl acetate (3.00 mL × 3), then washed with water (3.00 mL × 2) and saturated brine (3.00 mL × 1), and the organic phase was dried over anhydrous sodium sulfate, filtered. The filtrate was concentrated under reduced pressure, and the residue was purified by thin-layer chromatography (10/1, dichloromethane/methanol, Rf = 0.40) to obtain compound **76-3.** MS-ESI calculated for [M+H]⁺ 242, found 242.

### Step 3

Compound **76-3** (60.0 mg, 0.248 mmol), compound **26-1** (96.8 mg, 0.499 mmol), potassium carbonate (103 mg, 0.745 mmol), and [1,1'-bis(diphenylphosphino)ferrocene]dichloropalladium(II) dichloromethane complex (20.3 mg, 24.8 µmol) were dissolved in 1,4-dioxane (3.00 mL) and water (0.30 mL), and the reaction mixture was stirred and reacted at 100°C for 12 hours under nitrogen atmosphere. The reaction mixture was filtered, and the filtrate was concentrated under reduced pressure, and the residue was purified by preparative high performance liquid chromatography (3_Phenomenex Luna C18 75 × 30 mm × 3 µm; mobile phase A: 0.05% hydrochloric acid aqueous solution; mobile phase B: acetonitrile; B%: 20% to 50%, 10 min) to obtain the hydrochloride of compound **76.** ¹H NMR (400 MHz, CD₃OD) δ 8.30 (s, 1H), 7.72 (d, *J*=8.8 Hz, 1H), 7.65 (d, *J*=5.6 Hz, 1H), 7.58-7.56 (m, 2H), 3.83-3.80 (m, 1H), 3.70-3.63 (m, 1H), 2.48 (s, 3H), 2.12-2.10 (m, 2H), 1.84-1.82 (m, 2H), 1.45-1.41 (m, 2H), 1.30-1.29 (m, 2H). MS-ESI calculated for [M+H]⁺ 356, found 356.

### Example 77

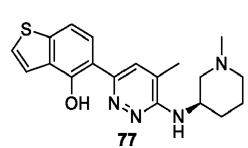

### Synthetic Route:

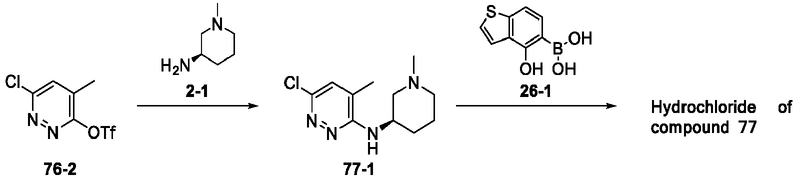

### Step 1

Compound **76-2** (500 mg, 1.77 mmol), compound **2-1** (399 mg, 2.65 mmol), and *N,N-*diisopropylethylamine (1.23 mL, 7.06 mmol) were dissolved in dimethylformamide (0.500 mL), and the reaction mixture was stirred and reacted at 100°C for 1 hour. The reaction was quenched with water (1.00 mL), and the reaction mixture was extracted with ethyl acetate (3.00 mL × 3), then washed with water (3.00 mL × 2) and saturated brine (3.00 mL × 1), and the organic phase was dried over anhydrous sodium sulfate, filtered. The filtrate was concentrated under reduced pressure, and the residue was purified by thin-layer chromatography (10/1, dichloromethane/methanol, Rf = 0.40) to obtain compound **77-1.** MS-ESI calculated for [M+H]⁺ 241, found 241.

### Step 2

Compound **77-1** (110 mg, 0.457 mmol), compound **26-1** (178 mg, 0.918 mmol), potassium carbonate (189 mg, 1.37 mmol), and [1,1'-bis(diphenylphosphino)ferrocene]dichloropalladium(II) dichloromethane complex (37.3 mg, 45.7 µmol) were dissolved in 1,4-dioxane (6.00 mL) and water (0.60 mL), and the reaction mixture was stirred and reacted at 100°C for 12 hours under nitrogen atmosphere. The reaction mixture was filtered, and the filtrate was concentrated under reduced pressure, and the residue was purified by preparative high performance liquid chromatography (3_Phenomenex Luna C18 75 × 30 mm × 3 µm; mobile phase A: 0.05% hydrochloric acid aqueous solution; mobile phase B: acetonitrile; B%: 14% to 34%, 8 min) to obtain the hydrochloride of compound **77.** ¹H NMR (400 MHz, CD₃OD) δ 8.28 (s, 1H), 7.73 (d, *J*=5.2 Hz, 1H), 7.67-7.65 (m, 2H), 7.58 (d, *J*=8.4 Hz, 1H), 4.57-4.46 (m, 1H), 3.86-3.81 (m, 1H), 3.60-3.55 (m, 1H), 3.06-3.00 (m, 1H), 2.97 (s, 3H), 2.93-2.91 (m, 1H), 2.50 (s, 3H), 2.28-2.15 (m, 2H), 2.02-1.95 (m, 1H), 1.84-1.74 (m, 1H). MS-ESI calculated for [M+H]⁺ 355, found 355.

### Biological activity test:

### Experiment example 1: Experiment of detecting IC₅₀ of NLRP3 inhibitor by using THP-1 cells

The chemical names and structural formulas of the compounds of the present disclosure for experimental use are shown in the preparation examples for each compound.
1. Experimental principle: This experiment utilized the human-derived monocyte cell line THP1 to investigate the inhibitory activity (IC₅₀) of NLRP3 inhibitor on cellular IL-1β secretion. PMA (12-O-tetradecanoylphorbol-13-acetate) was used to differentiate the monocyte cell line THP1 into mature macrophages, which were then stimulated using LPS (lipopolysaccharide), an agonist of the Toll-like receptor TLR4, to activate the transcriptional activity of the inflammasome NLRP3 and the expression of pro-IL-1β, a precursor of IL-1β. At this time, the inhibitor of NLRP3 was added, and then ATP (adenosine triphosphate) was added to further mature and activate NLRP3, and activate downstream caspase-1. Activated caspase-1 may enzymatically process pro-IL-1β into mature IL-1β that can be secreted. The NLRP3 inhibitor can effectively inhibit the ATP-induced maturation and activation of NLRP3, and the activation of downstream caspase-1, thereby inhibiting the maturation and secretion of IL-1β.
2. Experimental materials:
   2.1 Reagents: as shown in Table 2 below.

**Table 2 List of experimental reagents**

| **Name** | **Supplier** | **Article number or serial number** | **Storage conditions** |
|---|---|---|---|
| PMA | Sigma | 79346 | -20°C |
| LPS | InvivoGen | tlrl-eblps | -20°C |
| ATP | | | -20°C |
| 1640 Culture medium | Gibco | 22400-089 | 4°C |
| FBS | HyClone | SV30087.03 | -80°C |
| Penicillin- streptomycin | HyClone | SV30010 | 4°C |
| β-Mercaptoethanol | Sigma | M3148 | Room temperature |
| NEAA non-essential amino acids | Gibco | 1140-050 | 4°C |
| Human soluble protein kit | BD | 558265 | Room temperature |
| Human IL-1β Flex Set | BD | 558279 | Room temperature |
| 96-well flat-bottom plate | Corning | 3599 | Room temperature |
| 96-well U-bottom plate | Corning | 3799 | Room temperature |

### 2.2 Instruments:

Flow Cytometer, Supplier: BD, article number or serial number: LSRFortessa.

### 3. Experimental steps:

(1) The density of THP1 cells was adjusted to 5 × 10⁵ cells/mL, then PMA was added, and the final concentration was adjusted to 100 ng/mL. The cells were inoculated 200 µL/well into a 96-well flat-bottom plate, stimulated overnight (<16 hours if possible) at 37°C and 5% CO₂.
(2) The supernatant was discarded the next day, and then the cells were carefully washed twice with Dulbecco's phosphate-buffered saline (200 µL/time).
(3) The cells were stimulated with LPS at a final concentration of 100 ng/mL, added to a 96-well plate (200 µL/well), and cultured at 37°C and 5% CO₂ for 3 hours.
(4) The test compounds were added to the wells, and the screening concentrations were 5 µM, 1 µM, 200 nM, 40 nM, 8 nM, 1.6 nM, 0.32 nM, and 0.064 nM, respectively. The cells were incubated for 1 hour in a 37°C, 5% CO₂ incubator.
(5) ATP was added to each well with a final concentration of 5 mM, and cultured overnight (>18 hours) at 37°C and 5% CO₂.
(6) On the third day, 5 µL of the supernatant was taken, diluted 10-fold, and the content of IL-1β in the supernatant was detected by cytometric bead array (CBA).

### 4. The experimental results are shown in Table 3:

**Table 3 Test results of the experiment of detecting IC₅₀ of NLRP3 inhibitor by using THP-1 cells**

| **Test compound** | **IC₅₀ (nM)** |
|---|---|
| Compound **2** | 0.24 |
| Compound **4** | 5.32 |
| Hydrochloride of compound **5** | 4.34 |
| Hydrochloride of compound **7** | 5.72 |
| Compound **12a** | 0.97 |
| Compound **12b** | 3.13 |
| Compound **13** | 1.55 |
| Hydrochloride of compound **19** | 0.76 |
| Hydrochloride of compound **20** | 0.08 |
| Compound **21** | 62.7 |
| Compound **22** | 9.2 |
| Compound **23** | 4.7 |
| Compound **24a** | 28 |
| Compound **25** | 31.1 |
| Compound **26** | 0.52 |
| Compound **27a** | 41.5 |
| Compound **27b** | 25.5 |
| Compound **28** | 18.7 |
| Compound **30** | 3.39 |
| Compound **31** | 2.09 |
| Compound **38** | 0.07 |
| Compound **39** | 0.09 |
| Compound **40** | 0.19 |
| Compound **41** | 6.873 |
| Formate of compound **42** | 0.274 |
| Hydrochloride of compound **43** | 1.757 |
| Hydrochloride of compound **44a** | 0.076 |
| Hydrochloride of compound **44b** | 4.192 |
| Formate of compound **45** | 0.078 |
| Formate of compound **46** | 0.289 |
| Formate of compound **47** | 0.69 |
| Compound **48** | 2.867 |
| Compound **49** | 0.882 |
| Compound **50** | 1.936 |
| Compound **51** | 0.142 |
| Compound **53** | 0.74 |
| Hydrochloride of compound **57** | 3.37 |
| Hydrochloride of compound **58** | 0.85 |
| Hydrochloride of compound **59** | 1.73 |
| Hydrochloride of compound **60** | 0.436 |
| Hydrochloride of compound **64** | 21.1 |
| Hydrochloride of compound **65** | 6.07 |
| Hydrochloride of compound **66** | 8.18 |
| Hydrochloride of compound **67** | 16.6 |
| Compound **68a** | 33.4 |
| Compound **68b** | 24.3 |
| Compound **70a** | 23.4 |
| Compound **70b** | 3.33 |
| Hydrochloride of compound **72** | 0.318 |
| Hydrochloride of compound **73** | 3.209 |
| Hydrochloride of compound **74** | 19.437 |

Conclusion: The compounds of the present disclosure have significant inhibitory activity on the maturation and secretion of IL-1β in THP-1 cells.

### Experimental example 2: In vivo pharmacokinetic evaluation of compounds in CD-1 mice

Experimental purpose: To test the in vivo pharmacokinetics of the compounds in CD-1 mice
Experimental materials:
   CD-1 mice (male, 7 to 9 weeks old)
Experimental operation:
   The rodent pharmacokinetic characteristics after intravenous injection and oral administration of the compounds were tested using a standard protocol. The candidate compound was prepared as a clear solution during the experiment and given to mice for single intravenous injection and oral administration. The vehicle for intravenous injection and oral administration was a mixed vehicle composed of 5% dimethyl sulfoxide and 95% 5% solutol. Four male CD-1 mice were used in this project. Two mice were administered intravenously (IV), and plasma samples were collected at 0 hours (before administration) and 0.0833, 0.25, 0.5, 1, 2, 4, 8, and 24 hours after administration. The other two mice were administered orally by gavage (PO), and plasma samples were collected at 0 hours (before administration) and 0.25, 0.5, 1, 2, 4, 8, and 24 hours after administration. The plasma concentration was quantitatively analyzed by LC-MS/MS analysis method, and the pharmacokinetic parameters were calculated, such as peak concentration (Cₘₐₓ), clearance rate (CL), half-life (T_{1/2}), tissue distribution (Vdss), area under drug-time curve (AUC₀₋ₗₐₛₜ), bioavailability (F).

The experimental results are shown in Table 4:

**Table 4 In vivo pharmacokinetic test results of compounds in CD-1 mice**

| **Compound** | Dose **IV/PO (mg/kg )** | Peak concentration **Cmax (nM)** | Clearance rate **CL (mL/min/ kg)** | Tissue distributi -on **Vdss (L/kg)** | Half-life **T_{1/2}** (IV, h) | Area under drug-time curve **AUC₀₋ₗₐₛₜ PO (nM.hr)** | Bioavailability **F (%)** |
|---|---|---|---|---|---|---|---|
| Compound **4** | 1/2 | 2905 | 0.795 | 1.72 | 25.7 | 54253 | 102 |
| Hydrochloride of | 2/10 | 4295 | 28.7 | 7.03 | 3.26 | 15977 | 96.5 |
| compound **20** | | | | | | | |
| Compound **22** | 1/2 | 3220 | 9.56 | 0.718 | 1.13 | 5326 | 62.4 |
| Compound **23** | 1/2 | 3840 | 6.49 | 0.618 | 1.24 | 10361 | 79.9 |
| Compound **26** | 1/2 | 2067 | 16.6 | 1.71 | 1.75 | 3898 | 78.8 |
| Hydrochloride of compound **60** | 2/10 | 22150 | 12.8 | 1.08 | 1.45 | 48071 | 147 |

Conclusion: The compounds of the present disclosure have good in vivo pharmacokinetic properties in CD-1 mice, including good oral bioavailability, oral exposure, half-life, and clearance rate, etc.

### Experimental example 3: In vivo pharmacokinetic evaluation of compounds in SD rats

Experimental purpose: To test the in vivo pharmacokinetics of the compounds in SD rats
Experimental materials:
SD rats (male, 150 to 180g)
Experimental operation:
The rodent pharmacokinetic characteristics after intravenous injection and oral administration of the compounds were tested using a standard protocol. The candidate compound was prepared as a clear solution during the experiment and given to SD rats for single intravenous injection and oral administration. The vehicle for intravenous injection and oral administration was a mixed vehicle composed of 5% dimethyl sulfoxide and 95% 5% solutol. Four male SD rats were used in this project. Two SD rats were administered intravenously, and plasma samples were collected at 0.083, 0.25, 0.5, 1, 2, 4, 8, and 24 hours after administration. The other two SD rats were administered orally by gavage, and plasma samples were collected at 0.25, 0.5, 1, 2, 4, 8, and 24 hours after administration. The plasma concentration was quantitatively analyzed by LC-MS/MS analysis method, and the pharmacokinetic parameters were calculated, such as peak concentration (Cₘₐₓ), clearance rate (CL), half-life (T_{1/2}), tissue distribution (Vdss), area under drug-time curve (AUC₀₋ₗₐₛₜ), bioavailability (F). The experimental results are shown in Table 5:

**Table 5 In vivo pharmacokinetic test results of compounds in SD rats**

| **Compound** | Dose **IV/P O (mg/ kg)** | Peak concentration **Cmax (nM)** | Clearance rate **CL (mL/min/ kg)** | Tissue distribution **Vdss** (L/kg) | Half-life **T_{1/2} (IV, h)** | Area under drug-time curve **AUC**_{**0**-}**ₗₐₛₜ PO (nM.hr)** | Bioavailability **F** (%) |
|---|---|---|---|---|---|---|---|
| Hydrochloride of compound **20** | 1/2 | 204 | 48.5 | 6.79 | 1.81 | 926 | 49.7 |
| Compound **26** | 1/2 | 2914 | 12.3 | 0.789 | 1.1 | 7401 | 108 |
| Compound **39** | 1/2 | 573 | 15.3 | 2.63 | 2.19 | 4534 | 85.1 |
| Formate of compound **42** | 1/2 | 2096 | 6.08 | 0.837 | 1.72 | 9150 | 71.2 |
| Compound **53** | 1/2 | 1518 | 10.3 | 0.796 | 1.12 | 4688 | 49.8 |
| Hydrochloride of compound **60** | 1/2 | 2611 | 10.0 | 0.402 | 1.08 | 6658 | 71.1 |

Conclusion: The compounds of the present disclosure have good in vivo pharmacokinetic properties in SD rats, including good oral bioavailability, oral exposure, half-life, and clearance rate, etc.

### Experimental example 4: Inhibition test of compounds on hERG potassium ion channel

Experimental purpose: To detect the effect of the compounds to be tested on the hERG potassium channel by an automated patch clamp method.

### Experimental methods

### 1. Cell culture

1.1 CHO-hERG cells were cultured in a 175 cm² culture flask. When the cell density reached 60 to 80%, the culture medium was removed, and the cells were washed once with 7 mL of PBS (phosphate buffered saline), and then digested with 3 mL of digestion solution.
1.2 After the digestion was completed, 7 mL of culture medium was added to neutralize, then the mixture was centrifuged, and the supernatant was aspirated, and then 5 mL of culture medium was added to re-suspend, ensuring 2 to 5 × 10⁶/mL of cell density.

### 2. The solutions were prepared as shown in Table 6:

**Table 6 Composition of intracellular fluid and extracellular fluid**

| Reagent | Extracellular fluid (mM) | Intracellular fluid (mM) |
|---|---|---|
| CaCl₂ | 1 | 1 |
| MgCl₂ | 1.25 | 1 |
| KCl | 5 | 140 |
| NaCl | 140 | - |
| Glucose | 10 | - |
| 4-Hydroxyethylpiperazine ethanesulfonic acid buffer solution | 10 | 10 |
| Ethylene glycol bis-aminoethylether-N,N,N',N'-tetraacetic acid chelating agent | - | 10 |
| pH | Adjust pH to 7.4 with NaOH | Adjust pH to 7.4 with KOH |

| | | |
|---|---|---|
| Note: "-"indicates that there is no such reagent. | | |

### 3. Electrophysiological recording process

The process of single cell high impedance sealing and whole cell mode formation were all automatically completed by Qpatch instrument, after obtaining the whole cell recording mode, the cells were clamped at -80 mV, before giving a 5-second +40 mV depolarization stimulus, a 50 millisecond -50 mV prevoltage was given first, and then repolarized to -50 mV for 5 seconds, then returned to -80 mV. This voltage stimulation was applied every 15 seconds and after recording for 2 minutes, extracellular fluid was recordings for 5 minutes, and then the administration process was started. The compound concentration was given from the lowest test concentration, each test concentration was given for 2.5 minutes, and 3 µM of Cisapride as the positive control compound was given after all concentrations were continuously given. At least 3 cells (n ≥ 3) were tested at each concentration.

### 4. Compound preparation

4.1 The mother solution of the compound was diluted with DMSO, and 10 µL of mother solution of the compound was added to 20 µL of DMSO solution, and was 3-fold diluted continuously to 6 DMSO concentrations.
4.2 4 µL of compounds with 6 DMSO concentrations were added to 396 µL of extracellular fluid, 100-fold diluted to 6 intermediate concentrations, and then 80 µL of the compounds with 6 intermediate concentrations were added to 320 µL of extracellular fluid, 5-fold diluted to the final concentration to be tested.
4.3 The highest test concentration was 40.00 µM, in a total of 6 concentrations of 40.00, 13.33, 4.44, 1.48, 0.49, and 0.16 µM respectively.
4.4 The content of DMSO in the final test concentration was not more than 0.2%, and this concentration of DMSO had no effect on hERG potassium channel.
4.5 The whole dilution process of compound preparation was completed by Bravo instrument.

### 5. Data analysis

The experimental data were analyzed by GraphPad Prism 5.0 software.

### 6. Test results

The results of the IC₅₀ values of the compounds on the hERG potassium ion channel are shown in Table 7.

**Table 7 Results of IC₅₀ values of the compounds on the hERG potassium ion channel**

| **Test compound** | **hERG IC₅₀ (µM)** |
|---|---|
| Hydrochloride of compound **7** | 37.5 |
| Compound **12a** | 31.5 |
| Compound **13** | 21.1 |
| Hydrochloride of compound **20** | 10.3 |
| Compound **21** | 10.6 |
| Compound **22** | >40 |
| Compound **23** | >40 |
| Compound **48** | 42.6 |
| Compound **53** | 17.1 |
| Hydrochloride of compound **57** | >40 |
| Hydrochloride of compound **59** | >40 |
| Hydrochloride of compound **60** | >40 |
| Compound **70a** | 34.19 |
| Compound **70b** | >40 |

Conclusion: The compounds of the present disclosure have no significant inhibitory effect on hERG potassium channels.

## Claims

1. A compound of formula (VI) or a pharmaceutically acceptable salt thereof, 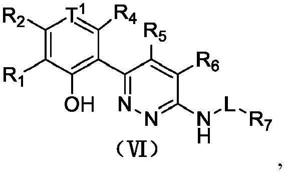 wherein
T¹ is selected from N and CR₃;
L is selected from a single bond and C(=O);
R₁ is selected from H, F, Cl, Br, I, -OH, -NH₂, -CN, C₁₋₃ alkyl, and C₁₋₃ haloalkyl;
R₂ is selected from H, F, Cl, Br, I, -OH, -NH₂, -CN, C₁₋₄ alkyl, C₁₋₄ alkoxy, -S(=O)₂-C₁₋₃ alkyl, C₃₋₆ cycloalkyl, C₃₋₆ cycloalkenyl, phenyl, and 5- to 6-membered heteroaryl, wherein the C₁₋₄ alkyl, C₁₋₄ alkoxy, -S(=O)₂-C₁₋₃ alkyl, C₃₋₆ cycloalkyl, C₃₋₆ cycloalkenyl, phenyl, and 5- to 6-membered heteroaryl are each independently and optionally substituted by 1, 2, 3, or 4 Rₐ;
or, R₁ and R₂ together with the carbon atom to which they are attached form ring A, wherein the ring A is selected from C₃₋₁₂ cycloalkyl, C₃₋₁₂ cycloalkenyl, 3- to 12-membered heterocycloalkyl, 3- to 12-membered heterocycloalkenyl, C₆₋₁₂ aryl, and 5- to 12-membered heteroaryl, wherein the C₃₋₁₂ cycloalkyl, C₃₋₁₂ cycloalkenyl, 3- to 12-membered heterocycloalkyl, 3- to 12-membered heterocycloalkenyl, C₆₋₁₂ aryl, and 5- to 12-membered heteroaryl are each independently and optionally substituted by 1, 2, 3, or 4 R_{b};
provided that when L is selected from the single bond, R₂ is not selected from Cl, CH₃, CF₃, and -OCF₃;
R₃ and R₄ are each independently selected from H, F, Cl, Br, I, -OH, -NH₂, -CN, and C₁₋₃ alkyl, wherein the C₁₋₃ alkyl is optionally substituted by 1, 2, or 3 R_{c};
or, R₂ and R₃ together with the carbon atom to which they are attached form ring B, wherein the ring B is selected from phenyl, wherein the phenyl is optionally substituted by 1, 2, 3, or 4 R_{b};
R₅ and R₆ are each independently selected from H, F, Cl, Br, I, -OH, -NH₂, -CN, C₁₋₃ alkyl, and C₁₋₃ haloalkyl;
R₇ is selected from H, C₁₋₃ alkyl, C₃₋₆ cycloalkyl, 3- to 12-membered heterocycloalkyl, and 5- to 6-membered heteroaryl, wherein the C₁₋₃ alkyl, C₃₋₆ cycloalkyl, 3- to 12-membered heterocycloalkyl, and 5- to 6-membered heteroaryl are each independently and optionally substituted by 1, 2, or 3 R_{d};
Rₐ is each independently selected from F, Cl, Br, I, =O, -OH, -NH₂, -CN, and -CH₃;
R_{b} is each independently selected from F, Cl, Br, I, =O, -OH, -NH₂, -CN, and -CH₃;
R_{c} is each independently selected from F, Cl, Br, I, =O, -OH, -NH₂, and -CN;
R_{d} is each independently selected from F, Cl, Br, I, =O, -OH, -NH₂, -CN, C₁₋₃ alkyl, C₁₋₃ alkoxy, C₁₋₃ alkylamino, 3- to 6-membered heterocycloalkyl, C₃₋₆ cycloalkyl, 3- to 6-membered heterocycloalkyl-CH₂-, C₃₋₆ cycloalkyl-CH₂-, and 3- to 6-membered heterocycloalkyl-C(=O)-, wherein the C₁₋₃ alkyl, C₁₋₃ alkoxy, C₁₋₃ alkylamino, 3- to 6-membered heterocycloalkyl, C₃₋₆ cycloalkyl, 3- to 6-membered heterocycloalkyl-CH₂-, C₃₋₆ cycloalkyl-CH₂-, and C₃₋₆ cycloalkyl-C(=O)- are each independently and optionally substituted by 1, 2, or 3 R;
R is each independently selected from F, Cl, Br, I, =O, -OH, -NH₂, -CN, -C(=O)NH₂, - CH₃, -OCH₃, and -N(CH₃)₂;
the 5- to 6-membered heteroaryl, 3- to 12-membered heterocycloalkyl, 3- to 12-membered heterocycloalkenyl, 5- to 12-membered heteroaryl, 3- to 6-membered heterocycloalkyl, and 3-to 6-membered heterocycloalkyl-CH₂- each independently comprises 1, 2, 3, or 4 atoms or atom groups each independently selected from N, O, S, and NH.

2. The compound or the pharmaceutically acceptable salt thereof according to claim 1, wherein R₁ is selected from H.

3. The compound or the pharmaceutically acceptable salt thereof according to claim 1, wherein R₂ is selected from H, -CN, -CH₃, 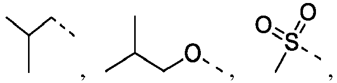 cyclopropyl, cyclobutyl, cyclopentyl, cyclohexyl, cyclopentenyl, cyclohexenyl, phenyl, and pyridyl, wherein the -CH₃, 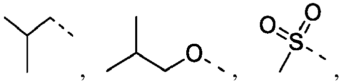 cyclopropyl, cyclobutyl, cyclopentyl, cyclohexyl, cyclobutenyl, cyclopentenyl, cyclohexenyl, phenyl, and pyridyl are each independently and optionally substituted by 1, 2, 3, or 4 Rₐ.

4. The compound or the pharmaceutically acceptable salt thereof according to claim 3, wherein R₂ is selected from H, -CN, -CH₃, 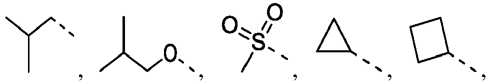 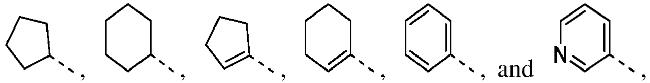 wherein the -CH₃, 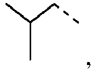 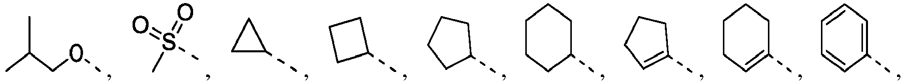 and 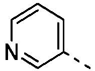 are each independently and optionally substituted by 1, 2, 3, or 4 Rₐ.

5. The compound or the pharmaceutically acceptable salt thereof according to claim 4, wherein R₂ is selected from H, -CN, -CF₃,

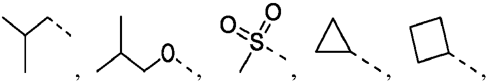

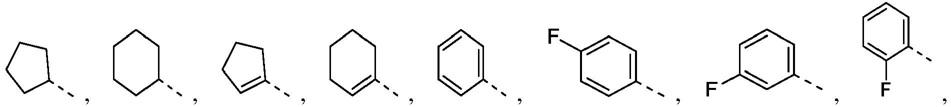

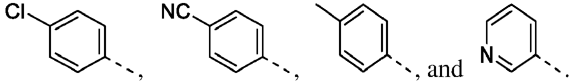

6. The compound or the pharmaceutically acceptable salt thereof according to claim 1, wherein ring A is selected from 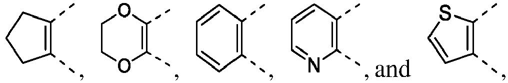 wherein the 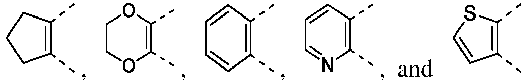 are each independently and optionally substituted by 1, 2, 3, or 4 R_{b}.

7. The compound or the pharmaceutically acceptable salt thereof according to claim 1, wherein ring A is selected from

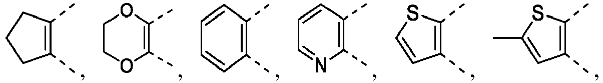

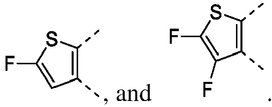

8. The compound or the pharmaceutically acceptable salt thereof according to claim 1, wherein R₃ is selected from H.

9. The compound or the pharmaceutically acceptable salt thereof according to claim 1, wherein R₄ is selected from H and -CH₃.

10. The compound or the pharmaceutically acceptable salt thereof according to claim 1, wherein R₅ is selected from H and -CH₃.

11. The compound or the pharmaceutically acceptable salt thereof according to claim 1, wherein R₆ is selected from H and -CH₃.

12. The compound or the pharmaceutically acceptable salt thereof according to claim 1, wherein R_{d} is selected from F, Cl, Br, -OH, -CH₃, -CH₂-CH₃, -CH(CH₃)₂, -CH₂-CH₂-CH₃, - N(CH₃)₂, 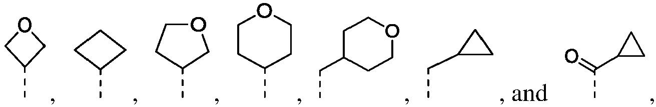 wherein the -CH₃, -CH₂-CH₃, -CH(CH₃)₂, -CH₂-CH₂-CH₃, -N(CH₃)₂, 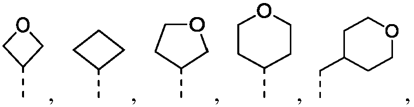 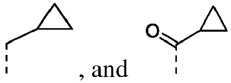 are each independently and optionally substituted by 1, 2, or 3 R.

13. The compound or the pharmaceutically acceptable salt thereof according to claim 12, wherein R_{d} is selected from F, Cl, Br, -OH, -CH₃, -CH₂C(=O)NH₂, -CH₂CN, -CH₂-CH₃, - CH₂CF₃, -CH(CH₃)₂, -CH₂-CH₂-CH₃, -N(CH₃)₂,

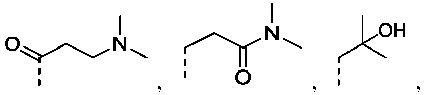

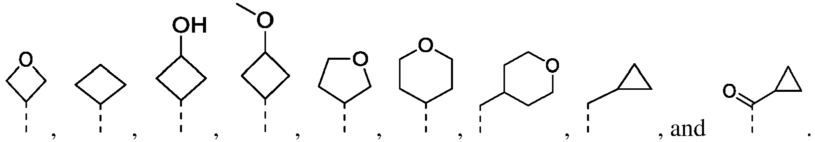

14. The compound or the pharmaceutically acceptable salt thereof according to claim 1, wherein R₇ is selected from H, -CH₂-CH₃, -CH(CH₃)₂, -CH₂-CH₂-CH₃, pyrrolidinyl, tetrahydrofuryl, piperidinyl, quinuclidinyl, 2-oxa-8-azaspiro[4.5]decyl, cyclohexyl, octahydroindolizinyl, and pyridyl, wherein the -CH₂-CH₃, -CH(CH₃)₂, -CH₂-CH₂-CH₃, pyrrolidinyl, tetrahydrofuryl, piperidinyl, quinuclidinyl, 2-oxa-8-azaspiro[4.5]decyl, 1-oxa-8-azaspiro[4.5]decyl, cyclohexyl, octahydroindolizinyl, and pyridyl are each independently and optionally substituted by 1, 2, or 3 R_{d}.

15. The compound or the pharmaceutically acceptable salt thereof according to claim 14, wherein R₇ is selected from H, -CH₂-CH₃, -CH(CH₃)₂, -CH₂-CH₂-CH₃,

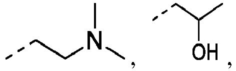

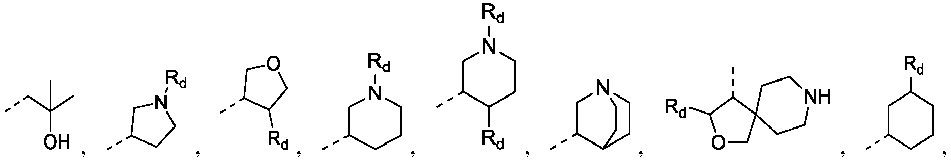

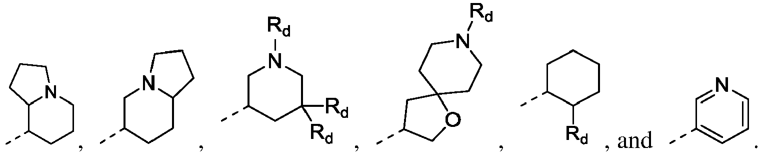

16. The compound or the pharmaceutically acceptable salt thereof according to claim 13 or 15, wherein R₇ is selected from H,

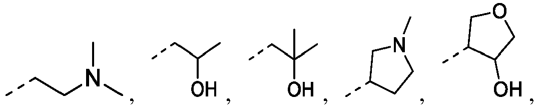

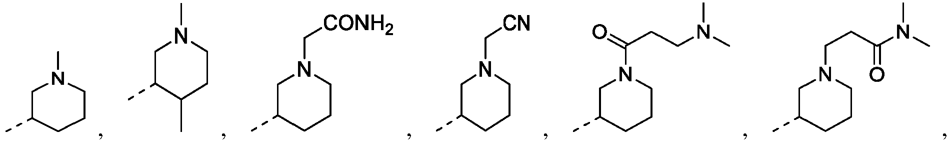

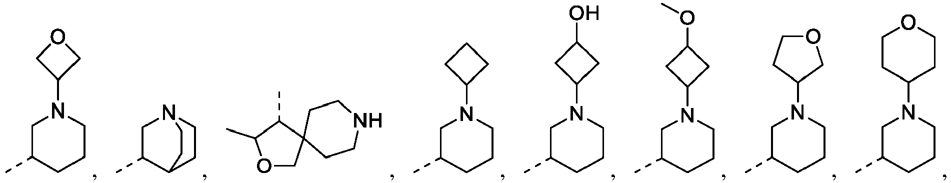

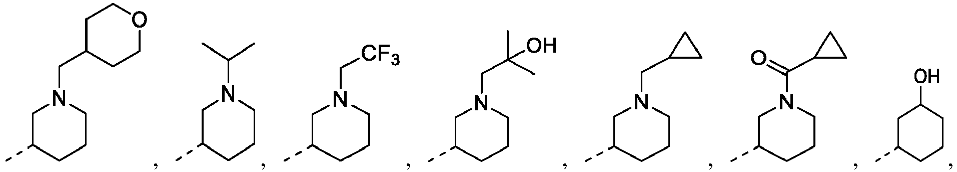

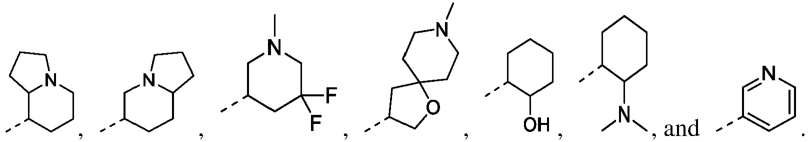

17. The compound or the pharmaceutically acceptable salt thereof according to claim 1, wherein the structural moiety 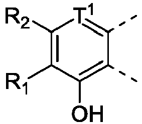 is selected from

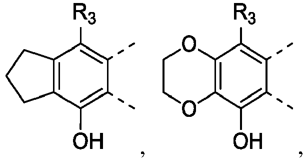

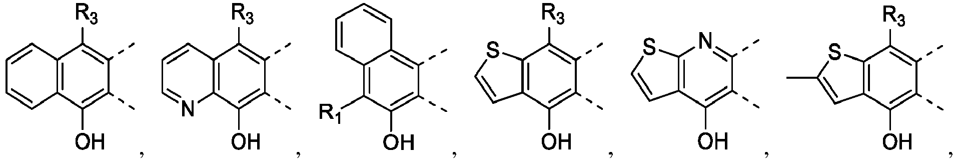

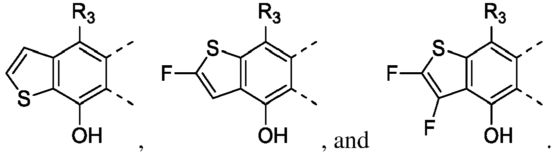

18. The compound or the pharmaceutically acceptable salt thereof according to any one of claims 1 to 9, wherein the structural moiety 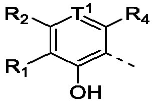 is selected from

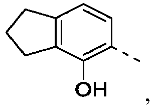

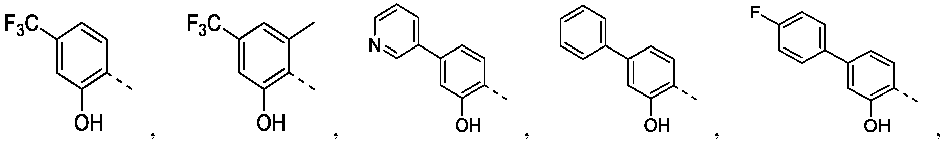

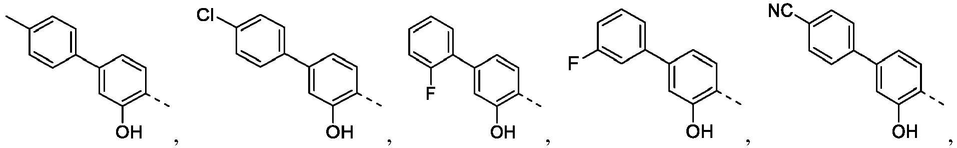

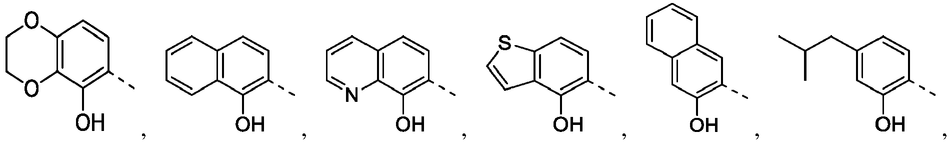

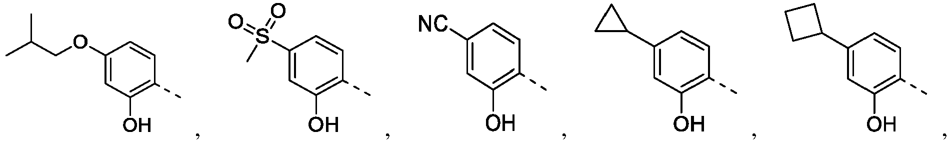

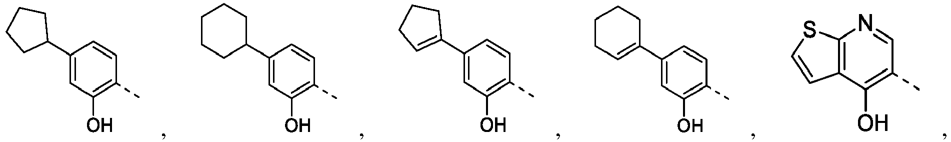

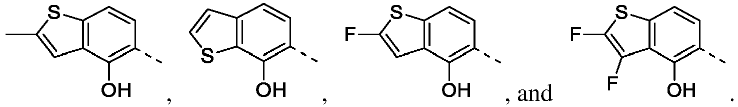

19. The compound or the pharmaceutically acceptable salt thereof according to claim 1, wherein the compound has a structure of formula (I) or (VI-1): 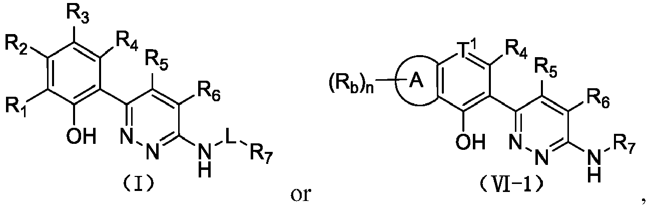 wherein T¹, L, R₁, R₂, R₃, R₄, R₅, R₆, R₇, R_{b}, n, and ring A are as defined in claim 1.

20. The compound or the pharmaceutically acceptable salt thereof according to claim 1, wherein the compound has a structure of formula (I-1), (I-2), or (I-4): 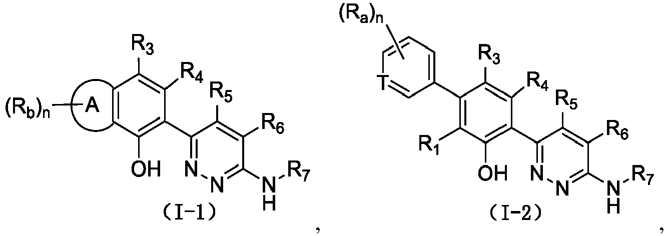 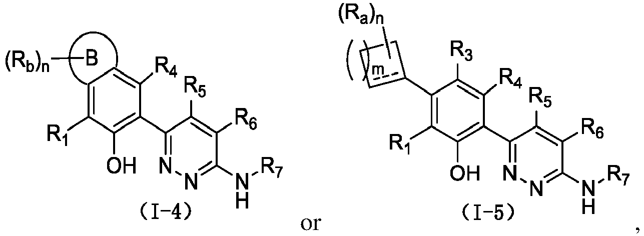
wherein T is selected from CH and N;
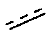 is a single bond or a double bond;
n is selected from 0, 1, 2, 3, or 4;
m is selected from 0, 1, 2, 3, or 4;
ring A, ring B, Rₐ, R_{b}, R₁, R₃, R₄, R₅, R₆, and R₇ are as defined in claim 1.

21. The compound or the pharmaceutically acceptable salt thereof according to claim 1, wherein the compound has a structure of formula (I-3): 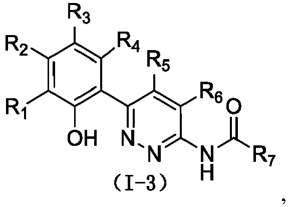 wherein R₁, R₂, R₃, R₄, R₅, R₆, and R₇ are as defined in claim 1.

22. The compound or the pharmaceutically acceptable salt thereof according to claim 20, wherein the compound has a structure of formula (I-1A): 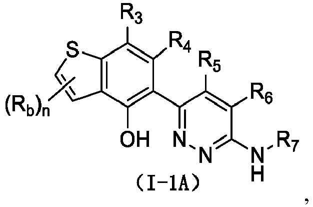 wherein
n is selected from 0, 1, or 2;
R_{b}, R₃, R₄, R₅, R₆, and R₇ are as defined in claim 20.

23. A compound of the following formula or a pharmaceutically acceptable salt thereof,

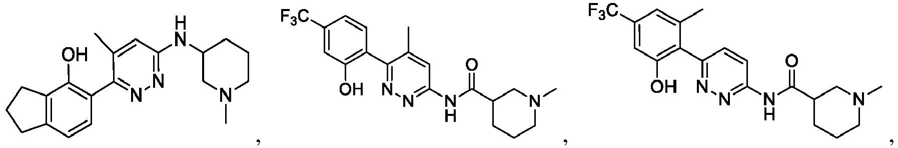

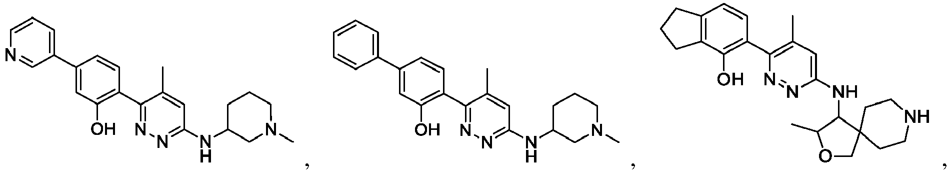

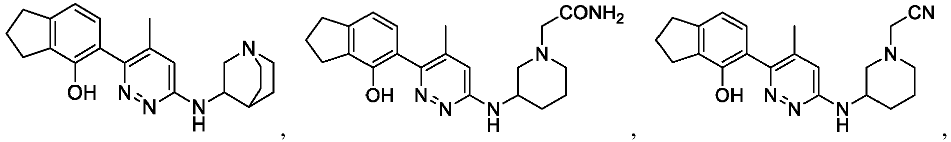

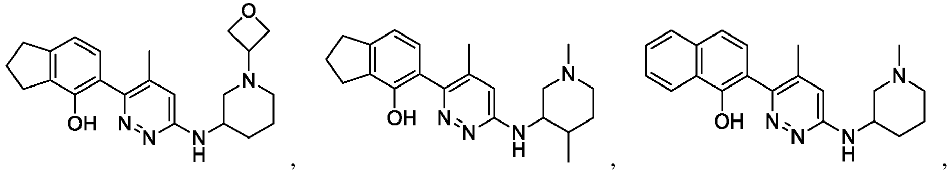

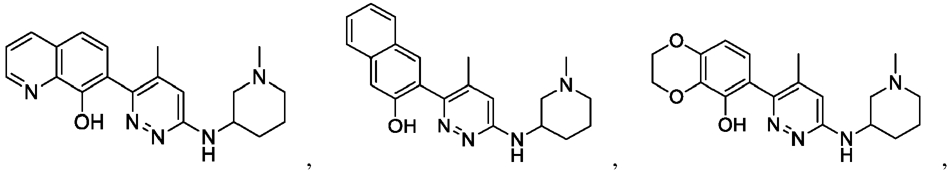

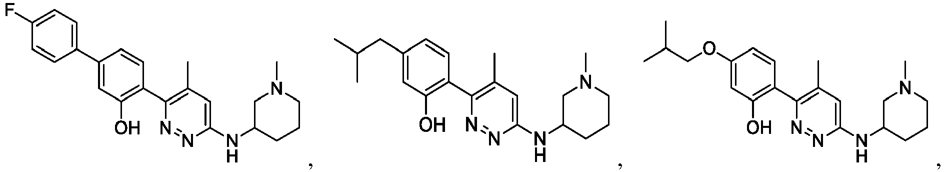

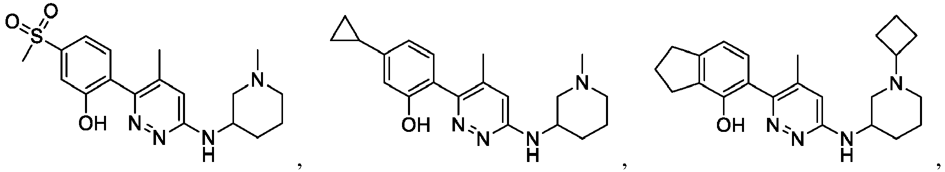

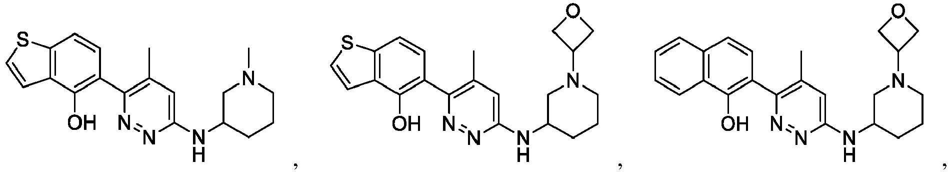

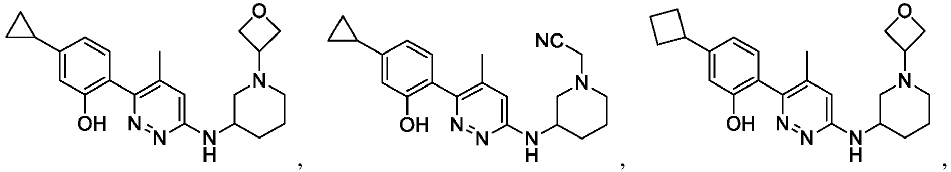

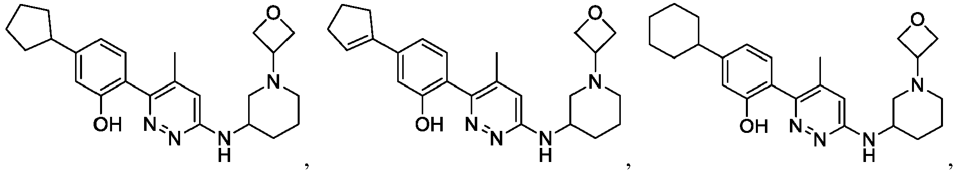

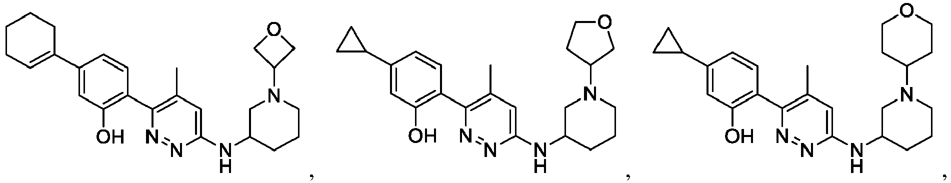

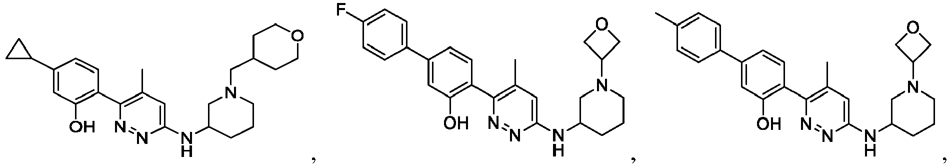

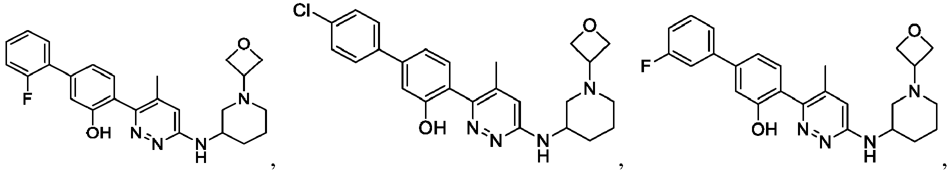

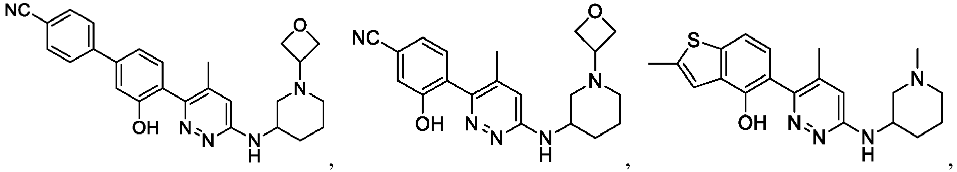

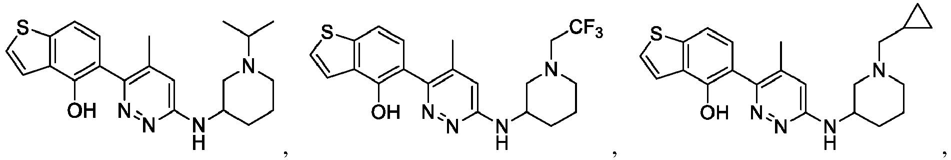

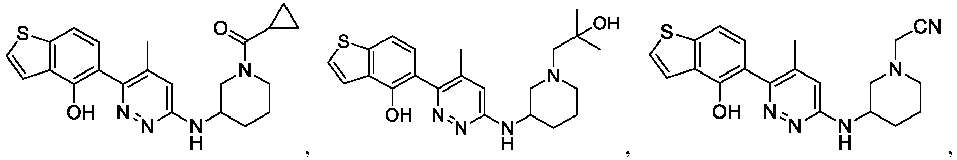

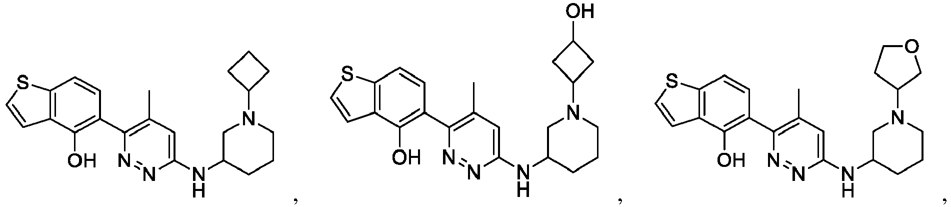

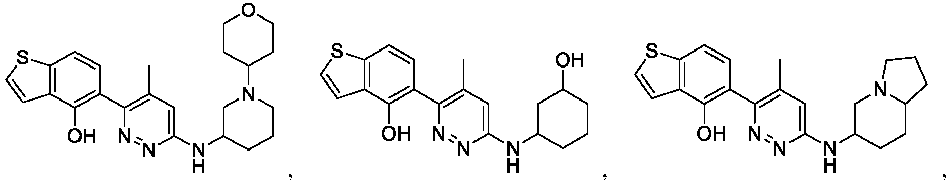

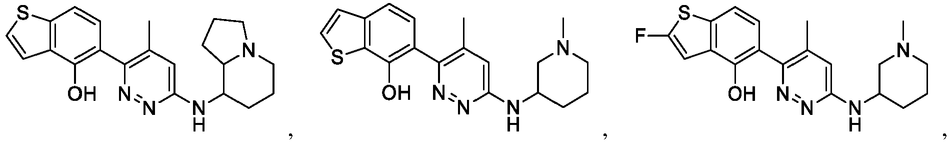

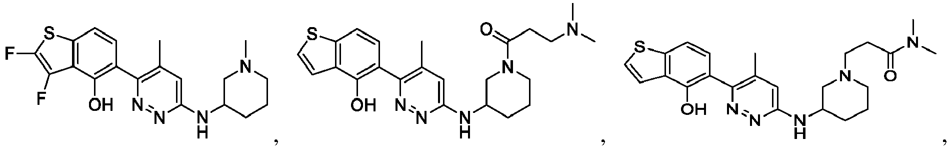

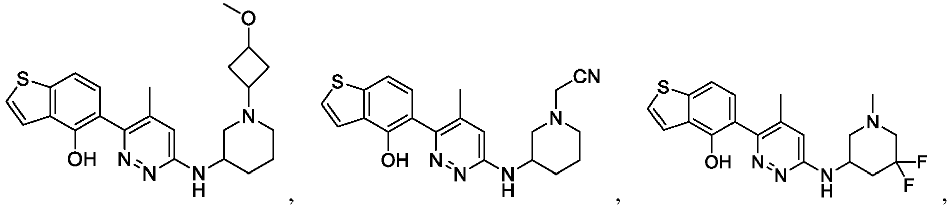

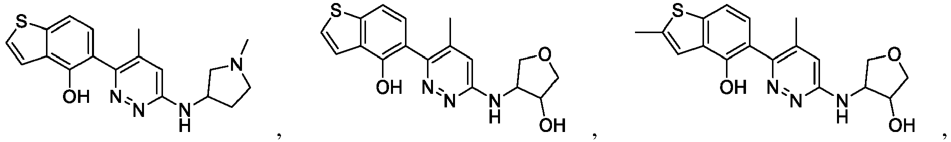

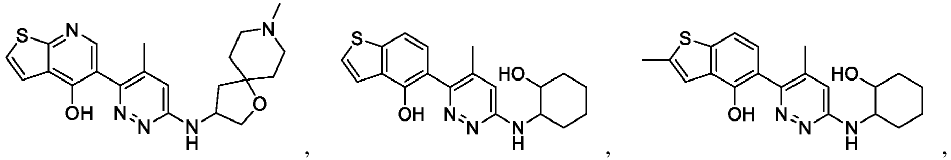

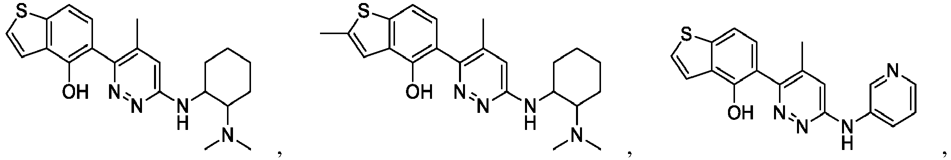

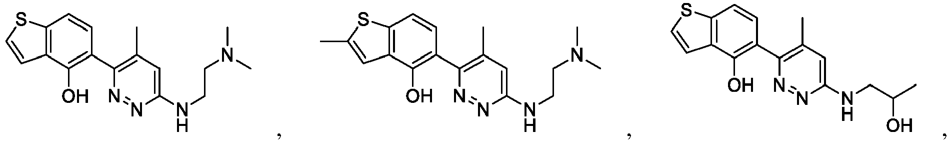

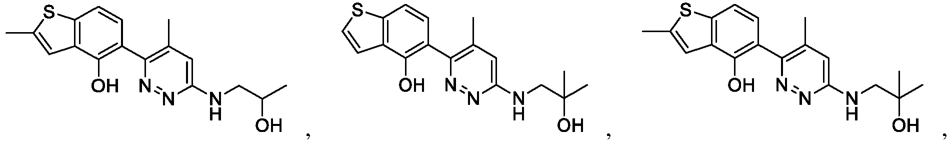

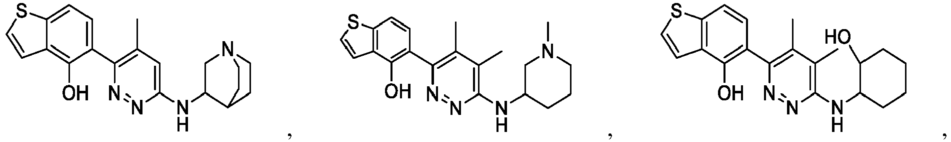

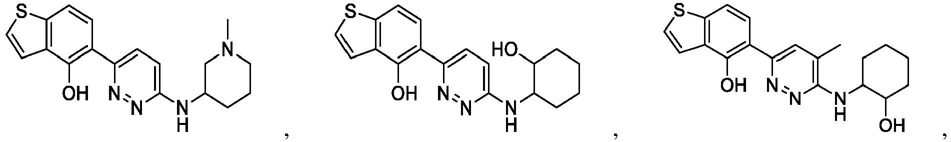

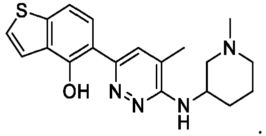

24. The compound or the pharmaceutically acceptable salt thereof according to claim 23, which is selected from,

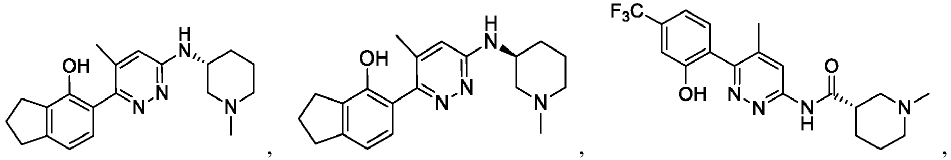

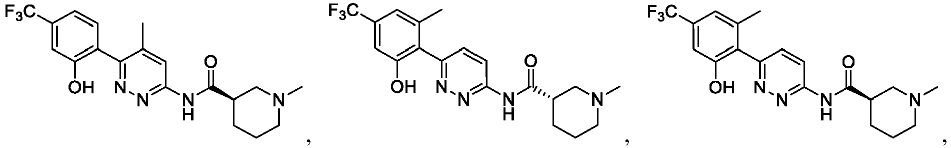

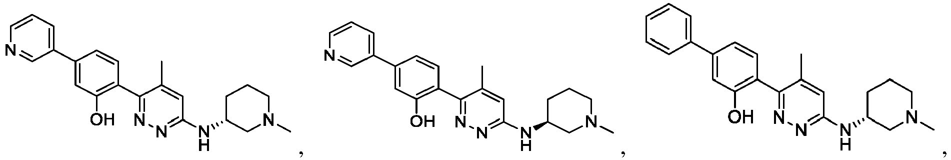

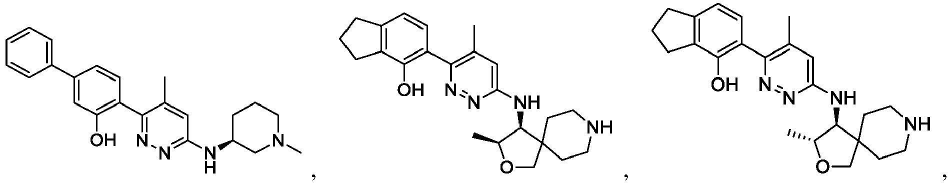

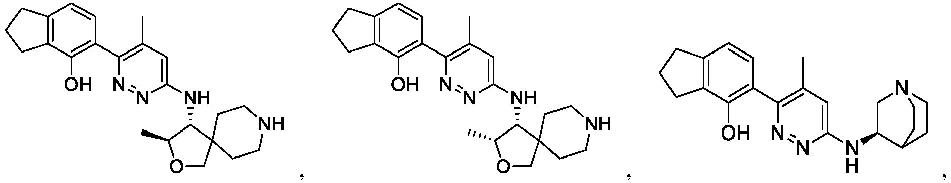

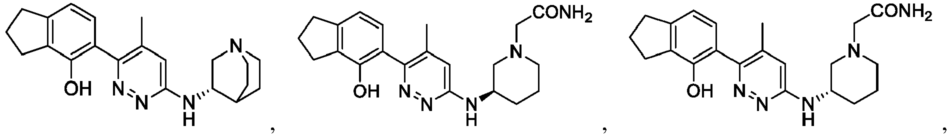

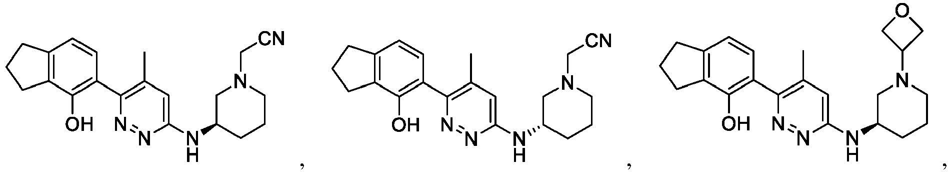

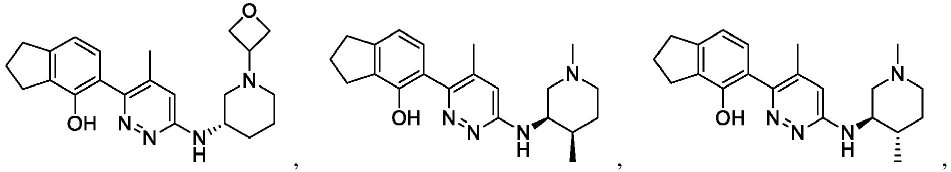

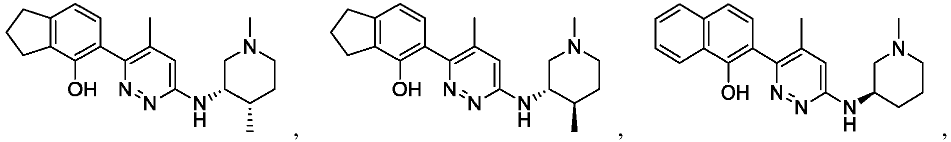

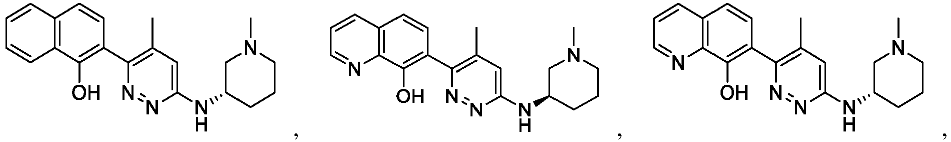

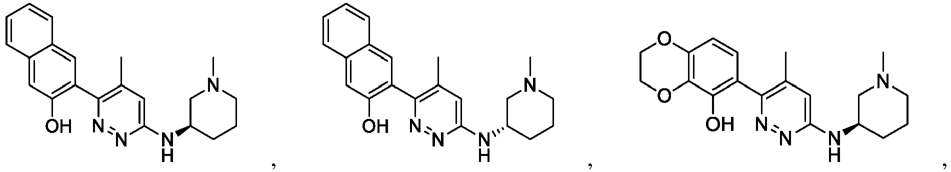

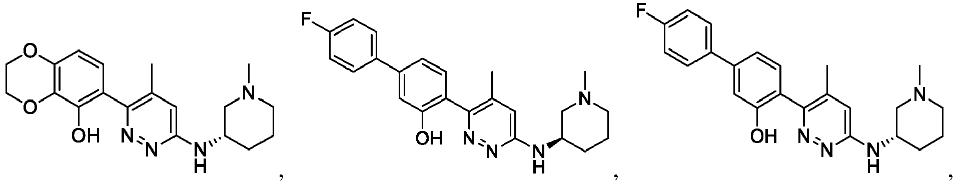

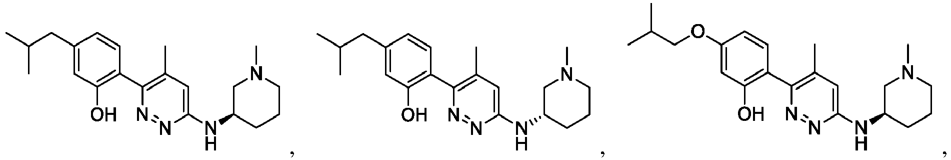

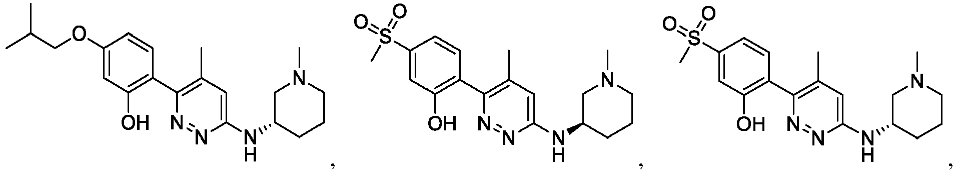

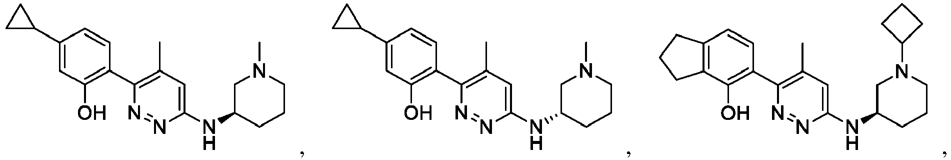

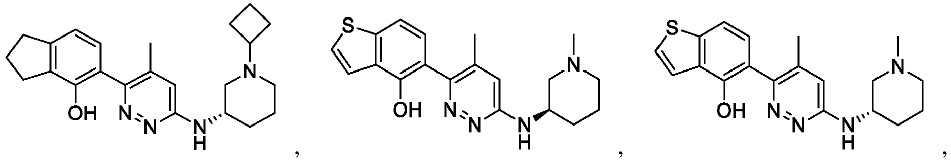

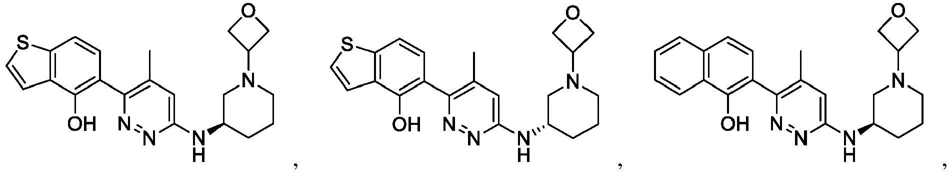

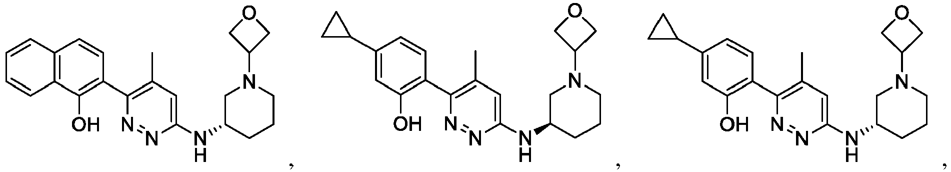

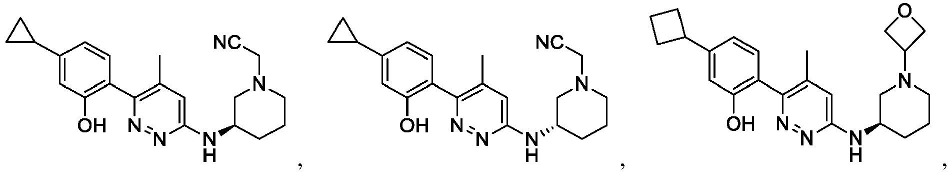

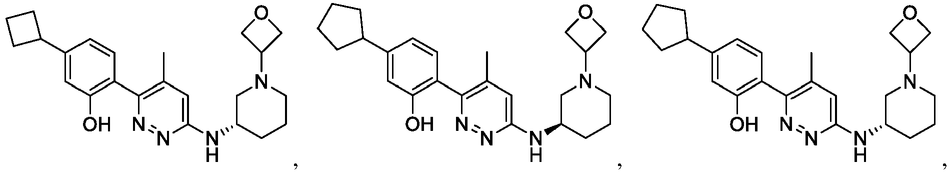

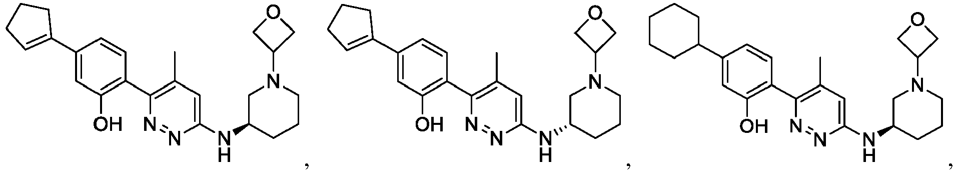

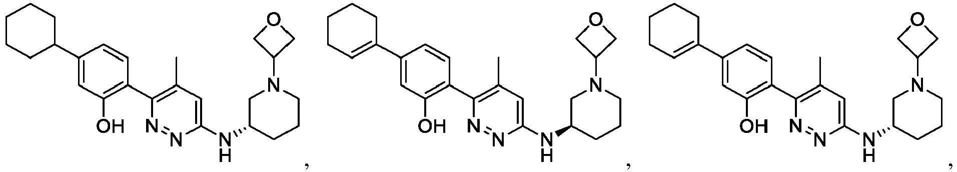

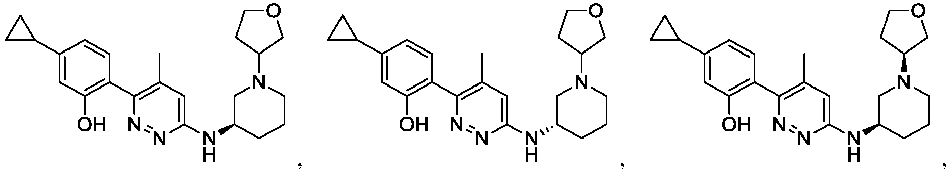

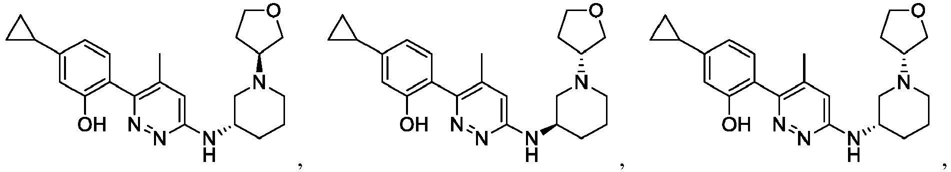

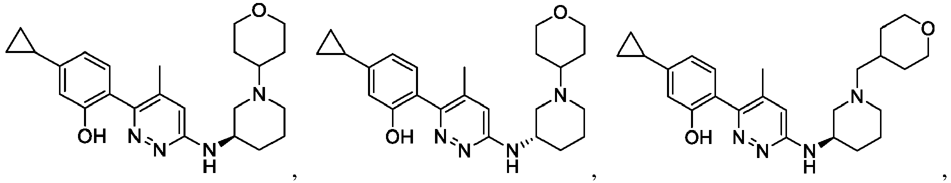

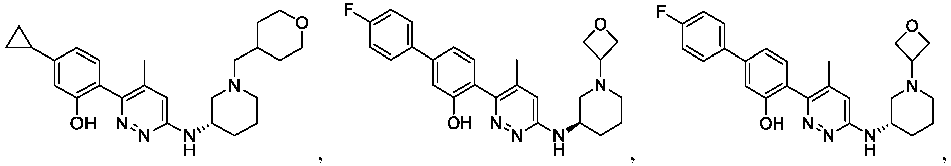

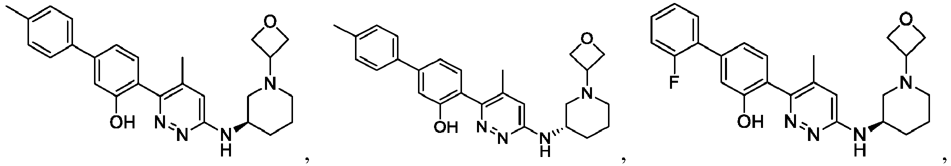

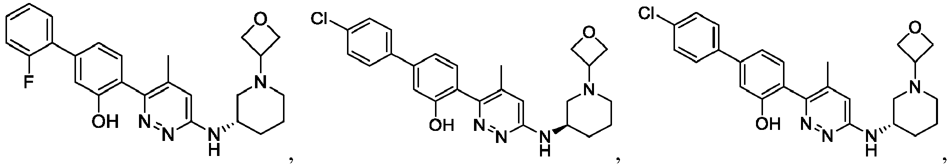

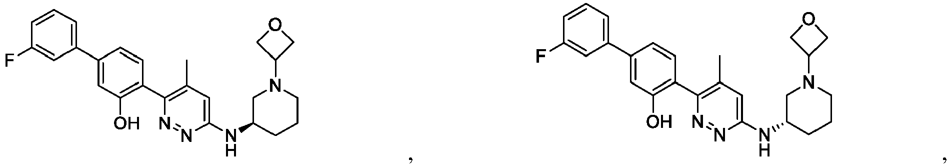

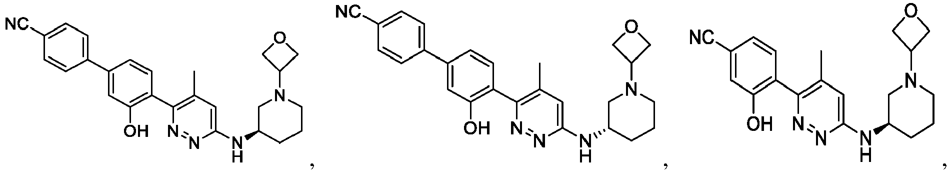

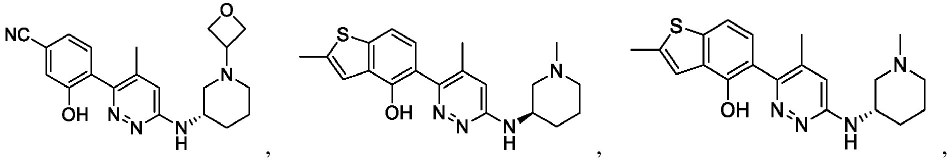

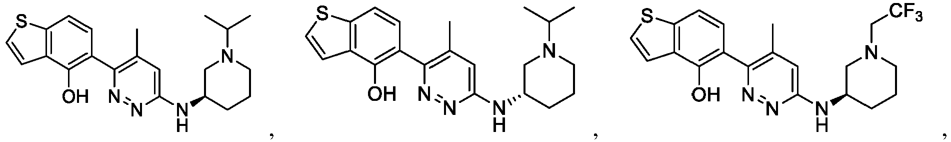

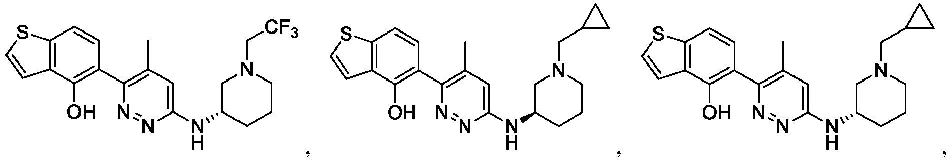

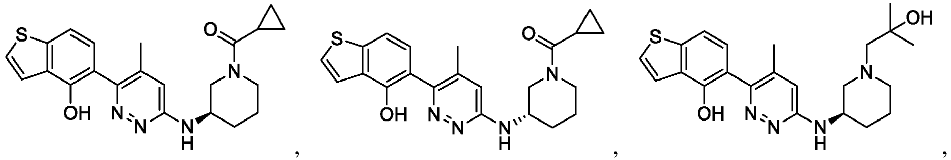

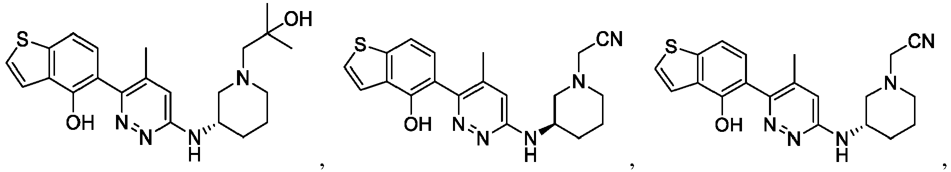

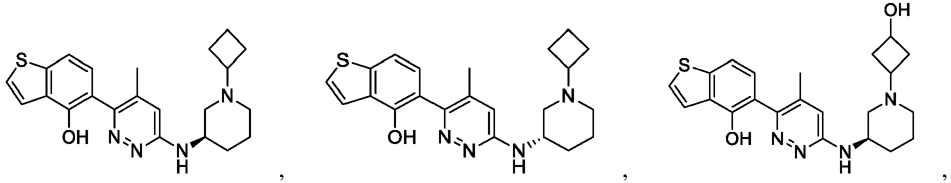

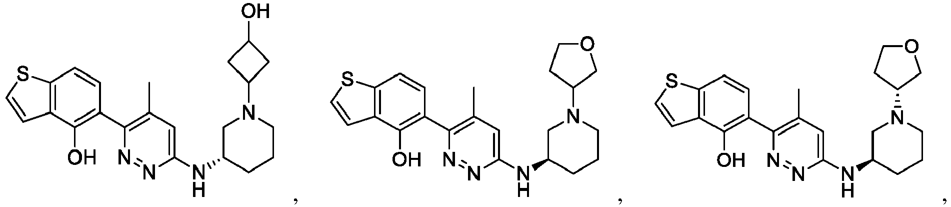

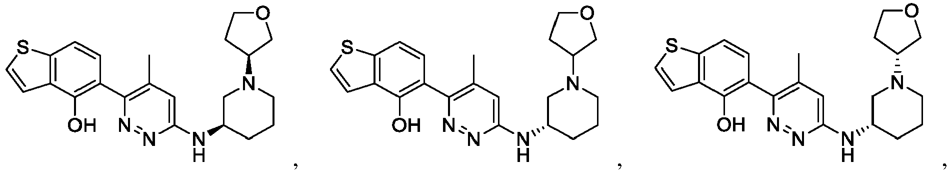

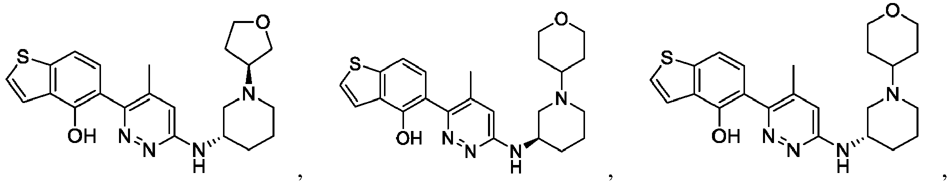

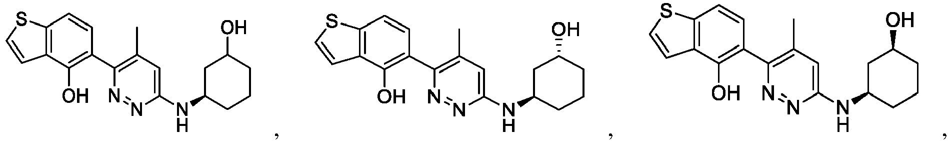

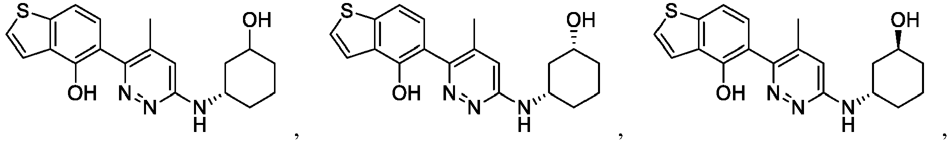

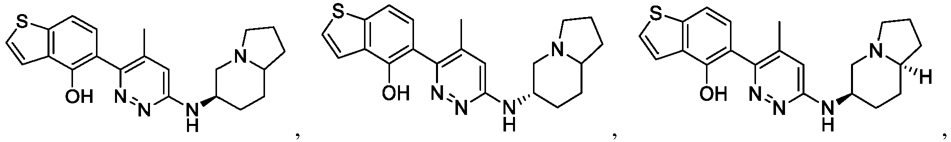

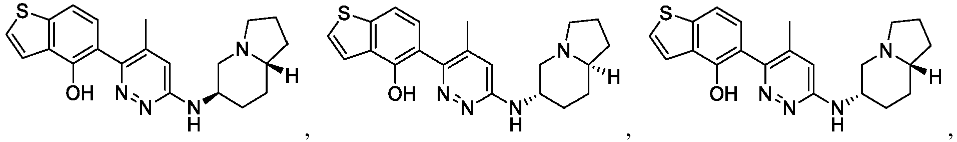

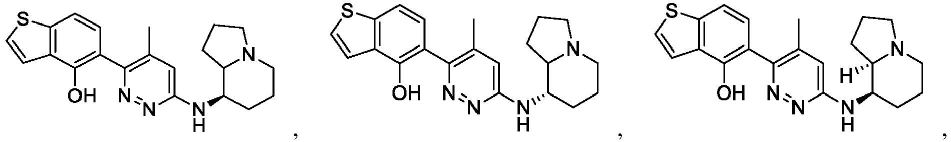

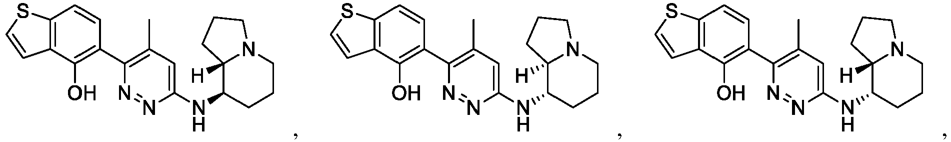

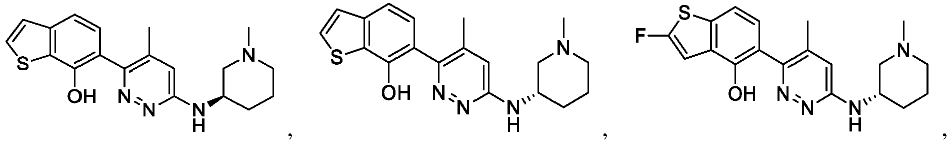

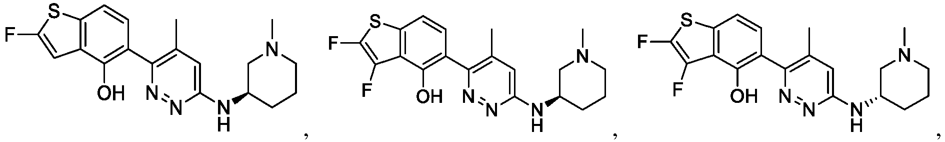

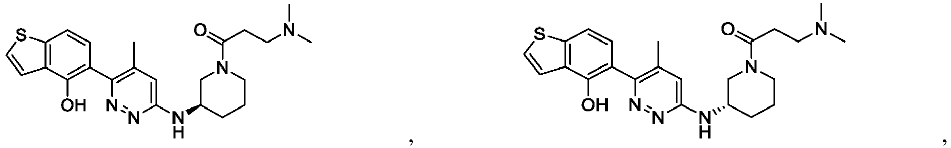

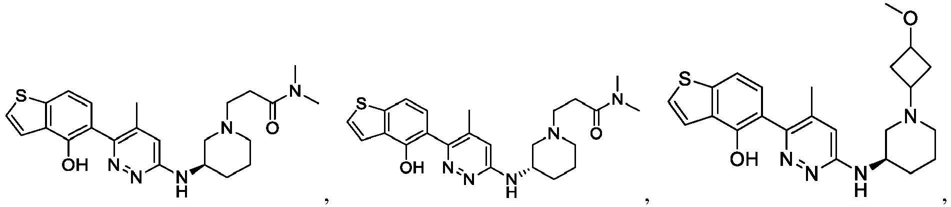

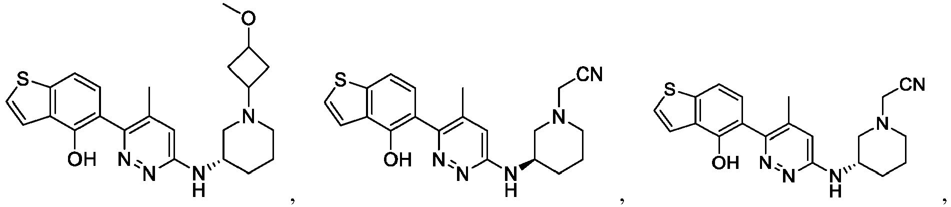

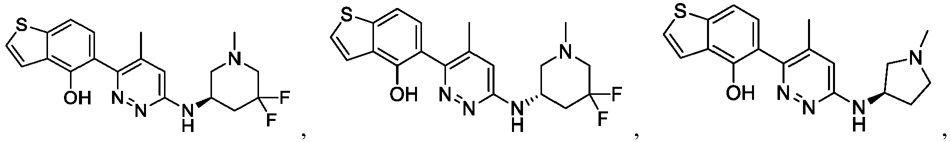

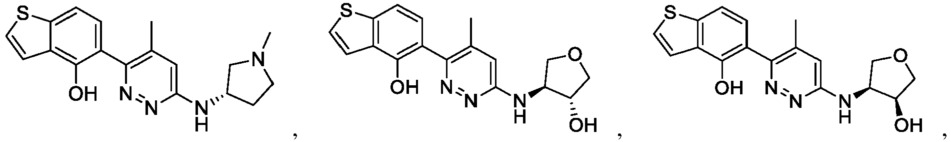

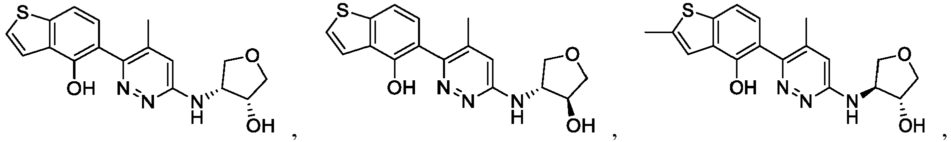

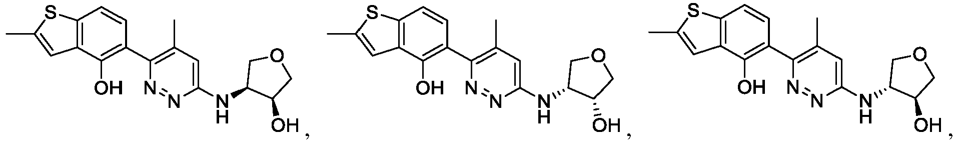

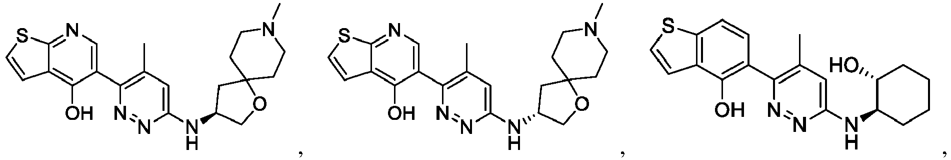

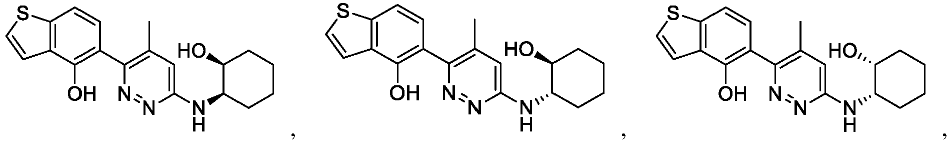

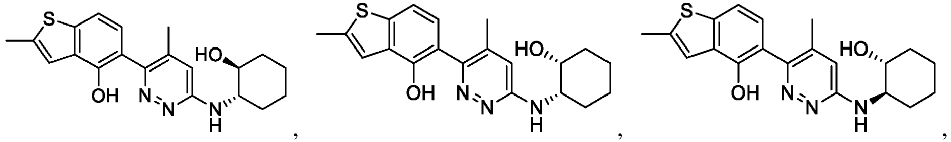

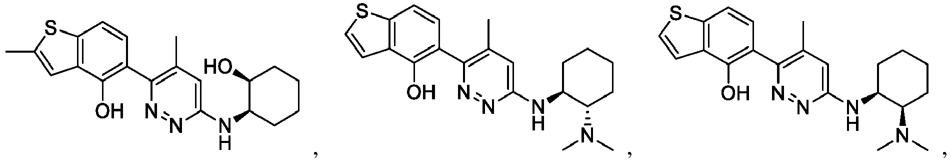

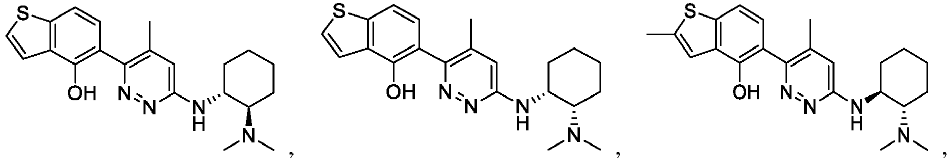

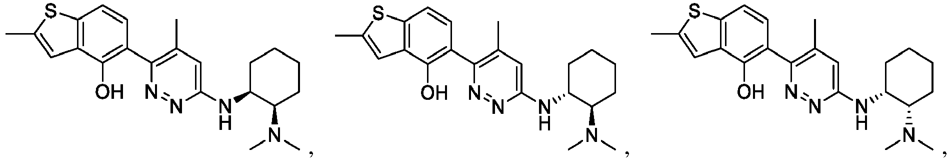

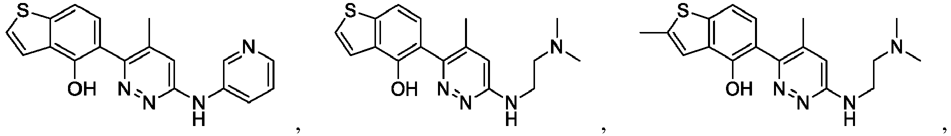

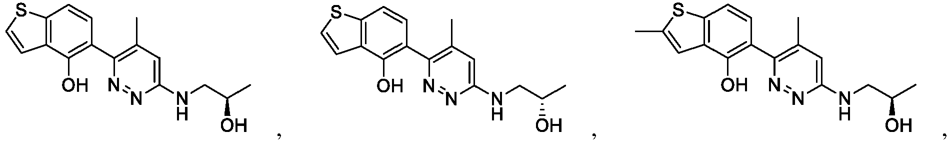

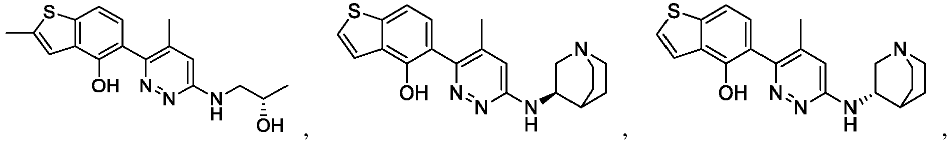

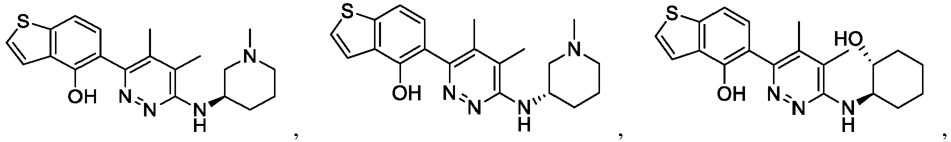

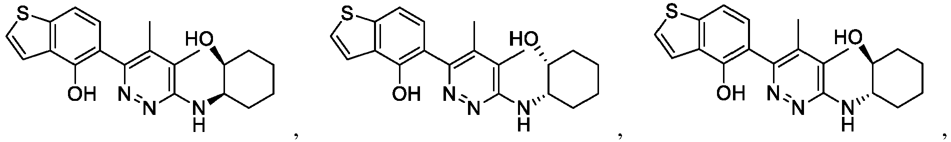

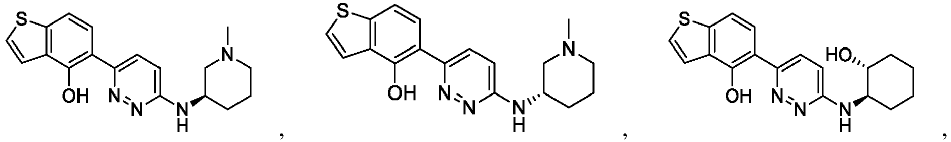

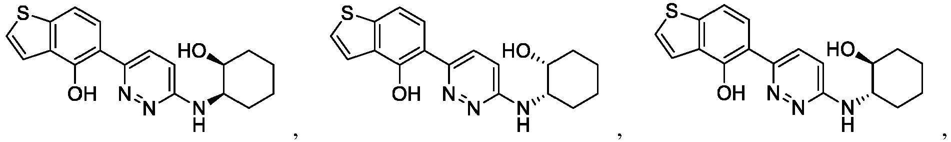

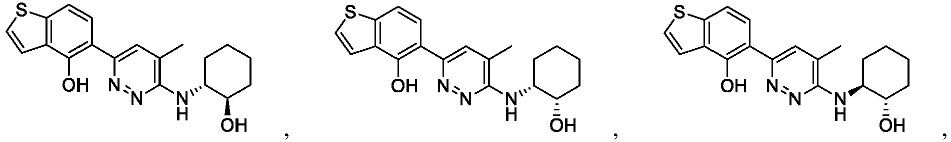

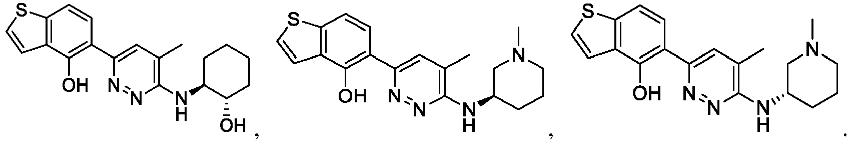

25. A use of the compound or the pharmaceutically acceptable salt thereof according to any one of claims 1 to 24 in the manufacture of a medicament for the treatment of Parkinson's disease.
